# EUROPEAN PATENT APPLICATION

(11) **EP 1 724 351 A2**
(43) Date of publication of application: **22.11.2006**
(21) Application number: 06076156.6
(22) Date of filing: 09.03.2000
(51) Int. Cl.: C12N 15/12, C07K 14/705, C07K 16/28, C12Q 1/68, A01K 67/027, C12N 15/62, A61K 38/00, G01N 33/566

(54) **Human Kallikrein-like genes**

(30) Priority: 11.03.1999 US 124260 P; 01.04.1999 US 127386 P; 21.07.1999 US 144919 P
(62) Divisional of application: 00908878.2
(71) Applicant: MOUNT SINAI HOSPITAL, Toronto, Ontario M5G 1X5 (CA)
(72) Inventor: Yousef, George M., Toronto, Ontario M5T 1B3 (CA); Diamandis, Eleftherios, Toronto, Ontario M5G 2X2 (CA)
(74) Representative: Holliday, Louise Caroline

(57) **Abstract**

The present invention relates to an isolated KLK-L1, KLK-L2, KLK-L3, KLK-L4, KLK-L5, OR KLK-L6 nucleic acid molecule of at least 30 nucleotides which hybridizes to SEQ ID NO. 1, 13, 21, 43, 56, or 65, respectively, or the complement of SEQ ID NO. 1, 13, 21, 43, 56, or 65, under stringent hybridization conditions.

## Description

### FIELD OF THE INVENTION

The invention relates to nucleic acid molecules, proteins encoded by such nucleic acid molecules; and use of the proteins and nucleic acid molecules

### BACKGROUND OF THE INVENTION

Kallikreins and kallikrein-like proteins are a subgroup of the serine protease enzyme family and exhibit a high degree of substrate specificity (1). The biological role of these kallikreins is the selective cleavage of specific polypeptide precursors (substrates) to release peptides with potent biological activity (2). In mouse and rat, kallikreins are encoded by large multigene families. In the mouse genome, at least 24 genes have been identified (3). Expression of 11 of these genes has been confirmed; the rest are presumed to be pseudogenes (4). A similar family of 15-20 kallikreins has been found in the rat genome (5) where at least 4 of these are known to be expressed (6).

Three human kallikrein genes have been described, i.e. prostatic specific antigen (PSA or KLK3) (7), human glandular kallikrein (KLK2) (8) and tissue (pancreatic-renal) kallikrein (KLK1) (9). The PSA gene spans 5.8 Kb of sequence which has been published (7); the KLK2 gene has a size of 5.2 Kb and its complete structure has also been elucidated (8). The KLK1 gene is approximately 4.5 Kb long and the exon sequences and the exon/intron junctions of this gene have been determined (9).

The mouse kallikrein genes are clustered in groups of up to 11 genes on chromosome 7 and the distance between the genes in the various clusters can be as small as 3-7 Kb (3). All three human kallikrein genes have been assigned to chromosome 19q13.2 - 19q 13.4 and the distance between PSA and KLK2 has been estimated to be 12 Kb (9).

A major difference between mouse and human kallikreins is that two of the human kallikreins (KLK2 and KLK3) are expressed almost exclusively in the prostate while in animals none of the kallikreins is localized in this organ. Other candidate new members of the human kallikrein gene family include protease M (10) (also named Zyme (11) or neurosin (12) and the normal epithelial cell-specific gene-1 (NES1) (13). Both genes have been assigned to chromosome 19q13.3 (10,14) and show structural homology with other serine proteases and the kallikrein gene family (10-14).

### SUMMARY OF THE INVENTION

In efforts to precisely define the relative genomic location of PSA, KLK2, Zyme and NES1 genes, an area spanning approximately 300 Kb of contiguous sequence on human chromosome 19 (19q13.3 -q13.4) was examined. The present inventors were able to identify the relative location of the known kallikrein genes and, in addition, they identified other kallikrein- like genes which exhibit both location proximity and structural similarity with the known members of the human kallikrein family. The novel genes exhibit homology with the currently known members of the kallikrein family and they are co-localized in the same genomic region. These new genes, like the already known kallikreins have utility in various cancers including those of the breast, testicular, and prostate.

The kallikrein-like proteins described herein are individually referred to as "KLK-L1, KLK-L2, KLK-L3, KLK-L4, KLK-L5, or KLK-L6", and collectively as "kallikrein-like proteins" or "KLK-L Proteins". The genes encoding the proteins are referred to as "*klk-11, klk-12, klk-13, klk-14, klk-15, or klk-16*", and collectively as "kallikrein-like genes" or "*klk-1* genes".

Broadly stated the present invention relates to an isolated nucleic acid molecule which comprises:
(i) a nucleic acid sequence encoding a protein having substantial sequence identity with an amino acid sequence of KLK-L1, KLK-L2, KLK-L3, KLK-L4, KLK-L5, or KLK-L6 as shown in SEQ.ID.NO. 2, 3, 14, 22, 23, 44, 45, 57, 58, 59, 60, 66, or 67, respectively;
(ii) a nucleic acid sequence encoding a protein comprising an amino acid sequence of KLK-L1, KLK-L2, KLK-L3, KLK-L4, KLK-L5, or KLK-L6 as shown in SEQ.ID.NO. 2, 3, 14, 22, 23, 44, 45, 57, 58, 59, 60, 66, or 67, respectively;
(iii) nucleic acid sequences complementary to (i);
(iv) a degenerate form of a nucleic acid sequence of (i);
(v) a nucleic acid sequence capable of hybridizing under stringent conditions to a nucleic acid sequence in (i), (ii) or (iii);
(vi) a nucleic acid sequence encoding a truncation, an analog, an allelic or species variation of a protein comprising an amino acid sequence of KLK-L1, KLK-L2, KLK-L3, KLK-L4, KLK-L5, or KLK-L6 as shown in SEQ.ID.NO. 2, 3, 14, 22, 23, 44, 45, 57, 58, 59, 60, 66, or 67, respectively; or
(vii) a fragment, or allelic or species variation of (i), (ii) or (iii).

Preferably, a purified and isolated nucleic acid molecule of the invention comprises:
(i) a nucleic acid sequence comprising the sequence of SEQ.ID.NO. 1, 13, 21, 43, 56, or 65 wherein T can also be U;
(ii) nucleic acid sequences complementary to (i), preferably complementary to the full nucleic acid sequence of SEQ.ID.NO. 1, 13, 21, 43, 56, or 65;
(iii) a nucleic acid capable of hybridizing under stringent conditions to a nucleic acid of (i) or (ii) and preferably having at least 18 nucleotides; or
(iv) a nucleic acid molecule differing from any of the nucleic acids of (i) to (iii) in codon sequences due to the degeneracy of the genetic code.

The invention also contemplates a nucleic acid molecule comprising a sequence encoding a truncation of a KLK-L protein, an analog, or a homolog of a KLK-L Protein or a truncation thereof. (KLK-L Proteins and truncations, analogs and homologs of KLK-L Proteins are also collectively referred to herein as "KLK-L Related Proteins").

The nucleic acid molecules of the invention may be inserted into an appropriate expression vector, i.e. a vector that contains the necessary elements for the transcription and translation of the inserted coding sequence. Accordingly, recombinant expression vectors adapted for transformation of a host cell may be constructed which comprise a nucleic acid molecule of the invention and one or more transcription and translation elements linked to the nucleic acid molecule.

The recombinant expression vector can be used to prepare transformed host cells expressing KLK-L Related Proteins. Therefore, the invention further provides host cells containing a recombinant molecule of the invention. The invention also contemplates transgenic non-human mammals whose germ cells and somatic cells contain a recombinant molecule comprising a nucleic acid molecule of the invention, in particular one which encodes an analog of a KLK-L Protein, or a truncation of a KLK-L Protein.

The invention further provides a method for preparing KLK-L Related Proteins utilizing the purified and isolated nucleic acid molecules of the invention. In an embodiment a method for preparing a KLK-L Related Protein is provided comprising (a) transferring a recombinant expression vector of the invention into a host cell; (b) selecting transformed host cells from untransformed host cells; (c) culturing a selected transformed host cell under conditions which allow expression of the KLK-L Related Protein; and (d) isolating the KLK-L Related Protein.

The invention further broadly contemplates an isolated KLK-L Protein comprising an amino acid sequence as shown in SEQ.ID.NO. 2, 3, 14, 22, 23, 44, 45, 57, 58, 59, 60, 66, or 67.

The KLK-L Related Proteins of the invention may be conjugated with other molecules, such as proteins, to prepare fusion proteins. This may be accomplished, for example, by the synthesis of N-terminal or C-terminal fusion proteins.

The invention further contemplates antibodies having specificity against an epitope of a KLK-L Related Protein of the invention. Antibodies may be labeled with a detectable substance and used to detect proteins of the invention in tissues and cells.

The invention also permits the construction of nucleotide probes which are unique to the nucleic acid molecules of the invention and/or to proteins of the invention. Therefore, the invention also relates to a probe comprising a nucleic acid sequence of the invention, or a nucleic acid sequence encoding a protein of the invention, or a part thereof. The probe may be labeled, for example, with a detectable substance and it may be used to select from a mixture of nucleotide sequences a nucleic acid molecule of the invention including nucleic acid molecules coding for a protein which displays one or more of the properties of a protein of the invention.

The invention still further provides a method for identifying a substance which binds to a protein of the invention comprising reacting the protein with at least one substance which potentially can bind with the protein, under conditions which permit the formation of complexes between the substance and protein and detecting binding. Binding may be detected by assaying for complexes, for free substance, or for non-complexed protein. The invention also contemplates methods for identifying substances that bind to other intracellular proteins that interact with a KLK-L Related Protein. Methods can also be utilized which identify compounds which bind to KLK-L gene regulatory sequences (e.g. promoter sequences).

Still further the invention provides a method for evaluating a compound for its ability to modulate the biological activity of a KLK-L Related Protein of the invention. For example a substance which inhibits or enhances the interaction of the protein and a substance which binds to the protein may be evaluated. In an embodiment, the method comprises providing a known concentration of a KLK-L Related Protein, with a substance which binds to the protein and a test compound under conditions which permit the formation of complexes between the substance and protein, and removing and/or detecting complexes.

Compounds which modulate the biological activity of a protein of the invention may also be identified using the methods of the invention by comparing the pattern and level of expression of the protein of the invention in tissues and cells, in the presence, and in the absence of the compounds.

The proteins of the invention and substances and compounds identified using the methods of the invention, and peptides of the invention may be used to modulate the biological activity of a KLK-L Related Protein of the invention, and they may be used in the treatment of conditions such as cancer (e.g. breast, testicular, and prostate cancer). Accordingly, the substances and compounds may be formulated into compositions for administration to individuals suffering from cancer.

Therefore, the present invention also relates to a composition comprising one or more of a protein of the invention, a peptide of the invention, or a substance or compound identified using the methods of the invention, and a pharmaceutically acceptable carrier, excipient or diluent. A method for treating or preventing cancer is also provided comprising administering to a patient in need thereof, a KLK-L Related Protein of the invention, or a composition of the invention.

Other objects, features and advantages of the present invention will become apparent from the following detailed description. It should be understood, however, that the detailed description and the specific examples while indicating preferred embodiments of the invention are given by way of illustration only, since various changes and modifications within the spirit and scope of the invention will become apparent to those skilled in the art from this detailed description.

### BRIEF DESCRIPTION OF THE DRAWINGS

The invention will now be described in relation to the drawings in which:

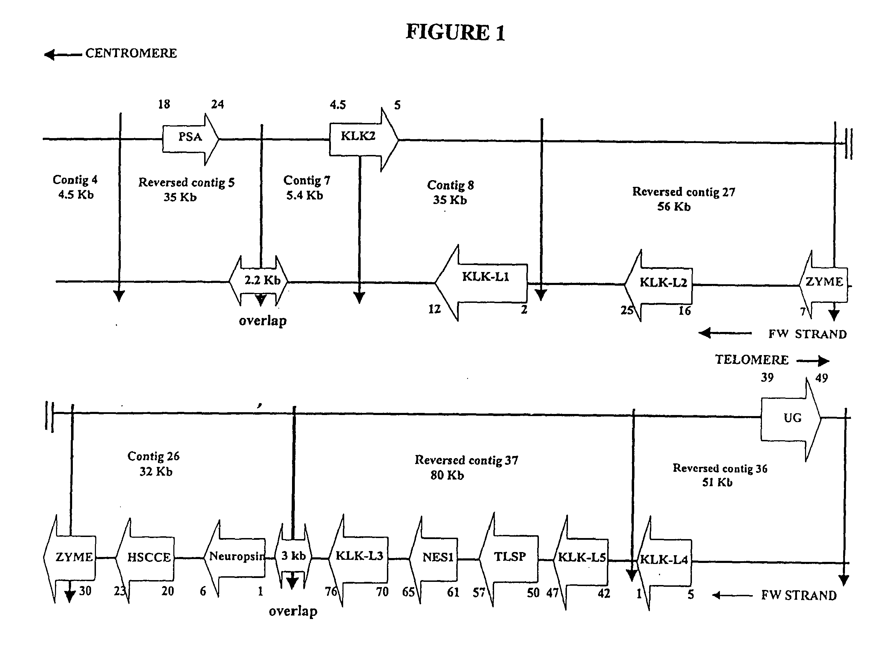

Figure 1 shows an approximate 300 Kb of contiguous genomic sequence around chromosome 19q13.3 - q13.4 represented by 8 contigs, each one shown with its length in Kb. The contig numbers refer to those reported in the Lawrence Livermore National Laboratory website. Note the localization of the seven known genes (PSA, KLK2, Zyme, NES1, HSCCE, neuropsin and TLSP) (see abbreviations for full names of these genes). All genes are represented with arrows denoting the direction of transcription. The gene with no homology to human kallikreins is termed UG (unknown gene). The five new kallikrein-like genes (KLK-L1 to KLK-L5) were numbered from the most centromeric to the most telomeric. Numbers just below or just above the arrows indicate appropriate Kb lengths in each contig. Gene lengths and distances between genes are rounded to the nearest 6.5 kb. The site of the gap is marked with an asterisk.

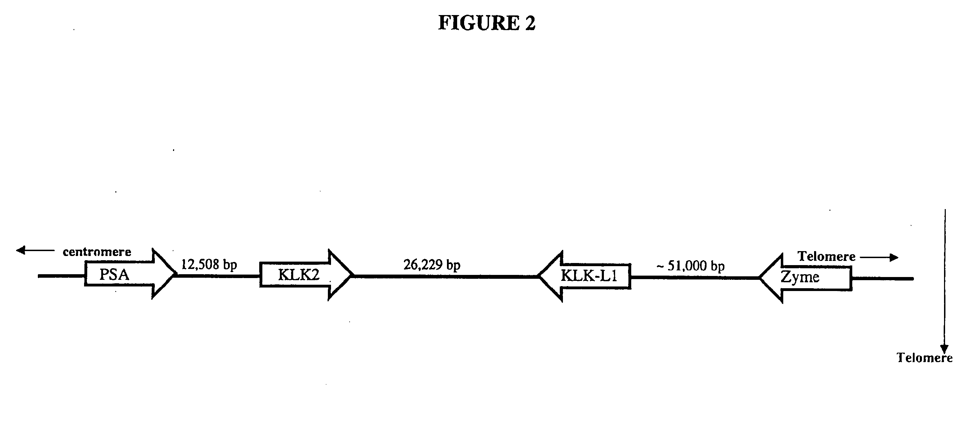

Figure 2 shows a contiguous genomic sequence around chromosome 19q 13.3- q13.4. Genes are represented by horizontal arrows denoting the direction of the coding sequence. Distances between genes are in base pairs.

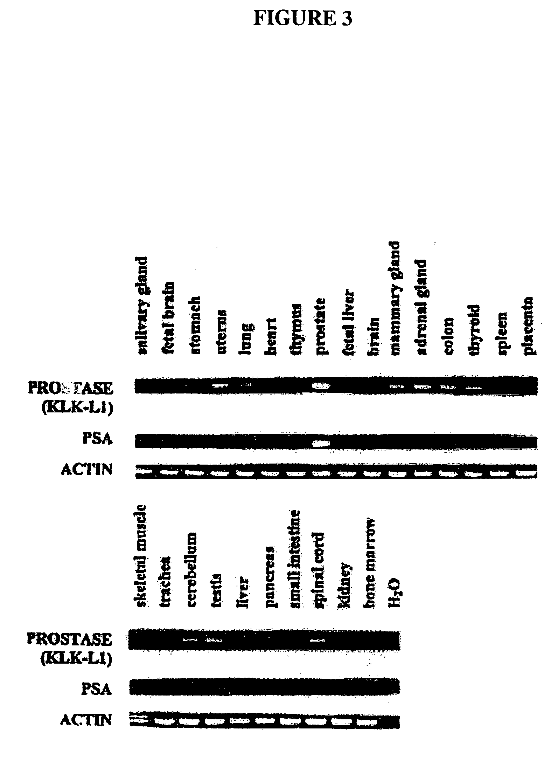

Figure 3 shows tissue expression of the prostase/KLK-L1 gene as determined by RT-PCR. Actin and PSA are control genes. Interpretations are presented in Table 9.

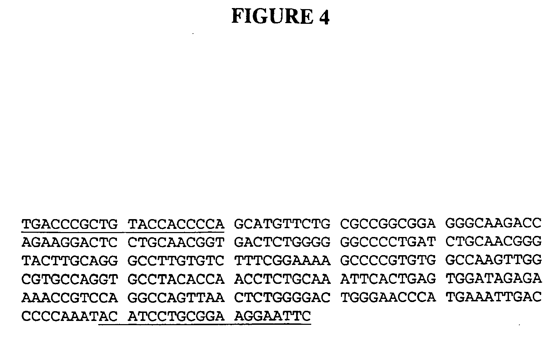

Figure 4 shows the sequence of PCR product obtained with cDNA from female breast tissue using prostase/KLK-L1 primers. Primer sequences are underlined. The sequence is identical to the sequence obtained from prostatic tissue.

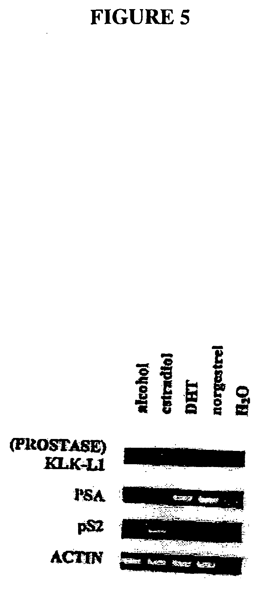

Figure 5 is a blot showing the results of experiments for hormonal regulation of the prostase/KLK-L1 gene in the BT-474 breast carcinoma cell lines. DHT = dihydrotestosterone. Steroids were added at 10⁻⁸ M final concentrations. Actin (not regulated by steroid hormones), pS2 (up-regulated by estrogens) and PSA (up-regulated by androgens and progestins), are control genes. Prostase/KLK-L1 is up-regulated by androgens and progestins.

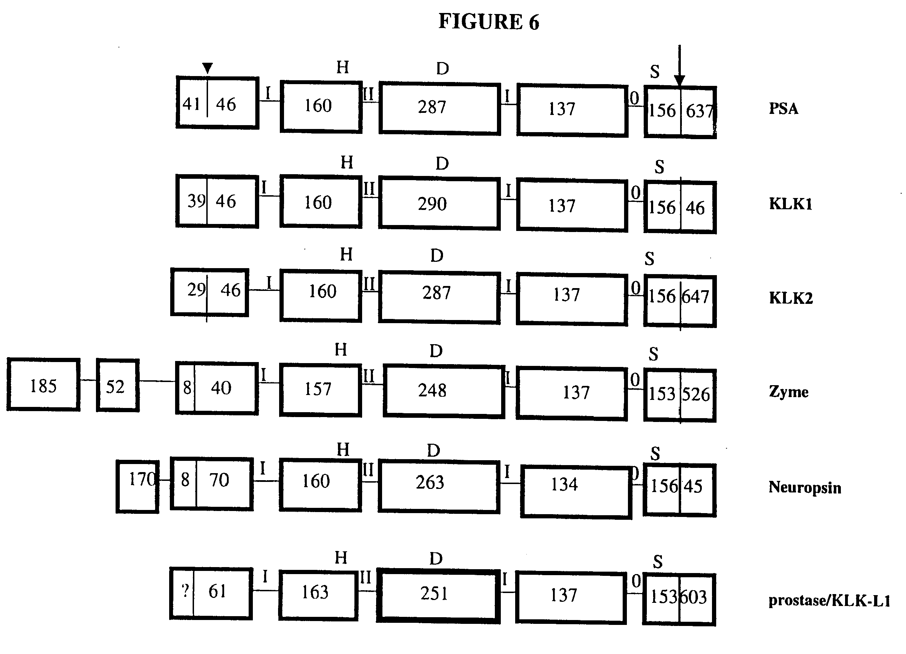

Figure 6 is a schematic diagram showing comparison of the genomic structure of PSA, KLK1, KLK2, zyme, neuropsin and prostase/KLK-L1 genes. Exons are shown by open boxes and introns by the connecting lines. Arrow head shows the start codons and the vertical arrow represents stop codons. Letters above boxes indicate relative positions of the catalytic triad; H denotes histidine, D aspartic acid and S serine. Roman numbers indicate intron phases. The intron phase refers to the location of the intron within the codon; I denotes that the intron occurs after the first nucleotide of the codon, II the intron occurs after the second nucleotide, 0 the intron occurs between codons. Numbers inside boxes indicate exon lengths in base pairs.

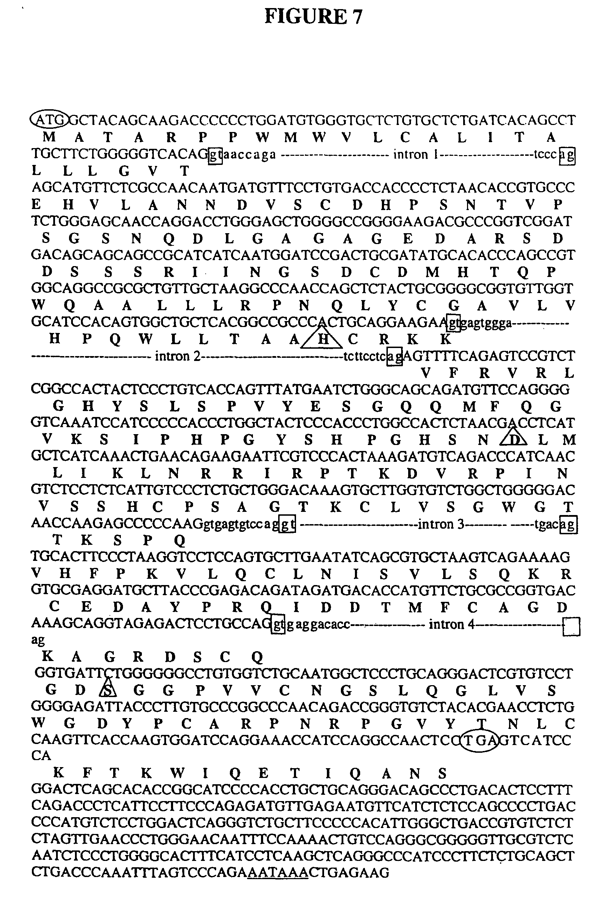

Figure 7 shows the genomic organization and partial genomic sequence of the KLK-L2 gene. Intronic sequences are not shown except for the splice junctions. Introns are shown with lower case letters and exons with capital letters. The start and stop codons are encircled and the exon -intron junctions are boxed. The translated amino acids of the coding region are shown underneath by a single letter abbreviation. The catalytic residues are inside triangles. Putative polyadenylation signal is underlined.

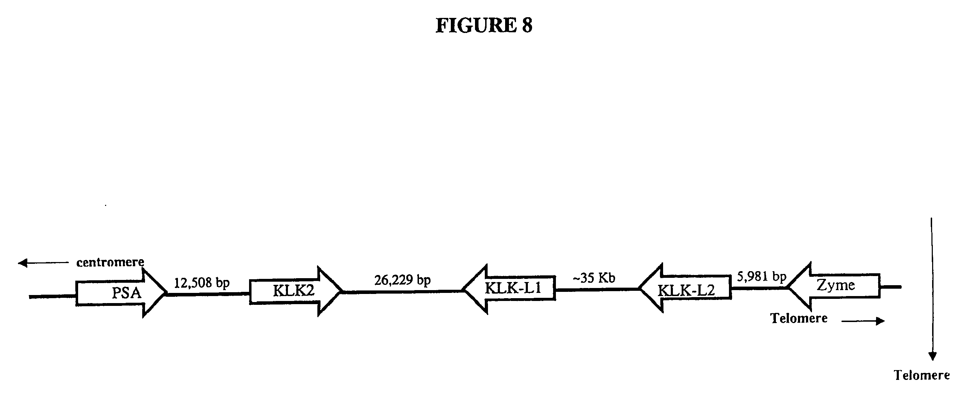

Figure 8 shows an approximate 300 Kb region of almost contiguous genomic sequence around chromosome 19q13.3- q13.4.Genes are represented by horizontal arrows denoting the direction of the coding sequence. Distances between genes are mentioned in base pairs .

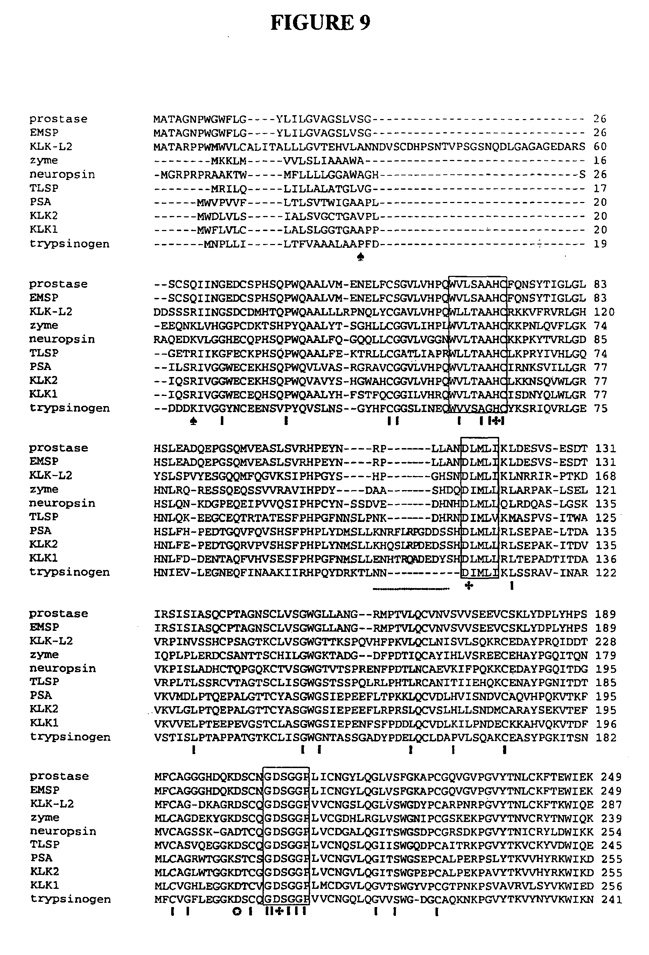

Figure 9 shows the alignment of the deduced amino acid sequence of KLK-L2 with members of the kallikrein multi-gene family. Genes are (from top to bottom) : Prostase/KLK-L1, enamel matrix serine proteinase 1 (EMSP1) (GenBank accession # NP_004908), KLK-L2, zyme (GenBank accession # Q92876), neuropsin (GenBank accession # BAA28673), trypsin-like serine protease (TLSP) (GenBank accession # BAA33404), PSA (GenBank accession # P07288), KLK2 (GenBank accession # P20151), KLK1 (GenBank accession # NP_002248 and trypsinogen (GenBank accession # P07477). (See SEQ.ID. NOs. 68-77) Dashes represent gaps to bring the sequences to better alignment. The residues of the catalytic triad are represented by ( 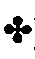 ) and the 29 invariant serine protease residues by (I or 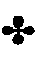 ). Conserved areas around the catalytic triad are boxed. The predicted cleavage sites are indicated by ( 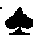 ). The dotted area represents the kallikrein loop sequence. The trypsin like cleavage pattern is indicated by ( 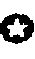 ).

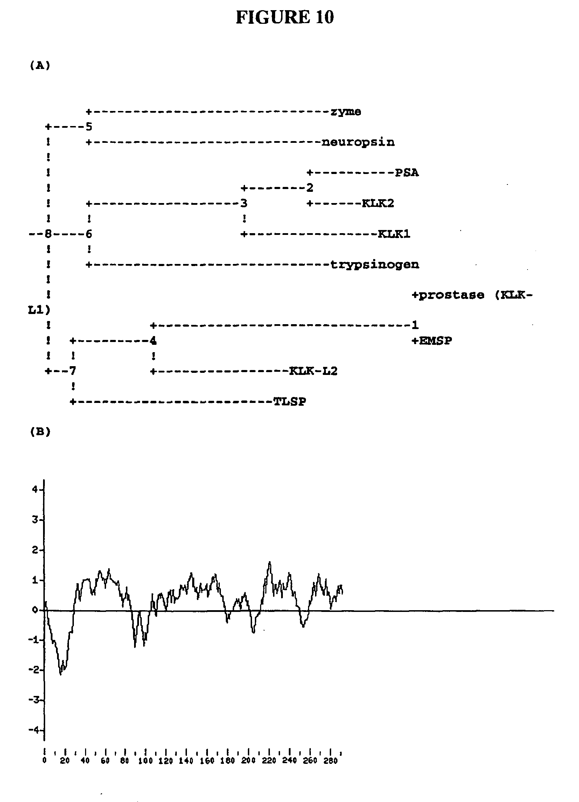

Figure 10(A) shows a dendrogram of the predicted phylogenetic tree for some kallikrein genes. Neighbor-joining/UPGMA method was used to align KLK-L2 with other members of the kallikrein gene family. Gene names and accession numbers are listed in Figure 9. The tree grouped the classical kallikreins (KLK1, KLK2, and PSA) together and aligned the KLK-L2 gene in one group with EMSP, prostase, and TLSP. (B) Plot of hydrophobicity and hydrophilicity of KLK-L2.

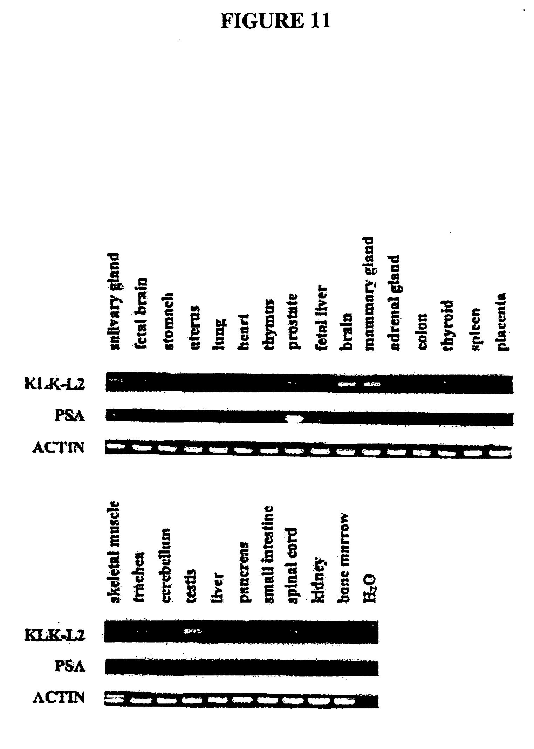

Figure 11 is a blot showing tissue expression of KLK-L2 gene as determined by RT-PCR. Actin and PSA are control genes. Interpretations are presented in Table 12.

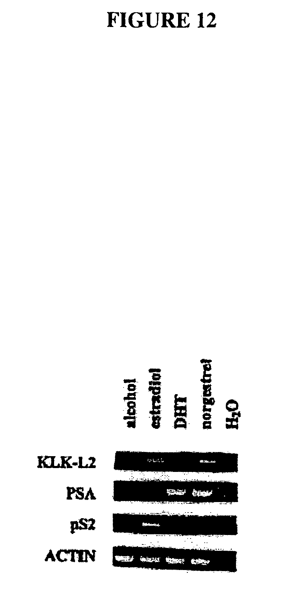

Figure 12 is a blot showing hormonal regulation of the KLK-L2 gene in BT-474 breast carcinoma cell lines. DHT = dihydrotestosterone. Steroids were at 10⁻⁸ M final concentrations. Actin (not regulated by steroid hormones), pS2 (up-regulated by estrogens) and PSA (upregulated by androgens and progestins), are control genes. KLK-L2 is upregulated by estrogens and progestins.

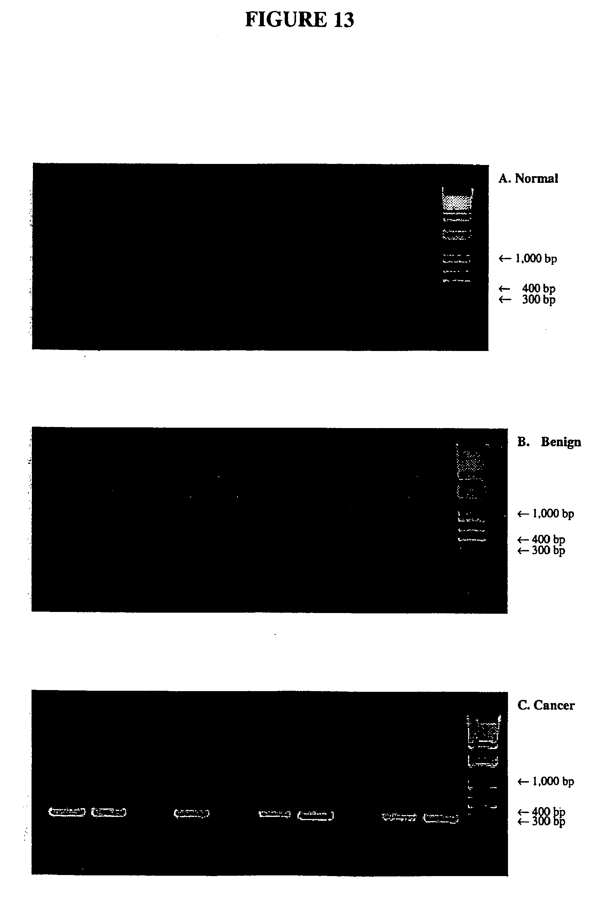

Figure 13 are blots of EtBr-stained agarose gels. Total RNA was extracted from normal, benign, and cancer tissues and used to generate cDNA. PCR was performed on cDNA

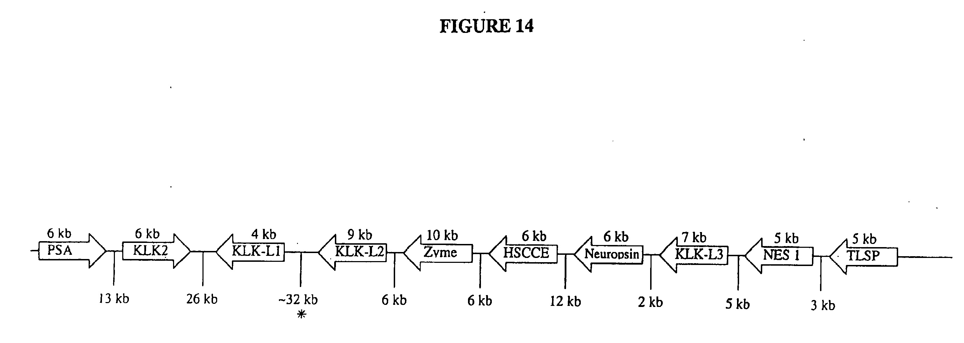

Figure 14 shows an approximate 300 Kb region of almost contiguous genomic sequence around chromosome 19q 13.3- q 13.4.Genes are represented by horizontal arrows denoting the direction of the coding sequence. Gene lengths and distances between genes are rounded to the nearest 0.5 kb. The site of the gap is marked with an asterisk. Telomeric to TLSP there are likely another three kallikrein-like genes.

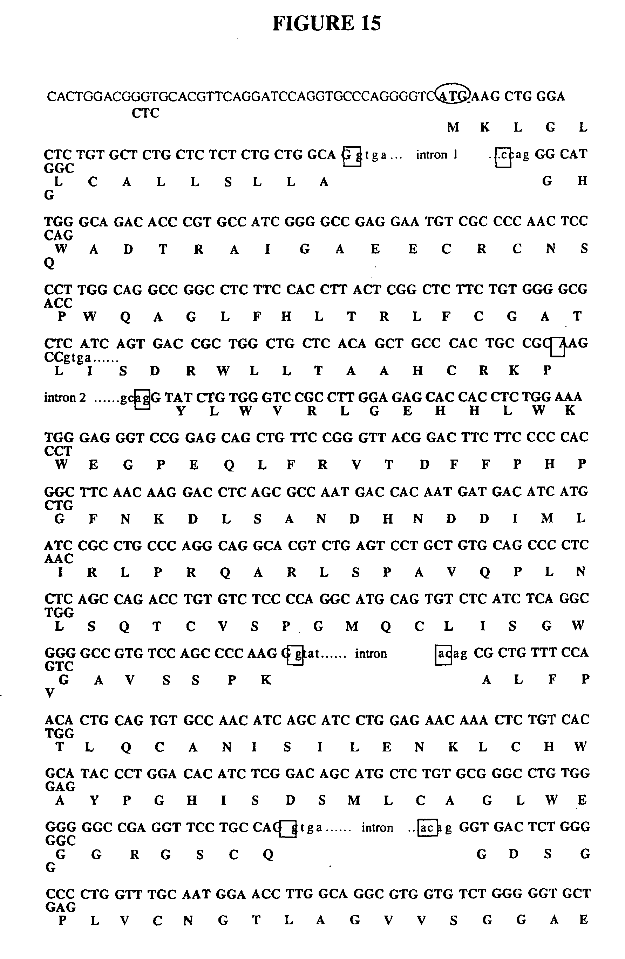

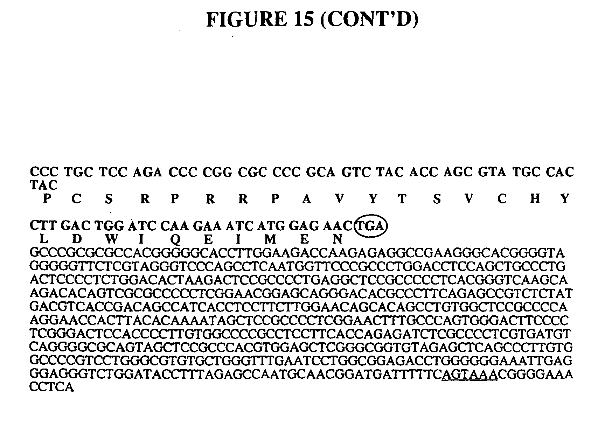

Figure 15 shows the genomic organization and partial genomic sequence of the KLK-L3 gene. Intronic sequences are not shown except for the splice junctions. Introns are shown with lower case letters and exons with capital letters. For the full sequence, see SEQ.ID. NO. 21. The start and stop codons are encircled and the exon -intron junctions are boxed. The translated amino acids of the coding region are shown underneath by a single letter abbreviation. The catalytic residues are inside triangles. Putative polyadenylation signal is underlined.

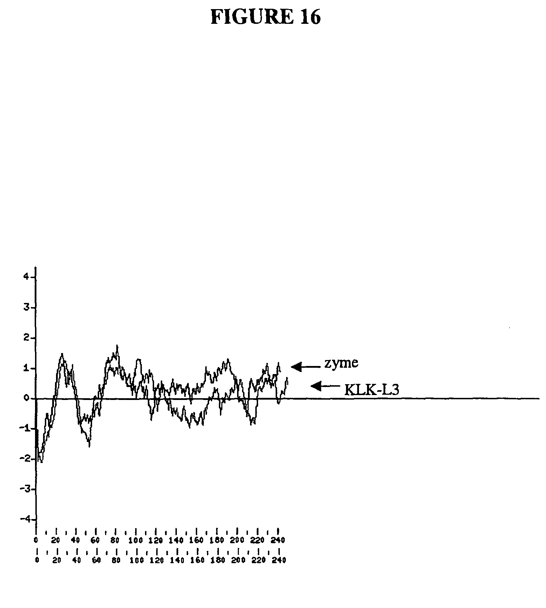

Figure 16 is a plot of hydrophobicity and hydrophilicity, comparing the pattern of the KLK-L3 with that of the zyme gene. Note the hydrophobic region around the first twenty amino acids, likely representing the signal peptide.

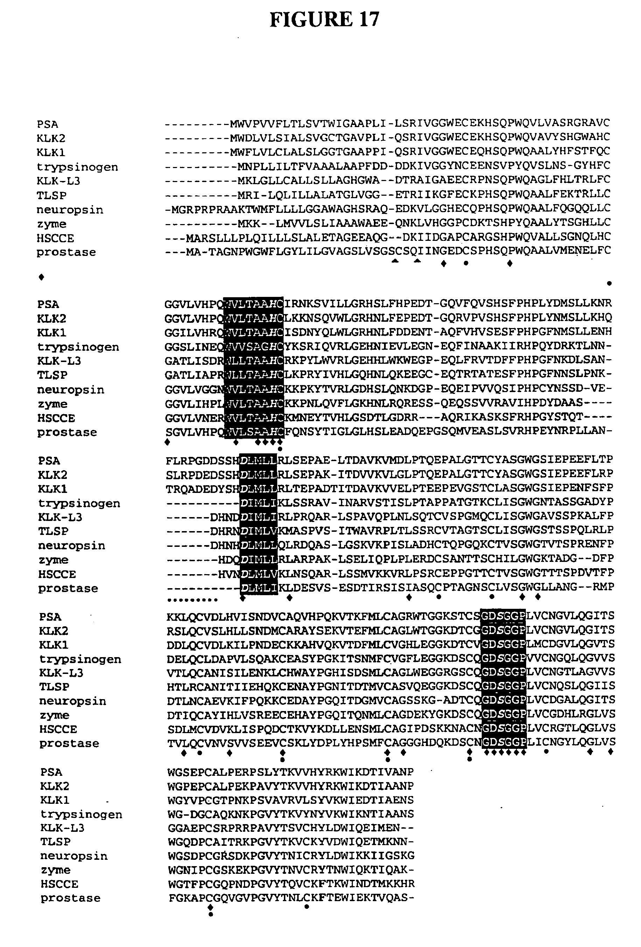

Figure 17 is an alignment of the deduced amino acid sequence of KLK-L3 with members of the kallikrein multi-gene family. Genes are (from top to bottom and in brackets is the GenBank accession #): PSA (P07288), KLK2 (P20151), KLK1 (NP002248), trypsinogen (P07477), KLK-L3 (AF135026), trypsin-like serine protease (TLSP) (BAA33404), neuropsin (BAA28673), zyme (Q92876), human stratum corneum chymotryptic enzyme (HSCCE) (AAD49718), and/prostase/KLK-L1 (AAD21581). (See SEQ.ID. NOs. 78 to 84). Dashes represent gaps to bring the sequences to better alignment. The residues of the catalytic triad are bold and in italics, and the 29 invariant serine protease residues are denoted by (◆). Cysteine residues are marked by (•). Conserved areas around the catalytic triad are highlited in black. The arrow heads (▲) represent the potential cleavage sites. The dotted area represents the kallikrein loop sequence.

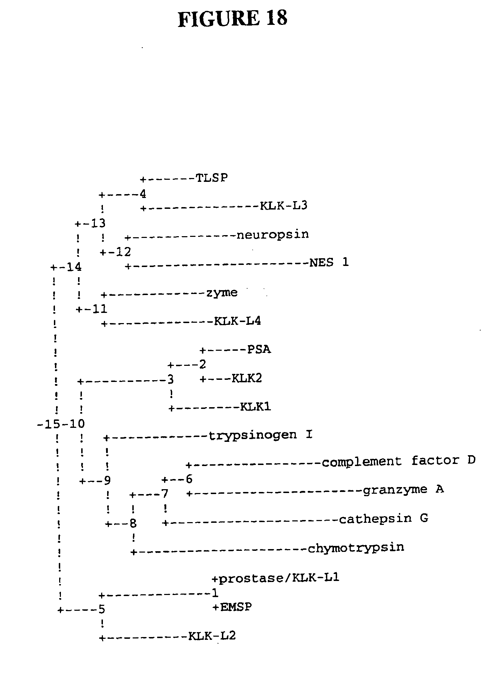

Figure 18 is a dendrogram of the predicted phylogenetic tree for some serine proteases and kallikrein genes. Neighbor-joining/UPGMA method was used to align KLK-L3 with other members of the kallikrein gene family. Gene names and accession numbers are listed in Figure 17. The tree grouped the classical kallikreins (KLK1, KLK2, and PSA) together and aligned the KLK-L3 gene in one group with TLSP, neuropsin, and NES 1 genes. KLK-L4 (SEQ.ID.NO. 43) lies further telomeric to TLSP (21).

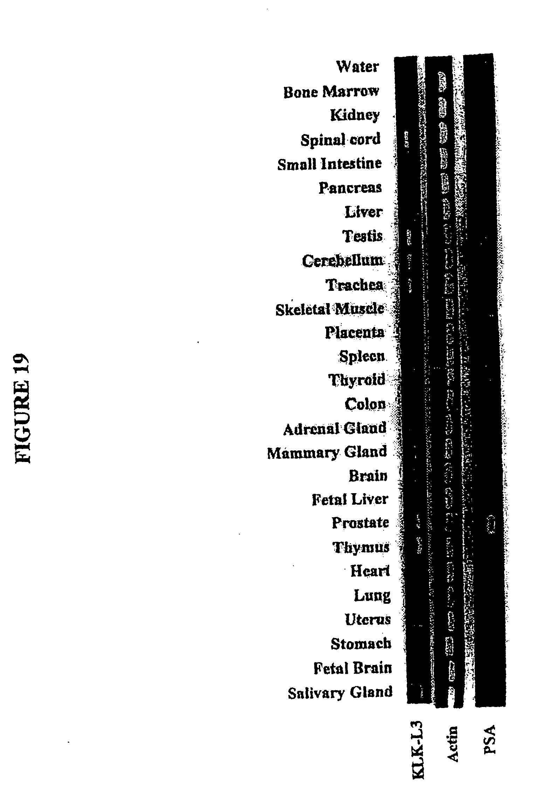

Figure 19 is a blot showing tissue expression of the KLK-L3 gene as determined by RT-PCR. Actin and PSA are control genes.

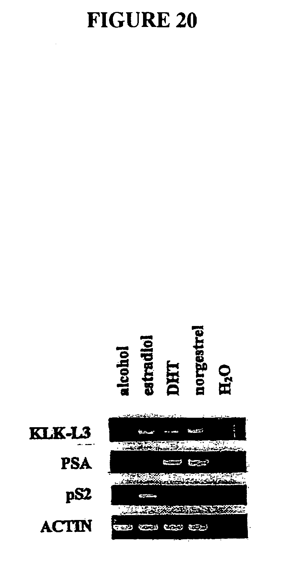

Figure 20 shows hormonal regulation of the KLK-L3 gene in the BT-474 breast carcinoma cell line. DHT = dihydrotestosterone. Steroids were at 10⁻⁸ M final concentrations. Actin (not regulated by steroid hormones), pS2 (up-regulated by estrogens) and PSA (upregulated by androgens and progestins), are control genes. KLK-L3 is upregulated by progestins, estrogens and androgens, in that order.

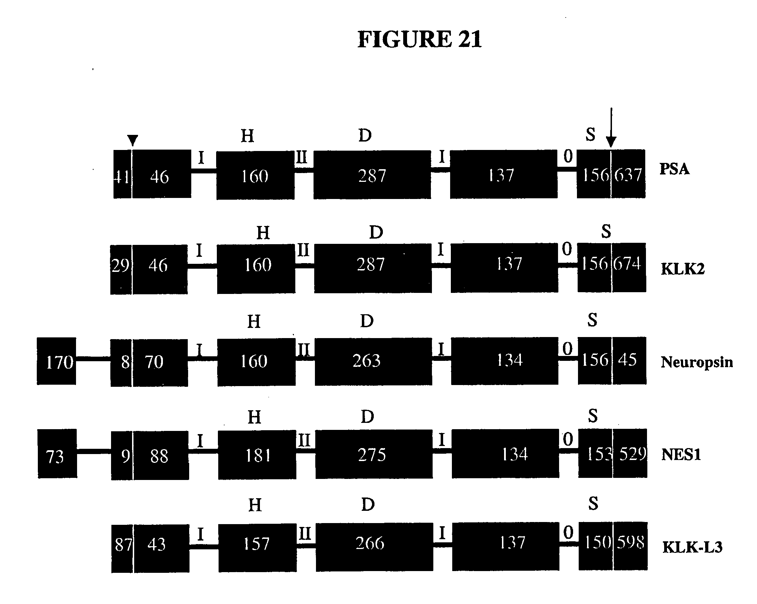

Figure 21 is a schematic diagram showing the comparison of the genomic structure of PSA, KLK2,neuropsin, NES1, and KLK-L3 genes. Exons are shown by black boxes and introns by the connecting lines. Arrowheads show the start codon, and arrows show the stop codon. Letters above boxes indicate relative positions of the catalytic triad; H denotes histidine, D aspartic acid and S serine. Roman numbers indicate intron phases. The intron phase refers to the location of the intron within the codon; I denotes that the intron occurs after the first nucleotide of the codon, II the intron occurs after the second nucleotide, 0 the intron occurs between codons. Numbers inside boxes indicate exon lengths in base pairs.

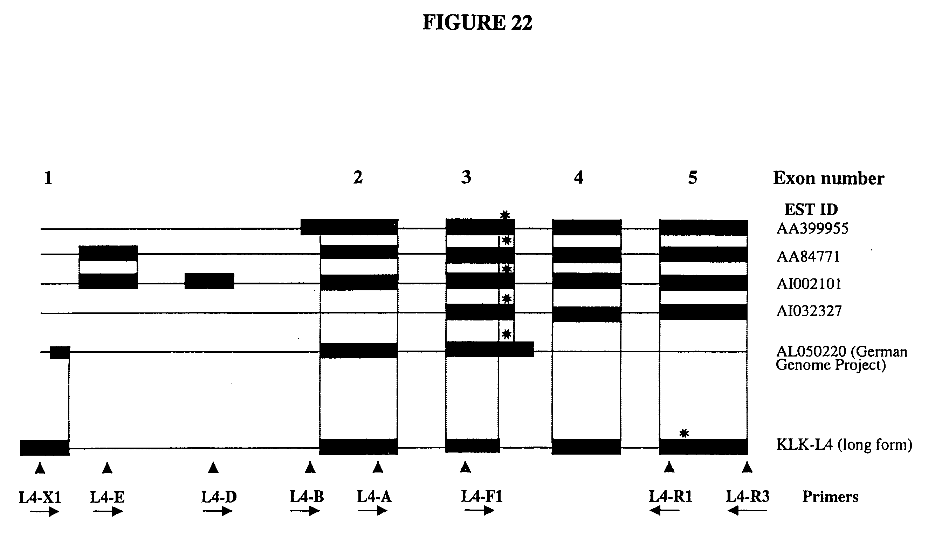

Figure 22 shows a comparative genomic structure of the ESTs (Table 16), the clone from The German Genome Project, and the long form of KLK-L4. Exons are represented by solid bars and introns by the connecting lines. Exon numbers on top of solid bars refer to GenBank submission #AF135024. The EST IDs represent GenBank accession numbers. Asterisks represent the positions of stop codons. Horizontal arrows indicate the direction of the PCR primers (described in Table 15) and arrowheads their position along the exons. Vertical dotted lines show alignment of identical fragments.

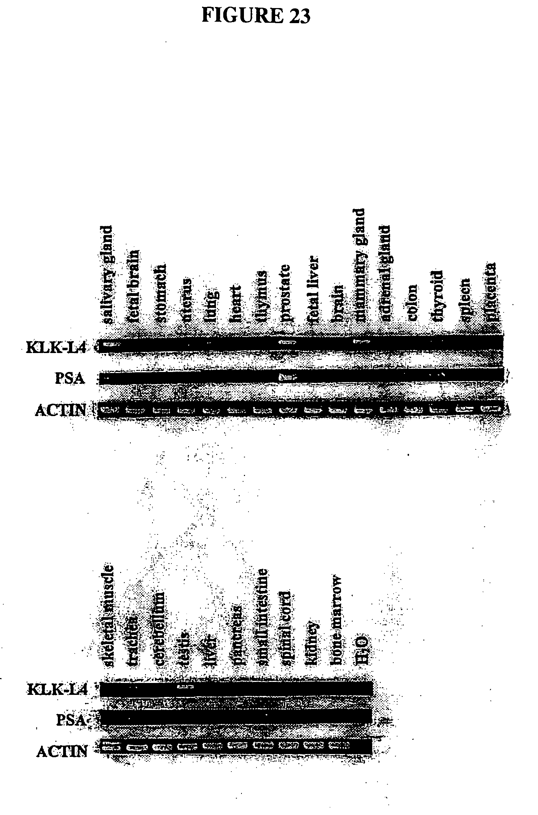

Figure 23 shows tissue expression of the KLK-L4 gene as determined by RT-PCR. Actin and PSA are control genes. KLK-L4 is highly expressed in breast, prostate, salivary gland and testis.

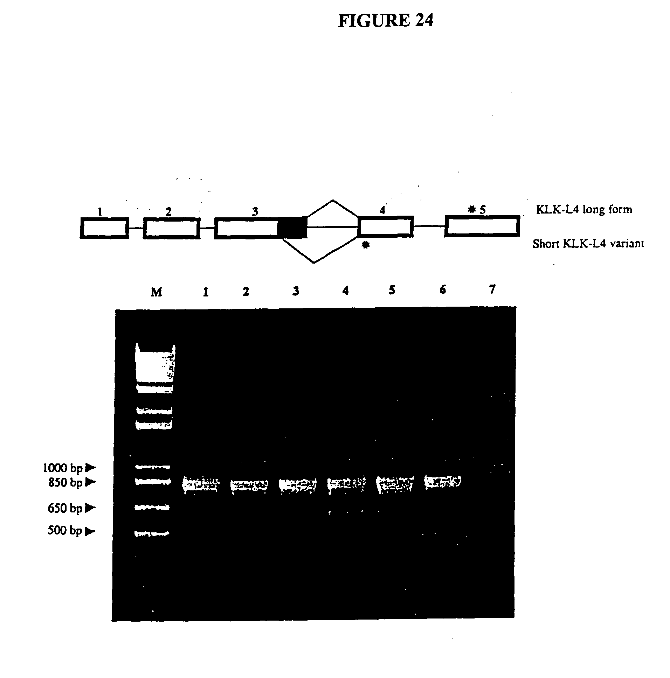

Figure 24 in the Upper Panel is a Diagram showing the comparative genomic structure of the long KLK-L4 form and the short KLK-L4 variant. Exons are represented by boxes and introns by the connecting lines. Exon numbers refer to SEQ. ID. NO. 43 and GenBank Accession No. AF135024. The black region indicates the extra fragment (214 bp) that is found in the long, but not in the short form of the gene. The positions of the stop codons of the two forms are marked with asterisks. Frame shifting occurs as a result of utilization of an alternative splice site, and a stop codon is generated at the beginning of exon 4 in the short form. The Lower Panel shows PCR products of the amplification of the KLK-L4 gene using L4-R1 and L4-X1 primers (Figure 22 and Table 15). Note the predominant long form and a minor band representing the short form of KLK-L4 mRNA. (M); Markers with sizes in bp shown on the left. Tissues used: (1), salivary gland; (2), mammary gland; (3), prostate; (4), testis; (5), uterus; (6), breast cancer tissue; (7), negative control.

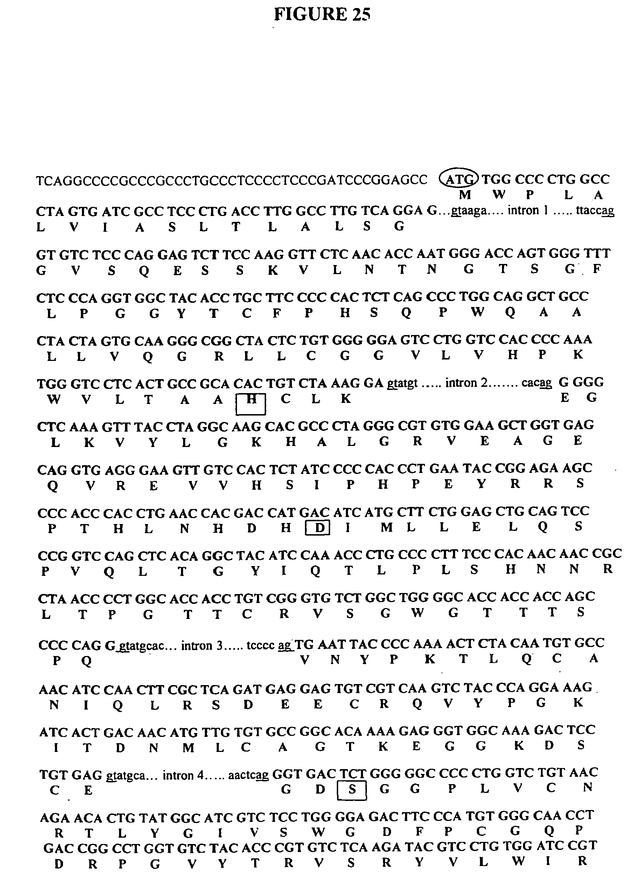

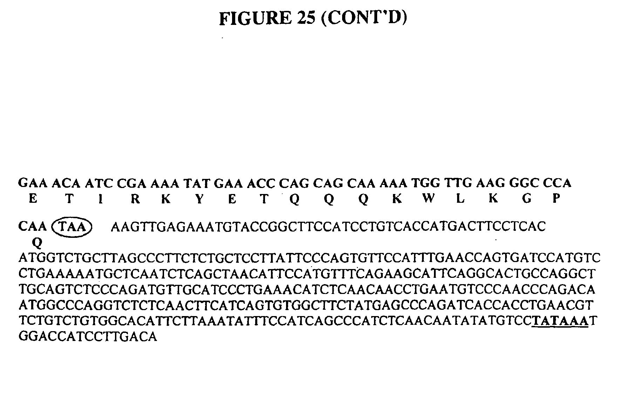

Figure 25 shows the genomic organization and partial genomic sequence of the KLK-L4 gene. Intronic sequences are not shown except for the splice junction areas. Introns are shown with lower case letters and exons with capital letters. For full sequence, see SEQ. ID. NO.43 or GenBank Accession #AF135024. The start and stop codons are encircled and the exon -intron junctions are underlined. The translated amino acids of the coding region are shown underneath by a single letter abbreviation. The catalytic residues are boxed. The putative polyadenylation signal is underlined.

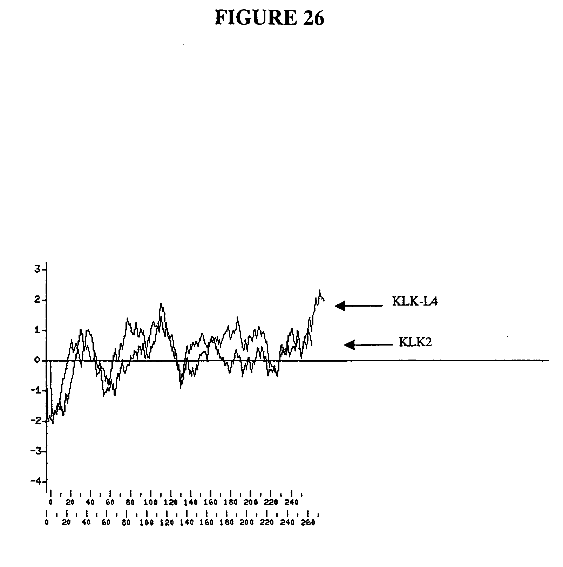

Figure 26 is a plot of hydrophobicity and hydrophilicity of the KLK-L4 protein, as compared with the glandular kallikrein gene 2 (KLK2). Note the hydrophobic region at the amino terminus, suggesting presence of a signal peptide.

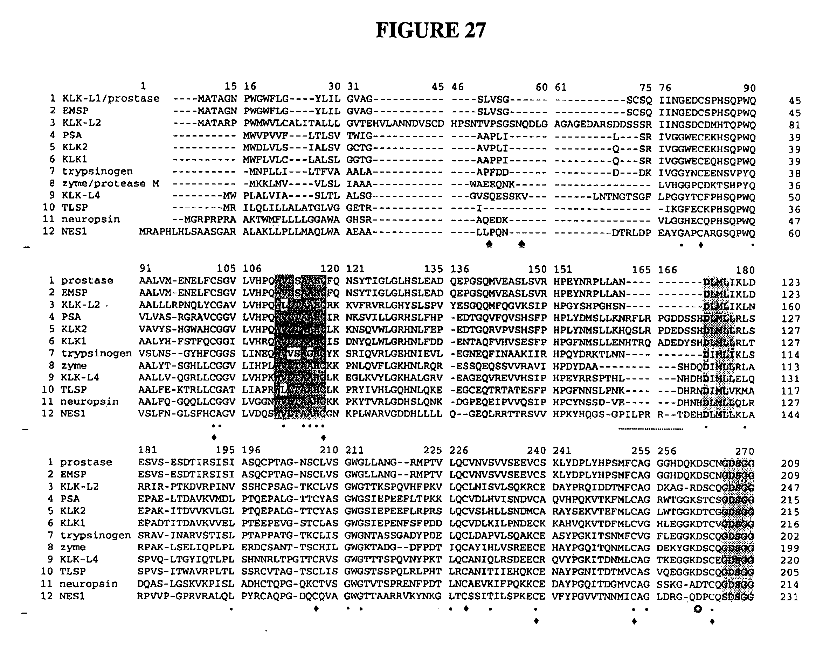

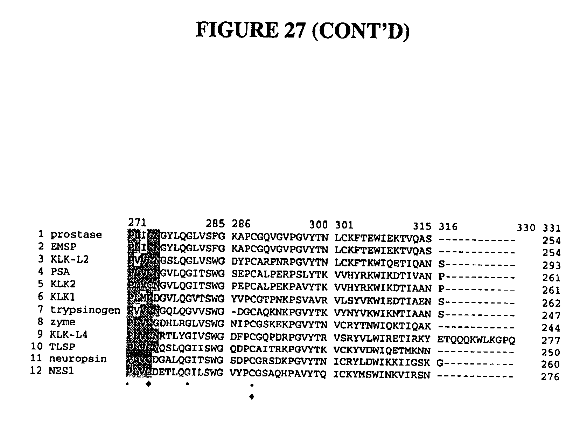

Figure 27 shows an alignment of the deduced amino acid sequence of KLK-L4 with members of the kallikrein multi-gene family. Genes are (from top to bottom, and in brackets are the GenBank accession #): KLK-L1/prostase (AAD21581), enamel matrix serine proteinase 1 (EMSP) (NP_004908), KLK-L2 (AF135028), PSA (P07288), KLK2 (P20151), KLK1 (NP_002248), trypsinogen (P07477), zyme (Q92876), KLK-L4 (AF135024), trypsin-like serine protease (TLSP) (BAA33404), KLK-L3 (AF135026),neuropsin (BAA28673), and the normal epithelial cell-specific 1 gene (NES1) (043240). (See SEQ.ID. NOs. 78-88). Dashes represent gaps to bring the sequences to better alignment. The residues of the catalytic triad are typed in bold, and conserved motifs around them are highlighted in grey.The 29 invariant serine protease residues are denoted by (•), and the cysteine residues by (◆). The predicted cleavage sites are indicated by ( 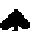 ). The dotted area represents the kallikrein loop sequence. The trypsin-like cleavage pattern of KLK-L4 with the D residue, is indicated by ( 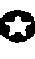 ).

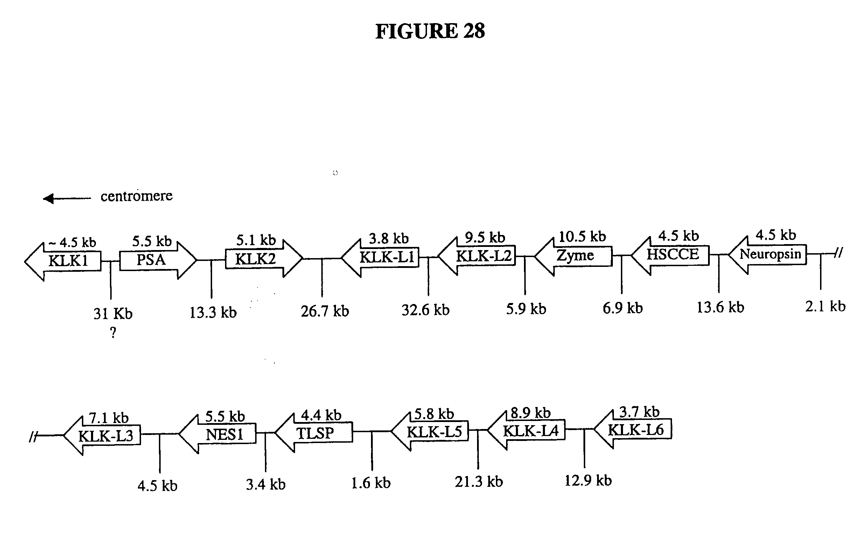

Figure 28 shows an approximate 300 Kb region of almost contiguous genomic sequence around chromosome 19q13.3- q13.4.Genes are represented by horizontal arrows denoting the direction of the coding sequence. Their lengths are shown on top of each arrow. Distances between genes are mentioned in base pairs below the arrows. The distance between KLK1 and PSA is not accurately known. For gene names, see under abbreviations.

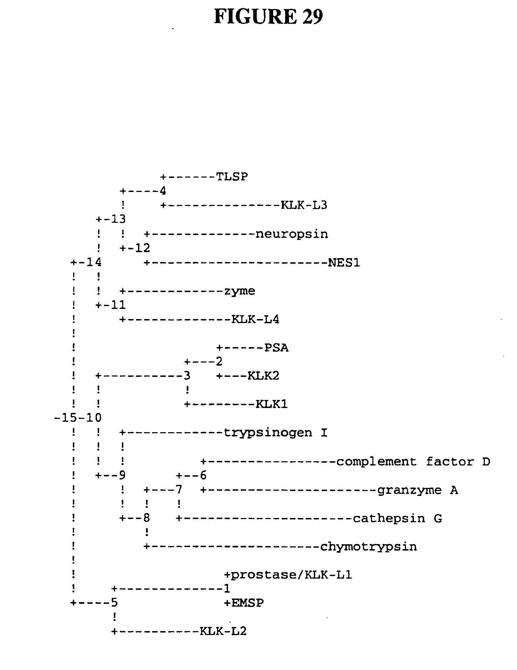

Figure 29 shows is a dendrogram of the predicted phylogenetic tree for some kallikrein and serine protease genes. The neighbor-joining/UPGMA method was used to align KLK-L4 with other serine proteases and members of the kallikrein gene family. The tree grouped the classical kallikreins (KLK1, KLK2, and PSA) together and aligned the KLK-L4 gene in one group with zyme, NES1, neuropsin, KLK-L3, and TLSP. Other serine proteases were aligned in different groups, as shown.

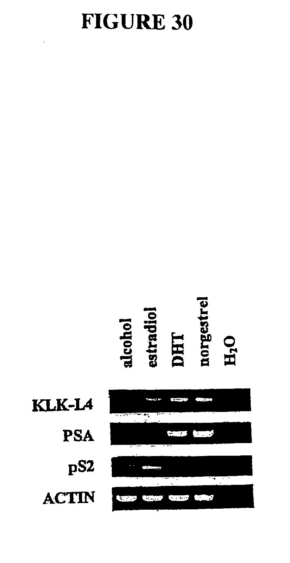

Figure 30 is a blot showing the hormonal regulation of the KLK-L4 gene in the BT-474 breast carcinoma cell line. DHT = dihydrotestosterone. Steroids were added at 10⁻⁸ M final concentrations. Actin (not regulated by steroid hormones), pS2 (up-regulated by estrogens) and PSA (upregulated by androgens and progestins) are control genes. KLK-L4 is up-regulated by androgens and progestins and to a lesser extent by estrogens. H₂O was used to check for PCR specificity in all PCR reactions. For more details, see text.

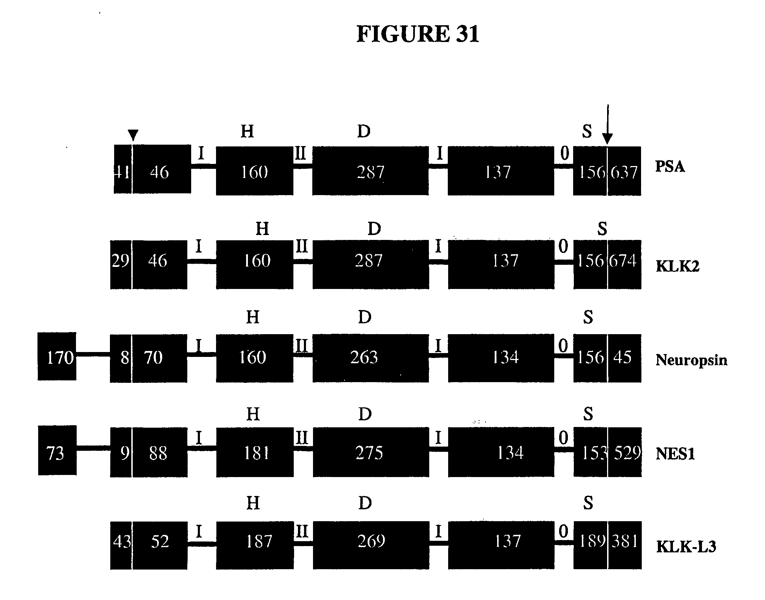

Figure 31 is a schematic diagram showing the comparison of the genomic structure of PSA, KLK2, neuropsin, NES1, and KLK-L4 genes. Exons are shown by black boxes and introns by the connecting lines. The arrowhead shows the start codons and the arrow the stop codons. Letters above boxes indicate the relative positions of the amino acids of the catalytic triad; H denotes histidine, D aspartic acid and S serine. Roman numbers indicate intron phases. The intron phase refers to the location of the intron within the codon; I, the intron occurs after the first nucleotide of the codon, II the intron occurs after the second nucleotide, 0 the intron occurs between codons. Numbers inside boxes indicate exon lengths in base pairs. The question mark indicates the possibility of more untranslated bases.

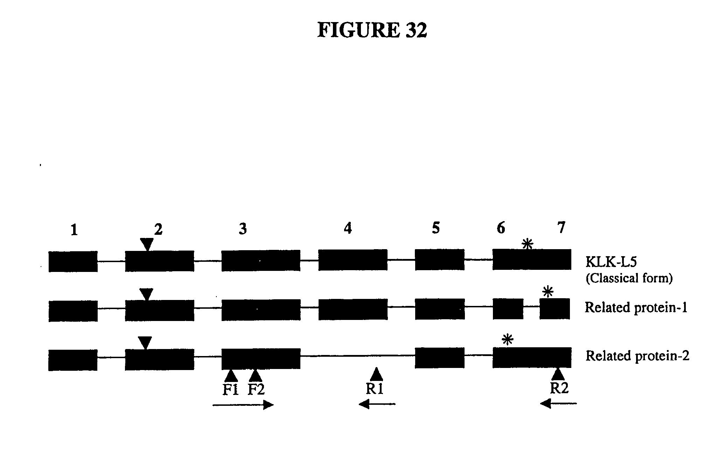

Figure 32 is a diagram showing the comparative genomic structure of the three splice forms of KLK-L5; the classical kallikrein form, related protein-1, and related protein-2. Exons are represented by solid bars and introns by the connecting lines. Exon numbers refer to SEQ.ID. NO.56 and GenBank Accession #AF135025. Start codons are represented by the inverted arrowhead (▼) and stop codons are represented by asterisks (*). Primer locations are represented by vertical arrowheads (▲) and their directions by horizontal arrows. For primer sequences and codes see Table 17 and SEQ.ID. NOs. 61-64, and 9-12.

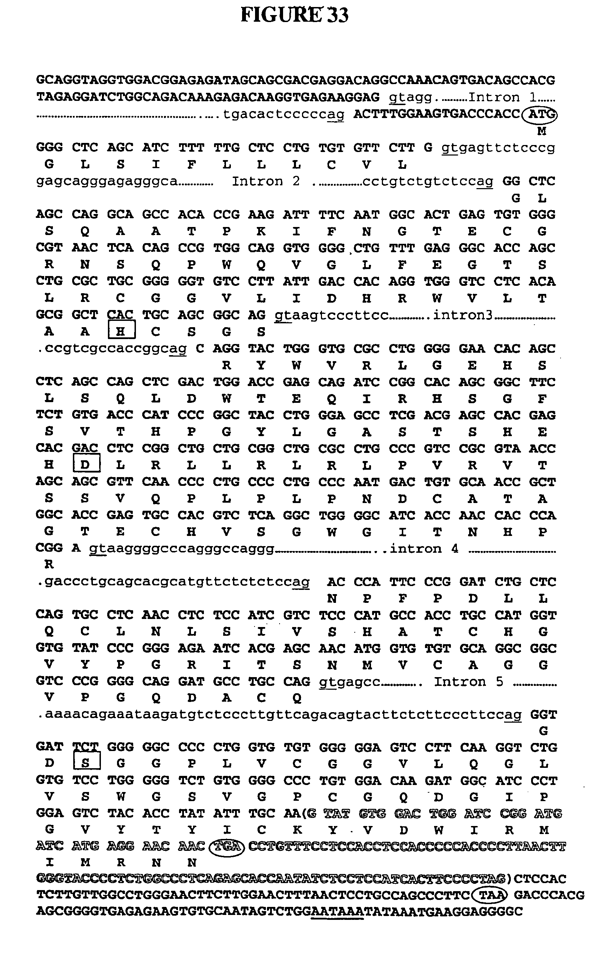

Figure 33 shows the genomic organization and partial genomic sequence of the KLK-L5 gene. Intronic sequences are not shown except for short sequences around the splice junctions. Introns are shown with lower case letters and exons with capital letters. For full sequence, see SEQ.ID.NO. 56. The start and stop codons are encircled and the exon -intron junctions are underlined. The translated amino acids of the coding region are shown underneath by a single letter abbreviation. The catalytic residues are boxed. Putative polyadenylation signal is underlined. The extra intron of the related protein-1 form is represented by non-bold capital letters between brackets. When this intron is spliced, the frame continues with codon AAC (asparagine, N, instead of lysine, K) until it encounters the stop codon TAA (encircled).

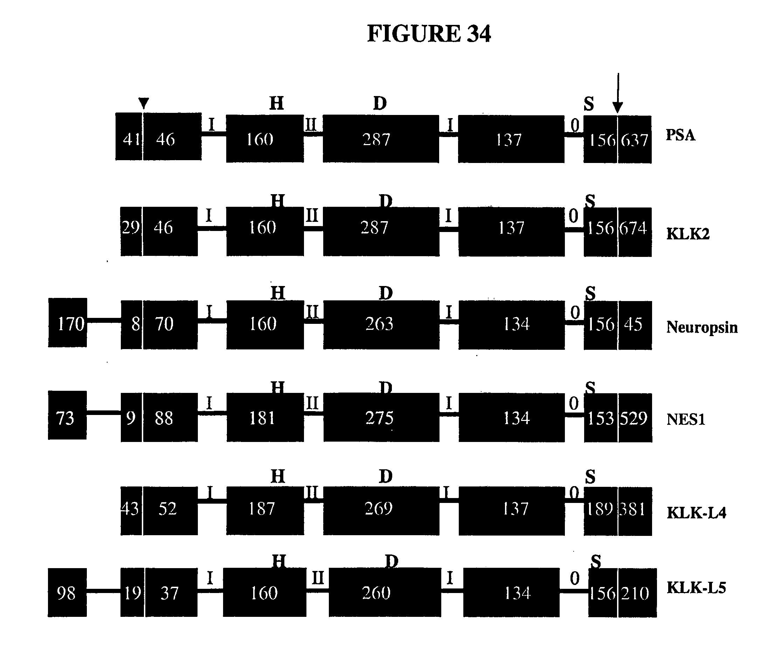

Figure 34 is a schematic diagram showing the comparison of the genomic structure of PSA, KLK2, neuropsin, NES1, KLK-L4 and KLK-L5 genes. Exons are shown by solid bars and introns by the connecting lines. Arrowhead marks the site of the start codon, and the arrow represents the stop codon. Letters above boxes indicate relative positions of the catalytic triad; H denotes histidine, D aspartic acid and S serine. Roman numbers indicate intron phases. The intron phase refers to the location of the intron within the codon; I denotes that the intron occurs after the first nucleotide of the codon, II the intron occurs after the second nucleotide, 0 the intron occurs between codons. Numbers inside boxes indicate exon lengths in base pairs. Question marks indicate that exact length is not accurately know.

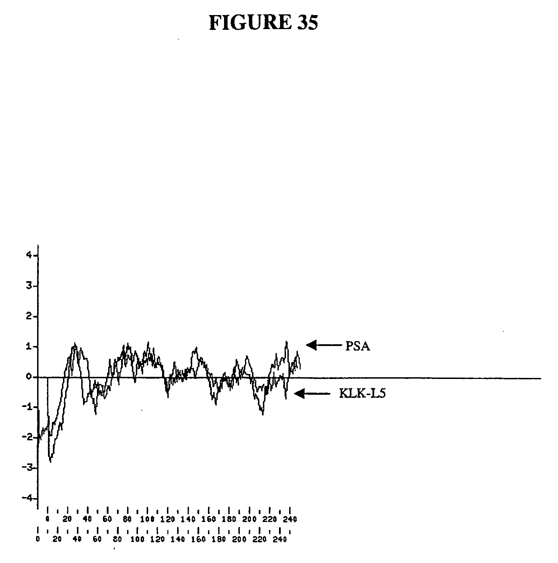

Figure 35 is a plot of hydrophobicity and hydrophilicity of KLK-L5 protein compared to prostate specific antigen (PSA). The hydrophobic N-terminus may harbor a signal and activation peptide.

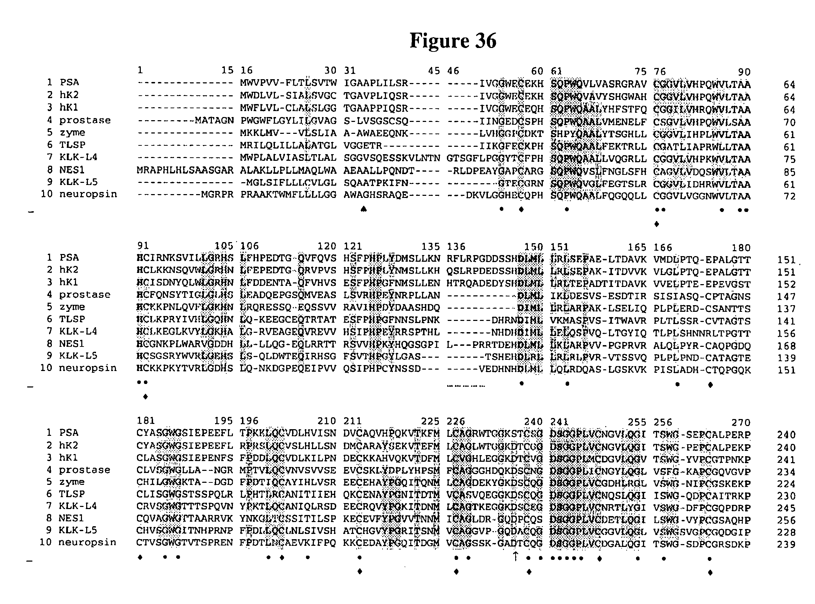

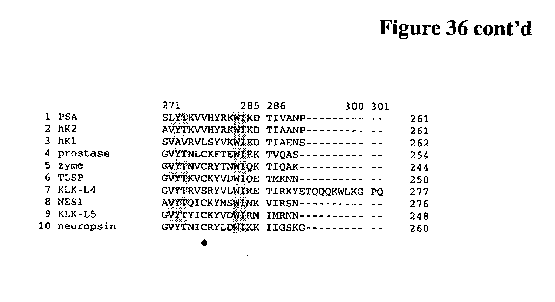

Figure 36 shows an alignment of the deduced amino acid sequence of KLK-L5 with members of the kallikrein multigene family. (See SEQ.ID. NOs. 78-81, 83, 84). Dashes represent gaps to bring the sequences to better alignment. The residues of the catalytic triad are represented by bold letters, and the 29 invariant serine protease residues are marked with (•). The cysteine residues are marked by (◆). Conserved areas are highlighted in grey. The predicted cleavage sites in signal peptide are indicated by ( 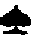 ). The dotted area represents the kallikrein loop sequence. A vertical arrow marks the trypsin like cleavage site.

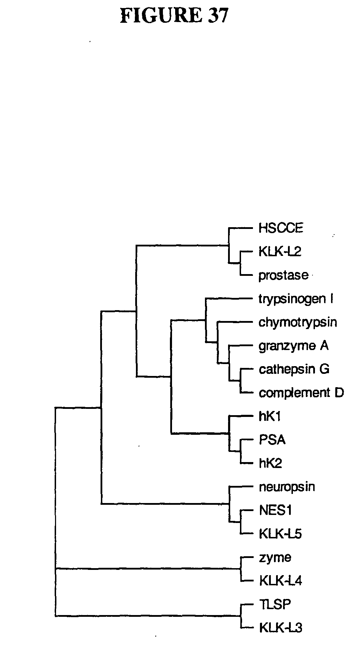

Figure 37 is a dendrogram of the predicted phylogenetic tree for some serine proteases and other kallikrein proteins. Neighbor-joining/UPGMA method was used to align KLK-L5 with other serine proteases and members of the kallikrein gene family. The tree grouped the classical kallikreins (hK1, hK2, and PSA) together and aligned the KLK-L5 protein in one group with NES 1 and neuropsin. Other serine proteases were aligned in different groups.

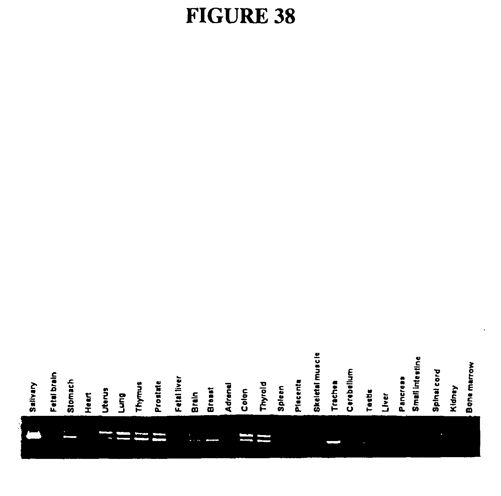

Figure 38 shows tissue expression of the KLK-L5 gene as determined by RT-PCR. The upper band (905 base pairs, bp) is the classical form (see Figure 32, the middle (776 bp) the related protein-1, and the lower band (644 bp) the related protein-2. For splice variant discussion see text. The primers used were L5-F2 and L5-R2, as shown in Table 17.

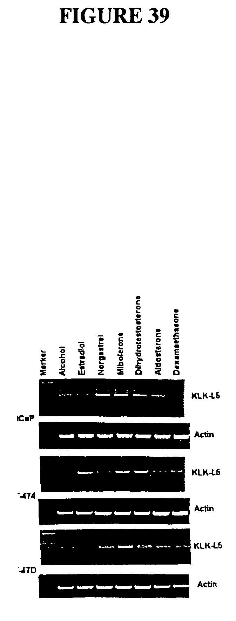

Figure 39 shows hormonal regulation of the KLK-L5 gene in the LnCaP prostatic carcinoma cell line, BT-474 and T-47D breast carcinoma cell lines. Steroids were at 10⁻⁸ M final concentration. Actin (not regulated by steroid hormones) was used as a control gene. Note detection of three isoforms only in LNCaP.

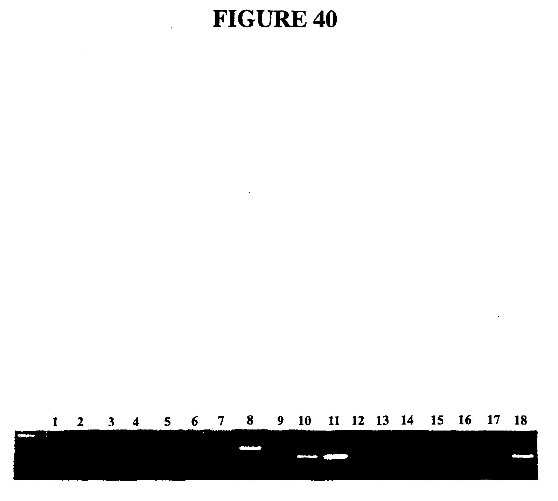

Figure 40 shows the expression of the KLK-L5 gene in breast cancer (1-17) and normal (18) tissues. Note complete absence of expression in 12 cancer tissues. For isoforms see also Figure 38.

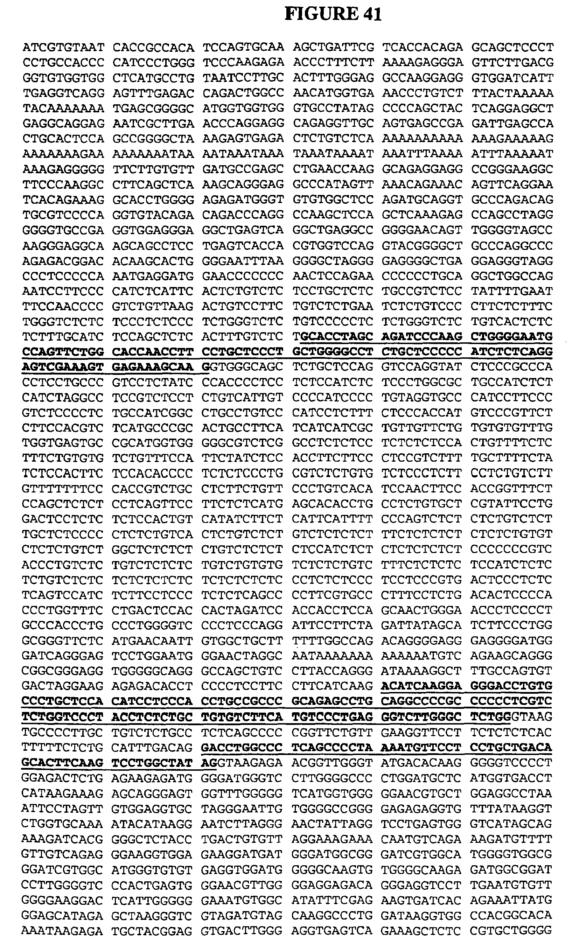

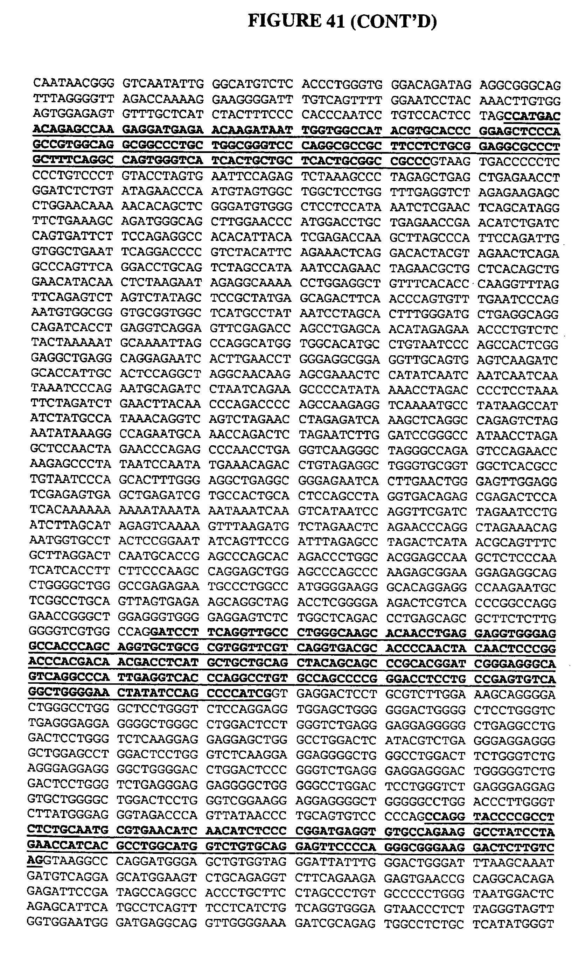

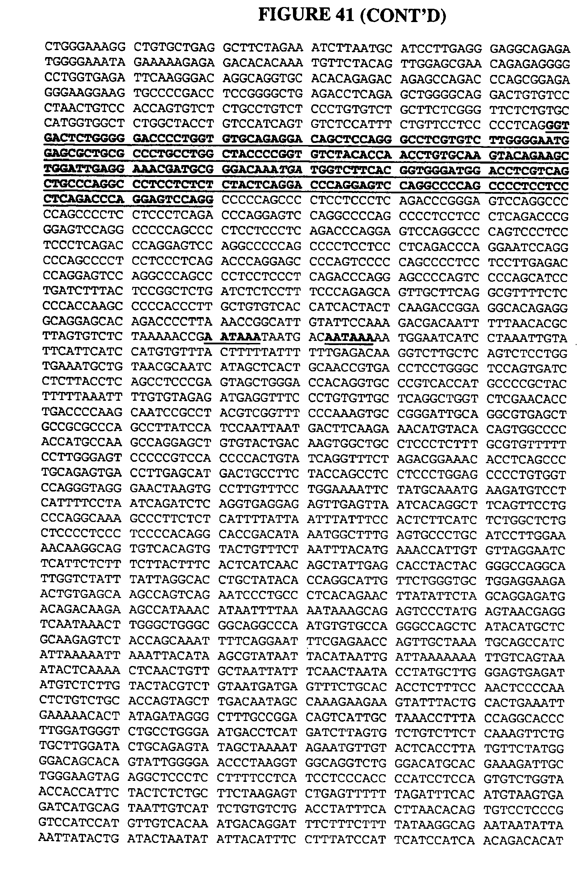

Figure 41 shows the full structure of a KLK-L6 nucleic acid molecule;

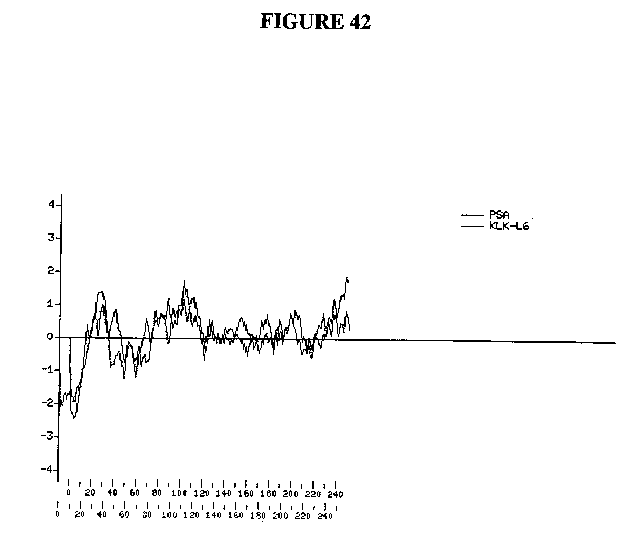

Figure 42 is a plot of hydrophobicity and hydrophilicity of KLK-L6 protein compared to prostate specific antigen (PSA).

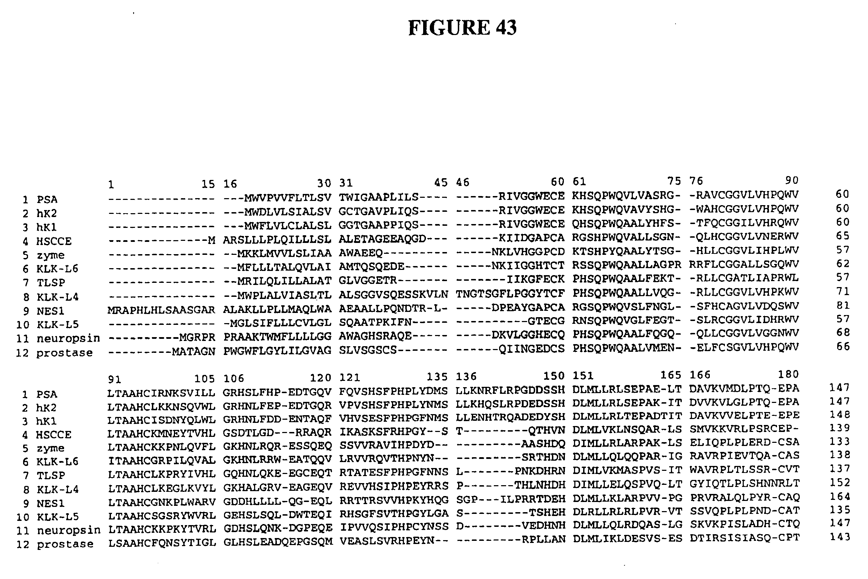

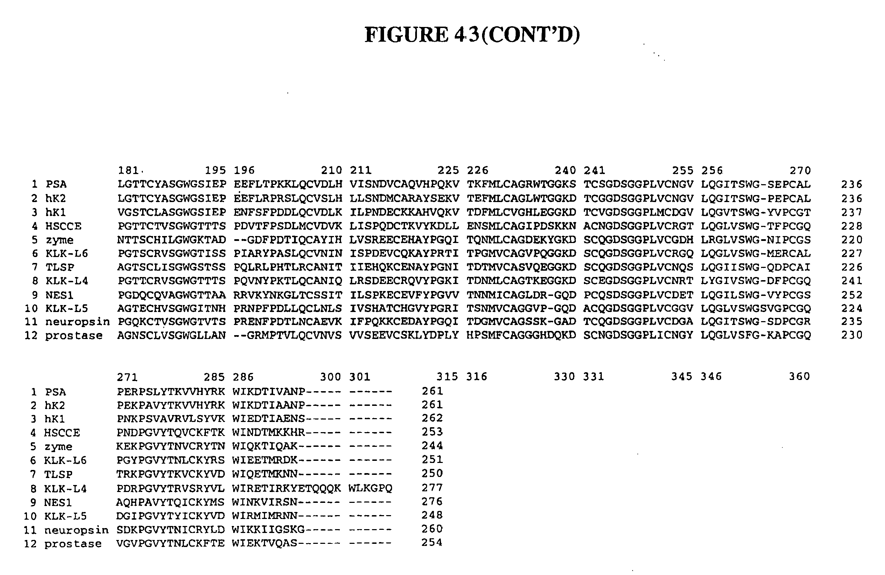

Figure 43 shows an alignment of the deduced amino acid sequence of KLK-L6 with members of the kallikrein multigene family. (See SEQ.ID. NOs. 78-81, 83, 84). Dashes represent gaps to bring the sequences to better alignment.

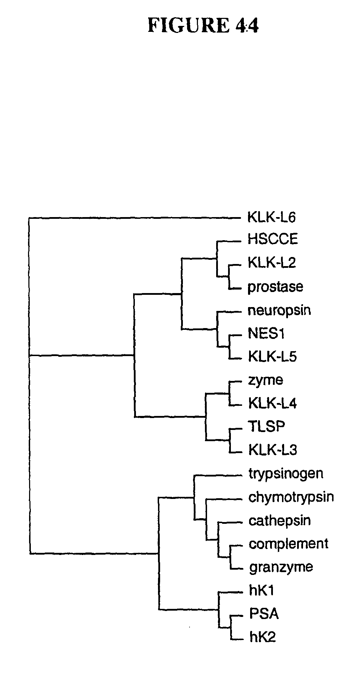

Figure 44 is a dendrogram of the predicted phylogenetic tree for some serine proteases and other kallikrein proteins. Neighbor-joining/UPGMA method was used to align KLK-L6 with other serine proteases and members of the kallikrein gene family.

### DETAILED DESCRIPTION OF THE INVENTION

In accordance with the present invention there may be employed conventional molecular biology, microbiology, and recombinant DNA techniques within the skill of the art. Such techniques are explained fully in the literature. See for example, Sambrook, Fritsch, & Maniatis, Molecular Cloning: A Laboratory Manual, Second Edition (1989) Cold Spring Harbor Laboratory Press, Cold Spring Harbor, N.Y); DNA Cloning: A Practical Approach, Volumes I and II (D.N. Glover ed. 1985); Oligonucleotide Synthesis (M..J. Gait ed. 1984); Nucleic Acid Hybridization B.D. Hames & S.J. Higgins eds. (1985); Transcription and Translation B.D. Hames & S.J. Higgins eds (1984); Animal Cell Culture R.I. Freshney, ed. (1986); Immobilized Cells and enzymes IRL Press, (1986); and B. Perbal, A Practical Guide to Molecular Cloning (1984).

### 1. Nucleic Acid Molecules of the Invention

As hereinbefore mentioned, the invention provides an isolated nucleic acid molecule having a sequence encoding a KLK-L Protein. The term "isolated" refers to a nucleic acid substantially free of cellular material or culture medium when produced by recombinant DNA techniques, or chemical reactants, or other chemicals when chemically synthesized. An "isolated" nucleic acid may also be free of sequences which naturally flank the nucleic acid (i.e., sequences located at the 5' and 3' ends of the nucleic acid molecule) from which the nucleic acid is derived. The term "nucleic acid" is intended to include DNA and RNA and can be either double stranded or single stranded. In an embodiment, a nucleic acid molecule encodes a KLK-L Protein comprising an amino acid sequence as shown in SEQ.ID.NO. 2, 3, 14, 22, 23, 44, 45, 57, 58, 59, 60, 66, or 67, preferably a nucleic acid molecule comprising a nucleic acid sequence as shown in SEQ.ID.NO. 1, 13, 21, 43, 56, or 65.

The invention includes nucleic acid sequences complementary to a nucleic acid encoding a KLK-L Protein comprising an amino acid sequence as shown in SEQ.ID.NO. 2, 3, 14, 22, 23, 44, 45, 57, 58, 59, 60, 66, or 67, preferably the nucleic acid sequences complementary to a full nucleic acid sequence shown in SEQ.ID.NO. 1, 13, 21, 43, 56, or 65.

The invention includes nucleic acid molecules having substantial sequence identity or homology to nucleic acid sequences of the invention or encoding proteins having substantial identity or similarity to the amino acid sequence shown in in SEQ.ID.NO. 2, 3, 14, 22, 23, 44, 45, 57, 58, 59, 60, 66, or 67. Preferably, the nucleic acids have substantial sequence identity for example at least 30%, 35%, 40%, 45%, 50%, 55%, 60%n, 65%, 70%, 75%, 80%, or 85% nucleic acid identity; more preferably 90% nucleic acid identity; and most preferably at least 95%, 96%, 97%, 98%, or 99% sequence identity. "Identity" as known in the art and used herein, is a relationship between two or more amino acid sequences or two or more nucleic acid sequences, as determined by comparing the sequences. It also refers to the degree of sequence relatedness between amino acid or nucleic acid sequences, as the case may be, as determined by the match between strings of such sequences. Identity and similarity are well known terms to skilled artisans and they can be calculated by conventional methods (for example see Computational Molecular Biology, Lesk, A.M. ed., Oxford University Press, New York, 1988; Biocomputing: Informatics and Genome Projects, Smith, D.W. ed., Academic Press, New York, 1993; Computer Analysis of Sequence Data, Part I, Griffin, A.M. and Griffin, H.G. eds., Humana Press, New Jersey, 1994; Sequence Analysis in Molecular Biology, von Heinje, G. Acadmeic Press, 1987; and Sequence Analysis Primer, Gribskov, M. and Devereux, J. eds. M. Stockton Press, New York, 1991, Carillo, H. and Lipman, D., SIAM J. Applied Math. 48:1073, 1988). Methods which are designed to give the largest match between the sequences are generally preferred. Methods to determine identity and similarity are codified in publicly available computer programs including the GCG program package (Devereux J. et al., Nucleic Acids Research 12(1): 387, 1984); BLASTP, BLASTN, and FASTA (Atschul, S.F. et al. J. Molec. Biol. 215: 403-410,1990). The BLAST X program is publicly available from NCBI and other sources (BLAST Manual, Altschul, S. et al. NCBI NLM NIH Bethesda, Md. 20894; Altschul, S. et al. J. Mol. Biol. 215: 403-410, 1990).

Isolated nucleic acid molecules encoding a KLK-L Protein, and having a sequence which differs from a nucleic acid sequence of the invention due to degeneracy in the genetic code are also within the scope of the invention. Such nucleic acids encode functionally equivalent proteins (e.g., a KLK-L Protein) but differ in sequence from the sequence of a KLK-L Protein due to degeneracy in the genetic code. As one example, DNA sequence polymorphisms within the nucleotide sequence of a KLK-L Protein may result in silent mutations which do not affect the amino acid sequence. Variations in one or more nucleotides may exist among individuals within a population due to natural allelic variation. Any and all such nucleic acid variations are within the scope of the invention. DNA sequence polymorphisms may also occur which lead to changes in the amino acid sequence of a KLK-L Protein. These amino acid polymorphisms are also within the scope of the present invention.

Another aspect of the invention provides a nucleic acid molecule which hybridizes under stringent conditions, preferably high stringency conditions to a nucleic acid molecule which comprises a sequence which encodes a KLK-L Protein having an amino acid sequence shown in SEQ.ID.NO. 2, 3, 14, 22, 23, 44, 45, 57, 58, 59, 60, 66, or 67. Appropriate stringency conditions which promote DNA hybridization are known to those skilled in the art, or can be found in Current Protocols in Molecular Biology, John Wiley & Sons, N.Y. (1989), 6.3.1-6.3.6. For example, 6.0 x sodium chloride/sodium citrate (SSC) at about 45°C, followed by a wash of 2.0 x SSC at 50°C may be employed. The stringency may be selected based on the conditions used in the wash step. By way of example, the salt concentration in the wash step can be selected from a high stringency of about 0.2 x SSC at 50°C. In addition, the temperature in the wash step can be at high stringency conditions, at about 65°C.

It will be appreciated that the invention includes nucleic acid molecules encoding a KLK-L Related Protein including truncations of a KLK-L Protein, and analogs of a KLK-L Protein as described herein. It will further be appreciated that variant forms of the nucleic acid molecules of the invention which arise by alternative splicing of an mRNA corresponding to a cDNA of the invention are encompassed by the invention. (See for example, splice variants of KLK-L5, SEQ.ID.NO. 58, 59, and 60.)

An isolated nucleic acid molecule of the invention which comprises DNA can be isolated by preparing a labelled nucleic acid probe based on all or part of a nucleic acid sequence of the invention. The labeled nucleic acid probe is used to screen an appropriate DNA library (e.g. a cDNA or genomic DNA library). For example, a cDNA library can be used to isolate a cDNA encoding a KLK-L Related Protein by screening the library with the labeled probe using standard techniques. Alternatively, a genomic DNA library can be similarly screened to isolate a genomic clone encompassing a gene encoding a KLK-L Related Protein. Nucleic acids isolated by screening of a cDNA or genomic DNA library can be sequenced by standard techniques.

An isolated nucleic acid molecule of the invention which is DNA can also be isolated by selectively amplifying a nucleic acid encoding a KLK-L Related Protein using the polymerase chain reaction (PCR) methods and cDNA or genomic DNA. It is possible to design synthetic oligonucleotide primers from the nucleotide sequence of the invention for use in PCR. A nucleic acid can be amplified from cDNA or genomic DNA using these oligonucleotide primers and standard PCR amplification techniques. The nucleic acid so amplified can be cloned into an appropriate vector and characterized by DNA sequence analysis, cDNA may be prepared from mRNA, by isolating total cellular mRNA by a variety of techniques, for example, by using the guanidinium-thiocyanate extraction procedure of Chirgwin et al., Biochemistry, 18, 5294-5299 (1979). cDNA is then synthesized from the mRNA using reverse transcriptase (for example, Moloney MLV reverse transcriptase available from Gibco/BRL, Bethesda, MD, or AMV reverse transcriptase available from Seikagaku America, Inc., St. Petersburg, FL).

An isolated nucleic acid molecule of the invention which is RNA can be isolated by cloning a cDNA encoding a KLK-L Related Protein into an appropriate vector which allows for transcription of the cDNA to produce an RNA molecule which encodes a KLK-L Related Protein. For example, a cDNA can be cloned downstream of a bacteriophage promoter, (e.g. a T7 promoter) in a vector, cDNA can be transcribed *in vitro* with T7 polymerase, and the resultant RNA can be isolated by conventional techniques.

Nucleic acid molecules of the invention may be chemically synthesized using standard techniques. Methods of chemically synthesizing polydeoxynucleotides are known, including but not limited to solid-phase synthesis which, like peptide synthesis, has been fully automated in commercially available DNA synthesizers (See e.g., Itakura et al. U.S. Patent No. 4,598,049; Caruthers et al. U.S. Patent No. 4,458,066; and Itakura U.S. Patent Nos. 4,401,796 and 4,373,071).

Determination of whether a particular nucleic acid molecule encodes a KLK-L Related Protein can be accomplished by expressing the cDNA in an appropriate host cell by standard techniques, and testing the expressed protein in the methods described herein. A cDNA encoding a KLK-L Related Protein can be sequenced by standard techniques, such as dideoxynucleotide chain termination or Maxam-Gilbert chemical sequencing, to determine the nucleic acid sequence and the predicted amino acid sequence of the encoded protein.

The initiation codon and untranslated sequences of a KLK-L Related Protein may be determined using computer software designed for the purpose, such as PC/Gene (IntelliGenetics Inc., Calif.). The intron-exon structure and the transcription regulatory sequences of a gene encoding a KLK-L Related Protein may be confirmed by using a nucleic acid molecule of the invention encoding a KLK-L Related Protein to probe a genomic DNA clone library. Regulatory elements can be identified using standard techniques. The function of the elements can be confirmed by using these elements to express a reporter gene such as the lacZ gene which is operatively linked to the elements. These constructs may be introduced into cultured cells using conventional procedures or into non-human transgenic animal models. In addition to identifying regulatory elements in DNA, such constructs may also be used to identify nuclear proteins interacting with the elements, using techniques known in the art.

In a particular embodiment of the invention, the nucleic acid molecules isolated using the methods described herein are mutant *klk-l* gene alleles. The mutant alleles may be isolated from individuals either known or proposed to have a genotype which contributes to the symptoms of for example, cancer (e.g.. breast, testicular, brain, colon, and prostate cancer). Mutant alleles and mutant allele products may be used in therapeutic and diagnostic methods described herein. For example, a cDNA of a mutant *klk-l* gene may be isolated using PCR as described herein, and the DNA sequence of the mutant allele may be compared to the normal allele to ascertain the mutation(s) responsible for the loss or alteration of function of the mutant gene product. A genomic library can also be constructed using DNA from an individual suspected of or known to carry a mutant allele, or a cDNA library can be constructed using RNA from tissue known, or suspected to express the mutant allele. A nucleic acid encoding a normal *klk-l* gene or any suitable fragment thereof, may then be labeled and used as a probe to identify the corresponding mutant allele in such libraries. Clones containing mutant sequences can be purified and subjected to sequence analysis. In addition, an expression library can be constructed using cDNA from RNA isolated from a tissue of an individual known or suspected to express a mutant *klk-l* allele. Gene products made by the putatively mutant tissue may be expressed and screened, for example using antibodies specific for a KLK-L Related Protein as described herein. Library clones identified using the antibodies can be purified and subjected to sequence analysis.

The sequence of a nucleic acid molecule of the invention, or a fragment of the molecule, may be inverted relative to its normal presentation for transcription to produce an antisense nucleic acid molecule. An antisense nucleic acid molecule may be constructed using chemical synthesis and enzymatic ligation reactions using procedures known in the art.

### 2. Proteins of the Invention

An amino acid sequence of a KLK-L Protein comprises a sequence as shown in Tables 1 to 5 or SEQ.ID.NO. 2, 3, 14, 22, 23, 44, 45, 57, 58, 59, 60, 66, or 67.

In addition to proteins comprising an amino acid sequence as shown in Tables 1 to 5 or SEQ.ID.NO. 2, 3, 14, 22, 23, 44, 45, 57, 58, 59, 60, 66, or 67, the proteins of the present invention include truncations of a KLK-L Protein, analogs of a KLK-L Protein, and proteins having sequence identity or similarity to a KLK-L Protein, and truncations thereof as described herein (i.e.included in KLK-L Related Proteins). Truncated proteins may comprise peptides of between 3 and 70 amino acid residues, ranging in size from a tripeptide to a 70 mer polypeptide.

The truncated proteins may have an amino group (-NH2), a hydrophobic group (for example, carbobenzoxyl, dansyl, or T-butyloxycarbonyl), an acetyl group, a 9-fluorenylmethoxy-carbonyl (PMOC) group, or a macromolecule including but not limited to lipid-fatty acid conjugates, polyethylene glycol, or carbohydrates at the amino terminal end. The truncated proteins may have a carboxyl group, an amido group, a T-butyloxycarbonyl group, or a macromolecule including but not limited to lipid-fatty acid conjugates, polyethylene glycol, or carbohydrates at the carboxy terminal end.

The proteins of the invention may also include analogs of a KLK-L Protein, and/or truncations thereof as described herein, which may include, but are not limited to a KLK-L Protein, containing one or more amino acid substitutions, insertions, and/or deletions. Amino acid substitutions may be of a conserved or non-conserved nature. Conserved amino acid substitutions involve replacing one or more amino acids of a KLK-L Protein amino acid sequence with amino acids of similar charge, size, and/or hydrophobicity characteristics. When only conserved substitutions are made the resulting analog is preferably functionally equivalent to a KLK-L Protein. Non-conserved substitutions involve replacing one or more amino acids of the KLK-L Protein amino acid sequence with one or more amino acids which possess dissimilar charge, size, and/or hydrophobicity characteristics.

One or more amino acid insertions may be introduced into a KLK-L Protein. Amino acid insertions may consist of single amino acid residues or sequential amino acids ranging from 2 to 15 amino acids in length.

Deletions may consist of the removal of one or more amino acids, or discrete portions from a KLK-L Protein sequence. The deleted amino acids may or may not be contiguous. The lower limit length of the resulting analog with a deletion mutation is about 10 amino acids, preferably 20 to 40 amino acids.

The proteins of the invention include proteins with sequence identity or similarity to a KLK-L Protein and/or truncations thereof as described herein. Such KLK-L Proteins include proteins whose amino acid sequences are comprised of the amino acid sequences of KLK-L Protein regions from other species that hybridize under selected hybridization conditions (see discussion of stringent hybridization conditions herein) with a probe used to obtain a KLK-L Protein. These proteins will generally have the same regions which are characteristic of a KLK-L Protein. Preferably a protein will have substantial sequence identity for example, about 30%, 35%, 40%, 45%, 50%, 60%, 65%, 70%, 75%, 80%, or 85% identity, preferably 90% identity, more preferably at least 95%, 96%, 97%, 98%, or 99% identity, and most preferably 98% identity with an amino acid sequence shown in Tables 1 to 5 or SEQ.ID.NO. 2, 3, 14, 22, 23, 44, 45, 57, 58, 59, 60, 66, or 67.

A percent amino acid sequence homology, similarity or identity is calculated as the percentage of aligned amino acids that match the reference sequence using known methods as described herein.

The invention also contemplates isoforms of the proteins of the invention. An isoform contains the same number and kinds of amino acids as a protein of the invention, but the isoform has a different molecular structure. Isoforms contemplated by the present invention preferably have the same properties as a protein of the invention as described herein.

The present invention also includes KLK-L Related Proteins conjugated with a selected protein, or a marker protein (see below) to produce fusion proteins. Additionally, immunogenic portions of a KLK-L Protein and a KLK-L Protein Related Protein are within the scope of the invention.

A KLK-L Related Protein of the invention may be prepared using recombinant DNA methods. Accordingly, the nucleic acid molecules of the present invention having a sequence which encodes a KLK-L Related Protein of the invention may be incorporated in a known manner into an appropriate expression vector which ensures good expression of the protein. Possible expression vectors include but are not limited to cosmids, plasmids, or modified viruses (e.g. replication defective retroviruses, adenoviruses and adeno-associated viruses), so long as the vector is compatible with the host cell used.

The invention therefore contemplates a recombinant expression vector of the invention containing a nucleic acid molecule of the invention, and the necessary regulatory sequences for the transcription and translation of the inserted protein-sequence. Suitable regulatory sequences may be derived from a variety of sources, including bacterial, fungal, viral, mammalian, or insect genes (For example, see the regulatory sequences described in Goeddel, Gene Expression Technology: Methods in Enzymology 185, Academic Press, San Diego, CA (1990). Selection of appropriate regulatory sequences is dependent on the host cell chosen as discussed below, and may be readily accomplished by one of ordinary skill in the art. The necessary regulatory sequences may be supplied by the native KLK-L Protein and/or its flanking regions.

The invention further provides a recombinant expression vector comprising a DNA nucleic acid molecule of the invention cloned into the expression vector in an antisense orientation. That is, the DNA molecule is linked to a regulatory sequence in a manner which allows for expression, by transcription of the DNA molecule, of an RNA molecule which is antisense to the nucleic acid sequence of a protein of the invention or a fragment thereof. Regulatory sequences linked to the antisense nucleic acid can be chosen which direct the continuous expression of the antisense RNA molecule in a variety of cell types, for instance a viral promoter and/or enhancer, or regulatory sequences can be chosen which direct tissue or cell type specific expression of antisense RNA.

The recombinant expression vectors of the invention may also contain a marker gene which facilitates the selection of host cells transformed or transfected with a recombinant molecule of the invention. Examples of marker genes are genes encoding a protein such as G418 and hygromycin which confer resistance to certain drugs, β-galactosidase, chloramphenicol acetyltransferase, firefly luciferase, or an immunoglobulin or portion thereof such as the Fc portion of an immunoglobulin preferably IgG. The markers can be introduced on a separate vector from the nucleic acid of interest.

The recombinant expression vectors may also contain genes which encode a fusion moiety which provides increased expression of the recombinant protein; increased solubility of the recombinant protein; and aid in the purification of the target recombinant protein by acting as a ligand in affinity purification. For example, a proteolytic cleavage site may be added to the target recombinant protein to allow separation of the recombinant protein from the fusion moiety subsequent to purification of the fusion protein. Typical fusion expression vectors include pGEX (Amrad Corp., Melbourne, Australia), pMAL (New England Biolabs, Beverly, MA) and pRIT5 (Pharmacia, Piscataway, NJ) which fuse glutathione S-transferase (GST), maltose E binding protein, or protein A, respectively, to the recombinant protein.

The recombinant expression vectors may be introduced into host cells to produce a transformant host cell. "Transformant host cells" include host cells which have been transformed or transfected with a recombinant expression vector of the invention. The terms "transformed with", "transfected with", "transformation" and "transfection" encompass the introduction of a nucleic acid (e.g. a vector) into a cell by one of many standard techniques. Prokaryotic cells can be transformed with a nucleic acid by, for example, electroporation or calcium-chloride mediated transformation. A nucleic acid can be introduced into mammalian cells via conventional techniques such as calcium phosphate or calcium chloride coprecipitation, DEAE-dextran-mediated transfection, lipofectin, electroporation or microinjection. Suitable methods for transforming and transfecting host cells can be found in Sambrook et al. (Molecular Cloning: A Laboratory Manual, 2nd Edition, Cold Spring Harbor Laboratory press (1989)), and other laboratory textbooks.

Suitable host cells include a wide variety of prokaryotic and eukaryotic host cells. For example, the proteins of the invention may be expressed in bacterial cells such as *E. coli,* insect cells (using baculovirus), yeast cells, or mammalian cells. Other suitable host cells can be found in Goeddel, Gene Expression Technology: Methods in Enzymology 185, Academic Press, San Diego, CA (1991).

A host cell may also be chosen which modulates the expression of an inserted nucleic acid sequence, or modifies (e.g. glycosylation or phosphorylation) and processes (e.g. cleaves) the protein in a desired fashion. Host systems or cell lines may be selected which have specific and characteristic mechanisms for post-translational processing and modification of proteins. For example, eukaryotic host cells including CHO, VERO, BHK, HeLA, COS, MDCK, 293, 3T3, and WI38 may be used. For long-term high-yield stable expression of the protein, cell lines and host systems which stably express the gene product may be engineered.

Host cells and in particular cell lines produced using the methods described herein may be particularly useful in screening and evaluating compounds that modulate the activity of a KLK-L Related Protein.

The proteins of the invention may also be expressed in non-human transgenic animals including but not limited to mice, rats, rabbits, guinea pigs, micro-pigs, goats, sheep, pigs, non-human primates (e.g. baboons, monkeys, and chimpanzees) [see Hammer et al. (Nature 315:680-683, 1985), Palmiter et al. (Science 222:809-814, 1983), Brinster et al. (Proc Natl. Acad. Sci USA 82:44384442, 1985), Palmiter and Brinster (Cell. 41:343-345, 1985) and U.S. Patent No. 4,736,866)]. Procedures known in the art may be used to introduce a nucleic acid molecule of the invention encoding a KLK-L Related Protein into animals to produce the founder lines of transgenic animals. Such procedures include pronuclear microinjection, retrovirus mediated gene transfer into germ lines, gene targeting in embryonic stem cells, electroporation of embryos, and sperm-mediated gene transfer.

The present invention contemplates a transgenic animal that carries the *KLK-L* gene in all their cells, and animals which carry the transgene in some but not all their cells. The transgene may be integrated as a single transgene or in concatamers. The transgene may be selectively introduced into and activated in specific cell types (See for example, Lasko et al, 1992 Proc. Natl. Acad. Sci. USA 89: 6236). The transgene may be integrated into the chromosomal site of the endogenous gene by gene targeting. The transgene may be selectively introduced into a particular cell type inactivating the endogenous gene in that cell type (See Gu et al Science 265: 103-106).

The expression of a recombinant KLK-L Related Protein in a transgenic animal may be assayed using standard techniques. Initial screening may be conducted by Southern Blot analysis, or PCR methods to analyze whether the transgene has been integrated. The level of mRNA expression in the tissues of transgenic animals may also be assessed using techniques including Northern blot analysis of tissue samples, *in situ* hybridization, and RT-PCR. Tissue may also be evaluated immunocytochemically using antibodies against KLK-L Protein.

Proteins of the invention may also be prepared by chemical synthesis using techniques well known in the chemistry of proteins such as solid phase synthesis (Merrifield, 1964, J. Am. Chem. Assoc. 85:2149-2154) or synthesis in homogenous solution (Houbenweyl, 1987, Methods of Organic Chemistry, ed. E. Wansch, Vol. 15 I and II, Thieme, Stuttgart).

N-terminal or C-terminal fusion proteins comprising a KLK-L Related Protein of the invention conjugated with other molecules, such as proteins, may be prepared by fusing, through recombinant techniques, the N-terminal or C-terminal of a KLK-L Related Protein, and the sequence of a selected protein or marker protein with a desired biological function. The resultant fusion proteins contain KLK-L Protein fused to the selected protein or marker protein as described herein. Examples of proteins which may be used to prepare fusion proteins include immunoglobulins, glutathione-S-transferase (GST), hemagglutinin (HA), and truncated myc.

### 3. Antibodies

KLK-L Related Proteins of the invention can be used to prepare antibodies specific for the proteins. Antibodies can be prepared which bind a distinct epitope in an unconserved region of the protein. An unconserved region of the protein is one that does not have substantial sequence homology to other proteins. A region from a conserved region such as a well-characterized domain can also be used to prepare an antibody to a conserved region of a KLK-L Related Protein. Antibodies having specificity for a KLK-L Related Protein may also be raised from fusion proteins created by expressing fusion proteins in bacteria as described herein.

The invention can employ intact monoclonal or polyclonal antibodies, and immunologically active fragments (e.g. a Fab, (Fab)₂ fragment, or Fab expression library fragments and epitope-binding fragments thereof), an antibody heavy chain, and antibody light chain, a genetically engineered single chain Fv molecule (Ladner et al, U.S. Pat. No. 4,946,778), or a chimeric antibody, for example, an antibody which contains the binding specificity of a murine antibody, but in which the remaining portions are of human origin. Antibodies including monoclonal and polyclonal antibodies, fragments and chimeras, may be prepared using methods known to those skilled in the art.

### 4. Applications of the Nucleic Acid Molecules, KLK-L Related Proteins, and Antibodies of the Invention

The nucleic acid molecules, KLK-L Related Proteins, and antibodies of the invention may be used in the prognostic and diagnostic evaluation of cancer (e.g. breast, testicular, and prostate cancer) or other conditions, and the identification of subjects with a predisposition to cancer (Section 4.1.1 and 4.1.2). Methods for detecting nucleic acid molecules and KLK-L Related Proteins of the invention, can be used to monitor conditions including cancer, by detecting KLK-L Related Proteins and nucleic acid molecules encoding KLK-L Related Proteins. It would also be apparent to one skilled in the art that the methods described herein may be used to study the developmental expression of KLK-L Related Proteins and, accordingly, will provide further insight into the role of KLK-L Related Proteins. The applications of the present invention also include methods for the identification of compounds that modulate the biological activity of *KLK-L* or KLK-L Related Proteins (Section 4.2). The compounds, antibodies etc. may be used for the treatment of cancer (Section 4.3).

### 4.1 Diagnostic Methods

A variety of methods can be employed for the diagnostic and prognostic evaluation of conditions including cancer (e.g. breast, testicular, and prostate cancer), and the identification of subjects with a predisposition to such conditions. Such methods may, for example, utilize nucleic acid molecules of the invention, and fragments thereof, and antibodies directed against KLK-L Related Proteins, including peptide fragments. In particular, the nucleic acids and antibodies may be used, for example, for: (1) the detection of the presence of *KLK-L* mutations, or the detection of either over- or under-expression of *KLK-L* mRNA relative to a non-disorder state or the qualitative or quantitative detection of alternatively spliced forms of *KLK-L* transcripts which may correlate with certain conditions or susceptibility toward such conditions; and (2) the detection of either an over- or an under-abundance of KLK-L Related Proteins relative to a non- disorder state or the presence of a modified (e.g., less than full length) KLK-L Protein which correlates with a disorder state, or a progression toward a disorder state.

The methods described herein may be performed by utilizing pre-packaged diagnostic kits comprising at least one specific *KLK-L* nucleic acid or antibody described herein, which may be conveniently used, e.g., in clinical settings, to screen and diagnose patients and to screen and identify those individuals exhibiting a predisposition to developing a disorder.

Nucleic acid-based detection techniques are described, below, in Section 4.1.1. Peptide detection techniques are described, below, in Section 4.1.2. The samples that may be analyzed using the methods of the invention include those which are known or suspected to express *KLK-L* or contain KLK-L Related Proteins. The samples may be derived from a patient or a cell culture, and include but are not limited to biological fluids, tissue extracts, freshly harvested cells, and lysates of cells which have been incubated in cell cultures.

Oligonucleotides or longer fragments derived from any of the nucleic acid molecules of the invention may be used as targets in a microarray. The microarray can be used to simultaneously monitor the expression levels of large numbers of genes and to identify genetic variants, mutations, and polymorphisms. The information from the microarray may be used to determine gene function, to understand the genetic basis of a disorder, to diagnose a disorder, and to develop and monitor the activities of therapeutic agents.

The preparation, use, and analysis of microarrays are well known to a person skilled in the art. (See, for example, Brennan, T. M. et al. (1995) U.S. Pat. No. 5,474,796; Schena, et al. (1996) Proc. Natl. Acad. Sci. 93:10614-10619; Baldeschweiler et al. (1995), PCT Application WO95/251116; Shalon, D. et al. (1995) PCT application WO95/35505; Heller, R. A. et al. (1997) Proc. Natl. Acad. Sci. 94:2150-2155; and Heller, M. J. et al. (1997) U.S. Pat. No. 5,605,662.)

### 4.1.1 Methods for Detectine Nucleic Acid Molecules of the Invention

The nucleic acid molecules of the invention allow those skilled in the art to construct nucleotide probes for use in the detection of nucleic acid sequences of the invention in samples. Suitable probes include nucleic acid molecules based on nucleic acid sequences encoding at least 5 sequential amino acids from regions of a KLK-L Protein, preferably they comprise 15 to 30 nucleotides. A nucleotide probe may be labeled with a detectable substance such as a radioactive label which provides for an adequate signal and has sufficient half-life such as ³²P, ³H, ¹⁴C or the like. Other detectable substances which may be used include antigens that are recognized by a specific labeled antibody, fluorescent compounds, enzymes, antibodies specific for a labeled antigen, and luminescent compounds. An appropriate label may be selected having regard to the rate of hybridization and binding of the probe to the nucleotide to be detected and the amount of nucleotide available for hybridization. Labeled probes may be hybridized to nucleic acids on solid supports such as nitrocellulose filters or nylon membranes as generally described in Sambrook et al, 1989, Molecular Cloning, A Laboratory Manual (2nd ed.). The nucleic acid probes may be used to detect genes, preferably in human cells, that encode KLK-L Related Proteins. The nucleotide probes may also be useful in the diagnosis of cancer; in monitoring the progression of cancer; or monitoring a therapeutic treatment.

The probe may be used in hybridization techniques to detect genes that encode KLK-L Related Proteins. The technique generally involves contacting and incubating nucleic acids (e.g. recombinant DNA molecules, cloned genes) obtained from a sample from a patient or other cellular source with a probe of the present invention under conditions favorable for the specific annealing of the probes to complementary sequences in the nucleic acids. After incubation, the non-annealed nucleic acids are removed, and the presence of nucleic acids that have hybridized to the probe if any are detected.

The detection of nucleic acid molecules of the invention may involve the amplification of specific gene sequences using an amplification method such as PCR, followed by the analysis of the amplified molecules using techniques known to those skilled in the art. Suitable primers can be routinely designed by one of skill in the art.

Genomic DNA may be used in hybridization or amplification assays of biological samples to detect abnormalities involving *klk- l* structure, including point mutations, insertions, deletions, and chromosomal rearrangements. For example, direct sequencing, single stranded conformational polymorphism analyses, heteroduplex analysis, denaturing gradient gel electrophoresis, chemical mismatch cleavage, and oligonucleotide hybridization may be utilized.

Genotyping techniques known to one skilled in the art can be used to type polymorphisms that are in close proximity to the mutations in a *klk-l* gene. The polymorphisms may be used to identify individuals in families that are likely to carry mutations. If a polymorphism exhibits linkage disequalibrium with mutations in a *klk-l* gene, it can also be used to screen for individuals in the general population likely to carry mutations. Polymorphisms which may be used include restriction fragment length polymorphisms (RFLPs), single-base polymorphisms, and simple sequence repeat polymorphisms (SSLPs).

A probe of the invention may be used to directly identify RFLPs. A probe or primer of the invention can additionally be used to isolate genomic clones such as YACs, BACs, PACs, cosmids, phage or plasmids. The DNA in the clones can be screened for SSLPs using hybridization or sequencing procedures.

Hybridization and amplification techniques described herein may be used to assay qualitative and quantitative aspects of *klk-l* expression. For example, RNA may be isolated from a cell type or tissue known to express *klk-l* and tested utilizing the hybridization (e.g. standard Northern analyses) or PCR techniques referred to herein. The techniques may be used to detect differences in transcript size which may be due to normal or abnormal alternative splicing. The techniques may be used to detect quantitative differences between levels of full length and/or alternatively splice transcripts detected in normal individuals relative to those individuals exhibiting cancer symptoms or other disease conditions.

The primers and probes may be used in the above described methods *in situ* i.e directly on tissue sections (fixed and/or frozen) of patient tissue obtained from biopsies or resections.

### 4.1.2 Methods for Detecting KLK-L Related Proteins

Antibodies specifically reactive with a KLK-L Related Protein, or derivatives, such as enzyme conjugates or labeled derivatives, may be used to detect KLK-L Related Proteins in various samples (e.g. biological materials). They may be used as diagnostic or prognostic reagents and they may be used to detect abnormalities in the level of KLK-L Related Proteins expression, or abnormalities in the structure, and/or temporal, tissue, cellular, or subcellular location of a KLK-L Related Protein. Antibodies may also be used to screen potentially therapeutic compounds *in vitro* to determine their effects on cancer, and other conditions. *In vitro* immunoassays may also be used to assess or monitor the efficacy of particular therapies. The antibodies of the invention may also be used *in vitro* to determine the level of *KLK-L* expression in cells genetically engineered to produce a KLK-L Related Protein.

The antibodies may be used in any known immunoassays which rely on the binding interaction between an antigenic determinant of a KLK-L Related Protein and the antibodies. Examples of such assays are radioimmunoassays, enzyme immunoassays (e.g. ELISA), immunofluorescence, immunoprecipitation, latex agglutination, hemagglutination, and histochemical tests. The antibodies may be used to detect and quantify KLK-L Related Proteins in a sample in order to determine its role in particular cellular events or pathological states, and to diagnose and treat such pathological states.

In particular, the antibodies of the invention may be used in immuno-histochemical analyses, for example, at the cellular and sub-subcellular level, to detect a KLK-L Related Protein, to localize it to particular cells and tissues, and to specific subcellular locations, and to quantitate the level of expression.

Cytochemical techniques known in the art for localizing antigens using light and electron microscopy may be used to detect a KLK-L Related Protein. Generally, an antibody of the invention may be labeled with a detectable substance and a KLK-L Related Protein may be localised in tissues and cells based upon the presence of the detectable substance. Examples of detectable substances include, but are not limited to, the following: radioisotopes (e.g., ³H, ¹⁴C, ³⁵S, ¹²⁵I, ¹³¹I), fluorescent labels (e.g., FITC, rhodamine, lanthanide phosphors), luminescent labels such as luminol; enzymatic labels (e.g., horseradish peroxidase, beta-galactosidase, luciferase, alkaline phosphatase, acetylcholinesterase), biotinyl groups (which can be detected by marked avidin e.g., streptavidin containing a fluorescent marker or enzymatic activity that can be detected by optical or calorimetric methods), predetermined polypeptide epitopes recognized by a secondary reporter (e.g., leucine zipper pair sequences, binding sites for secondary antibodies, metal binding domains, epitope tags). In some embodiments, labels are attached via spacer arms of various lengths to reduce potential steric hindrance. Antibodies may also be coupled to electron dense substances, such as ferritin or colloidal gold, which are readily visualised by electron microscopy.

The antibody or sample may be immobilized on a carrier or solid support which is capable of immobilizing cells, antibodies etc. For example, the carrier or support may be nitrocellulose, or glass, polyacrylamides, gabbros, and magnetite. The support material may have any possible configuration including spherical (e.g. bead), cylindrical (e.g. inside surface of a test tube or well, or the external surface of a rod), or flat (e.g. sheet, test strip). Indirect methods may also be employed in which the primary antigen-antibody reaction is amplified by the introduction of a second antibody, having specificity for the antibody reactive against KLK-L Related Protein. By way of example, if the antibody having specificity against a KLK-L Related Protein is a rabbit IgG antibody, the second antibody may be goat anti-rabbit gamma-globulin labeled with a detectable substance as described herein.

Where a radioactive label is used as a detectable substance, a KLK-L Related Protein may be localized by radioautography. The results of radioautography may be quantitated by determining the density of particles in the radioautographs by various optical methods, or by counting the grains.

### 4.2 Methods for Identifying or Evaluating Substances/Compounds

The methods described herein are designed to identify substances that modulate the biological activity of a KLK-L Related Protein including substances that bind to KLK-L Related Proteins, or bind to other proteins that interact with a KLK-L Related Protein, to compounds that interfere with, or enhance the interaction of a KLK-L Related Protein and substances that bind to the KLK-L Related Protein or other proteins that interact with a KLK-L Related Protein. Methods are also utilized that identify compounds that bind to *KLK-L* regulatory sequences.

The substances and compounds identified using the methods of the invention include but are not limited to peptides such as soluble peptides including Ig-tailed fusion peptides, members of random peptide libraries and combinatorial chemistry-derived molecular libraries made of D- and/or L-configuration amino acids, phosphopeptides (including members of random or partially degenerate, directed phosphopeptide libraries), antibodies [e.g. polyclonal, monoclonal, humanized, anti-idiotypic, chimeric, single chain antibodies, fragments, (e.g. Fab, F(ab)₂, and Fab expression library fragments, and epitope-binding fragments thereof)], and small organic or inorganic molecules. The substance or compound may be an endogenous physiological compound or it may be a natural or synthetic compound.

Substances which modulate a KLK-L Related Protein can be identified based on their ability to bind to a KLK-L Related Protein. Therefore, the invention also provides methods for identifying substances which bind to a KLK-L Related Protein. Substances identified using the methods of the invention may be isolated, cloned and sequenced using conventional techniques. A substance that associates with a polypeptide of the invention may be an agonist or antagonist of the biological or immunological activity of a polypeptide of the invention.

The term "agonist", refers to a molecule that increases the amount of, or prolongs the duration of, the activity of the polypeptide. The term "antagonist" refers to a molecule which decreases the biological or immunological activity of the polypeptide. Agonists and antagonists may include proteins, nucleic acids, carbohydrates, or any other molecules that associate with a polypeptide of the invention.

Substances which can bind with a KLK-L Related Protein may be identified by reacting a KLK-L Related Protein with a test substance which potentially binds to a KLK-L Related Protein, under conditions which permit the formation of substance-KLK-L Related Protein complexes and removing and/or detecting the complexes. The complexes can be detected by assaying for substance-KLK-L Related Protein complexes, for free substance, or for non-complexed KLK-L Related Protein. Conditions which permit the formation of substance-KLK-L Related Protein complexes may be selected having regard to factors such as the nature and amounts of the substance and the protein.

The substance-protein complex, free substance or non-complexed proteins may be isolated by conventional isolation techniques, for example, salting out, chromatography, electrophoresis, gel filtration, fractionation, absorption, polyacrylamide gel electrophoresis, agglutination, or combinations thereof. To facilitate the assay of the components, antibody against KLK-L Related Protein or the substance, or labeled KLK-L Related Protein, or a labeled substance may be utilized. The antibodies, proteins, or substances may be labeled with a detectable substance as described above.

A KLK-L Related Protein, or the substance used in the method of the invention may be insolubilized. For example, a KLK-L Related Protein, or substance may be bound to a suitable carrier such as agarose, cellulose, dextran, Sephadex, Sepharose, carboxymethyl cellulose polystyrene, filter paper, ionexchange resin, plastic film, plastic tube, glass beads, polyamine-methyl vinyl-ether-maleic acid copolymer, amino acid copolymer, ethylene-maleic acid copolymer, nylon, silk, etc. The carrier may be in the shape of, for example, a tube, test plate, beads, disc, sphere etc. The insolubilized protein or substance may be prepared by reacting the material with a suitable insoluble carrier using known chemical or physical methods, for example, cyanogen bromide coupling.

The invention also contemplates a method for evaluating a compound for its ability to modulate the biological activity of a KLK-L Related Protein of the invention, by assaying for an agonist or antagonist (i.e. enhancer or inhibitor) of the binding of a KLK-L Related Protein with a substance which binds with a KLK-L Related Protein. The basic method for evaluating if a compound is an agonist or antagonist of the binding of a KLK-L Related Protein and a substance that binds to the protein, is to prepare a reaction mixture containing the KLK-L Related Protein and the substance under conditions which permit the formation of substance-KLK-L Related Protein complexes, in the presence of a test compound. The test compound may be initially added to the mixture, or may be added subsequent to the addition of the KLK-L Related Protein and substance. Control reaction mixtures without the test compound or with a placebo are also prepared. The formation of complexes is detected and the formation of complexes in the control reaction but not in the reaction mixture indicates that the test compound interferes with the interaction of the KLK-L Related Protein and substance. The reactions may be carried out in the liquid phase or the KLK-L Related Protein, substance, or test compound may be immobilized as described herein. The ability of a compound to modulate the biological activity of a KLK-L Related Protein of the invention may be tested by determining the biological effects on cells.

It will be understood that the agonists and antagonists i.e. inhibitors and enhancers that can be assayed using the methods of the invention may act on one or more of the binding sites on the protein or substance including agonist binding sites, competitive antagonist binding sites, non-competitive antagonist binding sites or allosteric sites.

The invention also makes it possible to screen for antagonists that inhibit the effects of an agonist of the interaction of KLK-L Related Protein with a substance which is capable of binding to the KLK-L Related Protein. Thus, the invention may be used to assay for a compound that competes for the same binding site of a KLK-L Related Protein.

The invention also contemplates methods for identifying compounds that bind to proteins that interact with a KLK-L Related Protein. Protein-protein interactions may be identified using conventional methods such as co-immunoprecipitation, crosslinking and co-purification through gradients or chromatographic columns. Methods may also be employed that result in the simultaneous identification of genes which encode proteins interacting with a KLK-L Related Protein. These methods include probing expression libraries with labeled KLK-L Related Protein.

Two-hybrid systems may also be used to detect protein interactions *in vivo.* Generally, plasmids are constructed that encode two hybrid proteins. A first hybrid protein consists of the DNA-binding domain of a transcription activator protein fused to a KLK-L Related Protein, and the second hybrid protein consists of the transcription activator protein's activator domain fused to an unknown protein encoded by a cDNA which has been recombined into the plasmid as part of a cDNA library. The plasmids are transformed into a strain of yeast (e.g. *S. cerevisiae*) that contains a reporter gene (e.g. lacZ, luciferase, alkaline phosphatase, horseradish peroxidase) whose regulatory region contains the transcription activator's binding site. The hybrid proteins alone cannot activate the transcription of the reporter gene, However. interaction of the two hybrid proteins reconstitutes the functional activator protein and results in expression of the reporter gene, which is detected by an assay for the reporter gene product.

It will be appreciated that fusion proteins may be used in the above-described methods. In particular, KLK-L Related Proteins fused to a glutathione-S-transferase may be used in the methods.

The reagents suitable for applying the methods of the invention to evaluate compounds that modulate a KLK-L Related Protein may be packaged into convenient kits providing the necessary materials packaged into suitable containers. The kits may also include suitable supports useful in performing the methods of the invention.

### 4.3 Comnositions and Treatments

The proteins of the invention, substances or compounds identified by the methods described herein, antibodies, and antisense nucleic acid molecules of the invention may be used for modulating the biological activity of a KLK-L Related Protein, and they may be used in the treatment of conditions such as cancer (e.g. prostate, testicular, brain, uterine, thymus, ovarian, colon, ovarian, or breast cancer). Accordingly, the substances, antibodies, peptides, and compounds may be formulated into pharmaceutical compositions for administration to subjects in a biologically compatible form suitable for administration *in vivo.* By "biologically compatible form suitable for administration *in vivo*" is meant a form of the active substance to be administered in which any toxic effects are outweighed by the therapeutic effects. The active substances may be administered to living organisms including humans, and animals. Administration of a therapeutically active amount of a pharmaceutical composition of the present invention is defined as an amount effective, at dosages and for periods of time necessary to achieve the desired result. For example, a therapeutically active amount of a substance may vary according to factors such as the disease state, age, sex, and weight of the individual, and the ability of antibody to elicit a desired response in the individual. Dosage regima may be adjusted to provide the optimum therapeutic response. For example, several divided doses may be administered daily or the dose may be proportionally reduced as indicated by the exigencies of the therapeutic situation.

The active substance may be administered in a convenient manner such as by injection (subcutaneous, intravenous, etc.), oral administration, inhalation, transdermal application, or rectal administration. Depending on the route of administration, the active substance may be coated in a material to protect the substance from the action of enzymes, acids and other natural conditions that may inactivate the substance.

The compositions described herein can be prepared by per se known methods for the preparation of pharmaceutically acceptable compositions which can be administered to subjects, such that an effective quantity of the active substance is combined in a mixture with a pharmaceutically acceptable vehicle. Suitable vehicles are described, for example, in Remington's Pharmaceutical Sciences (Remington's Pharmaceutical Sciences, Mack Publishing Company, Easton, Pa., USA 1985). On this basis, the compositions include, albeit not exclusively, solutions of the active substances in association with one or more pharmaceutically acceptable vehicles or diluents, and contained in buffered solutions with a suitable pH and iso-osmotic with the physiological fluids.

Based upon their homology to genes encoding kallikrein, nucleic acid molecules of the invention may be also useful in the treatment of conditions such as hypertension, cardiac hypertrophy, arthritis, inflammatory disorders, neurological disorders, and blood clotting disorders.

Vectors derived from retroviruses, adenovirus, herpes or vaccinia viruses, or from various bacterial plasmids, may be used to deliver nucleic acid molecules to a targeted organ, tissue, or cell population. Methods well known to those skilled in the art may be used to construct recombinant vectors which will express antisense nucleic acid molecules of the invention. (See, for example, the techniques described in Sambrook et al (supra) and Ausubel et al (supra)).

The nucleic acid molecules comprising full length cDNA sequences and/or their regulatory elements enable a skilled artisan to use sequences encoding a protein of the invention as an investigative tool in sense (Youssoufian H and H F Lodish 1993 Mol Cell Biol 13:98-104) or antisense (Eguchi et al (1991) Annu Rev Biochem 60:631-652) regulation of gene function. Such technology is well known in the art, and sense or antisense oligomers, or larger fragments, can be designed from various locations along the coding or control regions.

Genes encoding a protein of the invention can be turned off by transfecting a cell or tissue with vectors which express high levels of a desired KLK-L-encoding fragment. Such constructs can inundate cells with untranslatable sense or antisense sequences. Even in the absence of integration into the DNA, such vectors may continue to transcribe RNA molecules until all copies are disabled by endogenous nucleases.

Modifications of gene expression can be obtained by designing antisense molecules, DNA, RNA or PNA, to the regulatory regions of a gene encoding a protein of the invention, ie, the promoters, enhancers, and introns. Preferably, oligonucleotides are derived from the transcription initiation site, eg, between -10 and +10 regions of the leader sequence. The antisense molecules may also be designed so that they block translation of mRNA by preventing the transcript from binding to ribosomes. Inhibition may also be achieved using "triple helix" base-pairing methodology. Triple helix pairing compromises the ability of the double helix to open sufficiently for the binding of polymerases, transcription factors, or regulatory molecules. Therapeutic advances using triplex DNA were reviewed by Gee J E et al (In: Huber B E and B I Carr (1994) Molecular and Immunologic Approaches, Futura Publishing Co, Mt Kisco N.Y.).

Ribozymes are enzymatic RNA molecules that catalyze the specific cleavage of RNA. Ribozymes act by sequence-specific hybridization of the ribozyme molecule to complementary target RNA, followed by endonucleolytic cleavage. The invention therefore contemplates engineered hammerhead motif ribozyme molecules that can specifically and efficiently catalyze endonucleolytic cleavage of sequences encoding a protein of the invention.

Specific ribozyme cleavage sites within any potential RNA target may initially be identified by scanning the target molecule for ribozyme cleavage sites which include the following sequences, GUA, GUU and GUC. Once the sites are identified, short RNA sequences of between 15 and 20 ribonucleotides corresponding to the region of the target gene containing the cleavage site may be evaluated for secondary structural features which may render the oligonucleotide inoperable. The suitability of candidate targets may also be determined by testing accessibility to hybridization with complementary oligonucleotides using ribonuclease protection assays.

Methods for introducing vectors into cells or tissues include those methods discussed herein and which are suitable for *in vivo, in vitro* and *ex vivo* therapy. For *ex vivo* therapy, vectors may be introduced into stem cells obtained from a patient and clonally propagated for autologous transplant into the same patient (See U.S. Pat. Nos. 5,399,493 and 5,437,994). Delivery by transfection and by liposome are well known in the art.

The nucleic acid molecules disclosed herein may also be used in molecular biology techniques that have not yet been developed, provided the new techniques rely on properties of nucleotide sequences that are currently known, including but not limited to such properties as the triplet genetic code and specific base pair interactions.

The invention also provides methods for studying the function of a polypeptide of the invention. Cells, tissues, and non-human animals lacking in expression or partially lacking in expression of a nucleic acid molecule or gene of the invention may be developed using recombinant expression vectors of the invention having specific deletion or insertion mutations in the gene. A recombinant expression vector may be used to inactivate or alter the endogenous gene by homologous recombination, and thereby create a deficient cell, tissue, or animal.

Null alleles may be generated in cells, such as embryonic stem cells by deletion mutation. A recombinant gene may also be engineered to contain an insertion mutation that inactivates the gene. Such a construct may then be introduced into a cell, such as an embryonic stem cell, by a technique such as transfection, electroporation, injection etc. Cells lacking an intact gene may then be identified, for example by Southern blotting, Northern Blotting, or by assaying for expression of the encoded polypeptide using the methods described herein. Such cells may then be fused to embryonic stem cells to generate transgenic non-human animals deficient in a polypeptide of the invention. Germline transmission of the mutation may be achieved, for example, by aggregating the embryonic stem cells with early stage embryos, such as 8 cell embryos, *in vitro;* transferring the resulting blastocysts into recipient females and; generating germline transmission of the resulting aggregation chimeras. Such a mutant animal may be used to define specific cell populations, developmental patterns and *in vivo* processes, normally dependent on gene expression.

The invention thus provides a transgenic non-human mammal all of whose germ cells and somatic cells contain a recombinant expression vector that inactivates or alters a gene encoding a KLK-L Related Protein. In an embodiment the invention provides a transgenic non-human mammal all of whose germ cells and somatic cells contain a recombinant expression vector that inactivates or alters a gene encoding a KLK-L Related Protein resulting in a KLK-L Related Protein associated pathology. Further the invention provides a transgenic non-human mammal which doe not express a KLK-L Related Protein of the invention. In an embodiment, the invention provides a transgenic non-human mammal which doe not express a KLK-L Related Protein of the invention resulting in a KLK-L Related Protein associated pathology. A KLK-L Related Protein pathology refers to a phenotype observed for a KLK-L Related Protein homozygous mutant.

A transgenic non-human animal includes but is not limited to mouse, rat, rabbit, sheep, hamster, dog, guinea pig, micro-pig, pig, cat, goat, and non-human primates, preferably mouse.

The invention also provides a transgenic non-human animal assay system which provides a model system for testing for an agent that reduces or inhibits a pathology associated with an KLK-L Related Protein, preferably a KLK-L Related Protein associated pathology, comprising:
(a) administering the agent to a transgenic non-human animal of the invention; and
(b) determining whether said agent reduces or inhibits the pathology (e.g. KLK-L Related Protein associated pathology) in the transgenic non-human animal relative to a transgenic non-human animal of step (a) which has not been administered the agent.

The agent may be useful in the treatment and prophylaxis of conditions such as cancer as discussed herein. The agents may also be incorporated in a pharmaceutical composition as described herein.

The activity of the proteins, substances, compounds, antibodies, nucleic acid molecules, agents, and compositions of the invention may be confirmed in animal experimental model systems. Therapeutic efficacy and toxicity may be determined by standard pharmaceutical procedures in cell cultures or with experimental animals, such as by calculating the ED₅₀ (the dose therapeutically effective in 50% of the population) or LD₅₀ (the dose lethal to 50% of the population) statistics. The therapeutic index is the dose ratio of therapeutic to toxic effects and it can be expressed as the ED₅₀/LD₅₀ ratio. Pharmaceutical compositions which exhibit large therapeutic indices are preferred.

The following non-limiting examples are illustrative of the present invention:

### Examples

### Example 1

### MATERIALS AND METHODS

### Identification of positive PAC and BAC genomic clones from a human genomic DNA library

The sequence of PSA, KLK1, KLK2, NES1 and Zyme genes is already known. Polymerase chain reaction (PCR)-based amplification protocols have been developed which allowed generation of PCR products specific for each one of these genes. Using these PCR products as probes, labeled with ³²P, a human genomic DNA PAC library and a human genomic DNA BAC library was screened for the purpose of identifying positive clones of approximately 100-150 Kb long. The general strategies for these experiments have been published elsewhere (14). The genomic libraries were spotted in duplicate on nylon membranes and positive clones were further confirmed by Southern blot analysis as described (14).

### DNA sequences on chromosome 19

The Lawrence Livermore National Laboratory participates in the sequencing of the human genome project and focuses on sequencing chromosome 19. Large sequencing information on this chromosome is available at the website of the Lawrence Livermore National Laboratory (http://www-bio.llnl.gov/genome/gemnome.html).

Approximately 300 Kb of genomic sequences were obtained from that website, encompassing a region on chromosome 19q13.3 - 13.4, where the known kallikrein genes are localized. This 300 Kb of sequence is represented by 8 contigs of variable lengths. By using a number of different computer programs, an almost contiguous sequence of the region was established as shown diagramatically in Figure 1 and Figure 28. Some of the contigs were reversed as shown in Figure I in order to reconstruct the area on both strands of DNA.

By using the published sequences of PSA. KLK2, NES 1 and Zyme and the computer software BLAST 2, using alignment strategies, the relative positions of these genes on the contiguous map were identified (Figure 28). These known genes served as hallmarks for further studies. An EcoR1 restriction map of the area is also available at the website of the Lawrence Livermore National Laboratory. Using this restriction map and the computer program WebCutter (http://www.firstmarket.com/cutter/cut2.html), a restriction study analysis of the available sequence was performed to further confirm the assignment and relative positions of these contigs along chromosome 19. The obtained configuration and the relative location of the known genes are presented in Figure 1.

### Gene prediction analysis

For exon prediction analysis of the whole genomic area, a number of different computer programs were used. All the programs were initially tested using known genomic sequences of the PSA, Zyme, and NES1 genes. The more reliable computer programs, GeneBuilder (gene prediction), GeneBuilder (exon prediction), Grail 2 and GENEID-3 were selected for further use.

### Protein homology searching

Putative exons of the new genes were first translated to the corresponding amino acid sequences. BLAST homology searching for the proteins encoded by the exons of the putative new genes were performed using the BLASTP program and the Genbank databases.

### RESULTS

### Relative position of PSA, KLK2, Zyme and NES1 on Chromosome 19

Screening of the human BAC library identified two clones which were positive for the Zyme gene (clones BAC 288H1 and BAC 76F7). These BACs were further analyzed by PCR and primers specific for PSA, NES1, KLK1 and KLK2. These analyses indicated that both BACs were positive for Zyme, PSA and KLK2 and negative for KLK1 and NES1 genes.

Screening of the human PAC genomic library identified a PAC clone which was positive for NES1 (clone PAC 34B1). Further PCR analysis indicated that this PAC clone was positive for NES1 and KLK1 genes and negative for PSA, KLK2 and Zyme. Combination of this information with the EcoR1 restriction map of the region allowed establishment of the relative positions of these four genes. PSA is the most centromeric, followed by KLK2, Zyme and NES 1. Further alignment of the known sequences of these genes with the 300 Kb contig enabled precise localization of all four genes and determination of the direction of transcription, as shown by the arrows in Figure 1. The KLK1 gene sequence was not identified on any of these contigs and appears to be further telomeric to NES1 (since it is co-localized on the same PAC as NES1).

### Identification of new genes

A set of rules was used to consider the presence of a new gene in the genomic area of interest as follows:
1. Clusters of at least 3 exons should be found.
2. Only exons with high prediction score ("good" or "excellent" quality, as indicated by the searching programs) were considered for the construction of the putative new genes.
3. Exons predicted were reliable only if they were identified by at least two different exon prediction programs.

By using this strategy, eleven putative new genes were identified of which three were found on subsequent homology analysis to be known genes not previously mapped i.e. the human stratum corneum chymotrypsin enzyme (HSCCE), human neuropsin, and trypsin-like serine protease (TLSP). Their relative location is shown in Figure 1. The five genes all have variable homologies with known human or animal kallikrein proteins and/or other known serine proteases (depicted as KLK-L1, KLK-L2, KLK-L3, KLK-L4 and KLK-L5 in Figure 1 and KLK-L1 to KLK-L6 in Figure 28).

In Tables 1 to 5, the preliminary exon structure and partial protein sequence for each one of the newly identified genes is shown. In Table 6, some proteins are presented which appear, on preliminary analysis, to be homologous to the proteins encoded by the putative new genes. SEQ. ID. NOs. 2, 3, 14, 22, 23, 44, 45, 57, 58, 59, 60, 66, and 67 show amino acid sequences of KLK-L1-KLK-L6. and SEQ. ID. NOs.1, 13, 21, 43, 56, and 65 show nucleic acid sequences of the genes encoding KLK-L1- KLK-L6.

### DISCUSSION

Prediction of protein-coding genes in newly sequenced DNA becomes very important after the establishment of large genome sequencing projects. This problem is complicated due to the exon-intron structure of the eukaryotic genes which interrupts the coding sequence in many unequal parts. In order to predict the protein-coding exons and overall gene structure, a number of computer programs were developed. All these programs are based on the combination of potential functional signals with the global statistical properties of known protein-coding regions (15). However, the most powerful approach for gene structure prediction is to combine information about potential functional signals (splice sites, translation start or stop signal etc.) together with the statistical properties of coding sequences (coding potential) along with information about homologies between the predicted protein and known protein families (16).

In mouse and rat, kallikreins are encoded by large multigene families and these genes tend to cluster in groups with a distance as small as 3.3 - 7.0 Kb (3). A strong conservation of gene order between human chromosome 19q13.1 - q13.4 and 17 loci in a 20-cM proximal part of mouse chromosome 7, including the kallikrein locus, has been documented (17).

In humans, only a few kallikrein genes were identified. In fact, only KLK1, KLK2 and KLK3 (PSA) are considered to represent the human kallikrein gene family (9), The work described herein provides strong evidence that a large number of kallikrein-like genes are clustered within a 300Kb region around chromosome 19q13.2 - q13.4. The three established human kallikreins (KLK1, KLK2, KLK3), Zyme and NES1, as well as the stratum corneum chymotrypticn enzyme, neuropsin, and TLSP (trypsin-like serine protease) and another five new genes , KLK-L1 to KLK-L5, may constitute a large gene family. This will bring the total number of kallikrein or kallikrein-like genes in this region of chromosome 19 to thirteen.

The human stratum corneum chymotryptic enzyme (19), neuropsin (20) and trypsin-like serine protease (TLSP) (21) are three previously characterized genes which have many structural similarities with the kallikreins and other members of the serine protease family. However, they have not been mapped in the past. Their precise mapping in the region of the kallikrein gene family indicates that these three genes, along with the ones that were newly identified, or are already known, constitute a family that likely originated by duplication of an ancestral gene. The relative localization of all these genes is depicted in Figure 1.

Kallikrein genes are a subfamily of serine proteases, traditionally characterized by their ability to liberate lysyl-bradykinin (kallidin) from kininogen (18). More recently, however, a new, structural concept has emerged to describe kallikreins. From accumulated sequence data, it is now clear that the mouse has many genes with high homology to kallikrein coding sequences (19-20). Richard and co-workers have contributed to the concept of a " kallikrein multigene family" to refer to these genes (21-22). This definition is not based much on specific enzymatic function of the gene product, but more on its sequence homology and their close linkage on mouse chromosome 7. In humans, only KLK1 meets the functional definition of a kallikrein. KLK2 has trypsin-like enzymatic activity and KLK3 (PSA) has very weak chymotrypsin-like enzymatic activity. These activities of KLK2 and KLK3 are not known to liberate biologically active peptides from precursors. Based on the newer definition, members of the kallikrein family include, not only the gene for the kallikrein enzyme, but also genes encoding other homologous proteases, including the enzyme that processes the precursors of the nerve growth factor and epidermal growth factor (8). Therefore, it is important to note the clear distinction between the enzyme kallikrein and a kallikrein or a kallikrein-like gene.

In carrying out the study only exons were considered which were predicted with "good" or "excellent" quality and only exons were considered which were predicted by at least two different programs. Moreover, the presence of a putative gene was only considered when at least three exons clustered coordinately in that region. Additional evidence that these new genes are indeed homologous to the known kallikreins and other serine proteases comes from comparison of the intron phases. As published previously (14), trypsinogen, PSA and NES1 have 5 coding exons of which the first has intron phase I (the intron occurs after the first nucleotide of the codon), the second has intron phase II (the intron occurs after the second nucleotide and the codon), the third has intron phase I and the fourth has intron phase 0 (the intron occurs between codons). The fifth exon contains the stop codon. The intron phases of the predicted new kallikrein-like genes follow these rules and are shown in the respective tables. Further support comes from the identification in the new genes, of the conserved amino acids of the catalytic domain of the serine proteases, as presented in Tables 1-5.

In order to test the accuracy of the computer programs, known genomic areas containing the PSA, Zyme and KLK2 genes were tested. Two of these programs (Grail 2 and GeneBuilder) were able to detect about 95% of the tested known genes. Matches with expressed sequence tag sequences (EST) can also be employed for gene structure prediction in the GeneBuilder program and this can significantly improve the power of the program especially at high stringency (e.g. >95% homology).

In mouse, ten of the kallikrein genes appear to be pseudogenes (9).

### Example 2

### PROSTASE/KLK-L1 in prostate and breast tissues

The fine mapping of the prostase/KLK-L1 gene and its chromosomal localization in relation to a number of other homologous genes also mapping to the same region are described. In addition, extensive tissue expression studies were carried out that demonstrate that, in addition to prostate (which shows the highest expression), that prostase/KLK-L1 is also expressed in female breasts, testis, adrenals, uterus, colon, thyroid, brain, spinal cord and salivary glands. Furthermore, the gene is up-regulated by androgens and progestins in the breast carcinoma cell line BT-474.

### Materials and Methods

### DNA sequences on chromosome 19

Large DNA sequencing data for chromosome 19 is available at the web site of the Lawrence Livermore National Laboratory (LLNL) (http://www-bio.lln1. gov/genome /genome.html). Approximately 300 Kb of genomic sequence was obtained from that web site, encompassing a region on chromosome 19q13.3 - 13.4, where the known kallikrein genes are localized. This sequence is represented by 9 contigs of variable lengths. By using the sequences of PSA, KLK2, NES1 and protease M and the alignment program BLAST 2 (37), the relative positions of these genes on the contiguous map were located.

### Gene prediction analysis

For exon prediction analysis of the whole genomic area, a number of different computer programs were used. Originally all these programs were tested using the known genomic sequences of the PSA, protease M and NES1 genes. The most reliable computer programs GeneBuilder (gene prediction)[http://125.itba.mi.cnr.it/-webgene/genebuilder.htm1 ] GeneBuilder (exon prediction) [http://125.itba.mi.cnr.it/~webgene/genebuilder.html] , Grail 2 [http://compbio.ornl.gov ], and GENEID-3 [http://apolo.imim.es/geneid.htmll] were selected for further use.

### Protein homology searching

Putative exons of the newly identified gene were first translated to the corresponding amino acid sequences. BLAST homology searching for the proteins encoded by the exons were performed using the BLASTP program and the GenBank databases (37).

### Searching expressed sequence tags (ESTs)

Sequence homology searching was performed using the BLASTN alogrithm (37) on the National Center for Biotechnology Information web server (http://www ncbi.nlm.nih.gov/BLAST/) against the human EST database (dbEST). Clones with > 95% homology were obtained from the I.M.A.G.E. (38) consortium through Research Genetics Inc, Huntsville, AL and from The Institute for Genomic Research (TIGR) (http://WWW.TIGR.ORG/ tdb/tdb.html) (Table 7). Clones were propagated, purified and then sequenced from both directions with an automated sequencer, using insert-flanking vector primers.

### Breast cancer cell line and stimulation experiments

The breast cancer cell line BT-474 was purchased from the American Type Culture Collection (ATCC), Rockville, MD. BT-474 cells were cultured in RPMI media (Gibco BRL, Gaithersburg, MD) supplemented with glutamine (200 mmol/L), bovine insulin (10 mg/L), fetal bovine serum (10%), antibiotics and antimycotics, in plastic flasks, to near confluency. The cells were then aliquoted into 24-well tissue culture plates and cultured to 50% confluency. 24 hours before the experiments, the culture media were changed into phenol red-free media containing 10% charcoal-stripped fetal bovine serum. For stimulation experiments, various steroid hormones dissolved in 100% ethanol were added into the culture media, at a final concentration of 10⁻⁸ M. Cells stimulated with 100% ethanol were included as controls. The cells were cultured for 24 hours, then harvested for mRNA extraction.

### Reverse transcriptase polymerase chain reaction

Total RNA was extracted from the breast cancer cells using Trizol reagent (Gibco BRL) following the manufacturer's instructions. RNA concentration was determined spectrophotometrically. 2 µg of total RNA was reverse transcribed into first strand cDNA using the Superscript™ preamplification system (Gibco BRL). The final volume was 20 µl. Based on the combined information obtained from the predicted genomic structure of the new gene and the EST sequences, two gene-specific primers were designed (Table 8), PCR was carried out in a reaction mixture containing 1 µl of cDNA, 10 mM Tris-HCl (pH 8.3), 50 mM KCl, 1.5 mM MgCl₂, 200 µM dNTPs (deoxynucleoside triphosphates), 150 ng of primers and 2.5 units of AmpliTaq Gold DNA polymerase (Roche Molecular Systems, Branchburg, NJ, USA) on a Perkin-Elmer 9600 thermal cycler. The cycling conditions were 94°C for 9 minutes to activate the Taq Gold DNA polymerase, followed by 43 cycles of 94°C for 30 s, 63°C for 1 minute and a final extension at 63°C for 10 min. Equal amounts of PCR products were electrophoresed on 2% agarose gels and visualized by ethidium bromide staining. All primers for RT-PCR spanned at least 2 exons to avoid contamination by genomic DNA.

### Tissue expression of KLK-L1

Total RNA isolated from 26 different human tissues was purchased from Clontech, Palo Alto, CA. cDNA was prepared as described above for the tissue culture experiments and used for PCR reactions with the primers described in Table 8 (SEQ. ID. Nos 5-12). Tissue cDNAs were amplified at various dilutions. Cloning and sequencing of the PCR products

To verify the identity of the PCR products, they were cloned into the pCR 2.1-TOPO vector (Invitrogen, Carlsbad, CA, USA) according to the manufacturer's instructions. The inserts were sequenced from both directions using vector-specific primers, by an automated DNA sequencer.

### Results

### Identification of the prostase/KLK-L1 gene

The exon prediction strategy of the 300Kb DNA sequences around chromosome 19q13.3 - q13.4 identified a novel gene with a structure reminiscent of a serine protease. The major features of this gene were its homology, at the amino acid and DNA level, with other human kallikrein genes; the conservation of the catalytic triad (histidine, aspartic acid, and serine), the number of exons and the complete conservation of the intron phases.

### EST sequence homology search

EST sequence homology search of the putative exons obtained from the gene prediction programs (as described above) against the human EST database (dbEST) revealed five expressed sequence tags (ESTs) with >95 % identity to the putative exons of the gene (Table 7). Positive clones were obtained and the inserts were sequenced from both directions. Alignment was used to compare between the EST sequences and the exons predicted by the computer programs, and final selection of the exon-intron splice sites was made according to the EST sequences. Furthermore, many of the ESTs were overlapping, further ensuring the accuracy of the data.

The coding sequence of the klk-L2 gene is shown in SEQ. ID. NO. 1 and GenBAnk Accession # AF135023. The exons of the gene are as follows: exon 1 (939-999); exon 2 (2263-2425); exon 3 (2847-3097); exon 4 (3181-3317); and exon 5 (4588-4740). The amino acid sequence of KLK-L2 proteins are shown in SEQ. ID. Nos. 2 and 3.

### Mapping and chromosomal localization of prostase /KLK-L1 gene

Alignment of the prostase/ KLK-L1 sequence and the sequences of other known kallikrein genes within the 300 Kb area of the contigs constructed at the Lawrence Livermore National Laboratory enabled precise localization of all genes and to determine the direction of transcription, as shown in Figure 2. The distance between PSA and KLK2 genes was calculated to be 12,508 bp. The prostase/KLK-L1 gene is 26,229 bp more telomeric to KLK2 and transcribes in the opposite direction. The zyme gene is about 51 Kb more telomeric to the prostase gene and transcribes in the same direction. The human stratum corneum chymotryptic enzyme gene, the neuropsin gene and the NES I gene are all further telomeric to zyme and all transcribe in the same direction as zyme.

### Tissue expression of the prostase/KLK-L1 gene

The tissues that express the prostase/KLK-L1 gene were assessed by RT-PCR. The experiments were performed at various dilutions of the cDNAs to obtain some information about the relative levels of expression. RT-PCR for actin was used as a positive control and RT-PCR for the PSA cDNA was used as another positive control with tissue restricted specificity. Positive ESTs for prostase/KLK-L1 were used as controls for the PCR procedure. The PSA gene was found to be highly expressed in the prostate, as expected, and to a lower extent in mammary and salivary glands as also expected from recent literature reports (24, 25). Very low expression of PSA in the thyroid gland, trachea and testis was also found, a finding that accords with recent RT-PCR data by others (26).

The tissue expression of prostase/KLK-L1 is summarized in Table 9 and Figure 3. This protease is primarily expressed in the prostate, testis, adrenals, uterus, thyroid, colon, central nervous system and mammary tissues, and, at much lower levels in other tissues. The specificity of the RT-PCR procedure was verified for prostase/KLK-L1 by cloning the PCR products from mammary, testicular and prostate tissues and sequencing them. One example with mammary tissue is shown in Figure 4. All cloned PCR products were identical in sequence to the cDNA sequence reported for the prostase/KLK-L1.

### Hormonal regulation of the prostase/KLK-L1 gene

The steroid hormone receptor-positive breast carcinoma cell line BT-474 was used as a model system to evaluate whether prostase/KLK-L1 expression is under steroid hormone regulation. As shown in Figure 5, the controls worked as expected i. e., actin positivity without hormonal regulation in all cDNAs, only estrogen up-regulation of the pS2 gene and up-regulation of the PSA gene by androgens and progestins. Prostase/KLK-L1 is up-regulated primarily by androgens and progestins, similarly to PSA. This up-regulation was dose-dependent and it was evident at steroid hormone levels ≥10⁻¹⁰ M.

### DISCUSSION

The KLK3 gene encodes for PSA, a protein that currently represents the best tumor marker available (24). Since in rodents there are so many kallikrein genes, the restriction of this family to only 3 genes in humans was somewhat surprising. More recently, new candidate kallikrein genes in humans have been discovered, including NES1 (13) and zyme/protease M/neurosin (10-12). The known kallikreins and the newly discovered kallikrein-like genes share the following similarities: (a) they encode serine proteases (b) they have five coding exons (c) they share significant DNA and protein homologies with each other (d) they map in the same locus on chromosome 19q 13.3-q 13.4, a region that is structurally similar to an area on mouse chromosome 7, where all the mouse kallikrein genes are localized (e) they appear to be regulated by steroid hormones. Prostase/KLK-L1 is a member of the same family since these common characteristics are also shared by the newly discovered gene.

The exact localization of the KLK-L1 gene and its position in relation to other genes in the area (Figure 2) was determined. Prostase/KLK-L1 lies between KLK2 and zyme.

Irwin et al. (27) have proposed that the serine protease genes can be classified into five different groups according to intron position. The established kallikreins (KLK1, KLK2, and PSA), trypsinogen and chymotrypsinogen belong to a group that has: (1) an intron just downstream from the codon for the active site histidine residue, (2) a second intron downstream from the exon containing the codon for the active site aspartic acid residue, and (3) a third intron just upstream from the exon containing the codon for the active site serine residue. As seen in Figure 6, the genomic organization of prostase/KLK-L1 gene is very similar to this group of genes. The lengths of the coding parts of exons 1-5 are 61,163, 263, 137 and 153 bp, respectively, which are close or identical to the lengths of the exons of the kallikrein genes and also, similar or identical to those of other newly discovered genes in the same chromosomal region like the NES1(14), zyme/protease M/neurosin (10-12) and neuoropsin (28) genes.

The sensitive RT-PCR protocol reveals that the KLK-L1 enzyme is expressed in prostatic tissue and it is also expressed in significant amounts in other tissues, including testis, female mammary gland, adrenals, uterus, thyroid, colon, brain, lung and salivary glands (Figure 3 and Table 9). The specificity of the RT-PCR primers was verified by sequencing the obtained PCR products, with one example shown in Figure 4 (SEQ.ID.NO. 4). Tissue culture studies with the breast carcinoma cell line BT-474 further confirm not only the ability of these cells to produce prostase/KLK-L1 but also its hormonal regulation (Figure 5).

An interesting theme is now developing involving the group of homologous genes on chromosome 19q13.3(PSA, KLK2, prostase, zyme, and NES1). The combined data suggest that all of them are expressed in prostate and breast tissues, and all of them are hormonally regulated. All these genes may be part of a cascade pathway that plays a role in cell proliferation, differentiation or apoptosis by regulating (positively or negatively) growth factors or their receptors or cytokines, through proteolysis (30). Also interesting is the linkage of locus 19q13 to solid tumors and gliomas (31) which raises the possibility that some of the genes in the region may be disrupted by rearrangements.

The KLK-1L gene encodes for a serine protease that shows homology with other members of the kallikrein gene family and maps to the same chromosomal location. Many structural features of the kallikreins are conserved in prostase/KLK-L1. The precise mapping of this gene between the two known genes KLK2 and zyme is presented. It is further demonstrated that prostase/KLK-L1 is expressed in many tissues, in addition to the prostate, including the female breast. This gene is also herein referred to as 'prostase'. It has been further demonstrated, using breast carcinoma cell lines, that prostase/KLK-L1 can be produced by these cells and that its expression is significantly up-regulated by androgens and progestins. Based on information for other homologous genes in the area (PSA, zyme, and NES1), prostase/KLK-L1 may be involved in the pathogenesis and/or progression of prostate, breast and possibly other cancers.

### Example 3

### IDENTIFICATION OF THE KLK-L2 GENE

### Materials and Methods

### DNA sequence on chromosome 19

Sequencing data of approximately 300Kb of nucleotides on chromosome 19q13.3-q13.4 was obtained from the web site of the Lawrence Livermore National Laboratory (LLNL) (http://www-bio.llnl.gov/genome/genome.html). This sequence was in the form of 9 contigs of different lengths. A restriction analysis study of the available sequences was performed using the "WebCutter" computer program (http://www.firstmarket. com/cutter/cut2.html) and with the aid of the EcoR1 restriction map of this area (also available from the LLNL web site) an almost contiguous stretch of genomic sequences was constructed. The relative positions of the known kallikrein genes: PSA (GenBank accession # X14810 ), KLK2 (GenBank accession # M18157), and zyme (GenBank accession # U60801) was determined using the alignment program BLAST 2 (37).

### New Gene Identification

A number of computer programs were used to predict the presence of putative new genes in the genomic area of interest. These programs were initially tested using the known genomic sequences of the PSA, protease M and NES 1 genes. The most reliable computer programs GeneBuilder (gene prediction) (http://125.itba.mi.cnr.it/~webgene/genebuilder.html) GeneBuilder (exon prediction) (http://125.itba.mi.cnr.it/~webgene/penebuilder.html), Grail 2 (http://compbio.ornl.gov) and GENEID-3 (http://apolo.imim.es/seneid.html) were selected for further use.

### Expressed sequence tag (EST) searching

The predicted exons of the putative new gene were subjected to homology search using the BLASTN algorithm (37) on the National Center for Biotechnology Information web server (http://www ncbi.nlm.nih.govBLAST/) against the human EST database (dbEST). Clones with > 95% homology were obtained from the I.M.A.G.E. consortium (38) through Research Genetics Inc, Huntsville, AL (Table 10). The clones were propagated, purified and sequenced from both directions with an automated sequencer, using insert-flanking vector primers.

### Rapid amplification of cDNA ends (5' RACE)

According to the EST sequence data and the predicted structure of the gene, two gene-specific primers were designed (R1 & R2) (Table 11). Two rounds of RACE reactions (nested PCR) were performed with 5µl Marathon Ready™ cDNA of human testis (Clontech, Palo Alto, CA, USA) as a template. The reaction mix and PCR conditions were conducted according to the manufacturer's recommendations. In brief, denaturation was done for 5 min at 94°C followed by 94° C for 5 sec followed by 72°C for 2 min for 5 cycles, then 94°C for 5 sec followed by 70° C for 2 min for 5 cycles then 94°C for 5 sec followed by 65°C for 2 min for 30 cycles for the first reaction and 25 cycles for the nested PCR reaction.

### Tissue expression

Total RNA isolated from 26 different human tissues was purchased from Clontech, Palo Alto, CA. cDNA was prepared as described below for the tissue culture experiments and used for PCR reactions with the primers described in Table 11 (SEQ. ID. Nos 9-12, 15-20). Tissue cDNAs were amplified at various dilutions.

### Breast cancer cell line and hormonal stimulation experiments

The breast cancer cell line BT-474 was purchased from the American Type Culture Collection (ATCC), Rockville, MD. Cells were cultured in RPMI media (Gibco BRL, Gaithersburg, MD) supplemented with glutamine (200 mmol/L), bovine insulin (10 mg/L), fetal bovine serum (10%), antibiotics and antimycotics, in plastic flasks, to near confluency. The cells were then aliquoted into 24-well tissue culture plates and cultured to 50% confluency. 24 hours before the experiments, the culture media were changed into phenol red-free media containing 10% charcoal-stripped fetal bovine serum. For stimulation experiments, various steroid hormones dissolved in 100% ethanol were added into the culture media, at a final concentration of 10⁻⁸ M. Cells stimulated with 100% ethanol were included as controls. The cells were cultured for 24 hours, then harvested for mRNA extraction

### Reverse transcriptase polymerase chain reaction

Total RNA was extracted from the breast cancer cells using Trizol reagent (Gibco BRL) following the manufacturer's instructions. RNA concentration was determined spectrophotometrically. 2 µg of total RNA was reverse-transcribed into first strand cDNA using the Superscript™ preamplification system (Gibco BRL). The final volume was 20 µl. Based on the combined information obtained from the predicted genomic structure of the new gene and the EST sequences, two gene-specific primers were designed (Table 11) and PCR was carried out in a reaction mixture containing 1 µl of cDNA, 10 mM Tris-HCl (pH 8.3), 50 mM KCl, 1.5 mM MgCl₂, 200µM dNTPs (deoxynucleoside triphosphates), 150 ng of primers and 2.5 units of AmpliTaq Gold DNA polymerase (Roche Molecular Systems, Branchburg, NJ, USA) on a Perkin-Elmer 9600 thermal cycler. The cycling conditions were 94°C for 9 minutes to activate the Taq Gold DNA polymerase, followed by 43 cycles of 94°C for 30 s, 63°C for 1 minute and a final extension at 63°C for 10 min. Equal amounts of PCR products were electrophoresed on 2% agarose gels and visualized by ethidium bromide staining. All primers for RT-PCR spanned at least 2 exons to avoid contamination by genomic DNA.

To verify the identity of the PCR products, they were cloned into the pCR 2.1-TOPO vector (Invitrogen, Carlsbad, CA, USA) according to the manufacturer's instructions. The inserts were sequenced from both directions using vector-specific primers, with an automated DNA sequencer.

### Structure analysis

Multiple alignment was performed using the Clustal X software package available at: ftp:/lftp.ebi.ac.uk/pub/software/dos/clustalw/clustalx/ (clustalx1.64b.msw.exe) and the multiple alignment program available from the Baylor College of Medicine (BCM), Houston, TX, USA (kiwi.imgen.bcm.tmc. edu:8808/search-launcher/launcher/html). Phylogenetic studies were performed using the Phylip software package available at: http://evolution.genetics. washington.edu/phylip/getme.html. Distance matrix analysis was performed using the "Neighbor-Joining/UPGMA" program and parsimony analysis was done using the "Protpars" program. Hydrophobicity study was performed using the BCM search launcher programs (http://dot.imgen.bcm.tmc.edu:9331/seq-search/struc-predict.html). Signal peptide was predicted using the "SignaIP" server (http://www.cbs.dtu.dk/services/ signal). Protein structure analysis was performed by "SAPS" (structural analysis of protein sequence) program (http://dot.imgen.bcm.tmc.edu:9331/seqsearch/struc-predict.html).

### RESULTS

Computer analysis of the genomic sequence predicted a putative new gene consisting of four exons. This gene was detected by all programs used and all exons had high prediction scores. EST sequence homology search of the putative exons against the human EST database (dbEST) revealed nine expressed sequence tag (EST) clones from different tissues with >95 % identity to the putative exons of the gene (Table 10). Positive clones were obtained and the inserts were sequenced from both directions. The "Blast 2 sequences" program was used to compare the EST sequences with the predicted exons, and final selection of the exon-intron splice sites was done according to the EST sequences. The presence of many areas of overlap between the various EST sequences allowed further verification of the structure of the new gene. The coding sequence of the gene is shown in SEQ. ID. NO. 13 and GenBank Accession #AF135028. The 3' end of the gene was verified by the presence of poly A stretches that are not present in the genomic sequence at the end of two of the sequenced ESTs. One of the sequenced ESTs revealed the presence of an additional exon at the 5'end. The nucleotide sequence of this exon matches exactly with the genomic sequence. To further identify the 5' end of the gene, 5' RACE was performed but no additional sequence could be obtained. However, as is the case with other kallikreins, the presence of further up-stream untranslated exon(s) could not be excluded. The amino acid sequence of KLK-L2 is shown in SEQ. ID. No. 14.

### Mapping and chromosomal localization of the KLK-L2 gene

Alignment of KLK-L2 gene and the sequences of other known kallikrein genes within the 300 Kb area of interest enabled precise localization of all genes and determination of the direction of transcription, as shown by the arrows in Figure 8. The PSA gene was found to be the most centromeric, separated by 12,508 base pairs (bp) from KLK2, and both genes are transcribed in the same direction (centromere to telomere). The prostase/KLK-L1 gene is 26,229 bp more telomeric and transcribes in the opposite direction, followed by KLK-L2. The distance between KLK-L1 and KLK-L2 is about 35 Kilobases (Kb). The zyme gene is 5,981 bp more telomeric and the latter 3 genes are all transcribed in the same direction (Figure 8).

### Structural characterization of the KLK-L2 gene and its protein product

The KLK-L2 gene, as presented in Figure 7, is formed of 5 coding exons and 4 intervening introns, spanning an area of 9.349 bp of genomic sequence on chromosome 19q13.3-q13.4. The lengths of the exons are 73, 262, 257,134, and 156 bp, respectively. The intron/exon splice sites (mGT....AGm) and their flanking sequences are closely related to the consensus splicing sites (-mGTAAGT ...CAGm-) (32). The presumptive protein coding region of the KLK-L2 gene is formed of 879 bp nucleotide sequence encoding a deduced 293-amino acid polypeptide with a predicted molecular weight of 32 KDa. There are two potential translation initiation codons (ATG) at positions 1 and 25 of the predicted first exon (numbers refer to SEQ. ID. NO. 13 and GenBank Accession #AF135028). It is assumed that the first ATG will be the initiation codon, since : (1) the flanking sequence of that codon (GCGGCCATGG) matches closely with the Kozak consensus sequence for initiation of translation (GCC A/G CCATGG) (33) and is exactly the same as that of the homologous zyme gene. At this initiation codon, the putative signal sequence at the N-terminus is similar to other trypsin-like serine proteases (prostase and EMSP) (Figure 9). The cDNA ends with a 328 bp of 3' untranslated region containing a conserved poly adenylation signal (AATAAA) located 11 bp up-stream of the poly A tail (at a position exactly the same as that of the zyme poly A tail)(11).

A hydrophobicity study of the KLK-L2 gene shows a hydrophobic region in the N-terminal region of the protein (Figure 10), suggesting that a presumed signal peptide is present. By computer analysis, a 29-amino acid signal peptide is predicted with a cleavage site at the carboxyl end of Ala²⁹. For better characterization of the predicted structural motif of the KLK-L2 protein, it was aligned with other members of the kallikrein multi-gene family, (Figure 9), and the predicted signal peptide cleavage site was found to match with the predicted signal cleavage sites of zyme (11), KLK1(1), KLK2 (8), and KLK-L1. Also, sequence alignment supports, by analogy, the presence of a cleavage site at the carboxyl end of Ser⁶⁶, which is the exact site predicted for cleavage of the activation peptide of all the other kallikreins aligned in Figure 9. Interestingly, the starting amino acid sequence of the mature protein (IING(S) DC) is conserved in the prostase and enamel matrix serine proteinase 1 (EMSP) genes. Thus, like other kallikreins, KLK-L2 is likely also synthesized as a preproenzyme that contains an N-terminal signal peptide (prezymogen) followed by an activation peptide and the enzymatic domain.

The presence of aspartate (D) in position 239 suggests that KLK-L2 will possess a trypsin-like cleavage pattern like most of the other kallikreins (e.g., KLK1, KLK2, TLSP, neuropsin, zyme, prostase, and EMSP) but different from PSA which has a serine (S) residue in the corresponding position, and is known to have a chymotrypsin like activity (Figure 9). The dotted region in Figure 9 indicates an 11-amino acid loop characteristic of the classical kallikreins (PSA, KLK1, and KLK2) but not found in KLK-L2 or other members of the kallikrein-like gene family (11).

### Homology with the kallikrein multi-gene family

The mature 227-amino acid sequence of the predicted protein was aligned against the GenBank database and the known kallikreins using the "BLASTP" and "BLAST 2 sequence" programs. KLK-L2 is found to have 54% amino acid sequence identity and 68% similarity with the enamel matrix serine proteinase 1 (EMSP1) gene, 50% identity with both trypsin like serine protease (TLSP) and neuropsin genes and 47%, 46%, and 42% identity with trypsinogen, zyme, and PSA genes, respectively. The multiple alignment study shows that the typical catalytic triad of serine proteases is conserved in the KLK-L2 gene (H¹⁰⁸, D¹⁵³, and S²⁴⁵) and, as the case with all other kallikreins, a well conserved peptide motif is found around the amino acid residues of the catalytic triad [i.e., histidine (WLLTAA*H*C), serine(GDSGGP), and aspartate(*D*LMLI)] (10, 11).

Twelve cysteine residues are present in the putative mature KLK-L2 protein, ten of them are conserved in all the serine proteases that are aligned in Figure 9, and would be expected to form disulphide bridges. The other two cysteines (C¹⁷⁸ and C²⁷⁹) are not found in PSA, KLK1, KLK2 or trypsinogen, however, they are found in similar positions in prostase, EMSP1, zyme, neuropsin, and TLSP genes and are expected to form an additional disulphide bond. Twenty nine "invariant" amino acids surrounding the active site of serine proteases have been described (39). Of these, twenty-six are conserved in KLK-L2. One of the non-conserved amino acids (Ser²¹⁰ instead of Pro) is also found in prostase and EMSP1 genes, the second (Leu¹⁰³ instead of Val) is also found in TLSP gene, and the third (Val¹⁷⁴ instead of Leu) is also not conserved in prostase or EMSP1 genes. According to protein evolution studies, each of these amino acid changes represents a conserved evolutionary substitution to a protein of the same group (39).

### Evolution of the KLK-L2 gene

To predict the phylogenetic relatedness of the KLK-L2 gene with other serine proteases, the amino acid sequences of the kallikrein genes were aligned together using the "Clustal X" multiple alignment program and a distance matrix tree was predicted using the Neighbor-joining/UPGMA method (Figure 10). Phylogenetic analysis separated the classical kallikreins (KLK1, KLK2, and PSA) and grouped the KLK-L2 with KLK-L1, EMSP1, and TLSP (40, 41).

### Tissue expression of the KLK-L2 gene

As shown in Table 12 and Figure 11, the KLK-L2 gene is primarily expressed in the brain, mammary gland, and testis but lower levels of expression are found in many other tissues. In order to verify the RT-PCR specificity, the PCR products were cloned and sequenced.

### Hormonal regulation of the KLK-L2 gene

A steroid hormone receptor positive breast cancer cell line (BT-474) was used as a model to verify whether the KLK-L2 gene is under steroid hormone regulation. PSA was used as a control known to be upregulated by androgens and progestins and pS2 as an estrogen upregulated control. The results indicate that KLK-L2 is up-regulated by estrogens and progestins (Figure 12).

### Expression of KLK-L2 in Ovarian Tissues

KLK-L2 is up-regulated (overexpressed) in ovarian tumors (Figure 13).

### Discussion

With the aid of computer programs for gene prediction and the available EST database, a new gene, named KLK-L2 (for kallikrein like gene 2) was identified. The 3' end of the gene was verified by the presence of "poly A" stretches in the sequenced ESTs which were not found in the genomic sequence, and the start of translation was identified by the presence of a start codon in a well conserved consensus Kozak sequence.

As is the case with other kallikreins, the KLK-L2 gene is composed of 5 coding exons and 4 intervening introns and, except for the second coding exon, the exon lengths are comparable to those of other members of the kallikrein gene family (Figure 6). The exon-intron splice junctions were identified by comparing the genomic sequence with the EST sequence and were further confirmed by the conservation of the consensus splice sequence (-mGT......AGm-) (32), and the fully conserved intron phases, as shown in Figure 6. Furthermore, the position of the catalytic triad residues in relation to the different exons is also conserved (Figure 6). As is the case with most other kallikreins, except PSA and HSCCE, KLK-L2 is more functionally related to trypsin than to chymotrypsin (34). The wide range of tissue expression of KLK-L2 should not be surprising since, by using the more sensitive RT-PCR technique instead of Northern blot analysis, many kallikrein genes were found to be expressed in a wide variety of tissues including salivary gland, kidney, pancreas, brain, and tissues of the reproductive system (uterus, mammary gland, ovary, and testis) (34). KLK-L2 is highly expressed in the brain. Another kallikrein, neuropsin, was also found to be highly expressed in the brain and has been shown to have important roles in neural plasticity in mice (35). Also, the zyme gene is highly expressed in the brain and appears to have amyloidogenic potential (11). Taken together, these data point to a possible role of KLK-L2 in the central nervous system.

It was initially thought that each kallikrein enzyme has one specific physiological substrate. However, the increasing number of substrates, which purified proteins can cleave *in vitro,* has led to the suggestion that they may perform a variety of functions in different tissues or physiological circumstances. Serine proteases encode protein cleaving enzymes that are involved in digestion, tissue remodeling, blood clotting etc., and many of the kallikrein genes are synthesized as precursor proteins that must be activated by cleavage of the propeptide. The predicted trypsin-like cleavage specificity of KLK-L2 makes it a candidate activator of other kallikreins or it may be involved in a "cascade" of enzymatic reactions similar to those found in fibrinolysis and blood clotting (36).

In conclusion, a new member of the human kallikrein gene family, KLK-L2 was characterized. This gene is hormonally regulated and it is mostly expressed in the brain, mammary gland and testis. KLK-L2 may be useful as a tumor marker.

### Example 4

### Materials and methods

### Strategy for new gene discovery

Sequencing data of approximately 300 kb, around chromosome 19q 13.3-q 13.4, was obtained from the web site of the Lawrence Livermore National Laboratory (LLNL) (http://www-bio.llnl.gov/ genome/genome. html). Different computer programs were used for putative new gene prediction, as previously described.

### RT-PCR for KLK-L3 cDNA

Total RNA isolated from 26 different human tissues was purchased from Clontech, Palo Alto, CA. cDNA was prepared as described below and used for PCR amplification. A primer set (L3-F1 and L3-R1) was used to identify the presence of the gene in tissues, and the reverse primer (L3-R1) was used with another primer (L3-F2) to amplify and clone the full cDNA of the gene. These primer sequences are shown in Table 13 (SEQ. ID. Nos. 9-12, 24-26). Tissue cDNAs were amplified at various dilutions.

### Reverse transcriptase polymerase chain reaction.

2 µg of total RNA was reverse-transcribed into first strand cDNA using the Superscript™ preamplification system (Gibco BRL, Gaithersburg, MD). The final volume was 20 µl. Based on the combined information obtained from the predicted genomic structure of the new gene and the EST sequence, two gene-specific primers (L3-F1 and L3-R1) were designed (Table 13, SEQ. ID. Nos. 9-12, 24-26) and PCR was carried out in a reaction mixture containing 1 µl of cDNA, 10 mM Tris-HCl (pH 8.3), 50 mM KCl, 1.5 mM MgCl₂, 200 µM dNTPs (deoxynucleoside triphosphates), 150 ng of primers and 2.5 units of AmpliTaq Gold DNA polymerase (Roche Molecular Systems, Branchburg, NJ, USA) on a Perkin-Elmer 9600 thermal cycler. The cycling conditions were 94°C for 9 minutes, followed by 43 cycles of 94°C for 30 s, 63°C for 1 minute, and a final extension at 63°C for 10 minutes. Equal amounts of PCR products were electrophoresed on 2% agarose gels and visualized by ethidium bromide staining. All primers for RT-PCR spanned at least 2 exons to avoid contamination by genomic DNA.

### Breast cancer cell line and hormonal stimulation experiments

The breast cancer cell line BT-474 was purchased from the American Type Culture Collection (ATCC), Rockville, MD. Cells were cultured in RPMI media (Gibco BRL, Gaithersburg, MD) supplemented with glutamine (200 mmol/L), bovine insulin (10 mg/L), fetal bovine serum (10%), antibiotics and antimycotics, in plastic flasks, to near confluency. The cells were then aliquoted into 24-well tissue culture plates and cultured to 50% confluency. 24 hours before the experiments, the culture media were changed into phenol red-free media containing 10% charcoal-stripped fetal bovine serum. For stimulation experiments, various steroid hormones dissolved in 100% ethanol were added into the culture media, at a final concentration of 10⁻⁸ M. Cells stimulated with 100% ethanol were included as controls. The cells were cultured for 24 hours, then harvested for total RNA extraction by the Trizol method (GibcoBRL). cDNA was prepared and amplified as described above. Control genes (PSA, pS2, and actin) were amplified as previously described herein.

### Cloning and sequencing of the PCR products.

To verify the identity of the PCR products, they were cloned into the pCR 2.1-TOPO vector (Invitrogen, Carlsbad, CA, USA) according to the manufacturer's instructions. The inserts were sequenced from both directions using vector-specific primers, with an automated DNA sequencer.

### Identification of positive PAC and BAC genomic clones from human genomic DNA libraries

The PCR product generated with primer set Z1S and Z1AS (Table 14, SEQ.ID.NOS. 27-42), was purified and then labeled with ³²P by the random primer method (Sambrook, supra) and used as a probe to screen a human genomic DNA BAC library, spotted in duplicate on nylon membranes, for identification of positive clones. The filters were hybridized in 15% formamide, 500 mM Na₂HPO₄, 7% SDS, 1% BSA (w/v) at 65°C overnight, then washed sequentially with 2X SSC, 1X SSC, 0.2X SSC, containing 0.1% SDS at 65°C, and then exposed to X-ray film as described (Sambrook, supra). Positive clones were obtained, plated on selective LB medium, and then a single colony was transferred into LB broth for overnight cultures. A PAC clone positive for NES1 was identified by a similar methodology as described elsewhere (14). PAC and BAC libraries were constructed by de Jong and associates (42). Purification of BAC and PAC DNA was done by a rapid alkaline lysis miniprep method, which is a modification of the standard Qiagen-Tip method. Positive clones were further confirmed by Southern blot analysis as described (Sambrook, supra).

### 5' Rapid amplification of cDNA ends (5' RACE)

According to the EST sequences and the computer-predicted structure of the KLK-L3 gene, two gene specific primers were designed. Two rounds of RACE reactions (nested PCR) were performed with 5µl Marathon Ready™ cDNA of human testis (Clontech) as a template. The reaction mix and PCR conditions were selected according to the manufacturer's recommendations. Positive bands were gel-purified using Qiagen Gel Purification kits according to manufacturer's recommendations.

### Gene-specific amplification of other genes from genomic DNA

According to the published sequence of prostatic specific antigen (PSA), human renal kallikrein (KLK1), human glandular kallikrein (KLK2), normal epithelial cell-specific 1gene (NES1), KLK-L1. KLK-L2 and zyme genes, gene-specific primers were designed for each of these genes (Table 14) and developed polymerase chain reaction (PCR)-based amplification protocols which allowed us to generate specific PCR products with genomic DNA as a template. The PCR reactions were carried out as described above but by using an annealing/extension temperature of 65°C.

### Structure analysis studies.

Multiple alignment was performed using the clustal X software package available at: ftp://ftp.ebi.ac.uk/pub/software/dos/clustalw/clustalx/(clustalx1.64b.msw.exe) and the multiple alignment program available from the Baylor College of Medicine (BCM) search launcher (kiwi.imgen.bcm.tmc.edu:8808/search-launcher/launcher/html). Phylogenetic studies were performed using the Phylip software package available from: (http://evolution.genetics.washington.edu/phylip/getme.html). Distance matrix analysis was performed using the "Neighbor-Joining/UPGMA" program and parsimony analysis was done using the "Prompters" program. Hydrophobicity study was performed using the BCM search launcher programs (http://dot.imgen.bcm.tmc.edu:9331/seq-search/struc-predict.html). Signal peptide was predicted using the SignalP WWW server (http://www.cbs.dtu.dk/services/ signal). Protein structure analysis was performed by SAPS (structural analysis of protein sequence) program (http://dot.imgen.bcm.tmc.edu:9331/seq-search/struc-predict.html).

### Results:

### Construction of a contiguous map of the human kallikrein locus on chromosome 19q13.3-q13.4

Sequence information around the human chromosome 19q13.3-q13.4 locus (the proposed kallikrein locus) is available at the Lawrence Livermore National Laboratory web site. Sequences of approximately 300 kb in length were obtained. These sequences were in the form of contigs of different lengths. A restriction analysis study of the contigs was performed using various computer programs. With the aid of the EcoR1 restriction map of this area which is also available at the LLNL web site, the relative positions of these contigs was defined in relation to each other. Some contigs were overlapping, enabling construction of a contiguous segment; however, three gaps were present. <BLAST> analysis of these segments against the GenBank database (37) enabled the precise location of two classical kallikreins, namely PSA and KLK2 to be defined. Other newly discovered serine proteases were localized which are homologous with the kallikrein genes, namely protease M/zyme/neurosin (10, 11, 12), human stratum corneum chymotryptic enzyme ( HSSCE) (55), neuropsin (28), normal epithelial cell-specific 1 gene ( NES1) (13), trypsin-like serine protease ( TLSP) (GenBank accession # AF164623), KLK-L1 (SEQ.ID.NO. 1) and KLK-L2 (SEQ.ID.NO. 13). The gaps in the 300 kb genomic sequence were partially filled as follows:
(a) The margins of the first gap were found to contain the 5' and 3' ends of the KLK2 gene; this gap was filled with the genomic structure of the KLK2 gene (GenBank Accession # M18157).
(b) The margins of the third gap (gaps are numbered from centromere to telomere) were found to have the 3' and 5' ends of the zyme gene mRNA sequence; thus, a radiolabeled probe specific for the zyme gene was used to screen a human BAC library and two positive clones were obtained. Restriction analysis was performed, followed by Southern blotting and a fragment containing the zyme gene was obtained and sequenced, thus filling this gap.
(c) The second gap (between KLK-L1 and KLK-L2 genes) still exists and the EcoR1 restriction map of this area was used to approximately define its length (Figure 14).

Further support for the relative locations of these genes was obtained by performing PCR reactions with gene-specific primers to screen genomic DNA clones. The most centromeric group of genes (PSA, KLK2, KLK-L1, KLK-L2 and zyme ) were found to be clustered in one genomic BAC clone, and the next group (HSCCE, neuropsin,KLK-L3 and NES1) were found to be clustered together in another clone, as expected from the data of Figure 14.

### Cloning of the KLK-L3 gene

A putative new gene, formed of three exons, was predicted by computer analysis of the genomic sequence. The predicted exons were subjected to sequence homology search against the human EST database (dbEST) and revealed an EST clone (GenBank accession # AA583908) which exhibited 99% homology with the putative gene. This EST was obtained, purified and sequenced and the sequence was aligned by BLAST software (37) against the genomic area that contains the putative gene. An additional exon, downstream of the predicted structure, was identified. The 3' end of the gene was verified by: (a) The presence of the serine residue (S) of the catalytic triad in a well-conserved region. This highly conserved motif (GDSGGP) always occurs at the beginning of the last exon in all known kallikreins. (b) The presence of a stop codon that is in frame with the predicted amino acid sequence. (c) The presence of a 19-poly A stretch at the end of the EST that was not found in the genomic sequence.

To verify the accuracy of the cDNA sequence of the gene, PCR reactions were performed using gene-specific primers for the first and last exons of the predicted structure of the gene (L3-F2 and L3-R1) with cDNA isolated from different human tissues as putative templates. A positive band of the expected size was isolated from testis cDNA and fully sequenced. Its sequence was aligned by BLAST against the genomic sequence to unequivocally define the exon/intron boundaries. For further characterization of the 5' end of the gene, 5'RACE reaction was performed using Marathon Ready cDNA from testis as a template. This allowed identification of an additional exon that contains the start codon and 5' untranslated region. The full sequence of the gene is shown in SEQ. ID. NO. 21 (GenBank Accession # AF135026) and the amino acid sequences of KLK-L3 proteins are shown in SEQ. ID. Nos. 22 and 23.

### Structural characterization of the KLK-L3 gene:

As shown in Figure 15, the KLK-L3 gene is formed of 5 coding exons and 4 intervening introns, although, as with other kallikreins, the presence of further upstream untranslated exon(s) could not be ruled out (14, 28). All of the exon /intron splice sites conform to the consensus sequence for eukaryotic splice sites (32). The gene further follows strictly the common structural features of the human kallikrein multigene family, as described below.

The predicted protein-coding region of the gene is formed of 753 bp, encoding a deduced amino acid polypeptide with a predicted molecular weight of 27.5 kDa. A potential translation initiation codon is found at position 28 of the predicted first exon (numbers of nucleotides refer to SEQ. ID. NO. 21 or GenBAnk Accession # AF135026. This codon does not match well with the consensus Kozak sequence (33), however, it has a purine at position (-3) which occurs in 97% of vertebrate mRNAs (43), and it is almost identical to the sequence of the zyme gene flanking the start codon. It should also be noted that most kallikreins do not have the consensus G nucleotide in position (+4).

Nucleotides 6803-6808 (AGTAAA) closely resemble a consensus polyadenylation signal (44) and are followed by a stretch of 19 poly A nucleotides not found in genomic DNA, after a space of 14 nucleotides. No other potential polyadenylation signals were discernable in the 3' untranslated region, suggesting that the above motif is indeed the polyadenylation signal. The same polyadenylation signal motif was predicted for the KLK1 and KLK2 genes.

Although the KLK-L3 protein sequence is unique, comparative analysis revealed that it is highly homologous to other members of the kallikrein multigene family. KLK-L3 shows 40% protein identity with the TLSP gene product and 38% and 33% identity with the KLK-L2 and KLK1 proteins, respectively. Hydrophobicity analysis revealed that the amino-terminal region is quite hydrophobic (Figure 16), consistent with the possibility that this region may harbor a signal sequence, analogous to other serine proteases. Computer analysis of the aminoacid sequence of KLK-L3 predicted a cleavage site between amino acids 19 and 20 (GWA-DT). Sequence alignment (Figure 17) also revealed a potential cleavage site (Arg²²), at a site homologous to other serine proteases (lysine (K) or arginine (R) is present in most cases). Several evenly distributed hydrophobic regions throughout the KLK-L3 polypeptide are consistent with a globular protein, similar to other kallikreins and serine proteases. The dotted region in Figure 17 indicates an 11-amino acid loop characteristic of the classical kallikreins (PSA, KLK1, and KLK2) but not found in KLK-L3 or other members of the kallikrein multi-gene family (11, 41).

Twenty nine "invariant" amino acids surrounding the active site of serine proteases have been described. Of these, twenty-six are conserved in KLK-L3. One of the unconserved amino acids (Ser¹⁶⁸ instead of Pro) is also found in prostase, KLK-L2 and enamel matrix serine proteinase (EMSP1) genes. The second (Leu⁵⁸ instead of Val) is also found in TLSP and KLK-L2 genes, and the third is Ala²⁶ instead of Gly. According to protein evolution studies, each of these changed amino acids represents a conserved evolutionary change to a protein of the same group (45). Twelve cysteine residues are present in the putative mature KLK-L3 protein, ten of them are conserved in all the serine proteases that are aligned in Figure 17, and would be expected to form disulphide bridges. The other two (C¹³⁶ and C²³⁸) are not found in PSA, KLK1, KLK2 or trypsinogen; however, they are found in similar positions in prostase, HSCCE, zyme neuropsin, and TLSP genes and are expected to form an additional disulphide bond.

To predict the phylogenetic relatedness of the KLK-L3 gene with other serine proteases, the amino acid sequences of the kallikrein genes were aligned together using the "Clustal X" multiple alignment program and a distance matrix tree was predicted using the Neighbor joining/UPGMA method (Figure 18). Phylogenetic analysis separated the classical kallikreins (KLK1, KLK2, and PSA) and grouped KLK-L3 with TLSP, neuropsin, zyme, HSCCE and prostase/KLK-L1, consistent with previously published studies (11, 41).

### Tissue expression and hormonal regulation of the KLK-L3 gene

As shown in Figure 19, the KLK-L3 gene is primarily expressed in thymus, testis, spinal cord, cerebellum, trachea, mammary gland, prostate, brain, salivary gland, ovary and skin (the latter two tissues are not shown in the figure). Lower levels of expression are seen in fetal brain, stomach, lung, thyroid, placenta, liver, small intestine, and bone marrow. No expression was seen in uterus, heart, fetal liver, adrenal gland, colon, spleen, skeletal muscle, pancreas, and kidney. In order to verify the RT-PCR specificity, representative PCR products were cloned and sequenced. Figure 20 shows that KLK-L3 gene is regulated by steroid hormones in the human breast cancer cell line BT-474.

### DISCUSSION

A human kallikrein gene locus has been defined, and the first detailed map describing the relative positions of the kallikreins and other kallikrein-like genes has been constructed (Figure 14). This map is consistent with previous reports on the localization of the classical kallikreins and the approximate mapping of some new kallikreins by radiation hybrid and FISH techniques (9, 14, 67). It should be noted, however, that the lengths of certain segments of this map (as depicted in Figure 14) are dependent on the EcoR1 restriction map of the area and are measured in terms of approximate kb units. Also, the measure of intervals between genes may change slightly in the future, since some kallikreins may have extra 5'exon(s) that have not as yet been identified. Kallikreins with verified 5'-untranslated exons include NES1 (14), zyme, and neuropsin (35). This map is also directional; it indicates that PSA and KLK2 genes are transcribed in the same direction (centromere to telomere) and that the rest of the kallikrein-like genes are transcribed in the reverse direction (Figure 14).

An early report indicated that KLK1 is located approximately 31 kb centromeric to PSA (9). The map described extends only 24 kb centromeric to PSA, and for this reason, KLK1 was not precisely localized. Thus, the exact location of the KLK1 gene is still to be defined from linear chromosome 19 sequencing data. The possibility still exists that this locus is extended further, and that other kallikrein-like genes may be located upstream of PSA or downstream from TLSP.

Traditionally, kallikreins are characterized by their ability to liberate lysyl-bradykinin (kallidin) from kininogen (2). In humans, only KLK1 meets this "functional" definition. KLK2 and KLK3 are assigned to the same family based on the strong structural similarities of the genes and proteins and the close localization of these genes on the same chromosomal region (20). More recently, a new structural concept has emerged to describe kallikreins. Richards and co-workers introduced the concept of a "kallikrein multigene family" in mice, to refer to these genes (20, 21). This definition is not based much on the specific enzymatic function of the gene product, but more on its sequence homology and its close linkage on mouse chromosome 7.

Irwin et al. (27) proposed that the serine protease genes can be classified into five different groups according to intron position as discussed above.The results indicate the presence of some more common structural features that are found in all kallikreins (including the newly identified KLK-L3 gene) : (1) All genes are formed of 5 coding exons and 4 intervening introns (with the possibility that some genes may have extra 5' untranslated exon(s) (24, 31, 35) (Figure 21). (2) The exon lengths are usually comparable. (3) The intron phases are always conserved (I-II-I-0) (see Figure 21 for description of intron phases). (4) These genes are clustered in the same chromosomal region, apparently without any intervening non kallikrein-like genes (Figure 14). Thus, all the recently identified serine proteases that are present in this region (zyme, HSCCE, neuropsin, NES1, prostase/KLK-L1, KLK-L2 and TLSP), together with the newly identified kallikrein-like gene (KLK-L3), could be considered members of the expanded human kallikrein multigene family.

The chromosomal band 19q13 is nonrandomly rearranged in a variety of human solid tumors including ovarian cancers (46), and the currently available data indicate that the kallikrein gene locus is related to many malignancies. At least three kallikrein genes (PSA, zyme and NES1) are down regulated in breast cancer (10, 13, 47, 48), and NES1 appears to be a novel tumor suppressor gene (29). Furthermore, PSA exhibits potent antiangiogenic activity (49). It is possible that some of these kallikreins are involved in a cascade pathway, similar to the coagulation or apoptotic process, whereby pro-forms of proteolytic enzymes are activated and then act upon downstream substrates. Such activity was found for the KLK2 gene product which acts upon and activates pro PSA (50, 51).

The expanded human kallikrein gene family has similar number of members as the rodent family of genes. Some new compelling data have raised the possibility that at least some of these genes behave as tumor suppressors (29), as negative regulators of cell growth (52), as antiangiogenic (49) and apoptotic (53) molecules. The paramount diagnostic value of some members is also well-established (24, 54). For these reasons, it is important to check all members of this family of genes as potential diagnostic or prognostic markers or as candidate therapeutic targets.

The newly identified KLK-L3 gene is expressed in many tissues, including skin, thymus, central nervous system, breast, prostate, and testis. The wide range of tissue expression of KLK-L3 should not be surprising since, by using the more sensitive RT-PCR technique, many kallikrein genes were found to be expressed in a wide variety of tissues. For example, PSA, KLK2, prostase/KLK-L1, and KLK-L2 are now known to be expressed in breast and many other tissues (30, 54).

Like many other kallikreins. KLK-L3 is regulated by steroid hormones but in a more complex fashion than PSA and KLK2 which are up-regulated by androgens and progestins (71). In the breast carcinoma cell line studied, KLK-L3 appears to be up-regulated by progestins > estrogens > androgens (Figure 20).

### Example 5

### Materials and Methods

### DNA sequence on chromosome 19 and prediction of new genes

Sequencing data of approximately 300Kb of nucleotides, around chromosome 19q13.3-q13.4, was obtained from the web site of the Lawrence Livermore National Laboratory (LLNL) (http://www http://www-bio.llnl.gov/genome/genome.html) and an almost contiguous stretch of genomic sequences was constructed. A number of computer programs were used to predict the presence of putative new genes in this genomic area.

### Expressed sequence tag (EST) searching

The predicted exons of the putative new gene were subjected to homology search using the BLASTN algorithm (37) on the National Center for Biotechnology Information web server (http://www ncbi.nim.nih.gov/BLAST/) against the human EST database (dbEST). Clones with > 95% homology were obtained from the I.M.A.G.E. consortium (38) through Research Genetics Inc, Huntsville, AL. The clones were propagated, purified and sequenced from both directions with an automated sequencer, using insert-flanking vector primers.

### Rapid amplification of cDNA ends (3' RACE)

According to the EST sequence data and the predicted structure of the gene, two gene-specific primers were designed and two rounds of RACE reactions (nested PCR) were performed with 5µl Marathon Ready™ cDNA of human testis (Clontech, Palo Alto, CA, USA) as a template. The reaction mix and PCR conditions used were according to the manufacturer's recommendations.

### Tissue expression

Total RNA isolated from 26 different human tissues was purchased from Clontech. cDNA was prepared as described below, and used for PCR reactions with different sets of primers (Table 15, SEQ.ID.NOs. 46-55, 9-12). Tissue cDNAs were amplified at various dilutions.

### Breast cancer cell line and hormonal stimulation experiments

The breast cancer cell line BT-474 was purchased from the American Type Culture Collection (ATCC), Rockville, MD. Cells were cultured in RPMI media (Gibco BRL, Gaithersburg, MD) supplemented with glutamine (200 mmol/L), bovine insulin (10 mg/L), fetal bovine serum (10%), antibiotics and antimycotics, in plastic flasks, to near confluency. The cells were then aliquoted into 24-well tissue culture plates and cultured to 50% confluency. 24 hours before the experiments, the culture media were changed into phenol red-free media containing 10% charcoal-stripped fetal bovine serum. For stimulation experiments, various steroid hormones dissolved in 100% ethanol were added into the culture media, at a final concentration of 10⁻⁸ M. Cells stimulated with 100% ethanol were included as controls. The cells were cultured for 24 hours, then harvested for mRNA extraction.

### Reverse transcriptase polymerase chain reaction

Total RNA was extracted from the breast cancer tissues and cell lines using Trizol™ reagent (Gibco BRL) following the manufacturer's instructions. RNA concentration was determined spectrophotometrically. 2 µg of total RNA was reverse-transcribed into first strand cDNA using the Superscript™ preamplification system (Gibco BRL). The final volume was 20 µl. Based on the combined information obtained from the predicted genomic structure of the new gene and the EST sequences, two gene-specific primers were designed (L4-F1 and L4-R1, see Table 15, SEQ.ID.NOs. 46 and 47) and PCR was carried out in a reaction mixture containing 1 µl of cDNA, 10 mM Tris-HCl (pH 8.3), 50 mM KCl, 1.5 mM MgCl₂, 200 µM dNTPs (deoxynucleoside triphosphates), 150 ng of primers and 2.5 units of AmpliTaq Gold DNA polymerase (Roche Molecular Systems, Branchburg, NJ, USA) on a Perkin-Elmer 9600 thermal cycler. The cycling conditions were 94°C for 9 minutes to activate the Taq Gold DNA polymerase, followed by 43 cycles of 94°C for 30 s, 63°C for 1 minute and a final extension at 63°C for 10 min. Equal amounts of PCR products were electrophoresed on 2% agarose gels and visualized by ethidium bromide staining. All primers for RT-PCR spanned at least 2 exons to avoid contamination by genomic DNA.

To verify the identity of the PCR products, they were cloned into the pCR 2.1-TOPO vector (Invitrogen, Carlsbad, CA, USA) according to the manufacturer's instructions. The inserts were sequenced from both directions using vector-specific primers, with an automated DNA sequencer.

### Normal and malignant breast tissues

Normal breast tissues were obtained from women undergoing reduction mammoplasties. Breast tumor tissues were obtained from female patients at participating hospitals of the Ontario Provincial Steroid Hormone Receptor Program. The normal and tumor tissues were immediately frozen in liquid nitrogen after surgical resection and stored in this manner until extracted. The tissues were pulverized with a hammer at dry ice temperature and RNA was extracted as described above, using Trizol reagent.

### Structure analysis

Multiple alignment was performed using the Clustal X software package available at: ftp://ftp.ebi.ac.uk/pub/softwareldos/clustalw/clustalx/[clustalx1.64b.msw.exe] and the multiple alignment program available from the Baylor College of Medicine (BCM), Houston, TX, USA [kiwi.imgen.bcm.tmc. edu:8808/search-launcher/launcher/html]. Phylogenetic studies were performed using the Phylip software package available at: http://evolution.genetics. washington.edu/phylip/getme.html. Distance matrix analysis was performed using the "Neighbor-Joining/UPGMA" program and parsimony analysis was done using the "Protpars" program. Hydrophobicity study was performed using the BCM search launcher programs [http://dot.imgen.bcm.tmc.edu:9331/seq-search/struc-predict.html]. Signal peptide was predicted using the "SignalP" server [http://www.cbs. dtu.dk/services/ signal]. Protein structure analysis was performed by the "SAPS" (structural analysis of protein sequence) program [http://dot.imgen.bcm.tmc.edu: 9331/seq-search/struc-predict.html].

### Results

### Cloning of the KLK-L4 gene

Computer analysis of the genomic sequence around chromosome 19 q13.3-q13.4 predicted a putative new gene formed of at least 3 exons. To experimentally verify the existence of this gene, the putative exons were subjected to sequence homology search against the human expressed sequence tag (EST) database (dbEST), and four EST clones with > 97% homology were identified (Table 16). All ESTs were cloned from testicular tissue. These clones were obtained and inserts were sequenced from both directions. Sequences were then compared with the computer-predicted structure and final selection of the intron/exon splice sites was made according to the EST sequences.

As shown in Figure 22, three ESTs match almost perfectly with the predicted 3 exons (exons 3, 4, 5) of the gene and one EST matches perfectly with predicted exons 3 and 5. However, each of the ESTs extends further upstream with different exonic patterns, suggesting the presence of different splice variants. Attempts to translate these clone sequences demonstrated the presence, in some ESTs, of interrupting stop codons in all three possible reading frames. A homology search of the three common exons against the GenBank database revealed a cDNA sequence from the German Human Genome Project. This clone has an identical exon 2 as the long form of KLK-L4 gene [this form will be described below] but has an extended exon 3 that ends with a stop codon (Figure 22). This clone was isolated from uterine tissue and is translated by software into a truncated protein product of 196 amino acids which is followed by a 3' untranslated region [GenBank accession # AL050220].

Screening of cDNAs from 26 different tissues by RT-PCR, using gene-specific primers for exons 3 and 5 [L4-FI and L4-R1] (Table 15 & Figure 22) revealed that this gene is expressed in many tissues. Four tissues that show the highest level of expression [salivary gland, mammary gland, prostate, and testis] (Figure 23) and uterus [the EST clone AL050220 was isolated from this tissue] were selected for identification of the full structure of the gene. Different PCR reactions were performed using one reverse primer (L4-R1) together with each of the forward primers located in upstream exons that were found in the different EST clones [primers L4-B, L4-D, L4-E] (Table 15 & Figure 22). The PCR reactions were performed under different experimental conditions, using the EST clones as positive controls, and the PCR products were sequenced. None of these forms were found in any of the tissues, except in testis where all three forms were found.

By RT-PCR of the KLK-L4 gene using primers L4-R1 and L4-F1, it was found that the gene is expressed in a wide variety of tissues (Figure 23). In order to obtain the structural forms that exist in these tissues, a homology study was performed. Aligning the predicted polypeptide of the KLK-L4 gene with all other kallikreins and kallikrein-like genes, suggested, by homology, that at least two more exons should be present upstream of the predicted three exons. The genomic fragment upstream of the third exon was subjected to further computer analysis for gene prediction, and exon 2 was identified based on: a) a consensus exon/intron splice site b) preservation intron phase II after this exon, in agreement to intron phases of all other known kallikreins c) presence of the histidine residue of the catalytic triad (H⁷⁶) surrounded by a well-conserved peptide motif [see below] just before the end of this exon d) comparable exon length to other kallikrein genes. A potential first exon was also predicted from the upstream genomic sequence, based on the preserved intron phase (phase I), and the existence of an in-frame start codon that is located at a comparable distance [in relation to other kallikreins] from the end of this exon. To verify this predicted structure, a PCR reaction was performed using one reverse primer (L4-R1) together with another forward primer that is located in the predicted first exon (primer L4-X1) (Table 15 & Figure 22). Two main PCR bands were obtained from the tissues examined; the expected 819 bp band (predominant) and an additional minor band of about 650 bp (Figure 24). Cloning and sequencing of these two bands revealed that the gene exists in two main forms in these tissues; the long form [SEQ. ID. No. 43 or GenBank Accession No. AF135024] and another form [referred to as the short KLK-L4 variant] that utilizes an upstream alternative splice donor site, located inside exon 3, thus creating an mRNA product that that is 214 bp shorter. This alternative splice site causes frame-shifting of the coding region that will generate a predicted stop codon at the beginning of exon 4, giving rise to a truncated protein product that does not contain the serine residue of the catalytic triad (Figures 24 and 25).

Aligning the long KLK-L4 form with the ESTs (Figure 22) demonstrated that all ESTs utilize a different splice donor site located 80 bp downstream from the end of exon 3. These additional 80 bp contain an in-frame stop codon at nucleotide position 5505 which will lead to the formation of a shorter polypeptide product. They also utilize an alternative polyadenylation signal located at position 8706 [numbers refer to SEQ. ID. No. 43 or or GenBank Accession No. AF135024]. The clone from the German Genome Project utilizes another splice donor site that is located further downstream, inside intron 3, and ends up with a poly A tail without having a fourth or fifth exon. The same stop codon (position 5505) will be in-frame, and therefore, a truncated protein product is predicted to be formed (Figure 22).

In order to obtain the 3'end of the gene, a 3'RACE reaction was performed, and an additional 375 bp fragment of 3' untranslated region, downstream from PCR primer L4-R1, was obtained. This fragment was further confirmed to be present in all tissues tested, by performing a PCR reaction using primers L4-F1 and L4-R3 (Table 15 & Figure 22). This fragment ends with a putative polyadenylation signal variant (TATAAA).

### Structural characterization of the KLK-L4 gene and its protein product

The long form of the KLK-L4 gene is presented in Figure 25 (SEQ.ID.NO. 43). KLK-L4 is formed of five coding exons and four intervening introns, spanning an area of 8,905 bp of genomic sequence on chromosome 19q13.3-q13.4. The lengths of the coding regions are 52, 187, 269,137 and 189 bp, respectively. The predicted protein coding region of the gene is formed of 831 bp, encoding a deduced 277-amino acid protein with a predicted molecular mass of 30.6 kDa (Figure 25). The intron/exon splice sites (mGT....AGm, where m is any base) and their flanking sequences are in agreement with the consensus splice site sequence. A potential translation initiation codon is present at position 45 of the predicted first exon [numbers refer to SEQ. ID. No. 43]. The cDNA extends at least 382 bp further downstream from the stop codon and a putative polyadenylation signal (TATAAA) is present at the end of this region (Figure 25).

Hydrophobicity analysis revealed that the amino-terminal region is quite hydrophobic (Figure 26), consistent with the possibility that this region may harbor a signal sequence, analogous to other serine proteases. Figure 26 also shows the presence of several evenly distributed hydrophobic regions throughout the KLK-L4 polypeptide, which are consistent with a globular protein, similar to other serine proteases (13). Computer analysis of the amino acid sequence of KLK-L4 predicted a cleavage site between amino acids 20 and 21 (GVS-QE). Sequence homology with other serine proteases (Figure 27) predicted another potential cleavage site (Lys25) in close proximity. Most other kallikreins are activated by cleavage after arginine or lysine. Thus, the protein product is very likely to be a secreted protein. The dotted region in Figure 27 indicates an 11-amino acid loop characteristic of the classical kallikreins (PSA, KLK1, and KLK2) which is not found in KLK-L4 or other members of the kallikrein multi-gene family (11,13, 35).

Amino acid sequences for KLK-L4 proteins are shown in SEQ.ID.NO. 44 and 45.

Sequence analysis of eukaryotic serine proteases indicates the presence of twenty nine invariant amino acids (39). Twenty eight of them are conserved in the KLK-L4 protein and the remaining amino acid (Q182 instead of P) is not conserved among all other kallikreins (Figure 27). Ten cysteine residues are present in the putative mature KLK-L4 protein. These are conserved in all the serine proteases that are aligned in Figure 27, and would be expected to form disulphide bridges. The presence of aspartate (D) in position 239 suggests that KLK-L4 will possess a trypsin-like cleavage pattern, similarly to most of the other kallikreins [e.g., KLK1, KLK2, TLSP, neuropsin, zyme, prostase, and EMSP] but different from PSA which has a serine (S) residue in the corresponding position, and is known to have chymotrypsin like activity (Figure 27) (2,40).

### Mapping and chromosomal localization of the KLK-L4 gene

Alignment of the KLK-L4 gene and the sequences of other known kallikrein genes within the 300 Kb area of interest [the human kallikrein gene family locus], enabled precise localization of all known genes and to determine the direction of transcription, as shown by the arrows in Figure 28. The PSA gene lies between KLK1 and KLK2 genes and is separated by 13, 319 base pairs (bp) from KLK2, and both genes are transcribed in the same direction (centromere to telomere). All other kallikrein-like genes are transcribed in the opposite direction. KLK-L4 is 13 kb centromeric from KLK-L6 [SEQ.ID.NO. 65], and 21 kb more telomeric to KLK-L5 [SEQ. ID. NO. 56].

### Homology with the kallikrein multi-gene family

Alignment of the amino acid sequence of the KLK-L4 protein (long form) against the GenBank database and the known kallikreins, using the BLAST algorithm (37), indicated that KLK-L4 has 51% amino acid sequence identity with the TLSP and zyme genes, 49% identity with KLK-L2 and 47% and 45% identity with PSA and KLK2 genes, respectively. Multiple alignment study shows that the typical catalytic triad of serine proteases is conserved in the KLK-L4 gene (H¹⁰⁸, D¹⁵³, and S²⁴⁵) and, as is the case with all other kallikreins, a well conserved peptide motif is found around the amino acid residues of the catalytic triad [i.e. histidine (WLLTAAHC), serine (GDSGGP), and aspartate (DLMLI)] (Figure 27) (1, 11, 13, 35). In addition, several other residues were found to be fully or partially conserved among the human kallikrein gene family, as further shown in Figure 27. To predict the phylogenetic relatedness of the KLK-L4 gene with other serine proteases, the amino acid sequences of the kallikrein genes were aligned together using the "Clustal X" multiple alignment program and a distance matrix tree was predicted using the Neighbor-joining/UPGMA method (Figure 29). Phylogenetic analysis separated the classical kallikreins (KLK1, KLK2, and PSA) and grouped KLK-L4 with zyme, TLSP, KLK-L3, neuropsin, and NES1 genes, consistent with previously published studies (41) and indicating that this group of genes probably arose from a common ancestral gene by duplication.

### Tissue expression and hormonal regulation of the KLK-L4 gene

As shown in Figure 23, the KLK-L4 gene is primarily expressed in mammary gland, prostate, salivary gland and testis, but, as is the case with other kallikreins, lower levels of expression are found in many other tissues. In order to verify the RT-PCR specificity, the PCR products were cloned and sequenced.

A steroid hormone receptor-positive breast cancer cell line (BT-474) was used as a model, to verify whether the KLK-L4 gene is under steroid hormone regulation. PSA was used as a control gene, known to be up-regulated by androgens and progestins and pS2 as an estrogen up-regulated control gene in the same cell line. Preliminary results indicate that KLK-L4 is up-regulated by progestins and androgens and to a lower extent by estrogens (Figure 30).

### Expression of KLK-L4 in breast cancer tissues and cell lines

To characterize the extent and frequency of expression of the KLK-L4 gene in breast tumors, cDNA derived from 3 normal and 19 malignant breast tissues and 3 breast cancer cell lines was used. The data were interpreted by comparison of band intensities. Out of the 19 tumors, KLK-L4 gene expression was undetectable in 7, lower than normal tissues in 9, comparable to the normal tissues in 1, and higher than normal tissues in 2 tumors. Without hormonal stimulation, the BT-474 and T-47D cell lines had no detectable KLK-L4 mRNA, while the MCF-7 cell line was positive. These preliminary results suggest that this gene is down-regulated in the majority (16/19) of breast tumors.

### Discussion

The established kallikreins (KLK1, KLK2, and PSA), trypsinogen and chymotrypsinogen belong to a group that has: (1) an intron just downstream from the codon for the active site histidine residue, (2) a second intron downstream from the exon containing the codon for the active site aspartic acid residue, and (3) a third intron just upstream from the exon containing the codon for the active site serine residue. Figure 31 shows that KLK-L4 meets the above mentioned criteria; moreover, is located in close proximity to other kallikrein genes on the chromosomal locus 19q13.3-q13.4 (Figure 28).

The preliminary findings, supporting that the KLK-L4 gene may be down-regulated in a subset of breast cancers, is not surprising. There is now growing evidence that many of the kallikreins and kallikrein-like genes that are clustered in the same chromosomal region (Figure 28) are related to malignancy. PSA is the best marker for prostate cancer so far (24). A recent report provided evidence that PSA has antiangiogenic activity, and that this activity may be related to its function as a serine protease (49). This study suggested that other serine proteases, including new members of the kallikrein multigene family of enzymes, should also be evaluated for potential antiangiogenic activity (49). Recent reports suggest that hK2 (encoded by the KLK2 gene) could be another useful diagnostic marker for prostate cancer (57, S8). NES1 appears to be a tumor suppressor gene (29). The protease M gene was shown to be differentially expressed in primary breast and ovarian tumors (10), and the human stratum corneum chymotryptic enzyme has been shown to be expressed at abnormally high levels in ovarian cancer (59). Another recently identified kallikrein-like gene, located close to KLK-L4 and tentatively named tumor-associated, differentially expressed gene-14 (TADG14) [an alternatively spliced form of neuropsin, see Figure 28] was found to be overexpressed in about 60% of ovarian cancer tissues (59). Also, prostase/KLK-L1, another newly discovered kallikrein-like gene, is speculated to be linked to prostate cancer (41). Thus, extensive new literature suggests multiple connections of many kallikrein genes to various forms of human cancer.

The removal of intervening RNA sequences (introns) from the pre-messenger RNA in eukaryotic nuclei is a major step in the regulation of gene expression (60). RNA splicing provides a mechanism whereby protein isoform diversity can be generated and the expression of particular proteins with specialized functions can be restricted to certain cell or tissue types during development (60). The sequence elements in the pre-mRNA at the 5' and 3' splice sites in metazoans have very loose consensus sequence; only the first and the last two bases (GT..AG) of the introns are highly conserved (Sambrook, supra). These sequences cannot be the sole determinants of splice site selection, since identical, but not ordinarily active, consensus sequences can be found within both exons and introns of many eukaryotic genes. Other protein factors and sequences downstream of the splice sites are also involved.

The existence of multiple splice forms is frequent among kallikreins. Distinct RNA species are transcribed from the PSA gene, in addition to the major 1.6-kb transcript (61). Several distinct PSA transcripts have been described by Reigman et al (7). Interestingly, one of these clones lacks the 3' untranslated region and the first 373 nucleotides of the open reading frame, and has an extended exon that contains a stop codon, a pattern that is comparable with some alternative forms of the KLK-L4 cDNA, as described here (Figure 22). Heuze et al., reported the cloning of a full-length cDNA corresponding to a 2.1 kb PSA mRNA. This form results from the alternative splicing of intron 4 and lacks the serine residue that is essential for catalytic activity (61). Also, Reigman et al reported the identification of two alternatively spliced forms of the human glandular kallikrein 2 (KLK2) gene (62). A novel transcript of the tissue kallikrein gene (KLK1) was also isolated from the colon (63). Interestingly, this transcript lacks the first two exons of the tissue kallikrein gene, but the last three exons were fully conserved, a pattern that is similar to the findings with some ESTs containing parts of the KLK-L4 gene (Figure 22). Neuropsin, a recently identified kallikrein-like gene, was found to have two alternatively spliced forms, in addition to the major form (59, 64). Here, the cloning of the KLK-L4 gene is described and the identification of a number of alternative mRNA forms. These forms may result from alternative-splicing (Sambrook, supra), retained intronic segment (7), or from the utilization of an alternative transcription initiation site (63). Because the long form of KLK-L4 and the major alternative splice variant [short KLK-L4 variant] (Figure 24) have an identical 5' sequence required for translation, secretion and activation, it is possible to assume that both mRNAs encode for a secreted protein (61).

In order to investigate the relative predominance of the long KLK-L4 and related forms, cDNA from various tissues was amplified by PCR. Although, in general, it is difficult to use PCR for quantitative comparisons between mRNA species, in this experiment, [mRNAs of comparable sizes, using one set of primers under identical conditions], such a comparison is reasonable (62). In all five normal tissues examined [breast, prostate, testis, salivary gland and uterus] the long form of KLK-L4 was the predominant, with minimal level of expression of the short form (Figure 24).

The presence of alternatively spliced forms may be related to malignancy. Recent literature suggests that distinct molecular forms of PSA could be expressed differently by malignant versus benign prostate epithelium (65). Aberrant PSA mRNA splicing in benign prostatic hyperplasia, as opposed to prostate cancer, has been described by Henttu et al (66). In addition, it has been recently postulated that different prostatic tissues potentially harboring occult cancer could account for the presence of various forms of PSA (65).

### Example 6

### Materials and Methods

### DNA sequence on chromosome 19

Sequencing data of approximately 300Kb of nucleotides on chromosome 19q13.3-q13.4 was obtained from the web site of the Lawrence Livermore National Laboratory (LLNL) (http://www-bio.llnl.gov/genome/genome.html), This sequence was in the form of 9 contigs of different lengths. Restriction enzyme analysis, long PCR strategies, followed by DNA sequencing, BAC and PAC library screening and end sequencing of selected clones, were used to construct a contiguous genomic region, representing the complete human kallikrein gene locus.

### New gene identification

A number of computer programs were used to predict the presence of putative new genes within the contiguous genomic area of interest. The ability of these programs for predicting new genes was first examined by using the genomic sequences of the known kallikreins as testing parameters. The most reliable computer programs; GeneBuilder (gene prediction) (http://125.itba.rni.cnr.it/ ~webgene/genebuilder.html). GeneBuilder (exon prediction) (http://125.itba.mi.cnr.it/-webgene/genebuilder.html), Grail 2 (http://compbio.ornl.gov), and GENEID-3 (http://apolo.imim.es/geneid.html) were selected for further use.

### Expressed sequence tag (EST) searching

The predicted exons of the putative new gene were subjected to homology search using the BLASTN algorithm (37) on the National Center for Biotechnology Information web server (http://www ncbi.nlm.nih.gov/BLAST/) against the human EST database (dbEST). A clone with > 95% homology was obtained from the I.M.A.G.E. consortium (38) through Research Genetics Inc, Huntsville, AL. This clone was propagated, purified and sequenced from both directions with an automated sequencer, using insert-flanking vector primers.

### Rapid amplification of cDNA ends (RACE)

According to the EST sequence and the predicted structure of the gene, two sets of gene-specific primers were designed for 5' and 3' RACE reactions. Two rounds of RACE reactions (nested PCR) were performed for each type of RACE with 5µl Marathon Ready™ cDNA of human testis and prostate (Clontech, Palo Alto, CA, USA) as templates. The reaction mix and PCR conditions were selected according to the manufacturer's recommendations. In brief, the initial denaturation was for 5 min at 94°C. followed by 94°C for 5 s and 72°C for 2 min, for 5 cycles; then, 94°C for 5 s and 70° C for 2 min, for 5 cycles; then, 94°C for 5 s and 65°C for 2 min for 30 cycles for the first reaction and 25 cycles for the nested PCR reaction.

### Tissue expression

Total RNA isolated from 26 different human tissues was purchased from Clontech, Palo Alto, CA. cDNA was prepared as described below for the tissue culture experiments and used for PCR reactions. After aligning all known kallikrein genes, two primers (KLK-L5-R1 and KLK-L5-F1) (Table 17, SEQ.ID.NOs. 61-64, 9-12, and Figure 32) were designed from areas with relatively low homology. Tissue cDNAs were amplified at various dilutions. Due to the high degree of homology between kallikreins, and to exclude non-specific amplification, PCR products were cloned and sequenced.

### Normal and malignant breast tissues

Normal breast tissues were obtained from women undergoing reduction mammoplasties. Breast tumor tissues were obtained from female patients at participating hospitals of the Ontario Provincial Steroid Hormone Receptor Program. The normal and tumor tissues were immediately frozen in liquid nitrogen after surgical resection and stored in this manner until extracted. The tissues were pulverized with a hammer under liquid nitrogen and RNA was extracted as described below, using Trizol reagent.

### Breast and prostate cancer cell lines and hormonal stimulation experiments

The breast cancer cell lines BT-474 and T-47D, and the LNCaP prostate cancer cell line were purchased from the American Type Culture Collection (ATCC), Rockville, MD. Cells were cultured in RPMI media (Gibco BRL, Gaithersburg, MD) supplemented with glutamine (200 mmol/L), bovine insulin (10 mg/L), fetal bovine serum (10%), antibiotics and antimycotics, in plastic flasks, to near confluency. The cells were then aliquoted into 24-well tissue culture plates and cultured to 50% confluency. 24 hours before the experiments, the culture media were changed into phenol red-free media containing 10% charcoal-stripped fetal bovine serum. For stimulation experiments, various steroid hormones dissolved in 100% ethanol were added into the culture media at a final concentration of 10⁻⁸ M. Cells stimulated with 100% ethanol were included as controls. The cells were cultured for 24 hours, then harvested for mRNA extraction.

### Reverse transcriptase polymerase chain reaction (RT-PCR)

Total RNA was extracted from the cell lines or tissues using Trizol reagent (Gibco BRL) following the manufacturer's instructions. RNA concentration was determined spectrophotometrically. 2 µg of total RNA was reverse-transcribed into first strand cDNA using the Superscript™ preamplification system (Gibco BRL). The final volume was 20 µl. Based on the combined information obtained from the predicted genomic structure of the new gene and the EST sequences, two gene-specific primers were designed (KLK-L5-F1 and KLK-L5-R1) (Table 17) and PCR was carried out in a reaction mixture containing 1 µl of cDNA, 10 mM Tris-HCl (pH 8.3), 50 mM KCl, 1.5 mM MgCl₂, 200 µM dNTPs (deoxynucleoside triphosphates), 150 ng of primers and 2.5 units of AmpliTaq Gold DNA polymerase (Roche Molecular Systems, Branchburg, NJ, USA) on a Perkin-Elmer 9600 thermal cycler. The cycling conditions were 94°C for 9 minutes to activate the Taq Gold DNA polymerase, followed by 43 cycles of 94°C for 30 s, 63°C for 1 minute and a final extension step at 63°C for 10 min. Equal amounts of PCR products were electrophoresed on 2% agarose gels and visualized by ethidium bromide staining. All primers for RT-PCR spanned at least 2 exons to avoid contamination by genomic DNA.

To verify the identity of the PCR products, they were cloned into the pCR 2.1-TOPO vector (Invitrogen, Carlsbad, CA, USA) according to the manufacturer's instructions. The inserts were sequenced from both directions using vector-specific primers, with an automated DNA sequencer.

### Structure analysis

Multiple alignment was performed using the Clustal X software package available at: ftp://ftp.ebi.ac.uk/pub/software/dos/ciusta)w/c)usta)x/ (clustalx1.64b.msw.exe) and the multiple alignment program available from the Baylor College of Medicine (BCM), Houston, TX, USA (kiwi.imgen.bcm.tmc. edu:8808/search-launcher/launcher/html). Phylogenetic studies were performed using the Phylip software package available at: http://evolution.genetics. washington.edu/phyiip/getme.htrnl. Distance matrix analysis was performed using the "Neighbor-Joining/UPGMA" program and parsimony analysis was done using the "Protpars" program. Hydrophobicity study was performed using the BCM search launcher programs (http://dot.imgen.bcm.tmc.edu:9331/seq-search/struc-predict.html). Signal peptide was predicted using the "SignalP" server (http://www.cbs.dtu.dk/services/ signal). Protein structure analysis was performed by "SAPS" (structural analysis of protein sequence) program (http://dot.imgen.bcm.tmc.edu:9331/seq-search/struc-predict.html).

### RESULTS

### Identification of the KLK-L5 gene

Computer analysis of the genomic area of interest (300Kb around chromosome 19q 13.3-q 13.4) predicted a putative gene comprised of at least three exons. Screening of the human expressed sequence tag (EST) database revealed an EST clone (GenBank Accession #394679) with 99% homology with the predicted exons. This clone was obtained, purified, and sequenced. The full-length sequence of the EST was compared with the genomic area containing the putative new gene and showed 100% homology with certain areas (exons), which were separated by introns. This alignment indicated that the new gene was comprised of 7 exons. Sequence homology comparisons and phylogenetic analysis revealed that this new gene is structurally similar to known kallikreins and other serine proteases (see below). Since four other new kallikrein-like genes were discovered in this area, this gene was named KLK-L5 (for kallikrein-like gene 5). Attempts to translate the coding region in all three possible reading frames indicated that only one reading frame will produce a full-length polypeptide chain without interrupting in-frame stop codons. Further support for the correctness of this reading frame was obtained by demonstrating that only this frame will preserve the three amino acid residues necessary for serine protease activity (catalytic triad) and the conserved motifs around them. An in-frame methionine start codon was found in the second exon. This start codon falls within a typical consensus Kozak sequence (CCACCATGG) (33). Thus, the gene will have at least one 5' untranslated exon, similarly to other kallikrein-like genes [e.g. zyme, the normal epithelial cell-specific 1 gene (NES1) (14), and neuropsin (35)]. 5' and 3' RACE reactions were performed in order to obtain the 5' and 3' ends of the gene. No more sequence was obtained by 5' RACE. However, 3' RACE enabled identification of the 3' untranslated region of the gene. The additional sequence ends with a poly-A stretch that does not exist in the genomic structure, thus marking the 3' end of the gene and the start of the poly-A tail.

### Splice variants of the KLK-L5 gene

Homology analysis of the KLK-L5 gene with other kallikreins revealed the presence of an additional 3' exon, an observation that has not been reported for any other member of the kallikrein multigene family. Furthermore, two different PCR bands were obtained with the 3' RACE. Sequencing of these bands revealed that this gene has at least two splice forms at its 3' end; one form in which the last exon is a single continuous fragment, and another form in which the last exon is split into two exons, with an intervening intron. In order to identify the full structure of other possible splice variants of the gene, PCR was performed using two primers (L5-F2 and L5-R2) (Table 17 and Figure 32). cDNA from 26 different tissues were used as templates and the reaction was performed under different experimental conditions (annealing temperature, MgCl₂ concentration). Three distinct bands were observed in many tissues. These bands were excised, gel-purified, and sequenced. As shown in Figure 32, the KLK-L5 gene was found to have 3 molecular forms:
1) One form (referred to, from now on, as the "classical" form) represents a typical kallikrein-like serine protease with five coding exons and four intervening introns (Figure 32). As is the case with some other kallikreins, a 5' untranslated exon is also present, and the possibility of further upstream untranslated exon(s) could not be excluded. Exons 1, 2 and 3 were present at the aforementioned EST. The start codon is present in the second exon (numbers refer to SEQ.ID.NO. 56 or GenBank Accession # AF135025). The stop codon is located in the sixth exon, followed by a 3' untranslated region, and a typical polyadenylation signal (AATAAA) is located 16 bp before the poly-A tail (Figure 33). This form of KLK-L5 spans a genomic length of 5,801 bp on chromosome 19q13.3-q13.4. The lengths of the coding regions of the exons are 37, 160, 260, 134, and 156 bp, respectively (Figures 33 and 34). The predicted protein-coding region is formed of 747 bp, encoding a deduced 248-amino acid protein with a predicted molecular mass of 26.7 kDa. The intron/exon splice sites (GT....AG) and their flanking sequences are in agreement with the consensus splice site sequence.
2) The second mRNA form, encoding the KLK-L5-related protein-1, is an alternatively spliced form in which the last exon is split into two separate exons with an additional intervening intron (Figure 32). This splitting of the last exon results in the utilization of another stop codon at position 9,478, thus creating a deduced 254-amino acid protein that is 6 amino acids longer than the "classical" KLK-L5 form and its carboxyterminal end is different in sequence by 19 amino acids (Figure 32). This variant has a predicted molecular mass of 27.1 kDa (for base numbering please see SEQ.ID.NO. 56 and GenBank Accession # AF135025).
3) The third mRNA form, encoding for KLK-L5-related protein-2, is similar to the classical form except that the fourth exon is missing (Figure 32). This leads to frame shifting of the coding region, and an earlier in-frame stop codon will be encountered at position 9,180. The protein-coding region of this form consists of 336 bp, encoding for a predicted 111-amino acid protein with a molecular mass of 12 kDa. This protein will lack both the serine and aspartate residues characteristic of serine proteases.

Amino acid sequences for KLK-L5 proteins are shown in SEQ. ID. NOs. 57 to 60.

### Structural analysis of the classical KLK-L5 gene

Figure 35 shows a comparative hydrophobicity analysis of the KLK-L5 and the prostate-specific antigen (PSA) proteins. The amino terminal regions of both genes are quite hydrophobic, indicating that this region of KLK-L5 is possibly harboring a signal peptide analogous to PSA. Figure 35 also shows the presence of several evenly distributed hydrophobic regions throughout the KLK-L5 polypeptide, which are consistent with a globular protein, similar to other serine proteases (13). Figure 36 shows the alignment of KLK-L5 protein with another 10 members of the same gene family. The dotted region in Figure 36 indicates an 11-amino acid loop characteristic of the classical human kallikreins (PSA, hK1 and hK2) but not found in KLK-L5 protein or other members of the kallikrein multigene family (11, 13, 35). Sequence analysis of eukaryotic serine proteases indicates the presence of twenty nine invariant amino acids (39). Twenty eight of them are conserved in the KLK-L5 polypeptide and the remaining amino acid (S156 instead of P) is not conserved among all other kallikreins (Figure 36). Twelve cysteine residues are present in the putative mature KLK-L5 protein, ten of them are conserved in all kallikreins, and the remaining two (C133, and C235) are present in most of the other kallikrein-like proteins but not in the classical kallikreins and they are expected to form an additional disulphide bridge (Figure 36).

The presence of aspartate (D) at position 194 suggests that KLK-L5 will possess a trypsin-like cleavage pattern, similarly to most of the other kallikreins (e.g., hK1 hK2, TLSP, neuropsin, zyme, prostase, and EMSP) but different from PSA, which has a serine (S) residue in the corresponding position, and is known to have chymotrypsin like activity (Figure 36) (54).

### Homology with other members of the kallikrein multigene family

Although the protein encoded by the KLK-L5 gene is unique, it has a high degree of homology with the other kallikrein-like genes. The KLK-L5 protein (classical form) has 48% amino acid sequence identity and 57% overall similarity with neuropsin, 46% identity with the normal epithelial cell-specific 1 gene product (NES1) and 38% identity with both PSA and hK2 proteins. Multiple alignment shows that the typical catalytic triad of serine proteases is conserved in the KLK-L5 protein (H⁶², D¹⁰⁸, and S²⁰⁰) (Figures 33 and 36). In addition, a well-conserved peptide motif is found around the amino acid residues of the catalytic triad as is the case with other serine proteases [i.e., histidine (VLTAA*H*C), serine (GDSGGP), and aspartate (*D*LRLL)] (11, 12) (Figure 36). Figure 36 also shows other amino acid residues that are completely conserved between kallikreins and kallikrein-like proteins. To predict the phylogenetic relatedness of the KLK-L5 protein with other serine proteases, the amino acid sequences of the kallikrein proteins were aligned together using the "Clustal X" multiple alignment program and a distance matrix tree was predicted using the Neighbor-joining/UPGMA and Protpars parsimony methods. Figure 37 shows the phylogenetic analysis which separated the classical kallikreins (hK1, hK2, and PSA) and clustered KLK-L5 with NES1 and neuropsin proteins in a separate group away from other serine proteases, consistent with previously published studies (27, 41) and indicating that this group of genes probably arose from a common ancestral gene, by gene duplication.

### Tissue expression and hormonal regulation of the KLK-L5 gene

As shown in Figure 38, the KLK-L5 gene is primarily expressed in the salivary gland, stomach, uterus, trachea, prostate, thymus, lung, colon, brain, breast and thyroid gland, but, as is the case with other kallikreins, lower levels of expression are found in some other tissues (testis, pancreas, small intestine, spinal cord). In order to verify the RT-PCR specificity, the PCR products were cloned and sequenced. The three splice forms of the gene were expressed in most of these tissues. However, the relative abundance of each form was different among tissues (Figure 38).

In order to investigate whether the KLK-L5 gene is under steroid hormone regulation, two breast cancer cell lines (BT-474 and T-47D) and a prostate cancer cell line (LNCaP) were used as models. In LNCaP cells, the gene was up-regulated only by androgen and progestin. Only in this cell line all 3 isoforms were expressed. In BT-474 cells, KLK-L5 was found to be up-regulated, at the mRNA level, by estrogen and androgen, and to a lesser extent by the progestin. The rank of potency was estrogen>androgen>progestin. However, the rank of potency for the T-47D cell line was androgen>progestin>estrogen . In both of these cell lines, only the short isoform (related protein-2) was present (Figure 39).

### KLK-L5 is down regulated in breast cancer

Expression of the KLK-L5 gene, at the mRNA level, was compared between seventeen breast cancer tissues and one normal breast tissue, by RT-PCR. The gene is not expressed at all in 12 tumors (Figure 40). In all breast tissues (normal and malignant) the short isoform (related protein-2) was predominant, with the exception of one tumor, which expressed only the classical form (Figure 40, lane 8). These results should be interpreted as preliminary, since the number of tumors and normal tissues tested is relatively small.

### Mapping and chromosomal localization of the KLK-L5 gene

The knowledge of extensive genomic sequence on chromosome 19q13.3-q13.4 enabled the precise localization of 14 kallikrein-like genes and determination of the direction of transcription, as shown by the arrows in Figure 28. Only PSA and KLK2 transcribe from centromere to telomere; the rest of the genes are transcribed in the reverse direction. The KLK1 gene was found to be the most centromeric, and the KLK-L6 gene the most telomeric (KLK-L6; SEQ.ID. NO.65). KLK-L5 is 21.3 Kb centromeric to KLK-L4 (SEQ.ID.NO. 43) and 1.6 kb more telomeric to the trypsin-like serine protease gene (TLSP) (Figure 28).

### DISCUSSION

As shown in Figure 34, kallikreins are characterized by the following common structural features: (a) All genes are formed of 5 coding exons and 4 intervening introns **[**some genes may have extra 5( untranslated exon(s)] (14, 35). (b) The exon lengths are usually comparable. (c) The intron phases are always conserved (I-II-1-0) (see legend of Figure 34 for definition of intron phases). (d) These genes are clustered in the same chromosomal region, without any intervening non-kallikrein-like genes (Figure 28). (e) The histidine residue of the catalytic triad of serine proteases is located near the end of the second coding exon; the aspartate residue in the middle of the third coding exon; and serine at the beginning of the fifth coding exon. As shown in Figure 34, all these criteria apply to the newly identified KLK-L5 gene. Thus, KLK-L5 should be considered a new member of the kallikrein multigene family.

Serine proteases and kallikreins are synthesized as "preproenzymes" that contain an N-terminal signal peptide (pre-zymogen), followed by a short activation peptide and the enzymatic domain (41, 54). PreproPSA has 24 additional residues that constitute the pre-region (signal peptide, 17 residues), and the propeptide (7 residues) (67). The signal peptide directs the protein to and across the endoplasmic reticulum (ER). In the ER, the signal peptide is removed and the resulting proPSA is transported to the plasma membrane, where it is secreted. The hydrophobicity study (Figure 35) indicates that the amino terminal region of the KLK-L5 protein is harboring a signal peptide. Also, computer analysis of the amino acid sequence of KLK-L5 predicted a cleavage site between amino acids 17 and 18 (SQA-AT). Thus, the protein product is very likely a secreted protein.

The presence of alternatively spliced forms is not a unique feature of the KLK-L5 gene; several other kallikreins are known to be expressed in various alternatively spliced forms. In addition to the major 1.6-kb transcript, several RNA species are transcribed from the PSA gene (61). Furthermore, others (69, 70) have described several PSA isoforms. Retained introns and loss of complete exons have been reported in some of these forms. In addition, Riegman et al. reported the identification of two alternatively spliced forms of the human glandular kallikrein (KLK2) gene (62) and Liu et al. isolated three alternative forms of the same gene (68). A novel transcript of the tissue kallikrein gene was isolated from the colon (63). Neuropsin, a recently identified kallkrein-like gene, was found to have two alternatively spliced forms in addition to the major form (35, 64). Here, the cloning of the classical kallikrein form and two unique splice forms of the KLK-L5 gene are described. Because the classical form and the splice forms all have the same 5' sequence required for translation, secretion and activation as do other kallikreins, i.e. a 5' leader sequence, a signal peptide, and a proregion, it is reasonable to assume that all three mRNA forms should produce a secreted protein. Preliminary findings identifying forms of KLK-L5 predominant in certain tissues are presented in Figures 35 and 40.

The preliminary results indicate that KLK-L5 is up-regulated by steroid hormones in breast and prostate cancer cell lines (Figure 39). These results are not surprising, since many other kallikrein genes are also regulated by steroid hormones. The differences in the rank of potency of steroid hormones among different cell lines could be attributed to differences in the abundance of the steroid hormone receptors between them as described elsewhere.

In conclusion, a new member of the human kallikrein gene family, KLK-L5, has been characterized which maps to the human kallikrein locus (chromosome 19q13.3-q13.4). This gene has two related splice forms in addition to the main form. KLK-L5 is expressed in a variety of tissues, appears to be down-regulated in breast cancer and its expression is influenced by steroid hormones. Since a few other kallikreins are already used as valuable tumour markers, KLK-L5 may also find a similar clinical application.

### Example 7

Using the Materials and Methods substantially as set out in Example 6, the present inventors identified another novel gene of the kallikrein multigen family referred to as KLK-L6. The full structure of the KLK-L6 gene is shown in Figure 41. Exons 1, 2, 3, 4, 5, 6, and 7 are at nucleic acids 1172-1281; 2561-2695; 2781-2842, 3714-3885; 5715-5968; 6466-6602; and 7258-7520. The nucleic acid sequence of the KLK-L6 gene is also shown in SEQ.ID.NO. 65 and amino acid sequences for the KLK-L6 protein are shown in SEQ.ID. Nos. 66 and 67. (See also GenBank Accession # AF161221).

Figure 42 shows a comparative hydrophobicity analysis of KLK-L6 and the prostate-specific antigen (PSA). The amino terminal regions of both genes are quite hydrophobic indicating that this region of KLK-L6 is possibly harboring a signal peptide analogous to PSA

Multiple alignment of KLK-L6 was carried out using the Clustal X software program as described herein (Figure 43).

To predict the phylogenetic relatedness of the KLK-L6 protein with other serine proteases, the amino acid sequences of the kallikrein proteins were aligned together using the "Clustal X" multiple alignment program and a distance matrix tree was predicted using the Neighbor-joining/UPGMA and Protpars parsimony methods. Figure 44 shows the phylogenetic analysis which separated the classical kallikreins (hK1, hK2, and PSA) and placed KLK-L6 in a separate group.

Having illustrated and described the principles of the invention in a preferred embodiment, it should be appreciated to those skilled in the art that the invention can be modified in arrangement and detail without departure from such principles. All modifications coming within the scope of the following claims are claimed.

All publications, patents and patent applications referred to herein are incorporated by reference in their entirety to the same extent as if each individual publication, patent or patent application was specifically and individually indicated to be incorporated by reference in its entirety.

### FULL CITATIONS FOR REFERENCES REFERRED TO IN THE SPECIFICATION

1. Evans BAE, Yun ZX, Close JA, Tregear GW, Kitamura N, Nakanishi S, et al. Structure and chromosomal localization of the human renal kallikrein gene. Biochemistry 1988;27:3124-3129.
2. Clements JA. The glandular kallikrein family of enzymes: Tissue-specific and hormonal regulation. Endocr Rev 1989;10:393-419.
3. Evans BA, Drinkwater CC, Richards RI. Mouse glandular kallikrein genes: Structure and partial sequence analysis of the kallikrein gene locus. J Biol Chem 1987;262:8027-8034.
4. Drinkwater CC, Evans BA, Richards RI. Kallikreins, kinins and growth factor biosynthesis. Trends Biochem Sci 1988b;13:169-172.
5. Ashley PL, MacDonald RJ. Tissue-specific expression of kallikrein-related genes in the rat. Biochemistry 1985;24:4520-5427.
6. Gerald WL, Chao J, Chao L. Sex dimorphism and hormonal regulation of rat tissue kallikrein mRNA. Biochim Biophys Acta 1986;867:16-23.
7. Riegman PHJ, Vlietstra RJ, van der Korput JAGM, Romijn JC, Trapman J. Characterization of the prostate-specific antigen gene: A novel human kallikrein-like gene. Biochem Biophys Res Commun 1989;159:95-102.
8. Schedlich LJ, Bennetts BH, Morris BJ. Primary structure of a human glandular kallikrein gene. DNA 1987;6:429-437.
9. Riegman PH, Vlietstra RJ, Suurmeijer L, Cleutjens CBJM, Trapman J. Characterization of the human kallikrein locus. Genomics 1992;14:6-11.
10. Anisowicz A, Sotiropoulou G, Stenman G, Mok SC, Sager R. A novel protease homolog differentially expressed in breast and ovarian cancer. Mol Med 1996;2:624-636.
11. Little SP, Dixon EP, Norris F, Buckley W, Becker GW, Johnson M, et al. Zyme, a novel and potentially amyloidogenic enzyme cDNA isolated from Alzheimer's disease brain. J Biol Chem 1997;272:25135-25142.
12. Yamashiro K, Tsuruoka N, Kodama S, Tsujimoto M, Yamamura Y, Tanaka T, et al. Molecular cloning of a novel trypsin-like serine protease (neurosin) preferentially expressed in brain. Biochim Biophys Acta 1997;1350:11-14.
13. Liu XL, Wazer DE, Watanabe K, Band V. Identification of a novel serine protease-like gene, the expression of which is down-regulated during breast cancer progression. Cancer Res 1996;56:3371-3379.
14. Luo L, Herbrick J-A. Scherer SW, Beatty B, Squire J, Diamandis EP. Structural characterization and mapping of the normal epithelial cell-specific 1 gene. Biochem Biophys Res Commun 1998;247:580-586.
15. Milanesi L, Koichanov N, Rogozin I, Kel A, Titov I. Sequence functional inference. In: "Guide to human genome computing", ed. M.J. Bishop, Academic Press, Cambridge, 1994, 249-312.
16. Burset M, Guigo R. Evaluation of gene structure prediction programs. Genomics 1996;34:353-367.
17. Nadeau J, Grant P, Kosowsky M. Mouse and human homology map. Mouse Genome 1991;89:31-36.
18. Schachter M. Kallikreins (kininogenases) - a group of serine proteases with bioregulatory actions. Pharmacol Rev 1980;31:1-17.
19. Morris BJ, Catanzaro DF, Richards RI, Mason AJ, Shine J. Kallikrein and renin: Molecular biology and biosynthesis. Clin Sci 1981;61:351s-353s.
20. Richards RI, Catanzaro DF, Mason AJ, Morris BJ, Baxter JD, Shine J. Mouse glandular kallikrein genes. Nucleotide sequence of cloned cDNA coding for a member of the kallikrein arginyl estero-peptidase group of serine proteases. J Biol Chem 1982;257:2758-2761.
21. Van Leeuwen BH, Evans BA, Tregear GW, Richards RI. Mouse glandular kallikrein genes. Identification, structure and expression of the renal kallikrein gene. J Biol Chem 1986; 261:5529-5535.
22. Evans BA, Richards RI. Genes for the α and γ subunits of mouse nerve growth factor. EMBO J 1985; 4:133-138.
23. Rogozin IB, Milanesi L, Kolchanov NA. Gene structure prediction using information on homologous protein sequence. Comput Applic Biosci 1996;12:161-170.
24. Diamandis, E.P. Prostate specific antigen-its usefulness in clinical medicine. Trends Endocrinol. Metab., 9: 310-316, 1998.
25. Diamandis, E. P., Yu H., and Sutherland, D.J. Detection of prostate-specific antigen immunoreactivity in breast tumours. Breast Cancer Res.Treat., 32: 301-310, 1994
26. Ishikawa, T., Kashiwagi, H., Iwakami, Y., et al. Expression of alpha-fetoprotein and prostate specific antigen genes in several tissues and detection of mRNAs in normal circulating blood by reverse trancriptase-polymerase chain reaction. Jpn. J. Oncol., 28:723-728, 1998.
27. Irwin, D.M., Robertson, K.A., and MacGillivary, R.T. J.Mol.Biol.212:31-45, 1988.
28. Yoshida, S., Taniguchi, M., Hirata, A., and Shiosaka, S. Sequence bovine prothrombin gene. J. Mol. Biol., 212: 31-45, 1988.
29. Goyal, J., Smith, K.M., Cowan, J.M., et al. The role of NES1 serine protease as a novel tumor suppressor. Cancer Res., 58: 4782-4786, 1998.
30. Diamandis, E.P., and Yu, H. New biological functions of prostate specific antigen? J. Clin. Endocrinol. Metab., 80 : 1515-1517, 1995.
31. Reifenberger, J., reifenberger, G., Liu, L., James, C.D. et al. Molecular genetic analysis of oligodendroglial tumors shows preferential allelic deletions on 19q and 1p.Am. J. Pathol.,145: 1175-90,1994.
32. Iida, Y. (1990). Quantification analysis of 5'-splice signal sequence in mRNA precursors. Mutations in 5'-splice signal sequence of human β-globin gene and β-thalassemia. J. Theor. Biol. 145:523-533.
33. Kozak, M. (1991). An analysis of vertebrate mRNA sequences: Intimations of translational control. J. Cell Biol. 115: 887-892.
34. Clements, J. (1997). The molecular biology of the kallikreins and their roles in inflamation. In: S. Farmer (ed.), The kinin system, pp. 71 -97. New York: Academic Press.
35. Yoshida, S., Taniguchi, M., Hirata, A., and Shiosaka, S. (1998). Sequence analysis and expression of human neuropsin cDNA and gene. Gene 213 :9-16.
36. Takayama, T. K, Fujikawa, K., Davie, E. W. (1997). Characterization of the precursor of prostate-specific antigen. Activation by trypsin and by human glandular kallikrein. J. Biol. Chem. 272: 21582-21588.
37. Altschul, S.F. et al., Nucleic Acids Res. 25: 3389-3402, 1997.
38. Lennon, G. et al, Genomics 33: 151-152, 1996.
39. Dayhoff, M, O., Natl. Biomed. Res. Found. 5 (Suppl 3) 79-81, 1998.
40. Simmer, J.P., et al, J. Dent. Res. 77:377-386, 1998.
41. Nelson, P.S. et al, PNAS 96: 3114-3119, 1999.
42. Osoegawa, K. et al, Genomics 52: 1-8, 1999.
43. Kozak, M., Nucleic Acid Res. 15: 8125-8148, 1987.
44. Proudfoot, N.J. and Brownlee, C.G., Nature 263: 211-214, 1976.
45. Miyata, T. et al, J. Mol Evol. 12: 219-236, 1979.
46. Mitelman, F., Catalog of Chromosome Aberrations in Cancer, 5th ed. Wiley-Liss, New York, pp. 3067-3198.
47. Yu, H. et al, Clin. Cancer res. 4: 1489-1497, 1998.
48. Sauter, E.R., Cancer Epidemol. Biomarkers Prev. 5:967-970, 1996.
49. Fortier, A.H. et al, J. Natl. Cancer Inst. 91: 1635-1640, 1999.
50. Kumar, A., 1998, Cancer res. 57: 3111-3114, 1997.
51. Lovgren, J. Biochem. Biophys. Res. Commun. 238: 549-555, 1987.
52. Lai, L.C. et al, Int. J. Cancer 66: 743-746, 1996.
53. Balbay, M.D. et al, Proc, Amer. Assoc. Cancer Res. 40: 225-226, 1999.
54. Rittenhouse, H.G. et al Crit. Rev. Clin. Lab. Sci. 35: 275-368, 1998.
55. Hansson, L et al, J. Biol. Chem. 269: 19420-19426, 1994.
56. Stephenson, S. et al J. Biol. Chem. 27: 23210-23214, 1999.
57. Stenman, U.H. Clin. Chem. 45: 753-754, 1999.
58. Black, M.H., Clin. Chem. 45: 790-799, 1999.
59. Underwood, L.J. et al, Cancer Res. 59:4435-4439, 1999.
60. Adams, M.D. et al, Curr. Opin. Cell Biol. 8:331-339, 1996.
61. Heuze, N. et al, cancer Res. 59: 2820-2824, 1999.
62. Riegman, P.H., Mol. Cell Endocrinol. 76: 181-190.
63. Chen, L.M. Braz. J. Med. Biol Res. 27: 1829-1838, 1994.
64. Mitsui, S. et al, Eur. J. Biochem. 260:627-634.
65. Baffa, R., Urology, 47:795-800,1996.
66. Henttu, P. et al, Int. J. Cancer 45: 654-660, 1990.
67. McCormack, R.T. et al, Urology 45: 729-744, 1995.
68. Liu, X.F., et al, Biochem. Biophys. Res. Commun. 264:833-839, 1999.
69. Riegman, P.H. et al, Biochem. Biophys. Res. Commun. 155: 181-188, 1988.
70. Lundwall, A. and H. Lilja, FEBS Lett. 214: 317-322, 1987.
71. Zarghami, N. et al, Br. J. Cancer 75: 579-88, 1997.

**TABLE 1. Predicted exons of the putative gene KLK-L1. The translated protein sequences of each exon (open reading frame) are shown.**

| Exon No . ¹ | Putative coding region² | | No. of bases | Translated protein sequence | EST match³ | Intron phase⁴ | Stop codon⁵ | Catalytic triad⁶ | Exon prediction program⁷ |
|---|---|---|---|---|---|---|---|---|---|
| | From(bp) | To (bp) | | | | | | | |
| 2 | 2263 | 2425 | 163 | SLVSGSCSQIINGEDCSPHSQPWQAALVMENELFCSGV LVHPQWVLSAAHCFQ | + | II | - | H | A,B,D |
| 3 | 2847 | 3109 | 263 | NSYTIGLGLHSLEADQEPGSQMVEASLSVRHPEYNRPL LANDLMLIKLDESVSESDTIRSISIASQCPTAGNSCLVSG WGLLANGELT | + | I | - | D | A,B,C,D |
| 4 | 3180 | 3317 | 137 | GRMPTVLQCVNVSVVSEEVCSKLYDPLYHPSMFCAGG GQDQKDSCN | + | 0 | - | | A,B,C,D |
| 5 | 4588 | 4737 | 150 | GDSGGPLICNGYLQGLVSFGKAPCGQVGVPGVYTNLC KFTEWIEKTVQAS | + | - | + | S | A,B,C |

| | | | | | | | | | |
|---|---|---|---|---|---|---|---|---|---|
| 1. Conventional numbering of exons in comparison to the five coding exons of PSA,as described in Ref. 14. 2. Nucleotide numbers refer to the related contig 3. (+) = >95% homology with published human EST sequences. 4.Intron phase:0=the intron occurs between codons;I=the intron occurs after the first nucleotide of the codon; II=the intron occurs after the second nucleotide of the codon. 5.(+) denotes the exon containing the stop codon. 6.H=histidine,D=aspartic acid,S=serine.The aminoacids of the catalytic triad are bold and underlined. 7. A = GeneBuilder (gene analysis), B = GeneBuilder (exon analysis), C = Grail 2, D = GENEID-3 | | | | | | | | | |

**TABLE 2. Predicted exons of the putative gene KLK-L2. The translated protein sequences of each exon (open reading frame) are shown***

| Exon No.¹ | Putative coding sequence² | | No. of bases | Translated protein sequence | EST match³ | Intron phase⁴ | Stop codon⁵ | Catalytic triad⁶ | Exon prediction program⁷ |
|---|---|---|---|---|---|---|---|---|---|
| | From(bp) | To(bp) | | | | | | | |
| 1 | 15,361 | 15,433 | 73 | MATARPPWMWVLCALITALLLGVT | + | 1 | - | - | - |
| 2 | 17,904 | 18,165 | 262 | EHVLANNDVSCDHPSNTVPSGSNQDLGAGAGEDARSDDSSSRIIN GSDCDMHTQPWQAALLLRPNQLYCGAVLVHPQWLLTAAHCRK K | + | II | - | H | A,B,C,D |
| 3 | 18,903 | 19,159 | 257 | VFRVRLGHYSLSPVYESGQQMFQGVKSIPHPGYSHPGHSNDLMLI KLNRRIRPTKDVRPINVSSHCPSAGTKCLVSGWGTTKSPQ | + | I | - | D | C,D |
| 4 | 19,245 | 19,378 | 134 | VHFPKVLQCLNISVLSQKRCEDAYPRQIDDTMFCAGDKAGRDSC Q | + | 0 | - | - | B,C |
| 5 | 24,232 | 24,384 | 153 | GDSGGPVVCNGSLQGLVSWGDYPCARPNRPGVYTNLCKFTKWI QETIQANS | + | - | + | S | A,B,C |

| | | | | | | | | | |
|---|---|---|---|---|---|---|---|---|---|
| * All footnotes same as Table 1 | | | | | | | | | |

**TABLE 3. Predicted exons of the putative gene KLK-L3. The translated protein sequences of each exon (open reading frame) are shown***

| Exon No.¹ | Putative coding region² | | No. of bases | Translated protein sequence | EST match³ | Intron phase⁴ | Stop codon⁵ | Catalytic triad⁶ | Exon prediction program⁷ |
|---|---|---|---|---|---|---|---|---|---|
| | From(bp) | To(bp) | | | | | | | |
| 1 | 70,473 | 70,584 | 112 | VHFPTPINHRGGPMEEEGDGMAYHKEALDAGCTFQDP | - | I | - | - | A,B,C,D |
| 2 | 70,764 | 70,962 | 199 | ACSSLTPLSLIPTPGHGWADTRAIGAEECRPNSQPWQAGLF HLTRLFCGATLISDRWLLTAAHCRK | + | II | - | H | A,B,C,D |
| 3 | 73,395 | 73,687 | 293 | PLTSEACPSRYLWVRLGEHHLWKWEGPEQLFRVTDFFPHP GFNKDLSANDHNDDIMLIRLPRQARLSPAVQPLNLSQTCV SPOMQCLISGWGAVSSPK | + | I | - | D | A,B,C,D |
| 4 | 76,305 | 76,441 | 137 | ALFPVTLQCANISILENKLCHWAYPGHISDSMLCAGLWEG GRGSCQ | + | 0 | - | - | A,B,C,D |
| 5 | 76,884 | 77633 | 749 | GDSGGPLVCNGTLAGVVSGGAEPCSRPRRPAVYTSVCHYL DWIQEIMEN | - | - | + | S | A,B |

| | | | | | | | | | |
|---|---|---|---|---|---|---|---|---|---|
| * All footnotes same as Table 1 | | | | | | | | | |

**TABLE 4. Predicted exons of the putative gene KLK-L4. The translated protein sequences of each exon (open reading frame) are shown**

| Exon No.¹ | Putative coding region² | | No. of bases | Translated protein sequence | EST match³ | Intron phase⁴ | Stop codon⁵ | Catalytic triad⁶ | Exon prediction program⁷ |
|---|---|---|---|---|---|---|---|---|---|
| | From(bp) | To(bp) | | | | | | | |
| 2 | 24,945 | 25,120 | 176 | ESSKVLNTNGTSGFLPGGYTCFPHSQPWQAALLVQGRLL CGGVLVHPKWVLTAAHCLKE | + | II | - | H | C |
| 3 | 25,460 | 25,728 | 269 | GLKVYLGKHALGRVEAGEQVREVVHSIPHPEYRRSPTHL NHDHDIMLLELQSPVQLTGYIQTLPLSHNNRLTPGTTCRV SGWGTTTSPQ | + | I | - | D | A,B,C,D |
| 4 | 26,879 | 27,015 | 137 | VNYPKTLQCANIQLRSDEECRQVYPGKITDNMLCAGTKE GGKDSCE | + | 0 | - | - | A,B,C,D |
| 5 | 28,778 | 28963 | 189 | GDSGGPLVCNRTLYGIVSWGDFPCGQPDRPGVYTRVSRY VLWIRETIRKYETQQQKWLKOPQ | + | - | + | S | A,B,C |

| | | | | | | | | | |
|---|---|---|---|---|---|---|---|---|---|
| * All footnotes same as Table 1 | | | | | | | | | |

**TABLE 5. Predicted exons of the putative gene KLK-L5. The translated protein sequences of each exon (open reading frame) are shown***

| Exon No.¹ | Putative coding region² | | No. of bases | Translated protein sequence | EST match³ | Intron phase⁴ | Stop codon⁵ | Catalytic triad⁶ | Exon prediction program⁷ |
|---|---|---|---|---|---|---|---|---|---|
| | From(bp) | To(bp) | | | | | | | |
| 2 | 1588 | 1747 | 160 | LSQAATPKIFNGTECGRNSQPWQVGLFEGTSLRCGGV - LIDHRWVL TAAHCSG | | II | - | H | A,B,C |
| 3 | 3592 | 3851 | 260 | SRYWVRLGEHSLSQLDWTEQIRHSGFSVTHPGYLGAS TSHEHDLRLLRLRLPVRVTSSVQPLPLPNDCATAGTEC HVSGWGITNHPR | + | I | - | D | A,B,C,D |
| 4 | 4806 | 4939 | 134 | NPFPDLLQCLNLSIVSHATCHGVYPGRITSNMVCAGG VPGQDACQ | + | 0 | - | - | A,B,C,D |

| | | | | | | | | | |
|---|---|---|---|---|---|---|---|---|---|
| * All footnotes same as Table 1 | | | | | | | | | |

**TABLE 6. Homology between the predicted amino acid sequences of the newly identified putative genes and protein sequences deposited in Genbank**

| **No.** | **Gene identity** | **Homolgous known protein** | Identity% (number of amino acids) |
|---|---|---|---|
| 1 | KLK-L1 | • Human stratum corneum chymotryptic enzyme | 44(101/227) |
| | | • Rat kallikrein | 40( 96/237) |
| | | • Mouse glandular kallikrein K22 | 39( 94/236) |
| | | • Human glandular kallikrein | 38( 93/241) |
| | | • Human prostatic specific antigen | 37( 91/241) |
| | | • Human protease M | 37( 87/229) |
| 2 | KLK-L2 | • Human neuropsin | 48(106/219) |
| | | • Human stratum corneum chymotryptic enzyme | 47(103/216) |
| | | • Human protease M | 45( 99/219) |
| | | • Human trypsinogen I | 45(100/221) |
| | | • Rat trypsinogen | 44( 98/220) |
| 3 | KLK-L3 | • Human neuropsin | 44(109/244) |
| | | • Rat trypsinogen 4 | 39(95/241) |
| | | • Human protease M | 38( 98/253) |
| | | • Human glandular kallikrein | 37( 94/248) |
| | | • Human prostatic specific antigen | 36( 89/242) |
| 4 | KLK-L4 | • Human protease M | 52(118/225) |
| | | • Human neuropsin | 51(116/225) |
| | | • Mouse neuropsin | 51(116/226) |
| | | • Human glandular kallikrein | 48(113/234) |
| | | • Human prostatic specific antigen | 47(108/227) |
| 5 | KLK-L5 | • Human neuropsin | 44( 81/184) |
| | | • Rat trypsinogen I | 42( 76/178) |
| | | • Rat trypsinogen II | 42( 75/178) |
| | | • Human protease M | 41( 73/178) |

**Table 7. Expressed sequence tags with >95% homology to exons of the prostase/KLK-L1 gene.**

| GenBank # | Source | Tissue | homologous exons |
|---|---|---|---|
| AA551449 | I.M.A.G.E. | prostate | 3,4,5 |
| AA533140 | I.M.A.G.E. | prostate | 4,5 |
| AA503963 | I.M.A.G.E. | prostate | 5 |
| AA569484 | I.M.A.G.E. | prostate | 5 |
| AA336074 | TIGR | endometrium | 2,3 |

**Table 8. Primers used for reverse transcription-polymerase chain reaction (RT-PCR) analysis of various genes.**

| **Gene** | **Primer name** | **Sequence**^{**1**} | **Product size** (base pairs) |
|---|---|---|---|
| Prostase (KLK-L1) | RS RAS | TGACCCGCTGTACCACCCCA GAATTCCTTCCGCAGGATGT | 278 |
| pS2 | PS2S PS2AS | GGTGATCTGCGCCCTGGTCCT AGGTGTCCGGTGGAGGTGGCA | 328 |
| PSA | PSAS PSAAS | TGCGCAAGTTCACCCTCA CCCTCTCCTTACTTCATCC | 754 |
| Actin | ACTINS ACTINAS | ACAATGAGCTGCGTGTGGCT TCTCCTTAATGTCACGCACGA | 372 |

| | | | |
|---|---|---|---|
| 1. All nucleotide sequences are given in the 5'→3' orientation. | | | |

**Table 9. Tissue expression of prostase/KLK-L1 by RT-PCR analysis**

| Expression level | | | |
|---|---|---|---|
| **High** | **medium** | **low** | **No Expression** |
| Prostate | Mammary gland | Salivary glands | Stomach |
| Testis | Colon | Lung | Heart |
| Adrenals | Spinal cord | Brain | Spleen |
| Uterus | | Bone marrow | Placenta |
| Thyroid | | Thymus | Liver |
| | | Trachea | Pancreas |
| | | Cerebellum | Kidney |
| | | | Fetal brain |
| | | | Fetal liver |
| | | | Skeletal muscle |
| | | | Small intestine |

**Table 10. EST clones with >95% homology to exons of KLK-L2**

| **GENBANK #** | **Tissue of Origin** | **I.M.A.GE. ID** | **Homologous exons** |
|---|---|---|---|
| W73140 | Fetal heart | 344588 | 4,5 |
| W73168 | Fetal heart | 344588 | 3,4,5 |
| AA862032 | Squamous cell carcinoma | 1485736 | 4,5 |
| AI002163 | Testis | 1619481 | 3,4,5 |
| N80762 | Fetal lung | 300611 | 5 |
| W68361 | Fetal heart | 342591 | 5 |
| W68496 | Fetal heart | 342591 | 5 |
| AA292366 | Ovarian tumor | 725905 | 1,2 |
| AA394040 | Ovarian tumor | 726001 | 5 |

**Table 11. Primers used for reverse transcription polymerase chain reaction (RT-PCR) analysis.**

| **Gene** | **Primer name** | **Sequence**^{**1**} | **Product size** (base pairs) |
|---|---|---|---|
| KLK-L2 | KS | GGATGCTTACCCGAGACAGA | 342 |
| | KAS | GCTGGAGAGATGAACATTCT | |
| pS2 | PS2S | GGTGATCTGCGCCCTGGTCCT | 328 |
| | PS2AS | AGGTGTCCGGTGGAGGTGGCA | |
| PSA | PSAS | TGCGCAAGTTCACCCTCA | 754 |
| | PSAAS | CCCTCTCCTTACTTCATCC | |
| Actin | ACTINS | ACAATGAGCTGCGTGTGGCT | 372 |
| | ACTINAS | TCTCCTTAATGTCACGCACGA | |
| KLK-L2 | R1 | CCGAGACGGACTCTGAAAACTTTCTTCC | |
| | R2 | TGAAAACTTTCTTCCTGCAGTGGGCGGC | |

| | | | |
|---|---|---|---|
| 1. All nucleotide sequence are given in the 5' 3' orientation. | | | |

**Table 12. Tissue expression of KLK-L2 by RT-PCR analysis.**

| **Expression Level** | | | |
|---|---|---|---|
| **high** | **Medium** | **low** | **No Expression** |
| Brain | Salivary gland | Uterus | Stomach |
| Mammary gland | Fetal brain | Lung | Adrenal gland |
| Testis | Thymus | Heart | Colon |
| | Prostate | Fetal liver | Skeletal muscle |
| | Thyroid | Spleen | |
| | Trachea | Placenta | |
| | Cerebellum | Liver | |
| | Spinal cord | Pancreas | |
| | | Small intestine | |
| | | Kidney | |
| | | Bone marrow | |

**TABLE 13. Primers used for reverse transcription polymerase chain reaction (RT-PCR) analysis.**

| **Gene** | **Primer name** | **Sequence**^{**1**} |
|---|---|---|
| KLK-L3 | L3-F1 | CATGCAGTGTCTCATCTCAG |
| | L3-F2 | CATGGAGGAGGAAGGAGATG |
| | L3-R1 | CTTCGGCCTCTCTTGGTCTT |
| | | |
| PSA | PSAS | TGCGCAAGTTCACCCTCA |
| | PSAAS | CCCTCTCCITACTTCATCC |
| | | |
| Actin | ACTINS | ACAATGAGCTGCGTGTGGCT |
| | ACTINAS | TCTCCTTAATGTCACGCACGA |

| | | |
|---|---|---|
| 1. All nucleotide sequence are given in the 5' → 3' orientation. | | |

**TABLE 14. Primers used for gene-specific PCR amplification of the kallikrein genes using DNA as a template.**

| **Primer name** | **Sequence**^{**1**} | **Coordinates** | **GenBank accession #** | **Gene name** |
|---|---|---|---|---|
| Z1S | GACCCTGACATGTGACATCTA | 979-999 | U62801 | Zyme |
| Z1AS | GCCACTGCCTGATGGAGACTG | 1422-1402 | | |
| | | | | |
| GL3-F1 | AACATCAGCATCCTGGAGAA | 7324-7343 | AF135026 | KLK-L3 |
| LL3-R1 | CTTCGGCCTCTCTTGGTCTT | 8051-8060 | | |
| | | | | |
| L2-1 | GGGTCAGAGCTGCAGAGAAG | 11104-11123 | AF135028 | KLK-L2 |
| L2-2 | GGGCCTGTCGTCTGCAATGG | 11522-11541 | | |
| | | | | |
| KLK-L1 | ATGGCCACAGCAGGAAATCC | 1411-1430 | AF113141 | KLK-L1 |
| | GGTCACTTGTCTGCGCAGAC | 1990-2019 | | |
| | | | | |
| PS PAS | CCCAACCCTGTGTTTTTCTC | 3634-3653 | M33105 | PSA² |
| | GGCCCTCCTCCCTCAGA | 4143-4118 | | |
| | | | | |
| K1S K1AS | ATCCCTCCATTCCCATCTTT | 2-22 | M18157 | KLK1³ |
| | CACATACAATTCTCTGGTTC | 324-30 | | |
| | | | | |
| K2S K2AS | AGTGACACTGTCTCAGAATT | 131-150 | AF024605 | KLK2⁴ |
| | CCCCAATCTCACCAGTGCAC | 580-561 | | |
| | | | | |
| NS NAS | GCTTCCCTACCGCTGTGCT | 552-570 | AF055481 | NES 1⁵ |
| | CACTCTGGCAAGGGTCCTG | 763-744 | | |

| | | | | |
|---|---|---|---|---|
| 1. all nucleotide sequences are given in the 5' → 3' orientation 2. prostate specific antigen 3. human renal kallikrein 4. human glandular kallikrein 5. normal epithelia cell-specific 1 gene. | | | | |

**TABLE 15. Primers used for reverse transcription polymerase chain reaction (RT-PCR) analysis.**

| **Gene** | **Primer name** | **Sequence**^{**1**} |
|---|---|---|
| KLK-L4 | L4-F1 | AACTCTACAATGTGCCAACA |
| | | |
| | G4-R1 | TTATTGTGGGCCCTTCAACC |
| | L4-R3 | GGATGGTCCATTTATAGGAC |
| | | |
| | L4-A | AGGCTGCCCTACTAGTGCAA |
| | L4-B | ATATTGCCTAGGTGGATGTG |
| | L4-D | AAGACTTCAAGGAGCCAAGC |
| | L4-E | GACCCTTCACCTCCCAAAAT |
| | | |
| | L4-X1 | CTAGTGATCGCCTCCCTGAC |
| | | |
| pS2 | PS2S | GGTGATCTGCGCCCTGGTCCT |
| | PS2AS | AGGTGTCCGGTGGAGGTGGCA |
| | | |
| PSA | PSAS | TGCGCAAGTTCACCCTCA |
| | PSAAS | CCCTCTCCTTACTTCATCC |
| | | |
| Actin | ACTINS | ACAATGAGCTGCGTGTGGCT |
| | ACTINAS | TCTCCTTAATGTCACGCACGA |

| | | |
|---|---|---|
| 1. All nucleotide sequence are given in the 5'→3' orientation. | | |

**TABLE 16. EST clones with >95% homology to exons of KLK-L4**

| **GenBank #** | **Tissue of origin** | **I.M.A.GE. ID** |
|---|---|---|
| AA399955 | Testis | 743113 |
| AA401397 | | |
| AA846771 | Testis | 1392889 |
| AI002101 | Testis | 1619045 |
| AI032327 | Testis | 1644236 |

**TABLE 17. Primers used for reverse transcription polymerase chain reaction (RT-PCR) analysis.**

| **Gene** | **Primer name** | **Sequence**^{**1**} |
|---|---|---|
| KLK-L5 | KLK-L5-F1 | TCAGCCAGGCAGCCACACCG |
| | KLK-L5-R1 | TTGGTGATGCCCCAGCCTGA |
| | | |
| | L5-F2 | CCACACCGAAGATTTTCAAT |
| | L5-R2 | GCCCCTCCTTCATTTATA |
| | | |
| PSA | PSAS | TGCGCAAGTTCACCCTCA |
| | PSAAS | CCCTCTCCTTACTTCATCC |
| | | |
| Actin | ACTINS | ACAATGAGCTGCGTGTGGCT |
| | ACTINAS | TCTCCTTAATGTCACGCACGA |

| | | |
|---|---|---|
| 1. All nucleotide sequence are given in the 5'→3' orientation. | | |

### Annex to the application documents - subsequently filed sequences listing

Sequence Listing

SEQ.ID. NO. 1
KLK-L1 na

1 tatctcatga gagagaataa gaacatgaaa agagaaagaa tgagagagag
agagagaaag
61 aaaaaggaga gtggagtcta ggatctgggc aggggtctcc tccctgggtc
cctagaccct
121 gctgccagcc ccttctgggc ccccaaccac tgcctggtca gagttgaggc
agcctgagag
181 agttgagctg gaagtttgca gcacctgacc cctggaacac atcccctggg
ggcaggccag
241 cccaggctga ggatgcttat aagccccaag gaggcccctg cggaggcagc
aggctggagc
301 tcagcccagc agtggaatcc aggagcccag aggtggccgg gtaagaggcc
tggtggtccc
361 ccactaaaag cctgcagtgt tcatgatcca actctcccta cagctccatg
tcgctggatt
421 ctcagcctct gtgccttctg tctccacatc tctctagaca gatctctcac
tgtctctagt
481 taggagtcac tgtctctagt taggggtctc tctgtctctc tgaatctata
tctccatgtc
541 taactctcag actgtctctg aggatatctc tcaagcactc tgtctctccg
gctctgattc
601 tctgtgtgtc ttccctccat gcttgtttgt gggtggctag acaccatctc
tccccattca
661 cagatggcta gatgctttct ctaaactttc ctttctacct agttctctct
ctctctcttt
721 tcccatctct ctctctcttt ttctctctca gtctctaaat ctgtctctct
aggttctggg
781 tccatggatg ggagaggggg tagatggtct aggctcttgc ctacctaata
acgtcccaga
841 gggaagaaag ggagggacaa agagagggat ggagagactt gggctgaaga
tccccagaca
901 cggctaagtc tcagtcctca tccccaggtg ctgacgtgat ggccacagca
ggaaatccct
961 ggggctggtt cctggggtac ctcatccttg gtgtcgcagg tatctgagta
tgcgtgtgtg
1021 tgtctgtccg tgcttggggg cacagtgttt gttaatgttc aggtgtgact
cagtgtcctc
1081 ttgcttgtga ctgcaaagct gcctgtgaga cggtaccgtg ttatccgtcc
gccatggctg
1141 tgcccctgca actccttgta tcgtggtaaa tttgtgtgtg gcagtgtgcc
tgggtgtgtg
1201 gttgtacctg tgagactctg acagtttgtg cctctgaata tctggtggag
tgacaacagt
1261 gtaatgatga tatggggaca ggggaagccg agggtgcagg agattgtgct
tcctggggcg
1321 tgatccattg ctgggaatct gtgcctgctt cctgggtctt cagtcctgag
atccccctct
1381 cccatcccca aggaactcac ctcacaggac tataaaacgg tgttttggtg
tgcatgggct
1441 tgtggcttgg tgtgactgtg ggcaaggctg ggagaggata ggagtgactc
ggcgcaggac
1501 cgactctttg agcatcagtc tgcgcagaca agtgacccga tccttgctcc
cagcaacaac
1561 tccaccccct gagctttaat tcaccccgaa ggacccgatc ctaccgctat
gagcctagac

1621 tcctctgttg aacccctcct gaccgtggct ttgcaccgcg atggcaccag
tctcacctcc
1681 agagctcacc ccagagccct gactccgccc cagaagccct ggtcccacct
tctgagactg
1741 cctctagcca taacccagct cttgaagcct tgatggcgcc cctgcgctgt
aaccccaacc
1801 ctaggagcac tgatcccgcc ttctcagccc acccccatgc cctgactctc
ctcccaggag
1861 ccctgactac cctgaatccc tgaccaggct cctgcaccgt gatcaccgcc
cctgggagcc
1921 ctaggcctat atcctggacc agcccctgaa gctccgatca tgacccctgc
accataaccc
1981 cacccccagg agccctgggt ccgccccctg ggcccgcccc cagccctgac
tcggcccccc
2041 aagagtcctg actgctcctg aagccctgac cacgcccctg ctcggtaacc
cctcccccaa
2101 gagccctggg cccgcctcct gagcccgttc ccagccctga ctccgccccg
aggagccctg
2161 actgctcctg aacctctgac cacgcccctg ctcggtaagc ccacccccag
gaaccctggg
2221 cccgcctcct ggtcccgatc ccatccctga ctccgccctc aggatctctc
gtctctggta
2281 gctgcagcca aatcataaac ggcgaggact gcagcccgca ctcgcagccc
tggcaggcgg
2341 cactggtcat ggaaaacgaa ttgttctgct cgggcgtcct ggtgcatccg
cagtgggtgc
2401 tgtcagccgc acactgtttc cagaagtgag tgcagaggta gggggagtgg
gcagggcctg
2461 ggtccggggg cggggcctaa tatcaggctc atcttggggt gctcaggggg
aaacagcggt
2521 gaaggctctg ggaggaggac ggaatgagcc tggatccggg gagcccagag
ggaagggctg
2581 ggaggcggga atcttgcttc ggaaggactc agagagccct gacttgaaat
ctcagcccag
2641 tgctgagtct ctagtgaact aaggcaagtt cttgtccctg aatttttgtg
aatgaggatt
2701 tgagaccatg gttaagtagc tcttagggtg tttagcgaag agggtggggt
tggggttagg
2761 agatggggat gggaatgggg ttgaagatga gaatggaggt aaggatgtag
ttgccacaaa
2821 actgacctgc cctccgtggc ccacagctcc tacaccatcg ggctgggcct
gcacagtctt
2881 gaggccgacc aagagccagg gagccagatg gtggaggcca gcctctccgt
acggcaccca
2941 gagtacaaca gacccttgct cgctaacgac ctcatgctca tcaagttgga
cgaatccgtg
3001 tccgagtctg acaccatccg gagcatcagc attgcttcgc agtgccctac
cgcggggaac
3061 tcttgcctcg tttctggctg gggtctgctg gcgaacggtg agctcacggg
tgtgtgtctg
3121 ccctcttcaa ggaggtcctc tgcccagtcg cgggggctga cccagagctc
tgcgtcccag
3181 gcagaatgcc taccgtgctg cagtgcgtga acgtgtcggt ggtgtctgag
gaggtctgca
3241 gtaagctcta tgacccgctg taccacccca gcatgttctg cgccggcgga
gggcaagacc
3301 agaaggactc ctgcaacgtg agagagggga aaggggaggg caggcgactc
agggaagggt
3361 ggagaagggg gagacagaga cacacagggc cgcatggcga gatgcagaga
tggagagaca

3421 cacagggaga cagtgacaac tagagagaga aactgagaga aacagagaaa
taaacacagg
3481 aataaagaga agcaaaggaa gagagaaaca gaaacagaca tggggaggca
gaaacacaca
3541 cacatagaaa tgcagttgac cttccaacag catggggcct gagggcggtg
acctccaccc
3601 aatagaaaat cctcttataa cttttgactc cccaaaaacc tgactagaaa
tagcctactg
3661 ttgacgggga gccttaccaa taacataaat agtcgattta tgcatacgtt
ttatgcattc
3721 atgatatacc tttgttggaa ttttttgata tttctaagct acacagttcg
tctgtgaatt
3781 tttttaaatt gttgcaactc tcctaaaatt tttctgatgt gtttattgaa
aaaatccaag
3841 tataagtgga cttgtgcagt tcaaaccagg gttgttcaag ggtcaactgt
gtacccagag
3901 ggaaacagtg acacagattc atagaggtga aacacgaaga gaaacaggaa
aaatcaagac
3961 tctacaaaga ggctgggcag ggtggctcat gcctgtaatc ccagcacttt
gggaggcgag
4021 gcaggcagat cacttgaggt aaggagttca agaccagcct ggccaaaatg
gtgaaatcct
4081 gtctgtacta aaaatacaaa agttagctgg atatggtggc aggcgcctgt
aatcccagct
4141 acttgggagg ctgaggcagg agaattgctt gaatatggga ggcagaggtt
gaagtgagtt
4201 gagatcacac cactatactc cagctggggc aacagagtaa gactctgtct
caaaaaaaaa
4261 aaaaaaaaag actttacaaa gagatgcaga gacactgaga cagataaaca
agccacaaag
4321 gagacaaagg agagacagac aaacagaaac agacagacca caagcccaag
agaagcagcc
4381 agcattcagg acataggaca tcgggaagca ggattagatg aagtcaggga
tctggaatgg
4441 gacttccaac agatatgttg ctgggctatg ttgttattga tgatggttct
gtctttgttt
4501 ctcagtctca tttagttcct ttctgagccc atatccattt ccacctctct
gtgttttgaa
4561 ttctgactct ccctctcttc acaacagggt gactctgggg ggcccctgat
ctgcaacggg
4621 tacttgcagg gccttgtgtc tttcggaaaa gccccgtgtg gccaagttgg
cgtgccaggt
4681 gtctacacca acctctgcaa attcactgag tggatagaga aaaccgtcca ggccagttaa

SEQ.ID. NO. 2

KLK-L1

SLVSGSCSQIINGEDCSPHSQPWQAALVMENELFCSGVLVHPQWVLSAAHCF
QNSYTIGLGLHSLEADQEPGSQMVEASLSVRHPEYNRPLLANDLMLIKLDESV
SESDTIRSISIASQCPTAGNSCLVSGWGLLANGELTGRMPTVLQCVNVSVVSE
EVCSKLYDPLYHPSMFCAGGGQDQKDSCNGDSGGPLICNGYLQGLVSFGKAP
CGQVGVPGVYTNLCKFTEWIEKTVQAS

SEQ.ID. NO. 3

KLK-L1

MATAGNPWGWFLGYLILGVAGSLVSGSCSQIINGEDCSPHSQPWQAALVME
NELFCSGVLVHPQWVLSAAHCFQNSYTIGLGLHSLEADQEPGSQMVEASLSV
RHPEYNRPLLANDLMLIKLDESVSESDTIRSISIASQCPTAGNSCLVSGWGLLA
NGRMPTVLQCVNVSVVSEEVCSKLYDPLYHPSMFCAGGGQDQKDSCNGDSG
GPLICNGYLQGLVSFGKAPCGQVGVPGVYTNLCKFTEWIEKTVQAS

SEQ.ID. NO. 4

Figure 4 Sequence

TGACCCGCTG TACCACCCCA GCATGTTCTG CGCCGGCGGA
GGGCAAGACC AGAAGGACTC
CTGCAACGGT GACTCTGGGG GGCCCCTGAT CTGCAACGGG
TACTTGCAGG GCCTTGTGTC
TTTCGGAAAA GCCCCGTGTG GCCAAGTTGG CGTGCCAGGT
GCCTACACCA ACCTCTGCAA
ATTCACTGAG TGGATAGAGA AAACCGTCCA GGCCAGTTAA
CTCTGGGGAC TGGGAACCCA
TGAAATTGAC CCCCAAATAC ATCCTGCGGA AGGAATTC

SEQ.ID. NO. 5

Table 8 Sequence

TGACCCGCTGTACCACCCCA

SEQ.ID. NO. 6

Table 8 Sequence

GAATTCCTTCCGCAGGATGT

SEQ.ID. NO. 7

Table 8 Sequence

GGTGATCTGCGCCCTGGTCCT

SEQ.ID. NO. 8

Table 8 Sequence

AGGTGTCCGGTGGAGGTGGCA

SEQ.ID. NO. 9

Table 8 Sequence

TGCGCAAGTTCACCCTCA

SEQ.ID. NO. 10

Table 8 Sequence

CCCTCTCCTTACTTCATCC

SEQ.ID. NO. 11

Table 8 Sequence

ACAATGAGCTGCGTGTGGCT

SEQ.ID. NO. 12

Table 8 Sequence

TCTCCTTAATGTCACGCACGA

SEQ.ID. NO. 13

KLK-L2 na

1 gggcccagag tgaaggcaag agaaggagtt gagagctccc tctgcaaagt
ggcttgagtc
61 tcccctgcct aaaatgcagg gagagggagg cagaaagaca gggaagagga
aggggtgggg
121 aagaaagaga gagagagaga gagacagaat aacacaacta cagaaacaca
gagagaacac
181 acagagagcc tgggacacag ggacacacag agtcagagag aaaagagaag
atagagaaag
241 acacaaatgg agacacagag gtgtaaagaa agagagatta acagagtccc
agatacacgc
301 aaaggggcag aagcacagtt ttcagggtgg tgtctatgat catcttcttt
tttttttttt
361 tttttttttt tttttgagac ggagtctcgc tctgtcgccc aggctggagt
gcagtggcgg
421 gatctcggct cactgcaagc tccgcctccc gggttcacgc cattctcctg
cctcagcctc
481 ccaagtagct gggactacag gcgcccgcca ctacgcccgg ctaatttttt
tgtattttta
541 gtagagacgg ggtttcaccg ttttagccgg gatggcctcg atctcctgac
ctcgtgatcc
601 gcccgcctcg gcctcccaaa gtgctgggat tacaggcgtg agccaccgcg
cccggccatg
661 atcatcttct tgactatgct gatgtgacaa gtacctaaag ccatcagact
ctacccttta
721 aatatgcagt ttgggccagg caccgtggct catgcctgta attccagcac
tttgggaggc
781 agaggtgggt gaatcacttg aggccaggag tttgagacca gcctggccaa
catggtgaaa
841 ctctgtcttt actaaaaaaa aaaaaaaaaa aaaaaaaatc agccgggtgt
cgtggggcac
901 acctgtaatc ccagctatgc tggaggctga ggcacgagag tcacttgaac
cctggaggcg

961 gaggttgcag tgggccgaga tcacatcacc gccctccagc ctgggcgaca
gagcaagact
1021 ctgtctcaaa taaataaata aacaaacgaa caagcagttt gttgtacctt
agttatatct
1081 aaaaaaaaaa tgctgtcaac aaatagagca gaagtgaaat aaaggaaaat
aaatgggcca
1141 agaactctaa ggtatatttg acaaatcatt cagaaccttt aaaaaagaaa
gaatcacaga
1201 ggcatagaaa gacagggagg aacagggaga cagaaacacc tgtggcccaa
ggagaacaaa
1261 acaaggctcc taagacagac aggaggagag agagagagag tgagtgagag
acagacagag
1321 aaaaagacag agagagagag acagagacag agagacagag aggcgagagg
gatagaaaga
1381 gagagagggg tggagagaga cacgagatat tgagagagac tcagaaagat
agccgaggga
1441 gaaccacaga gagatggaag aagactctga gaaaaaacca gagacaaaga
tggaaagagg
1501 agtatcgagg gtgaacagac agtggtggaa tgagcaaaat gcagagaaga
aagcaagcaa
1561 tccaggcgcc aagaatagtg acccagagtt ggtgagaagc cagatcctta
aggctggggg
1621 aggcagggaa ggggctggcc tggcttccgg agacccctcc ccattctccg
ggccagggag
1681 gtagggagtg acattccgga ctgggtgggg ggtgctctgg gggtggagat
aggggggagca
1741 ggaggagcta ttgctaaggc ccgataggca cctcattgcc cgggaatgtg
ccccagggag
1801 cagtgggtgg ttataactca ggcccggtgc ccagagccca ggaggaggca
gtggccagga
1861 aggcacaggc ctgagaagtc tgcggctgag ctgggagcaa atcccccacc
ccctacctgg
1921 gggacagggc aagtgagacc tggtgagggt ggctcagcag gcagggaagg
agaggtgtct
1981 gtgcgtcctg cacccacatc tttctctgtc ccctccttgc cctgtctgga
ggctgctaga
2041 ctcctatctt ctgaattcta tagtgcctgg gtctcagcgc agtgccgatg
gtggcccgtc
2101 cttgtggttc ctctctacct ggggaaataa ggtaggggag ggaggggaag
tgggttaagg
2161 gctccccgga tcgcctgggc ctcccaaccc tctgacattc cccatccagg
tgcagcggcc
2221 atggctacag caagaccccc ctggatgtgg gtgctctgtg ctctgatcac
agccttgctt
2281 ctgggggtca caggtaacca gaactctggg gtgggagggt tgtgggattg
ggaggactgt
2341 ctctgcggca ctagagcgcc tgtcccctgg ggaactgtgt gagcctgggc
atgactccgg
2401 gaccgggtga atgtgagtct ctgtctgtac ttgtggttgt gcgatcgtat
gtggccctgt
2461 gactgccacg gtgtgtgtcg ggagggggga tgccttttcc catatcaggt
gactgtgcgg
2521 caggtggcac tgaccctttg aggctgtgtg tgtggttttg tgattgtgtg
tgcatttaag
2581 attgtgtgtg gctccacagc tgtgtgggtg aatgcatgta gcactggggg
tgttcactgt
2641 gtgtttggct gtgtgtggtg acttggcatt gtatatgact gcaggtatct
gcagttcctg
2701 tccctgaggt cccgggattg cgtgcaacaa aagtggtcat caccatggaa
agctgtgact

2761 gtgtgctgct tgcaggcgat tatgtgattg tggctgagtg tgacgttatg
gatgcccgta
2821 tttgtgaccg tgtgactacc tgaagctctg tgtaggggtg actgtatgtg
actgtgtgtg
2881 tctgtgtgag gccgtgtaaa tgctactgta tgtgtgatgg tgcagctgtg
tgtctggagt
2941 ttctgtctct gcctggaggg atagagggtg caggggtagc tatctctggg
agatgggtgc
3001 caggtgactg acttgcagtg tgtgcctgtg tgcagaagag tatgtggcag
tctgaacatc
3061 tgtgcacaca cggcatctgt gcgtggcact gagacactgt ggatgagggt
gtgcgatccc
3121 gctaggctgc ccgggagcgt gtgtacctgg agacagagct gtatgttagc
tgcacctgtg
3181 gaggcaacat gggcgtgtct gcagaactgc gtgcgtgctt ggctgttact
gctgttgtgc
3241 gcgtggttct tggggtgagt tcgtgaatga tggtggtgcc agggccatca
gcaagggtaa
3301 gaaccaggcc gggcgcggtg gctcacgcct gtaatcccag ccctttggga
ggccgaggca
3361 ggcggatcac ctgaggtcgg gagatcgagg ccagcctgac caacatggag
aaccccgtct
3421 ctactaaaaa tacaaaaaat tagctggtgt ggtggcgcgt gcctgtaatc
ccagctactc
3481 gggagactgg ggcagaaaaa tcgcttgaac ccgggaggtg gaggttgcgg
tgagccgaga
3541 tcgcgccatt gcactccagc ctgggcaaca agagcgaaac tccgtctcga
aagaaaaaaa
3601 gaaaaaaaaa agggtaagaa ccagtgaatg ggcacgggag gactgatgat
ggagtggggc
3661 atgcatgtag tctgtaggtc tgtgtgtgag aggaggagat tgacaggatt
gagaaggcat
3721 gttttcatct gagaattcag aaacctaggc ctgctcttcc cctccatgtg
gccccctaag
3781 ctgagccctt ctttcctggt cctgctttcg gaaccctagc tccgcccatg
agctctgacc
3841 ccacctcctt tcctcaacca cgcccctagg ccagactcta gtggaccccg
cctaaggcca
3901 cacccctttg ggccaggctc caccccctat tctgtgggta ccttctagaa
ccccttcaa
3961 agtcagagct tttttttttt tttttttgga gacagtcttg ctctctctcc
caggctggag
4021 tgcagtggcg tgatctcggc tcactgcaac ctctgcctcc caggttcaag
tgattctcgt
4081 gcctccacct cctgagtagc tgggattaca ggtgcgcgcc accacgcctg
gctaatttt
4141 gtgtctttag tagagacagg gtttcacctt gttggccagg ctggtctcaa
actcccaacc
4201 tcaggtgatc cgcccacctc ggcctcccag agtgctgggg ttacaggcgt
gagccaccgc
4261 ccccagccca aagtcagagc tctttatagg agactctaac atgtaaccct
gaccctggcc
4321 ctaactaagt caattccaaa ccccttcctg cctccagccc tgaccccact
cactgaggcc
4381 tgaccccact tcttgagacc agttccatcc ctaaagccct ggtctccctc
ccatccccag
4441 gctccagccc ccacagcttt ggcactaccc ctgagcttgt ccaggaatcc
tgtacccaat
4501 tttaccctca catgtagttc tagccaattc caggaatctg tgaggtccag
ttagagtcca

4561 gtaaccctac ctgagcctgg gctctgtcct tgagcttgag cctgggcttg
agaggtgcca
4621 ctcttattct ccaggccctg cccctgcccc ctcagcatgt cagacaccca
ccctctagct
4681 ggtctggcct cttgagtctg aaacccaccc ccagcccaag ccccgcctct
gagccccgcc
4741 caacccattt tccgttccca gagcatgttc tcgccaacaa tgatgtttcc
tgtgaccacc
4801 cctctaacac cgtgccctct gggagcaacc aggacctggg agctggggcc
ggggaagacg
4861 cccggtcgga tgacagcagc agccgcatca tcaatggatc cgactgcgat
atgcacaccc
4921 agccgtggca ggccgcgctg ttgctaaggc ccaaccagct ctactgcggg
gcggtgttgg
4981 tgcatccaca gtggctgctc acggccgccc actgcaggaa gaagtgagtg
ggagttccaa
5041 gaggagggtt ggtggggacg gggaagtggg ggtgggggtg gggaagtggg
ggtgggggtg
5101 tcatggaggt gagggctggt ggggacgggg aagtggggtt gggggtgtca
tggaaggtga
5161 gggttggtgg ggatgggttg gggatgtggg agcaggagga ggtcgagttg
gggataggac
5221 taaggatgga gttttgcggg ggagcaaggt gggaggatga ggttggagag
gggagagtgt
5281 tgtggtaggg aatgggaagg agccaaggat gggttggatt tggggttagg
agcatatatt
5341 tgttgaatgg tttgggatgg aggtggaatt gggattggct ttagaattgg
gggtgggtga
5401 aaatcgggct ggggtggaaa tgaagatagc atggagatag ggttgagatt
gggagcagat
5461 atagaatgaa ggatggggat tggagttttg ggtggggttg gagatggttg
gatttgggct
5521 tgagaatgca tatggtgatg gcttctgggt agggaaagaa ttagggttgg
gaatgggatg
5581 ggtttggaat tgtgactggg atggggacag gcatgggatt ggagaccaag
agggagttga
5641 ggatggtttg gggaccgggg gtggggatgg gggtggggct ggggctgggt
gtggggttgg
5701 gattggcgtt ggacgtggag atagagatca gggttggtgg tgacctgccc
catcttcctc
5761 agagttttca gagtccgtct cggccactac tccctgtcac cagtttatga
atctgggcag
5821 cagatgttcc agggggtcaa atccatcccc caccctggct actcccaccc
tggccactct
5881 aacgacctca tgctcatcaa actgaacaga agaattcgtc ccactaaaga
tgtcagaccc
5941 atcaacgtct cctctcattg tccctctgct gggacaaagt gcttggtgtc
tggctggggg
6001 acaaccaaga gcccccaagg tgagtgtcca ggttcttctt gataccgacc
catctctgcc
6061 gccttccatc tttctccact tctcattgtg ttcctgtttg acagtgcact
tccctaaggt
6121 cctccagtgc ttgaatatca gcgtgctaag tcagaaaagg tgcgaggatg
cttacccgag
6181 acagatagat gacaccatgt tctgcgccgg tgacaaagca ggtagagact
cctgccaggt
6241 gaggacacct ctctttattc agcagataca cactgagtgc caactcggta
acatggagcg
6301 ttgccaaatt ctgagaatcc agcaattgcc aagacagtca ggacccctgt
tctcacagag

6361 ctcataccct agagtagtgg tgtttagtag aaataatgct gagctgctta
tgtcatttcc
6421 agttttttag tagccacatt aaaacaggta aaaaaggctg ggcgcagtgg
ctcacacctg
6481 taatcccagc actttgggag gctgaggcag gcagatcacc tttggtcagg
agtttgagac
6541 tagcctggcc aacatggcga aactctgtct ctaaaaaaaa atacaaaaat
tagcctggca
6601 tggtggcggg cgcctgtaat ctcagctgct caggaggccg agacacaaga
atcacttaaa
6661 cccaggaggt ggaggttgca gtgagctgag atcgtgccac tcactccaac
ctgggagaca
6721 gagtgacact tttgtctcaa aaagaaaaaa aaaaacaagt aaaaaagaaa
caggtgaagt
6781 taactttaat aacccaatgt atcccaaata caatcatttc aaagtgtaat
taatataaaa
6841 caattatgaa tgagatactt tacattcttt tcttgttttc atattaagtc
tttgaaagtg
6901 agtatatatg ttatgctgac agcacatctc aatttggact agctacattt
caggtgctca
6961 gtagccacat gtggctagca gttactgtat tggatggcac ggatctagag
ggaaagatca
7021 gggctgtttt gtatggttgg gcaggttgtg cactgcataa agataccata
tctaataggg
7081 gcactccgtg ttacagatgt cagttttggc agttttcagg cgtgtggtag
ttaagtgtct
7141 tgtttcaaca aaatctgtaa tatgacagtt ttctagcaag tgctggtaaa
atatcttgag
7201 gaaggaaaag agaaatctgg taggtatttt tacaagagaa tatttaatac
aggggattaa
7261 ttgcaaagct gctggaaggg ctggaggaac aaagttaaaa aataaaaaac
tctgtggtca
7321 agaatctgca taaatagggc aatttcagag agtggtaaag gttaacccca
aaataaaaca
7381 tggttttagg atagtaaaca ataaggccca atattcaaaa aggtggtcag
gggagcctcc
7441 ttggagaggt ggcatttgag cagagaatgg atgacacaaa gaagctaaac
tcgtgaagtt
7501 taaggggaaa gaaaaggcac gtgcaaaggc cctgaggcag taaggaattt
ggctgattca
7561 aagaagaaga ggaaaccaat gcaactggag aacaaaagtg ggggcaacag
tagaaagtga
7621 cgctggaggt gtaggcaggg gcgaatgctc tgcaagtatt tcttggtcac
caacacagag
7681 cttccctatg ttctaatgga agctgtatct gttgaggaag acagaattta
aaatcaaact
7741 gttacatcaa ccagcaccct tctctgtatt caggctccca agggatctag
aaggacgtaa
7801 gttaacaagc tctcattagc agggtgtgtg tttcaacagt agttaggaag
ctggggattc
7861 aggagtactc cagtcccatg gctatgaaaa gctcccccca aattgtacaa
acctgacaaa
7921 tgcaacacct ccccagctct ccccatttct tctctgtgcc ctgggtgtgg
gggggtgggt
7981 tgcgaggggg aaaactttta acagaagaaa gcacatctcg gccgggcgtg
gtggctcaca
8041 cctgtaatcc caacactttg ggaggccgag gcgggtggat cactaggtca
ggagatggag
8101 accatcctgg ctgacacggt gaaaccctgt ctctactaaa aacacaaaaa
attagccggg

8161 cgtggtggca ggcgcctgta gtcccagcta ctcgggaggc tgaggcagga
gaatggcctg
8221 aacccgggag gcggaacttg cagtgagccg aggttgcacc actgcactcc
agcctgggca
8281 acacagtgag actccgtctc aaaaaaaaaa aaagaaaaga aaagaaatca
catctcattc
8341 aagtggtggc atttaaaact atttagcctt tctgtaggca aggttagtat
cttgtttttc
8401 cagacctcaa ggtgtttttt tgtttgtttt ttcataccgg tgtgtggtct
gggtgtggcc
8461 actaaaagct acaagcaaga aataataaca actacaacaa tactaatacc
aatagtataa
8521 aaataatagc atctggctaa ttgctggaca ctgttttaag tggtttgcat
gcctcagctc
8581 attaactcat ttacctgtta ttattggccc tattttacaa acaaggagcc
aaggctcaga
8641 gcagttaact aacagcctct caaaagaaac tctgcagaga tattaaattt
aaaaaataat
8701 gagagaaatt aaaccacaag aaagttgaaa tttagaggta caggcagcta
agcttgtttg
8761 ctttgaaaca gtgtctgcta ctgggaaaaa ggcaagtctt ggctttccta
ataattgata
8821 ccaggactct gtaattcata ttttgcatgc atgtaagtaa gaaatgaagc
cgggtgcaat
8881 ggcacatgcc agtaatccca gcactctggg agactgaagt gggaagatca
cttgagctca
8941 ggagttcaag accagcctgg gcaactaaaa attaaaaaaa taaaaatact
aattgttttt
9001 attttagtag attttattca taccacttac atcattattg tagtatgtac
atatttattt
9061 cttttctttt cttttctttt cttttttgag acggagtctc gctctgtcac
ccaggctgga
9121 gtgcaatggc accatatcag ctcactgcag catgcgcctc ctgggttcaa
gcatttcttc
9181 cacctcagcc tcccaagtag ctgggataac aggcacccac caccatgcct
ggctattttt
9241 ttttttccgt agagatgggg ttccaccatg ttggccaggc tggtcttgaa
ctcctgacct
9301 ccagtgatct gcctgcctcg gcctcccaaa ttgctggtat tacaggtgtg
agccaccgtg
9361 cccaggtggg agatagacat ttctctctac ctcaaacaga ggtccactca
agctactttt
9421 cattttcttc ataaatatta gccgagtggc tattttgcac caggaatggt
tccaggtgct
9481 gtggatatgg catcaggcaa aacagaccaa aaacttcctg ccgcgtggac
ctcatgttcc
9541 ccaagtggaa gacaggcaat aaagagatag ataaatatgt agtaaattaa
aaaaaaaaaa
9601 aattagccgg gtgtggtggc ttgcacctgt agttccagct acttgggagg
ctgaggtggg
9661 agaattgctt gagcccaaac gtttgaggct gcggtaagcc atgactgcac
tgctgcactc
9721 cagacagcag cctgggtgac aaagcaagac gtttttgtca gaaagaaaaa
aaaaagagac
9781 gaagggagga aggagagaga aaggaaggaa ggaaggagaa agaaaggaag
gaaggagaaa
9841 gaaaggaagg aaggaaggag aaagaaagga agaaagagaa agaaagaaaa
agaaagaaag
9901 aaagaagaaa gaaaagagag aggaaggaag gaaagaagga aaagagggaa
aaaaatgact

9961 gttgaagagc agtgagtatt attataggag ggtaattata gggaggtatg
gggaattgaa
10021 gacaggaaac acaaattagt ccaagcgaat ggatttctat tgggagtgat
tctgccccta
10081 gaagacactg gcaataccag gagacatttt tggttgtcac aactatatgg
aggggcatta
10141 ctggcaacta atggatagat gccaagtgtg ctgttcaaca tgctatgatg
cacacggcag
10201 gcctccacaa caaaccatta tccagcttca gatgcccaca gtgcccagat
cgaggaaccc
10261 tcatccaggg gctgagaacc gtatttttgc agaagggagg tataaggatg
ggttggtgga
10321 gaatggggaa ggaaggtgtg tgtccagtaa gagaaataag gcctgcacag
gctggagggg
10381 agagtgagag agaaagggag gcggagagat acacgatgag ggagacaggc
tggaacagaa
10441 agtagagacg aagattcgag atgtggagag gaagggtcac agaccccccc
gaaatgatgt
10501 gtggacaaca ggaatctgga agaggaagat ggagtggaga gtgacaaatg
gggtctaaag
10561 gttgaacttg gaggccaggc atggtggctc acgcctgtaa tcccaacact
ttggaggctg
10621 aggtgggcga atcacttgag gccaggagtt cgagaccagc ctggccaaca
tggtgaaacc
10681 ccgtctctac aaaaaaaata caaaaaatta gccgggtgtg gtgatggaca
cctgtagtca
10741 cagctacttg ggaggctgag gcaggagaat tgcttgaacc cgggagatgg
aggctgcagt
10801 gagctgaggt caggccactg cgctccaacc tgggcaacag agtaagactc
catctcaaaa
10861 aaaaaaaagc tggatttgga gtgaaatatt aataacattc tccctctctc
tccttttgcc
10921 tgtgtctcca tctctgtctt tttctgcatt tcttcatctc tgtactttcc
atctctgtgt
10981 gtctgttccc atctgcttct ccatctatgg gcatctctgg gtctctcatg
tctccttctg
11041 cccactttgc cacatctctg cctctctcat gccccccttt ctctcctgca
gggtgattct
11101 ggggggcctg tggtctgcaa tggctccctg cagggactcg tgtcctgggg
agattaccct
11161 tgtgcccggc ccaacagacc gggtgtctac acgaacctct gcaagttcac
caagtggatc
11221 caggaaacca tccaggccaa ctcctgagtc atcccaggac tcagcacacc
ggcatcccca
11281 cctgctgcag ggacagccct gacactcctt tcagaccctc attccttccc
agagatgttg
11341 agaatgttca tctctccagc ccctgacccc atgtctcctg gactcagggt
ctgcttcccc
11401 cacattgggc tgaccgtgtc tctctagttg aaccctggga acaatttcca
aaactgtcca
11461 gggcgggggt tgcgtctcaa tctccctggg gcactttcat cctcaagctc
agggcccatc
11521 ccttctctgc agctctgacc caaatttagt cccagaaata aactgagaag

SEQ.ID. NO. 14

KLK-L2 aa

MATARPPWMWVLCALITALLLGVTEHVLANNDVSCDHPSNTVPSGSNQDLG

AGAGEDARSDDSSSRIINGSDCDMHTQPWQAALLLRPNQLYCGAVLVHPQW
LLTAAHCRKKVFRVRLGHYSLSPVYESGQQMFQGVKSIPHPGYSHPGHSNDL
MLIKLNRRIRPTKDVRPINVSSHCPSAGTKCLVSGWGTTKSPQVHFPKVLQCL
NISVLSQKRCEDAYPRQIDDTMFCAGDKAGRDSCQGDSGGPVVCNGSLQGL
VSWGDYPCARPNRPGVYTNLCKFTKWIQETIQANS

SEQ.ID. NO. 15

Table 11

GGATGCTTACCCGAGACAGA

SEQ.ID. NO. 16

Table 11

GCTGGAGAGAGATGAACATTCT

SEQ.ID. NO. 17

Table 11

GGTGATCTGCGCCCTGGTCCT

SEQ.ID. NO. 18

Table 11

AGGTGTCCGGTGGAGGTGGCA

SEQ.ID. NO. 19

Table 11

CCGAGACGGACTCTGAAAACTTTCTTCC

SEQ.ID. NO. 20

Table 11

TGAAAACTTTCTTCCTGCAGTGGGCGGC

SEQ.ID. NO. 21

KLK-L3

      1 cttgaaccca ggaggcagag gttgcagtga gctgagatcg cgccactgta
cttcagcctg
      61 ggtgtcagag caatactccg ttttggaaaa caaacaaaca aacaaacaaa
caaaaaacag

121 atggagcaac tgagagaggt cttgtgactt gcccaaagtc acacacctca
tcactaatca
181 cacctaatca ttgagatttg gacacacatg gttcagttcc agagtccatg
ctccaaacca
241 tgacgacaca gtgagagaac attcaagggg agcccagacc cagcttcata
accaggcctg
301 tgagcaggag aaagtggaag ggatcgtaag tgcccagggg aggcaaagat
ggactctgcc
361 tgaggatctc agagatttcc tggaggaggg agaattgagg ttgggtgttg
aaggatgagt
421 gggagttcac caggaaaaga aggatatgga gaaagacatt cactcattca
atgaacatct
481 cctgaggact tctgcaagcc ctgttccgcc tggaacgggg tgatgctggg
acacagagat
541 gagtcagacc tgggcccagc cctccagaag ctgtccacct ggtgagaagg
aatgatgagg
601 agagaggcag ggaggatggg gtgatggaag ggacaatggg gtgggggca
gggagatgga
661 tgaaaaaaat atatagcaaa tgttctcagg atttggcaaa gatcaggatg
tattaagaga
721 gagcacaggg cacttgctac ctggaaggtt gggcacctgg gtccttgggt
ggtggagccg
781 tggggaaggg ggcaggttat gacaagagtg ggttaatcca gatggaacca
gatttctcaa
841 cattctagga gagggccttg tccttgtggg aagaggccca aatccccagg
gcagggaagg
901 ttctgcaagg tgtgtaaacc tgtgcagctg cctgtggtct ctgcctcact
ccacctggat
961 ttccctcaat ctttcccgtg ttctgtctcc tcctcccact cctcctctca
tcttgggtcc
1021 ttctgtgcct gtacctccct ctctttgtat cttttgctct tgtgtctgag
tcctgactct
1081 gtcttccacc cctcgcctcc tttctgggtg gtccccctgc acatccctcc
agcctgccgt
1141 gggaggttgg tctctgcaca ccactgcttt atccaaaata aacctgctgc
accccaggac
1201 cttaggcttc aaggatctcc ctccttttcc aggacacaaa agattctgta
tcttgtagcc
1261 taaggtgatg aggaatgagg tctcccactc tgaagacccc agaggaggtg
cccacaacct
1321 ctccacaccc ccagcactcc tcctccattc agtcaagctc tggcccagca
agccgccagt
1381 tcatcccaaa aggggggtcc ccctgcactt acctcctctc ccaaggcccc
tgtcacagcc
1441 ccagggcttc cccctccccc aggtacattt cccaaccccg attaatcaca
ggggcggccc
1501 catggaggag gaaggagatg gcatggctta ccataaagaa gcactggacg
ccgggtgcac
1561 gttccaggat ccaggtgccc aggggtcatg aagctgggac tcctctgtgc
tctgctctct
1621 ctgctggcag gtgaggctcc caggctggct gccccttcac ggctgtacta
aggtcacctt
1681 gctcttccct cccatcccag gcttctgcct cctgccctct aggcttctca
gcatcctctc
1741 cctgccctcc cagcctgctc ttcgctgacc cctttgtccc tcatccccac
cccagggcat
1801 ggctgggcag acacccgtgc catcggggcc gaggaatgtc gccccaactc
ccagccttgg
1861 caggccggcc tcttccacct tactcggctc ttctgtgggg cgaccctcat
cagtgaccgc

1921 tggctgctca cagctgccca ctgccgcaag ccgtgagtga cccaggctgg
ccatgctggg
1981 gagggacaga ggctgggggt caggagaggg tgaggggtgc tttaggccag
aagtgcggag
2041 cctccacttc tgataccaca agttcaactc ttagaagtag gaagggtagc
ctcccaaatc
2101 ctaaaattct agagaccagc aatatctcat ttgagaagtc taagattcga
aacttaggct
2161 cttcgaatcc gagactgacc cagagaaatc cagaatcgta gaatcctaaa
atcttgaatt
2221 tatgaaattc tgcaatagcc tcagcaaatt ttagaatcat agattcgcag
actattagaa
2281 tcttagcagt ctgggtcagc actgcccaga ggaattatga tgccagccac
atgtgtaagt
2341 ttaaatttct ggtggacaca tttaaaaaat aaggaatgag taaaattaat
tctaatagat
2401 ttaacttgac atacccaaaa acttattttg acatgtaatc aatttttaaa
tacgtatgaa
2461 cgatacagtt tacttttgtt ttggtactaa gcctttgaaa tctgttctgt
attttacaca
2521 catagcctgt tacaaaatgg actagccaca tttcaagtgt tcaatagcca
taatggctag
2581 tgtgatccta gaatcttaaa ttcagagctt tctagattca ttgaatattg
aaactcacag
2641 tactagaatc tttgattcac agtatcctag aatattgaga ttcagataat
tctgtagtct
2701 taaactattt gaatcccaga ctcttaaatt tctaaggtta tagatttata
gaatgatgac
2761 attctagtct ttcttttttt tttttttttt tttttttgag acagagtctc
cctctatctc
2821 ccaggctgga gtgcagtggc acaatctcag ctcactgcaa cctctgcctc
tcgggttcaa
2881 gcaattctcc tgcctcagcc tcctgagtag ctgggattac aggtatgcac
caccatgcca
2941 ggctattttt tttttttttt tttttttagt agagacgggg gtttcaccat
attggccagg
3001 ctggtcttga actcctgacc ttgtgatctg cccgcctcgg cctcccaaag
tgctgggatt
3061 acaggcgtga gccaccgcgc ccagccaaaa ttctagtctt tttgtcctag
aacattaaaa
3121 ttctatgttc aaatcttaga tttaattcag ataatgttag aatcctggag
tttttttgat
3181 ccaggggaat ctggaatgtt agaatcttgg attcataaaa ctctaaacct
tgagcctcta
3241 gattctagaa tcatggataa tagtgtgtcg gaatctgaga attctagaat
cttaggttct
3301 gggcattcta atagtatcct ggaatccacc tgatgcagga atcctctctc
cattgcctct
3361 gaaaagtgac catccatact gttccaattt tcttccctcc atgagtaaag
cactgattgt
3421 ggtaagagat gctgtgtggg aatttcccat catgcattgc tccatgatgg
aacctccttt
3481 aacttaagcc tatacatcag actgggagaa cgatgttcag atttcagccg
aaagtgaagc
3541 aggagaaatg cagagatatg aaggtggaag agagtgagag gcaggggaag
ggtaggggga
3601 tgaagggatg taggggtgag gactactttt ccagatccag agccaagaca
gcaagaatga
3661 cagagagaga cagacacaga tgtttctggt tccccaaccc tgaattcgca
gtcattagcc

3721 tgctgcctaa tgtcagaggt cagaggctgg ggaatggact tgtcatcccc
gaaaggatcc
3781 cagctgtcta gggcatggac cagaaatgaa acaagtgcgc tgagactgtg
gtgagggctt
3841 aaggttagac accaggaaga catgcattga agggtgaagg atatgataga
caggaaaagc
3901 tgaggccaga gatgaccccc aatttgggga ttttccatat cccatcccct
ttcatacaca
3961 cgcacacgta tacacacaca ccacttagac atacagagcc gctcccacag
aagccaccag
4021 acctgtgggg gcaggggtgg ggcggttgtt atgtggtagg tggggtcccc
cgtgcccaca
4081 ccgttcctag ggacccaagt caccaccaag gctccaggtg agtagggagg
aaggtggctc
4141 actcagcctg ggactaggag cgggggcttt gtggggagag ctacaaagat
ggagacacac
4201 aaaacatcag agtggggacc agggacccag aggaggtgtg tgcctcgctt
aaaatcacag
4261 taccctgggc cagacataga tgatgagggt gcagagaggg tgtgtggctt
gcagagggtc
4321 acacagcacc ctgatggaca ggaaaagagg gctggggctg aaaggacttt
tacctttccc
4381 ccagcttgac ctctgaggcc tgtcccagca ggtatctgtg ggtccgcctt
ggagagcacc
4441 acctctggaa atgggagggt ccggagcagc tgttccgggt tacggacttc
ttcccccacc
4501 ctggcttcaa caaggacctc agcgccaatg accacaatga tgacatcatg
ctgatccgcc
4561 tgcccaggca ggcacgtctg agtcctgctg tgcagcccct caacctcagc
cagacctgtg
4621 tctccccagg catgcagtgt ctcatctcag gctggggggc cgtgtccagc
cccaagggta
4681 tgacctggcc cagaactctc tctgaaactt gctccctcac ccctctgtct
ctgccttttc
4741 atctctgtct tctccttttc tctctcctct ctctctctgt cagtctatct
atctgccaat
4801 cgatatattt aaccaaatat aagatgctag catttttaag atgtgccatt
atttcatgaa
4861 ctgcgaagaa gtggaagaag gaggaggagg agaagaaaaa aaggaggagg
aggaaagatc
4921 ccattagatc ccattgatta tataacacca ttttctggaa gacacattct
aatttcagag
4981 tgtttgtttg tttgtttgtt tgtttgtttt tgagacaggg tctcgctttg
ttgctcaggc
5041 tggagtgcag cggtgtgatc acggctcatt gcagctttga actcctgggc
tcaagtgatc
5101 ctctcgcctc aacctcccaa gtagctggga ttacagatat gcaccaccac
atcccacacc
5161 ggggtcattt ttttattatt tattattatt attattatta tctttttttt
tgtattttta
5221 gtagagacag aggtttcacc atattggcca ggctggtctc aaattcctga
cctggtgatc
5281 tgcccgcctt ggactcccaa agtgctggga aaacaggcat gagccactgc
acccagccaa
5341 aattctagtc ttttttaaat ctagtcatat cttagattta attcagataa
tgttagaatc
5401 ctggagtttt ttgatccagg ggaatctgga atgttagaat cttggattca
taaaactcta
5461 aacgttgagc ctctagattc tagaatcatg gatactagtg tgtcagaatc
tgagaattct

5521 agaatcttag attctgggca ttctaatagt atcctggaat ccacctgatg
caggaatcct
5581 ctctccattg cctctgaaaa gtgaccatcc atactgttcc aatttcttc
cctccatgaa
5641 taaagcactg attctggtaa aagatgctgg gtgggaattt cccatcatgc
attgctccat
5701 gatgggacct cctttaactt aagccttatg ctaaaaattt ttattatttt
tagcaaagat
5761 gaggtcttgc tatgttgtcc aggctagtct caaactcctg gcctcccaaa
gtgctgagat
5821 tacaagtgtg agccactgta cctggcccag agatgtttaa atgtgaaatg
cgttcatctt
5881 agaatgggaa taagaccatg tctctcagag tcacggatca ctgacccatt
agccaaattg
5941 ggtcagtgga ttggaaaaac agtctgaatt tgttgctgcc aatatctaaa
acttggaaag
6001 ttttatacaa aagccaggtt tctggattca cctgaaaaag tttgaagaac
tcacattccc
6061 aaaatagcaa gcattgggct gagtcaatgg aggctgcccc cttcagccaa
gataagttct
6121 ctgattcact ccaatggacc caaatggctc ctgtctccct gcacagcccc
cgtccccgac
6181 ttctgtttac caattctgtt tatcatatcc cttgatgcat cggagcctgc
acccatgtct
6241 tatatagatg cacatgtgta ttatatatcc atatccacat ctatactgac
tacactgtat
6301 ctggtatctc tgtctatgtc tctgtctcca tcagtgacca tcttcctgca
aatctctttc
6361 cttttatctc actgccttca ttccacccct tgaggtctgg gtctttttct
atttcttttt
6421 tttttttttt taagagactg agtcttgctc ttgttgccca ggctggagtg
cagtggtgtg
6481 atctcggctc actgcaacct ccacctcctg ggttttaagt gatcctcctg
cctcagcctc
6541 ccgagtagct gggactacag gtgtgcaaca gcatgcccag ctgatttttt
gtattttcag
6601 tagagacgga gtttcaccat gttggccagg atggtctcaa tctcttgacc
ttgtgatccg
6661 cccgcctcag cctcccaaag tgctagggag ttatatatgc atctcctctt
atctcttggc
6721 tctctgcatg catctttctg tttctcttcc ttcctttctt tttttttttt
tttttttttt
6781 tttttttttt ttttttgaga cggagtcttg ctctgtctcc caggctggag
tgcagtgacc
6841 agtctcggct cactgcaacc tccacctccc aggttcaagt gattctcgtg
cctcagcctc
6901 ccgagtagct gggattacag gcgcctgcca ccatgcctgg ctaattttg
tatatttagc
6961 agagatgggg tttcaccatg ttggctgggc tggtctcaaa ctcctgacct
caagcgatcc
7021 gccggcctcg gcctccaaaa cactgggatt acaggcatga gccacggtgc
ccggccagcc
7081 tctctctcta cttggccctc ttcctccttg tctccatttg tttctcttgt
gtgctatgac
7141 tgtctgtctg tcactgtctc ttgtctctat ctttgagagt cctaaatgtg
gctccattgg
7201 tcctttggaa aagctgcagg gaggactcag ggcagtgggg tgctgagtgt
gttggagaca
7261 gttgcagatc cttgacagtt ctcttccctg acagcgctgt ttccagtcac
actgcagtgt

7321 gccaacatca gcatcctgga gaacaaactc tgtcactggg cataccctgg
acacatctcg
7381 gacagcatgc tctgtgcggg cctgtgggag gggggccgag gttcctgcca
ggtgagacct
7441 tactctgggg aaaatgaggc tgtcctgcca agttttctag gatttagggg
agcagagggg
7501 tcggccccca gccttcctgg gtcaaaatga gaaggagact gggatacctg
gttcctggga
7561 gaggacggga ccagggcctg gactccttag tgtaaaagag aaaaggtctg
gaggtccaga
7621 cttctggatc tacaggagga gtgggctggg cgtccagagt ctgagtcctc
ggggaggagg
7681 aggttaggtc ctgcggggag gtgggccctc tgagctttta ctcctgggtc
tgaggaagaa
7741 gaggctggag atggaggact ctcggatgtt ggaggaggaa ggggctgggg
cctttctggg
7801 agggaggaag tggcccgtgt aattgtcatg aacagagtgg cctaacagtt
cctctgccct
7861 tctctcgcgt acagggtgac tctgggggcc ccctggtttg caatggaacc
ttggcaggcg
7921 tggtgtctgg gggtgctgag ccctgctcca gaccccggcg ccccgcagtc
tacaccagcg
7981 tatgccacta ccttgactgg atccaagaaa tcatggagaa ctgagcccgc
gcgccacggg
8041 ggcaccttgg aagaccaaga gaggccgaag ggcacggggt agggggttct
cgtagggtcc
8101 cagcctcaat ggttcccgcc ctggacctcc agctgccctg actcccctct
ggacactaag
8161 actccgcccc tgaggctccg ccccctcacg aggtcaagca agacacagtc
gcgccccctc
8221 ggaacggagc agggacacgc ccttcagagc ccgtctctat gacgtcaccg
acagccatca
8281 cctccttctt ggaacagcac agcctgtggc tccgccccaa ggaaccactt
acacaaaata
8341 gctccgcccc tcggaacttt gcccagtggg acttcccctc gggactccac
cccttgtggc
8401 cccgcctcct tcaccagaga tctcgcccct cgtgatgtca ggggcgcagt
agctccgccc
8461 acgtggagct cgggcggtgt agagctcagc cccttgtggc cccgtcctgg
gcgtgtgctg
8521 ggtttgaatc ctggcggaga cctgggggga aattgaggga gggtctggat
acctttagag
8581 ccaatgcaac ggatgatttt tcagtaaacg cgggaaacct ca

SEQ. ID. NO. 22

KLK-L3

VHFPTPINHRGGPMEEEGDGMAYHKEALDAGCTFQDPACSSLTPLSLIPTPGH
GWADTRAIGAEECRPNSQPWQAGLFHLTRLFCGATLISDRWLLTAAHCRKPL
TSEACPSRYLWVRLGEHHLWKWEGPEQLFRVTDFFPHPGFNKDLSANDHND
DIMLIRLPRQARLSPAVQPLNLSQTCVSPGMQCLISGWGAVSSPKALFPVTLQ
CANISILENKLCHWAYPGHISDSMLCAGLWEGGRGSCQGDSGGPLVCNGTLA
GVVSGGAEPCSRPRRPAVYTSVCHYLDWIQEIMEN

SEQ. ID. NO. 23

KLK-L3

MKLGLLCALLSLLAGHGWADTRAIGAEECRPNSQPWQAGLFHLTRLFCGAT
LISDRWLLTAAHCRKPYLWVRLGEHHLWKWEGPEQLFRVTDFFPHPGFNKD
LSANDHNDDIMLIRLPRQARLSPAVQPLNLSQTCVSPGMQCLISGWGAVSSPK
ALFPVTLQCANISILENKLCHWAYPGHISDSMLCAGLWEGGRGSCQGDSGGP
LVCNGTLAGVVSGGAEPCSRPRRPAVYTSVCHYLDWIQEIMEN

SEQ. ID. NO. 24

Table 13

CATGCAGTGTCTCATCTCAG

SEQ. ID. NO. 25

Table 13

CATGGAGGAGGAAGGAGATG

SEQ. ID. NO. 26

Table 13

CTTCGGCCTCTCTTGGTCTT

SEQ. ID. NO. 27

Table 14

GACCCTGACATTGGACATCTA

SEQ. ID. NO. 28

TABLE 14

GCCACTGCCTGATGGAGACTG

SEQ. ID. NO. 29

TABLE 14

AACATCAGCATCCTGGAGAA

SEQ. ID. NO. 30

TABLE 14
CTTCGGCCTCTCTTGGTCTT
SEQ. ID. NO. 31
TABLE 14
GGGTCAGAGCTGCAGAGAAG
SEQ. ID. NO. 32
TABLE 14
GGGCCTGTCGTCTGCAATGG
SEQ. ID. NO. 33
TABLE 14
ATGGCCACAGCAGGAAATCC
SEQ. ID. NO. 34
TABLE 14
GGTCACTTGTCTGCGCAGAC
SEQ. ID. NO. 35
TABLE 14
CCCAACCCTGTGTTTTTCTC
SEQ. ID. NO. 36
GGCCCTCCTCCCTCAGA

SEQ. ID. NO. 37
TABLE 14
ATCCCTCCATTCCCATCTTT
SEQ. ID. NO. 38
TABLE 14

CACATACAATTCTCTGGTTC

SEQ. ID. NO. 39

TABLE 14

AGTGACACTGTCTCAGAATT

SEQ. ID. NO. 40

TABLE 14

CCCCAATCTCACCAGTGCAC

SEQ. ID. NO. 41

TABLE 14

GCTTCCCTACCGCTGTGCT

SEQ. ID. NO. 42

TABLE 14

CACTCTGGCAAGGGTCCTG

SEQ. ID. NO. 43

KLK-L4 NA

1 caggaggttg cacactgttc ctcccacctc gccactgcac ccccaccaag
gatggaattg
61 gaggcggggg gcagattcca gggtcagggc tgtcaagagt gaatgaggcg
aggagacatt
121 caggagcaga gaggtttcag acgcggaggt tccgggcacg ccctcaacac
cccccttcacc
181 ttctcctcag gccccgcccg ccctgccctc ccctcccgat cccggagcca
tgtggcccct
241 ggccctagtg atcgcctccc tgaccttggc cttgtcagga ggtaagaatg
cgcgggggtg
301 gaggcgcggc ggccattcgg gacaatggta ggaggggtca ggccggaggg
ggagggggcg
361 tgggagccgc gagctccgcc ccccgcccac tcggggccgg gtccagtggg
gacagctcag
421 agctcttcct gcttgtccct gggtgacctg gtttcccggc tgaggttggc
cctccgaccc
481 cagacccttc acctcccaaa ataccctcgc agcagcccct cccgcgttca
aggcttcctg
541 tcctctctgg aaagctgaaa gacatgggtt cgcgtcctga cgctgccgct
ttgagccagt
601 agcctagcag ctgctttgtg cctaaattgt tttcatctgg aaaatgggct
taatctataa

661 gtgcttacca gagaaggtca ctgtgaatat tgaaacgagg taatgcgtcg agccttcagt
721 atgtcgcagg tagaagggac ttgaaagtta gccacttagc cgttattact ttattagtag
781 tattcctttt tttttttttt tttttttttg agatggaacc ttgctctgtc tcccaggctg
841 gaaggcagtg gcacgatctt ggcttactga aacctccgcc tcccgggttg aagcgattct
901 cctgcctcag cctcccgagt agctgggatt acaggcgccc gccaccacgc ccaactaatt
961 tttgtatttt cagcagagac ggggtttcgc catgttggtt aggctggtct cgaacttcta
1021 acttcaagta gcccgcgtca gcctcccaaa gtgccaggat tacaggcatg agccaccgag
1081 cccggcctct agtattctgt cttcatactc agccccttcc agaaccttct agattgttat
1141 tttaatcctt gggttgaccc caaacctatg tgacctcacc ccaaattggt agtccttaag
1201 atccttatgg atctttccca tctttccctg ccgttgtagg caggttctct ggaaaccccg
1261 ttcatgaatc atttattcat tcaacaaaca gctattaaac accggccact gtgctgggtg
1321 ctgtacaagc agagacacag tccctgctct cagcacctgg agtctagcgg ggacagacgc
1381 agatgttatt caaacaatta tccaaataat tagttaataa ttatcttgac atgaggtgaa
1441 gacttcaagg agccaagcca ggggcctaga gatgtaatgg cggcttcccg accagaggcc
1501 ttcccaaagg gcttgaccct tgagccaaga cctgaaaaag gagggatctg tgggtgcctg
1561 gcacctggca ccatccttgg cctgaaggtg gggtggcttt tctcctctgg cgacactccc
1621 tggattcatg cccgtgccac tcctgagtgc cacaccctag gctaggagac ccacacgcta
1681 cgccttgtgg agtcctcaac aacctggcga ggtaggtgca ttgtaattac tccaatttca
1741 tggcagagaa acctaggact caaagacaga aggctcctgc tccaatgaca ccggcgatgc
1801 ctgagtcaga atcctaatca aggttgtttt ccctgtccat atcctggact tgaggctctg
1861 aaaaccattt ttataacttt tgacctaatc atttgcttaa agttagcttt ttttcttctt
1921 ttttcactca aacaaaagca tgttcaactt tatattactg tcctgaatag agaatagaat
1981 tctttgtcat aaatagaagg taaggaagga aataaatcct gcacaatgaa aagaaaataa
2041 tatgtttatt gggttggacc acctgaaatt gctgatactt gacccttttt gaccttccta
2101 aaacaacttt tgcagatggt tcagtgtaat aaatgttagg tggcctgatg aggcttctgt
2161 gtcctcctgg ctttgaaaag tgagctcagt gaggattagg gaggtgttaa aaccatatta
2221 gcaccatcct gagactttat ccttgacaaa atcaggttta aaagagaact ggatgctggt
2281 tcagcgtctg agtgtgcgat ttaacgttac ttaaatctca tctctctacc atctaaaatg
2341 atcctgtgct caccgacaac ttctgtccct aactgcaaac cactgagcta atccaactgc
2401 ttgccctgta gttggggaaa ctagctaggg aggcagaggg acctcctgtt gtagctaata

2461 attaataata acatttccca ctgactgagt gctctccatg ccacctgctg
tgctgcacgg
2521 tttgaaatgc aggatcatct tgaattcttc aactgcgcaa tgagagatga
actattactt
2581 tttctacttg acagctgggg aaactgaggc tggtgatttg cataaggtca
cacagtcaca
2641 aaatggcatg catgttcagg attggattct ccctgtccca cggacccctg
ctgtgctttc
2701 aatgccagac acagtgcctg gcacacacag catttattta ttgagccccc
attgtgtgcc
2761 aggcgctgtg ttaggtcctg ggaatatggt actgaataaa gcagttaagg
tgcctgttgt
2821 caatggagct tacagtcaaa gtggagagat ttttaaaaac gaatacatac
aaatgtgaag
2881 agaaatgaat agcaatcatt gttctgatga agaccaactg gaagaatgta
atgggggagg
2941 agtcgggacc aggagagtca acattagacc aggtggtcag ggaaggcctt
tctgaagagg
3001 agacatttga gctgacctct cagaattaag aaggacccag acatacaacc
tctaaattct
3061 gagggtcatc cagtagaata ttccatatat gtatatatga aatatcctat
atctgtgctg
3121 tccaattatc cactagcccc ttcaggctat tgaacatttg aaatatggct
ggtgtgactt
3181 aagaactgaa tttttaattt agttttactt cattttaatt agtttaaatt
taaatagcca
3241 catgtagcta gtggctacca tattaaacaa cataggtctg gagaaaggac
tgtgcagaga
3301 gaggaaatag caagtataaa atgtctagta tgggggcatc caagatgatt
taaattcttc
3361 ttttctttaa atgcctggtg tgtttgaaga acaggcccat gaggctggac
tagaggaagt
3421 cagaagaaag aggttggaga tggggtcaaa gaggctggca agggccagac
agcacagagt
3481 cctgcacacc ttgggaaggc tttttggatt ttattttaaa gaaagttgag
cctgggaaca
3541 acatctgact ttctttgttt gaagagtcct cagcctactt tgagaagact
ggatcggagg
3601 gatgtaaaag tggaaggatt taggttaatg ttgtagtcat ttgggctaca
gaagatgggg
3661 catggaccaa gatggtggca gaagtgtgga gataactgga tatttgggag
ataaaaccaa
3721 taggaactgg ttgtgagtga tgaaggaaag aagagaagca aagatgactc
ccaggtttgg
3781 ggctgagcac tgaggtggga aatactggag cgaacagttt tgattgagaa
gaatcaagtt
3841 gggaatacaa agcttaagat gcctgtaagg catccaaatc aacagtgttt
gagttttgag
3901 cttaaagaag agttcagggc tggagatgat tagcctatag ctggtattta
aagccatgga
3961 ggcaaccagt atatatgcag tgaaaggata gagagatggg tggaaagatg
attggatgga
4021 tgcatggatg gatatatgga tagatggatg gatggatggt tggattggat
ggatggatgg
4081 atggatggat ggatggatgg atggatggat ggatgaataa atggaccagt
ggatggaggg
4141 acagatgagt ggatggatgg ttggatggat ggatggatgg atggatggat
agatggttag
4201 atgactacct aaatggatga atggatagat ggatgagtag acggatggac
aaatcaatag

4261 gatgaatggg ggatggatga ttggatagat tgatggatag atattgccta
ggtggatgtg
4321 taggtcagtc tcacttctac ctcctgaaat ccatcttctg gtagaatgat
ataaaaaatg
4381 catgtggaga gaaagtcagg ctcctgctta cctatcagca acatcctcat
tttgtgaact
4441 cttctgttaa cccccagtgg aggatttggt acttcctgag aaaataatgt
caccccttttg
4501 ccctaattca tctccacttg gtcaagaata gcaactgcca taggtcggca
aattcatctt
4561 cagttcctgg tcacccaggg caataatccg acccttaccc caaacccaga
aaccacaacc
4621 ccagggctcc tctgccccct ggatcccagt tttctaacaa tctctcttct
ttaccaggtg
4681 tctcccagga gtcttccaag gttctcaaca ccaatgggac cagtgggttt
ctcccaggtg
4741 gctacacctg cttcccccac tctcagccct ggcaggctgc cctactagtg
caagggcggc
4801 tactctgtgg gggagtcctg gtccacccca aatgggtcct cactgccgca
cactgtctaa
4861 aggagtatgt gggggccggg ggagcatggg gtagggatga gaatgggact
gggattgtgg
4921 atggggtaga gttggatttg aggatggagt tggagttagg gttggggatg
gacatgggag
4981 tgagaatgag gtttggggtt gagatatggg gattgggtat gggaatagaa
tcaaagtagg
5041 ggatttggat gggattgaag ttgaggatgg gggagatgta tttggagatg
aggaaggtag
5101 gatggagaag aagttaggtt ggggatggga agaggttggg gctgggatgg
ggatggaaat
5161 gggctcatct tctttcctaa ccaccttctt tctgcaccca cagggggctc
aaagtttacc
5221 taggcaagca cgccctaggg cgtgtggaag ctggtgagca ggtgagggaa
gttgtccact
5281 ctatccccca ccctgaatac cggagaagcc ccacccacct gaaccacgac
catgacatca
5341 tgcttctgga gctgcagtcc ccggtccagc tcacaggcta catccaaacc
ctgccccttt
5401 cccacaacaa ccgcctaacc cctggcacca cctgtcgggt gtctggctgg
ggcaccacca
5461 ccagccccca gggtatgcac ccacacaggt ggcctgaggc cccataggag
tggctgggga
5521 aacaggggca gagatgggag ggaaggtctg aggtaggttc ctttatatat
aaaaatataa
5581 ataagtaaat aaatatatat atttaaagtt agctgtatcc tttatataaa
tataaattca
5641 tgaatatata aaaatatgag tatataaatt catgaatata tagaaatata
aatagatcta
5701 atatatgaat atattatatg atgtatatta tgtattatat agtaatataa
ttatatatta
5761 tacaaaaagt atacaaatta aatgtatttt ataaattata aaatttatca
attatgtatt
5821 ttaaatatgt atttctgcat aatgtatata ttatatataa tctatattta
aattatatat
5881 tataaatgta ttttataaat gtatacattt atatatttat atactgtaaa
tgaattttat
5941 catttataat atataaatca tacatataaa atgtttatat ttctataatt
tataaaatgt
6001 ttaatatatt aaatatggtt attaatgaaa tgtctaataa ttcaatgtaa
taattaattc

6061 tatatcatta cttagtaagt ataatacatt atatatgtga atataaagtt
gatgtatata
6121 ccgacaagag ccctttgcat ctccctagca atccctgact ctctcccagc
ctcatgtttg
6181 tatctttctc ctcaacatgc cctgtctctc ttcctaccat tctatccaac
tctcccgtaa
6241 ctcttcccat ccctgttcct gcttttccca tctttaattc tctatttctg
accatctccc
6301 tattccaact ccctctctcc aactttctct ccccaccgct ggctccacca
ctctccttat
6361 caaccttcca ttctcttgtc ccttccctcc ttgtccttcc ctccactttt
ctcctcatct
6421 ctcccttcgc ctctctccca tgtccctcca tatttctgtc acttccgttg
ctttacccag
6481 ataggtgctc atctcttctc ccatctttct cttcccatct caattttcta
tctactcttt
6541 acccattcaa ctcgcctatt tcaccttcat cccatatcct atccaggtcg
gataccttag
6601 accttctctt tcttctcccc agtgaattac cccaaaactc tacaatgtgc
caacatccaa
6661 cttcgctcag atgaggagtg tcgtcaagtc tacccaggaa agatcactga
caacatgttg
6721 tgtgccggca caaaagaggg tggcaaagac tcctgtgagg tgaggccggg
aggctggtgg
6781 gtgccttgga caggatagaa agccagaatg gaagtgacag atgctgggga
aaaagctttg
6841 tttccagcct taggggaacc aatctttata agatacaatg tcccctcaca
taggaggtca
6901 agacaaaaag gggtacccag ggatggcagg aataattcat cataagcccc
agctttgact
6961 gagtggctgc caagatccct gtgttgagat gcataaaggt tggtattctt
tcacttgtga
7021 gtgatagaca accaactcaa actggcttaa acaaaatgca ggcttttgta
actgaaaatc
7081 caggttgtct ggctttaggc acagatggat ccaggtatgc aaattgtgtg
tttggaattc
7141 tgtctttctt ttaactctca gctcttcttt attctgtttt ggcttcattc
tcggttagat
7201 tcttcccatg acaagatggc cccagcagct ttgagcttac atcctaccct
ctaggcaacc
7261 ctattagaaa gagaacctct cttttccaat agttcacaca aaagtcttaa
gcatgattct
7321 cactaggctg acctaagtca tgtgtcttga gccatcactc caccagagct
gtgggattct
7381 ctgatgggcc aagcctgagt cacatagtta actgtgggtg ctggagaggg
gcagggacaa
7441 actgcatgga ttggaagtgg agaagggcag ttccccaaat gaaaaatca
ggagaggctg
7501 ttaccaaaat aaggggaaat ggccaagtac agtagttcat gcctgtaatc
ccagcacttt
7561 gggaggctga ggtgagagga ttacttgagc ccaggagttt gagaccagcc
tgggcaacat
7621 agtgagactc tgtctctaca aaaagaaaaa aaagttttta aattagccag
gtgtggtgga
7681 gtacaactgc agtcctagtt actcgggagg ctgaggcaga aggactattt
gaacccagga
7741 gttcaaggct gcagtgaggt atgatcatgc cactgcactc cagcctgggt
gatagagcaa
7801 ggccctgtct ctaaaacaaa aagaaataaa tagagcaaga cactgtctct
aataaataaa

7861 taaataaaaa tttaaaaatg aatgtttaat tttttaaaaa taagaggaaa
tggatactac
7921 atgagcaaaa aatagccttc atcaataaag aagttgagat tggattcagt
gagaaagagt
7981 atgatactat attaatgata tgtgccttga tcgattagtg atgtctgcct
tgggcccagg
8041 aagagaaata gacttacacg tgtgttgcat accctgccca gatatgaatg
ggttcactca
8101 atagtgagag acacaaatga gccttaaata ggagcagggt cagctggtgt
ggggcagggg
8161 gtgatttagt accagggaaa caaaaatggg tatgaagtaa gttgttacca
ttttaatgaa
8221 actgaggaac agagaaaaac acagaaattt ctctgtgtct ctctttctct
gggcctatct
8281 ctgtctttct gtccctattt ctgtctcttg ctgtctgtcc ctctgtgttt
gtcttcttgt
8341 ctgtttctca ctgtcttcat tgctttctct cacactgtgt gtgtctgact
ctgcctctct
8401 gagtctcctt ctctgtgtgt gtctctctcc atctttcact ctctccccac
acctccctgt
8461 ccctgccttg tttagcccca gcaaggaccc acctctctct ctctttcttt
ccccaactca
8521 gggtgactct gggggccccc tggtctgtaa cagaacactg tatggcatcg
tctcctgggg
8581 agacttccca tgtgggcaac ctgaccggcc tggtgtctac acccgtgtct
caagatacgt
8641 cctgtggatc cgtgaaacaa tccgaaaata tgaaacccag cagcaaaaat
ggttgaaggg
8701 cccacaataa aagttgagaa atgtaccggc ttccatcctg tcaccatgac
ttcctcacat
8761 ggtctgctta gcccttctct gctccttatt cccagtgttc catttgaacc
agtgatccat
8821 gtcctgaaaa atgctcaatc tcagctaaca ttccatgttt cagaagcatt
caggcactgc
8881 caggcttgca gtctcccaga tgttgcatcc ctgaaacatc tcaacaacct
gaatgtccca
8941 acccagacaa tggcccaggt ctctcaactt catcagtgtg gcttctatga
gcccagatca
9001 ccacctgaac gttctgtctg tggcacattc ttaaatattt ccatcagccc
atctcaacaa
9061 tatatgtcct ataaatggac catccttgac aacatcctct aactcttcaa
gtatttattc
9121 aatgccagta tcctagacct tctatttttt gcactcaaga aggctctaga
ctcccatgat
9181 agttcatcct gaaaatattc tcttatgccc acaatcttct gccctgacaa
cattctgtgt
9241 acctctgtga ctcaccacag ctaacattgg atcctcagaa tatttcattc
tcacactgtt
9301 atgggtgtct cagaagtccc aacccaacct acatcccaca ttcttccaat
accccacctc
9361 tgccaacatt ccctctctga atcaatggca ccctagtctc tagagttata
gggttcagta
9421 taccaaaggg tcttcttgcc tgaactttat tgtctaccaa atattccgtc
ttgtatcccc
9481 tccatgaaca tccttggtca gtgtcccttg ctgttacatc tttgtgcatg
accctaaaat
9541 gtagtgcaaa tccttgcttt ggacaagtta taaaactcac agtctctgtg
ctttctcatc
9601 tgtaaaatgg gttcataatt ttttttaatt gtaacattat tacaagaata
aatgtcaagc

9661 atttatcact attattattt gcatggttcc cataaaatat taccttagaa
tgttaataac
9721 agcccttcga atttgcagag tgtccaaaaa aagtgttgca ctgatttatt
ttcctcagga
9781 gacatttctt cagtgttgac tatgtgcaag cactctcctg ggtgttgtta
aatatagttt
9841 atttactcaa caaatatttg tacctatcaa gagccaggca ctgttgcaga
gacaagtgat
9901 aaccaatgag ttaaacagat aaaaacttct gcccttgtag aacttacatt
cttttcaaga
9961 agtctccata acaatgaata aagaaatagg ctgtcaggtg gtgctgcaag
ccatagcaag
10021 aaatgaaaca agggccatat gtggtagctc atgcctgtaa taccaacact
gggaggccaa

SEQ.ID.NO. 44

KLK-L4 AA

ESSKVLNTNGTSGFLPGGYTCFPHSQPWQAALLVQGRLLCGGVLVHPKWVL
TAAHCLKEGLKVYLGKHALGRVEAGEQVREVVHSIPHPEYRRSPTHLNHDH
DIMLLELQSPVQLTGYIQTLPLSHNNRLTPGTTCRVSGWGTTTSPQVNYPKTL
QCANIQLRSDEECRQVYPGKITDNMLCAGTKEGGKDSCEGDSGGPLVCNRTL
YGIVSWGDFPCGQPDRPGVYTRVSRYVLWIRETIRKYETQQQKWLKGPQ

SEQ.ID.NO. 45

KLK-L4 AA

MWPLALVIASLTLALSGGVSQESSKVLNTNGTSGFLPGGYTCFPHSQPWQAA
LLVQGRLLCGGVLVHPKWVLTAAHCLKEGLKVYLGKHALGRVEAGEQVRE
VVHSIPHPEYRRSPTHLNHDHDIMLLELQSPVQLTGYIQTLPLSHNNRLTPGTT
CRVSGWGTTTSPQVNYPKTLQCANIQLRSDEECRQVYPGKITDNMLCAGTKE
GGKDSCEGDSGGPLVCNRTLYGIVSWGDFPCGQPDRPGVYTRVSRYVLWIRE
TIRKYETQQQKWLKGPQ

SEQ.ID.NO. 46

Table 15

AACTCTACAATGTGCCACA

SEQ.ID.NO. 47

Table 15

TTATTGTGGGCCCTTCAACC

SEQ.ID.NO. 48

Table 15

GGATGGTCCATTTATAGGAC

SEQ.ID.NO. 49

Table 15

AGGCTGCCCTACTAGTGCAA

SEQ.ID.NO. 50

Table 15

ATATTGCCTAGGTGGATGTG

SEQ.ID.NO. 51

Table 15

AAGACTTCAAGGAGCCAAGC

SEQ.ID.NO. 52

Table 15

GACCCTTCACCTCCCAAAAT

SEQ.ID.NO. 53

Table 15

CTAGTGATCGCCTCCCTGAC

SEQ.ID.NO. 54

Table 15

GGTGATCTGCGCCCTGGTCCT

SEQ.ID.NO. 55

Table 15

AGGTGTCCGGTGGAGGTGGCA

SEQ.ID.NO. 56

KLK-L5 na

1 attgaggggg gatcccagca ggtcccattt gttcagattc ttctgggcct
ttctgtgttg
61 catttcttcc tctgcaggac acctgtcaca tgagggtctt caggggaaaa
ggaattctag
121 tgtctgtgac tgcttcaaag gagaagtaag gggagaggag aggaaagcag
gaggaggttg
181 gagagaacct cttgttcctg aggtcttcca atctccttca gctcaaagca
ctcagcatgc
241 tgaagttcca gactctgggc tatcacgttc tgacatgcaa cacaggcaac
accccagctc
301 catccacgtt tctccaaaag cacaggcatg acgtcatatg gtgacaaaca
cccttgtcca
361 aaggaagccc ataggatacg ctaattctag attcacaaat actctagagg
aactcacaca
421 atgggatggg ccagtgcccc acacagagta tgaggcctcc caccttggtt
gaatatcttt
481 ttcttttttt tctttttttt tttttttgag acggagtctc actctgtcac
ccaggctgga
541 gtgcagtggc tcgatctcgg ctcactgcaa cctctgcctc ccaggttcaa
gtgattctcc
601 tgcctcagcc tctggagtag ctggaattac aggtgcccac caccacaact
ggctaatttt
661 tgtattttta gtagagacga gggtccacca tgttgaccag gcttgtctcg
aactcctggt
721 ctcaagtgat ctgcccacct cggcctagtg ctgggattac agacgtgagt
caccacgccc
781 ggccccacct tggttaaatt tctgaaaatc atttggtaaa gtgaggaccc
ctccagctga
841 gacactgcca ggaaacagct attgagtctc ttagcaccca cagcattaaa
acaaacccaa
901 aacattttag gcctcgttga gttctggagg caaaatattc ctcatctaca
aatttatttt
961 attttatttt ttaataaaag ttattattat ttttttttat agagagacag
ggtcttgctc
1021 tgtgacccag actagagtgc agtggtgtga ccatggttca ctgcagcctc
cacctcctgg
1081 gctcaagcga tcctcccacc tcagcctgcc aagtagctgg gaccacaggt
atgcccctcc
1141 cccaggctaa ctttttttta ctttttgtgg agatgaggtc tcactatgtt
gcccatgctg
1201 gtcttttttt tttttttttt tttttttgag acgggagtgc aggctggagt
gcccaggctg
1261 gagtgcagtg gcacaatcat aactcactgc agcctcaaac tcctggggtc
aagtgatcct
1321 cccacctcag cctcccacat aactaggact acaggcctgt gtcaccataa
tgcctggtta
1381 atttttttag tttttgtttg tagaaacagg gtctcactat gttacccagg
ctggcctcta
1441 actcctggcc tcaatccatc ctcccatctc aacctcccaa agtgctagga
ttatagccac
1501 gagccaccat gcccggcccc atacttatat ttacttgtag tgagaacact
taaaccctac
1561 tcggtcagta attttcaagt acacaataca ttgttactaa ctatatacat
ggtattttta
1621 atttgcatga atgctcctta aaatcagcga gcccgcctta tttttgtat
tcaattttat
1681 tggatataaa ttccacatca gtaaacctga ttctcttaaa aatctacaca
gaaaaaaaaa
1741 aagagagaga ggttgagttg ggtagttgtt ggtatttttg tttttgtttc
tttgcttatt

1801 cagttgaggt ttattgtaaa acttgacacc agaaaaagga agaagtggcg
tttttgtgct
1861 gtgagcgtgt gacgtgtgtc ctttccagag aaaggacagt catggtgctt
ttttatcctc
1921 tctgcccaaa gaaggaaagc tcttaacagc cagcaggagg ctttgtaggg
accagcgtta
1981 tcaacgccag tcgcgctgac caatgatgag ttaaagcagt taggtcgttt
ctaagagcaa
2041 atcaaaagct aaggttctgt gattctgaaa atgagacacg gacagagact
ggagacccag
2101 agagaaagtg aaggactaaa agacagtcat agggtgggag tttgctctcc
tctgttttgt
2161 tctgggtttt tttgtttgtt tgtgcgctct gtctgaccgc ttttcttttt
tttctttttc
2221 tttctttttt tttttttttt ttttgagatg gagtcttgct ctgccgccca
ggctggagtg
2281 cggtggcaca atctcggctc actgcaagct ccgcctccca cgttcaaggg
attctcctgc
2341 ctcagcctcc cgagcagctg ggattacagg catgcaccac cacactcagc
taatttttgt
2401 atttttagta gagatggggt ttcaccatat tgaccaggct ggtcttgaac
tcctggcgtc
2461 aagtgacttg cccgcctcgg cctcccaaag tgttgggatt acaggcgtga
gccaccgcgc
2521 ctggccctga ctgcttttct ccttggtttg tttgtcaatc cccttcctc
tgagccgaat
2581 tccctttttg ttctcatttt ctctctctgt cccctctctc tctccttctt
tctctccttc
2641 cattcctcct agatgaagca aaaactcaga taaaccagca cagaggccag
gtatggtggc
2701 tcacacctgt aatcccggca ctttgggaag ccaaggcagg caggttgctt
gaggccagga
2761 gttcaagacc agcctggccc acatggtgaa accccgtctc tactaaaaat
acaaaaatta
2821 gccggacatg gtggcacgtg cctgtaatcc caggtactca agaggtggag
gttgcagtga
2881 gcggagatca cagccctgca ctccagcctg ggtgacagag cgagactcca
tctcaaaacg
2941 aaaaacaaaa aacagcacaa agttcccttg tcctgtgact cattctctct
ctctctttct
3001 accatttctc cttccctgtg tctttttttt ttctctctgt gggttttatt
taagcaatag
3061 aagttcttag caaagaaaaa ctttatggaa ttagattgat ccacttcata
tgtacatata
3121 tgaactcagt tcagaaactc tcttctaccc ctgcctgatc acctatttgg
aagtctgttc
3181 cttcaactct tcttctcttt ctgggactct ttctagcttg ggcttcctgc
ccctcccgtc
3241 cactctcctg ctttcacagc ctctccttcc ccctgcccct cccctgcact
gcatggggat
3301 gggccccagg tgtccaaggt ctccccaccc tcctttgtca ctggagtcag
gattagaacc
3361 cagctcccta gtcaccttga gtcatcagtc ctggggctgc tgacgggctt
gcagaggaga
3421 gagggagtgg ggctgggtct tcccaccctg ggtcctttcc tccttcccca
ctccgtttag
3481 ctgtaaagct caattaagtg tgattagctg agaagagttt ctgcagaatt
agagcacgcc
3541 ccacccctgt cttcgtggtc cccttccctt aacccggaaa ctggatgggc
caggacaaag

3601 agagttaaga gctttgtcag tggtctgtct ggagcgacag atggaaggaa
agggaccggt
3661 tgagcaacat gacaggtggc tgaggagcca ggtgcagagt ggtagagttg
gctggcggag
3721 tggccagcac atgagaagac aggcaggtag gtggacggag agatagcagc
gacgaggaca
3781 ggccaaacag tgacagccac gtagaggatc tggcagacaa agagacaagg
tgagaaggag
3841 gtaggcgact gccaatgagg gagtgacaca caggggagca ggtagagaga
ggacaagcag
3901 gtcatcccct tggtgacctt caaagagaag cagagagggc agaggtgggg
ggcacaggga
3961 aagggtgacc tctgagattc ccctttcccc ccagactttg gaagtgaccc
accatggggc
4021 tcagcatctt tttgctcctg tgtgttcttg gtgagttctc ccggagcagg
gagagggcag
4081 gactgcgact ggatcccttc acccccatga ggaggcccca ccaccctccc
catctcagct
4141 ctggccccca gcctggtggt gaggaggaga ggggctttct ctgtgcctcc
atttacctgc
4201 agctctcagg gtactgctca cctcggtctc ccctattttt tgatccctct
tcccttctgt
4261 ccctctctga atctctgtct ctccatttcc ctcctatgtg taagcatctt
tctccctggg
4321 tgtctttgat gtttcatggt ctttttctat cactgggtct ctctctcttt
ctctctcttt
4381 ctcgtctctc tttctcctct ctctctcctg cctgtttctc tctctcactc
tgtgtgtctc
4441 tccatctctg tatcttttct tcctctctct gacccatgcc cctgtctgtc
tccagggctc
4501 agccaggcag ccacaccgaa gattttcaat ggcactgagt gtgggcgtaa
ctcacagccg
4561 tggcaggtgg ggctgtttga gggcaccagc ctgcgctgcg gggtgtcct
tattgaccac
4621 aggtgggtcc tcacagcggc tcactgcagc ggcaggtaag tcccttcctg
gggtgggcga
4681 agggaggact atgggaaggc aagcgctggg ggtaggatca caagggaggg
tggtgcccac
4741 tgggaagaag ctgatcctgc aacaagagag tctgaggtta gaccaggagt
ggaacttcct
4801 tagcagtggg cctggggtgg tgctgggcag ggtgaggtat gttgggtgga
gggccgggga
4861 gggtcctgga acctgccctc ctgcctctcc cattcctgca tgtacccttt
ctttcctata
4921 tgacatctgc cactcacccc agccattcct tgacccagtc tgggcccggg
gcccaggtct
4981 cacccaagct ctttttcttt ttcttttttt tatttttttg agacagggtc
tcgctctgtc
5041 gcccaggctg ctgtgcaatg gcgtgatcac agctcactgc tgtctctgcc
tcccaggttc
5101 aagtgattct cctgccccag cctcctgagt agctgggatt acaggcaccc
gccaccatgc
5161 ccagctaatt tttgtatttt ttgtagagac agggttttgc catgttggcc
aggctggtct
5221 cgaactcctg gcctcaaatg acctgcccgt cttggcctcc caaagtgctg
ggattacagg
5281 tgtgagccac tgcacccggc caacatgacc caaactcttt gtgcaacttc
agaatctatg
5341 cctggcacct ctctgggcct cagtagactg atgttctgga atttttttct
tttctttct

5401 tttttttttt ttttggagac agagtcttgc tctttctgtc atccaagctg
gagtgcagtg
5461 atgctatctt ggctcactac agcctcaacc acctgggctc aagtgatcct
cacacctcag
5521 cctcccaagg agctaagact acaggcctgc gccaccacac ctggctaatt
tttaaatttt
5581 ttttgtagag acagggtttt gctatgttac ccaggctggt ctcaaactcc
tcagctcaag
5641 caatcttcct gccttgacct cccaaagtgc tgggattaca ggcatgagcc
actgtgcctg
5701 gcctggaact ttttttgtga aaggggagat cagatgcaaa gaaacagaga
ctcagggaga
5761 gagagggcca gcagcaggat gcagagaggc cattcatcaa cccactcgtt
caatcatgaa
5821 cccactcgtc cacgcatgag catggagggc acatgctccg tgccaggcgg
tgggaataag
5881 gcagtgaaca aggtccactg atgtccctgc cttcatgggc ttcaccagcc
gagagaatca
5941 gaaagagagg cctggcgcgg tggctcacac ctgtaatccc agcactttgg
gaggccgagg
6001 cgggcggatc acttgaggtc aggagtttga gaccagcctg acacacatgg
tgaaacctta
6061 tctctactaa aaatacaaaa attagctggg catggtggca tgcttctgta
atcccagcta
6121 cttgggaggc tgaggcaggt gaattgcttg aacctgggag gtggaggttg
tagtgagcca
6181 agatggtgcc actgcactcc agcctgggcg acagagcgag actcggtctt
gaaaaaaaaa
6241 aaaaaaaaaa aaaggagaga gagagacaca gatgcaggga catggtagga
gaaacaggga
6301 acacccaaga tggaaagagg gtgatggagg ttgggaataa gagcctgtaa
gagagactcg
6361 gagaatgaga gttgcgggtg agaggacaga cagtgagggg cagaacagtg
gggagcggca
6421 ggagcgcctg agtgtccgtg gaggggtgca aggtggggga ctgcgtgcct
gccacccgct
6481 cagccgtcgc caccggcagc aggtactggg tgcgcctggg ggaacacagc
ctcagccagc
6541 tcgactggac cgagcagatc cggcacagcg gcttctctgt gacccatccc
ggctacctgg
6601 gagcctcgac gagccacgag cacgacctcc ggctgctgcg gctgcgcctg
cccgtccgcg
6661 taaccagcag cgttcaaccc ctgcccctgc ccaatgactg tgcaaccgct
ggcaccgagt
6721 gccacgtctc aggctggggc atcaccaacc acccacggag taaggggccc
agggccaggg
6781 gtcaggggtc aggatgggta caagtctggg atgcagggcg agaggtcgaa
tcatgacacc
6841 tcagaggaag gatgggtaaa gggtcagggt gtgggatggg acatcaggat
catggtttgg
6901 ggtcagagat tatggtggat tggggtcttg ggagccaaag gggttaaagg
actgggtatg
6961 aagtcaggga tcagaggtca gaggtcagag tgtgtcagag gtcatcacac
tggagcaaaa
7021 ggcatatata tatatatatg tatgtatagg atatgggcat tgtgggtcat
gggtctgggg
7081 ttagaggtca ccgtagaatt aaggtcatgg gatccagagg ttgtacaatc
tggtcaaaat
7141 ctgaggatgg aaattgggat tctatccaaa atcacatatc tgagattgga
ggtcatagcg

7201 tttggggtgt ggggcccgaa gtttggggtc atggaggctg gggcccaata
aactaggatc
7261 aggggacact ggcgttggaa gcagtgaggt ttggaagatg cagagctgag
gttggaggtt
7321 aaggtaaaga cagggacatg gggtcaggag acagaagata tgagatcaag
ctgggatcat
7381 aaggtaataa gacagaaggt caaagatcac agtagctggc attgaagagg
gtcaggtctg
7441 gattcgttgt ctctgacgct ggagagacaa gaaagttctt gagttatgcc
actcaaagtc
7501 aaatgtcaaa gatcaaagag accgtcaatc atctggggtc atgattcata
tgaaattaag
7561 tcataaatat gtaacttgga ggtttcggga ttgtagtaca ggtcggtgag
gggcaggggt
7621 attgacatgg atgggccaca tccagggaag agggacgtgg cctcaaagtg
gggagattta
7681 ggggaccctg cagcacgcat gttctctctc cagacccatt cccggatctg
ctccagtgcc
7741 tcaacctctc catcgtctcc catgccacct gccatggtgt gtatcccggg
agaatcacga
7801 gcaacatggt gtgtgcaggc ggcgtcccgg ggcaggatgc ctgccaggtg
agccagtgca
7861 ggcagcgtgc gtggtcacca ggacaggaag tgaaggggag gggctggaag
caggagggga
7921 actgatggag gatgaatcag ggaaagggga tgctgcagag agacggggtc
aaaaaggaag
7981 ggagaggctg gttacggagg ctcacacctg taatcccagc actttgggag
gccgaggcgg
8041 gcggatcact tgaggtcagg agttcaagac aagcctggcc aacacggtga
gactctgaat
8101 ctactaaaaa taccagaatt agccgggggt ggtggtgcaa gcctgtggcc
ccagctactt
8161 ggaaggctga ggcaggagaa tcgcttgatc ccgggaggcg gaggttgcag
tgagctgaga
8221 tcacgccact gcactccagc ctgggcgaca gagccagact ctgtctcaaa
acaaaataat
8281 taataataat aataataata ataataataa taatggagga gaggcccagg
ataagggagg
8341 gagagagaca gggagtaaaa gggaggaccg gggaatggag gagggggagg
ggcagggaga
8401 gagagggagg aagggaacag agaaggaaag atggggcagg ggttacagag
agagacagca
8461 aaacagacgg agaggactgg gagcccagac agggaaccag ctgtttctgg
ggctctaagt
8521 ctttcccata ccatcctcca gttggtgctg tcccagactg agagagattt
gaggatggcg
8581 gtctctcccc tcattggtca gggccccagc cattgtcctt gagagaactc
tgtgcttttg
8641 atggagtcct gcccaccttc cctgggattg gtcatttttg atggcactct
ctcccctcat
8701 tggtcagaac cccaggcatt gtccttgaga gaacctctat cctttatgga
gtcccacccct
8761 cctcccctgg gattggtcat tgataatagt gttctctctc ctcattggtc
agggccccag
8821 ccattgtcct tgagagaatg ctcgactctt tatgttgtct tgacagcctc
ccctgagatt
8881 ggtcattaat gactgtgctc tctctcctca ttggtcaggg ccccagccat
tgtccttgag
8941 agaacctctg tcctttatgg agttccaccc ttcttccctg ggattggccc
ctagagacag

9001 tggttcttct cttttggtta gccattgcca ttgtcctccg ggaaagtgat
tatactcttt
9061 tgtctaatga ccagacttgg agccctcccc aaggcccagg actgggttga
agggttgggg
9121 aggaaaacag aaataagatg tctcccttgt tcagacagta cttctcttcc
cttccagggt
9181 gattctgggg gccccctggt gtgtggggga gtccttcaag gtctggtgtc
ctgggggtct
9241 gtggggccct gtggacaaga tggcatccct ggagtctaca cctatatttg
caagtatgtg
9301 gactggatcc ggatgatcat gaggaacaac tgacctgttt cctccacctc
cacccccacc
9361 ccttaacttg ggtacccctc tggccctcag agcaccaata tctcctccat
cacttcccct
9421 agctccactc ttgttggcct gggaacttct tggaacttta actcctgcca
gcccttctaa
9481 gacccacgag cggggtgaga gaagtgtgca atagtctgga ataaatataa
atgaaggagg
9541 ggccatgtct gtccatttga agtcctcatg ctggttgaga ctggaagaag
gactcagcag
9601 tttccctatc tcataggagt agaaacagag ctcaaataag gccaggcaca
gtggctcaca
9661 cctgtaatcc catcactttg ggaagctgag gcaggtggat cacctgaggt
caggaactcg
9721 ggaccagcct ggtcaacata gtgaaacccc aactctacta aaaatgcaaa
aattagccag
9781 gcatggtggc gcatgcctgt aatcccagct actcaggagg ctgagacagg
agaatagcat
9841 gaacccgtga ggcagaggct gcagcgagcc gagattgaac cattacactc
cagcctgggc
9901 gacagagcga gactccatct caaaaacaaa caaacaaaaa acccagtgct
caaataggat
9961 gagggtcttc cctgagtagt tactcagaaa tggagtagaa aaagttactt
ttaataatat
10021 aggccgggtg cagtggccca cgcctgtaat cccagcactt tgggaggccg
aggtgggagg
10081 atggcttgag ctcagatttc gagatcagcc tggcaacaca gtgaaatctt
gtcactacaa
10141 aaacacaaaa aattagctgg gtgtggtggt gcgtgcctgt agtcccagct
acttgggaag
10201 ctgaggtggg aggatcaccc gagccgggga ggtggaggct gcaaagagcc
gagatcatgc
10261 cactgcactc cagcctgggc aataaagtga gaccttgtct caaaaacaaa
aacccagcaa
10321 tataaataag acacatgttt cttcatctgg cataatagaa atagtgccca
gagcttataa
10381 gcttttcaag agtccacaaa agacccgaaa aagaaaaaga aaattgttag
ctccaaaata
10441 ccagatgaaa gctgcaaagt caacatttat gaccatttaa tccaatgtcc
ataaaacgta
10501 gcattctttc cactagccaa ctgcagttta ctttcttgta atgaagcata
cattgtatct
10561 ttaatgtggg acgtggcttt gttctaataa gacgaagggt ggagtgcagg
cttggaaagc
10621 aggagagctc agcctacgtc tttaatcctc ctgcccaccc cttggattct
gtctccactg
10681 ggactcaaga ggtgaggaga gaccatctcc ccaaatgcac tgaagggaaa
ctggaggagg
10741 gagggagtga ggggtgatca taccagcgga ggcacatttg ctgagccccc
ccgcagtctg

10801 ctctttccaa gtggaccctc ctggaagcct gatcccaacc tcccctgcaa
gcaggtctgt
10861 cacccccatc tctcagatga agaaactgag ccttgcaggg gtggagtccc
ttgtccccac
10921 gtcataaggg tagtcatagt agtaggaaga ggaagcacct aggtttgagg
ccagggctgg
10981 ctgctgtcag aacctaggcc ctcccctgcc ttgctccaca cctggtcagg
ggagagaggg
11041 gaggaaagcc aagggaaggg acctaactga aaacaaacaa gctgggagaa
gcaggaatct
11101 gcgctcgggt tccgcagatg cagaggttga ggtggctgcg ggactggaag
tcatcgggca
11161 gaggtctcac agcagccagt aagtgaacag ctggactcgg gctgcctggg
cggcagggag
11221 aagcgggcag gggaagggtc agcagaggag cgaggcccca gaggagccct
ggggtggagc
11281 acagccaagg gctctgttcc ctttcctgga ctcggcttcc acaggccctg
acctgcctcc
11341 cccaccctcc ggtcctgccc ctgtgcctgg cagcagcccc acctgtgtga
catcccagca
11401 caccccccct ctccttgcaa aggagaaggg agcggcctag gggaggccag
gggcccacct
11461 gggctggggc tgtggagagg gagtggctgg gacgggagga aaaagagaga
cggagattag
11521 atggaagaag agggatttca agacaaattg ccagagatgc agtcagagag
actgactgag
11581 agacacaaag atagaaggaa ttagagaaag ggccacacag agccagacag
agagagaaga
11641 gtggagatgg agacagggac gaggacagag aaaggcagac agacacatag
ggacagaaag
11701 agaaaaatca cacaaagtca gaattactga atgacaggga atgacacata
gaacgagaca
11761 cagattcaga gactcagggc agggaaagga aggctgcaga cagacagaca
gacagaggga

SEQ.ID.NO. 57

KLK-L5 AA

LSQAATPKIFNGTECGRNSQPWQVGLFEGTSLRCGGVLIDHRWVLTAAHCSG
SRYWVRLGEHSLSQLDWTEQIRHSGFSVTHPGYLGASTSHEHDLRLLRLRLP
VRVTSSVQPLPLPNDCATAGTECHVSGWGITNHPRNPFPDLLQCLNLSIVSHA
TCHGVYPGRITSNMVCAGGVPGQDACQ

SEQ.ID.NO. 58

KLK-L5 AA – alternatively spliced

MGLSIFLLLCVLGLSQAATPKIFNGTECGRNSQPWQVGLFEGTSLRCGGVLID
HRWVLTAAHCSGRPIPGSAPVPQPLHRLPCHLPWCVSRENHEQHGVCRRRPG
AGCLPG

SEQ.ID.NO. 59

KLK-L5 AA – alternatively spliced

MGLSIFLLLCVLGLSQAATPKIFNGTECGRNSQPWQVGLFEGTSLRCGGVLID
HRWVLTAAHCSGSRYWVRLGEHSLSQLDWTEQIRHSGFSVTHPGYLGASTS
HEHDLRLLRLRLPVRVTSSVQPLPLPNDCATAGTECHVSGWGITNHPRNPFPD
LLQCLNLSIVSHATCHGVYPGRITSNMVCAGGVPGQDACQGDSGGPLVCGG
VLQGLVSWGSVGPCGQDGIPGVYTYICNSTLVGLGTSWNFNSCQPF

SEQ.ID.NO. 60

KLK-L5-AA

MGLSIFLLLCVLGLSQAATPKIFNGTECGRNSQPWQVGLFEGTSLRCGGVLID
HRWVLTAAHCSGSRYWVRLGEHSLSQLDWTEQIRHSGFSVTHPGYLGASTS
HEHDLRLLRLRLPVRVTSSVQPLPLPNDCATAGTECHVSGWGITNHPRNPFPD
LLQCLNLSIVSHATCHGVYPGRITSNMVCAGGVPGQDACQGDSGGPLVCGG
VLQGLVSWGSVGPCGQDGIPGVYTYICKYVDWIRMIMRNN

SEQ.ID.NO. 61

Table 17

TCAGCCAGGCAGCCACACCG

SEQ.ID.NO. 62

Table 17

TTGGTGATGCCCCAGCCTGA

SEQ.ID.NO. 63

Table 17

CCACACCGAAGATTTTCAAT

SEQ.ID.NO. 64

Table 17

GCCCCTCCTTCATTTATA

SEQ.ID.NO. 65

KLK-L6 NA

1 atcgtgtaat caccgccaca tccagtgcaa agctgattcg tcaccacaga
gcagctccct
61 cctgccaccc catccctggg tcccaagaga accctttctt aaaagaggga
gttcttgacg
121 ggtgtggtgg ctcatgcctg taatccttgc actttgggag gccaaggagg
gtggatcatt
181 tgaggtcagg agtttgagac cagactggcc aacatggtga aaccctgtct
ttactaaaaa
241 tacaaaaaaa tgagcggggc atggtggtgg gtgcctatag ccccagctac
tcaggaggct
301 gaggcaggag aatcgcttga acccaggagg cagaggttgc agtgagccga
gattgagcca
361 ctgcactcca gccggggcta aagagtgaga ctctgtctca aaaaaaaaaa
aaagaaaaag
421 aaaaaaagaa aaaaaaataa aataaataaa taaataaaat aaatttaaaa
atttaaaaat
481 aaagaggggg ttcttgtgtt gatgccgagc ctgaaccaag gcagaggagg
ccgggaaggc
541 ttcccaaggc cttcagctca aagcagggag gcccatagtt aaacagaaac
agttcaggaa
601 tcacagaaag gcacctgggg agagatgggt gtgtggctcc agatgcaggt
gcccagacag
661 tgcgtcccca ggtgtacaga cagacccagg ccaagctcca gctcaaagag
ccagcctagg
721 ggggtgccga ggtggaggga ggctgagtca ggctgaggcc ggggaacagt
tggggtagcc
781 aagggaggca agcagcctcc tgagtcacca cgtggtccag gtacggggct
gcccaggccc
841 agagacggac acaagcactg gggaatttaa ggggctaggg gaggggctga
ggagggtagg
901 ccctccccca aatgaggatg gaaccccccc aactccagaa cccccctgca
ggctggccag
961 aatccttccc catctcattc actctgtctc tcctgctctc tgccgtctcc
tattttgaat
1021 ttccaacccc gtctgttaag actgtccttc tgtctctgaa tctctgtccc
cttctctttc
1081 tgggtctctc tccctctccc tctgggtctc tgtccccctc tctgggtctc
tgtcactctc
1141 tctttgcatc tccagctctc actttgtctc tgcacctagc agatcccaag
ctggggaatg
1201 ccagttctgg caccaacctt cctgctccct gctggggcct ctgctccccc
atctctcagg
1261 agtcgaaagt gagaaagcaa ggtgggcagc tctgctccag gtccaggtat
ctcccgccca
1321 cctcctgccc gtcctctatc ccacccctcc tctccatctc tccctggcgc
tgccatctct
1381 catctaggcc tccgtctcct ctgtcattgt ccccatcccc tgtaggtgcc
catccttccc
1441 gtctcccctc tgccatcggc ctgcctgtcc catcctcttt ctcccaccat
gtcccgttct
1501 cttccacgtc tcatgcccgc actgccttca tcatcatcgc tgttgttctg
tgtgtgtttg
1561 tggtgagtgc cgcatggtgg gggcgtctcg gcctctctcc tctctctcca
ctgtttctc
1621 tttctgtgtg tctgtttcca ttctatctcc accttcttcc ctccgtcttt
tgcttttcta
1681 tctccacttc tccacacccc tctctccctg cgtctctgtg tctccctctt
cctctgtctt

1741 gtttttttcc caccgtctgc ctcttctgtt ccctgtcaca tccaacttcc
accggtttct
1801 ccagctctct cctcagttcc ttctctcatg agcacacctg cctctgtgct
cgtattcctg
1861 gactcctctc tctccactgt catatcttct cattcatttt cccagtctct
ctctgtctct
1921 tgctctcccc ctctctgtca ctctgtctct gtctctctct ttctctctct
ctctctgtgt
1981 ctctctgtct ggctctctct ctgtctctct ctccatctct ctctctctct
cccccccgtc
2041 accctgtctc tgtctctctc tgtctgtgtg tctctctgtc tttctctctc
tccatctctc
2101 tctgtctctc tctctctctc tctctctctc cctctctccc tcctcccgtg
actccctctc
2161 tcagtccatc tcttcctccc tctctcagcc ccttcgtgcc ctttcctctg
acactcccca
2221 ccctggtttc ctgactccac cactagatcc accacctcca gcaactggga
accctcccct
2281 gcccaccctg ccctggggtc ccctcccagg attccttcta gattatagca
tcttccctgg
2341 gcgggttctc atgaacaatt gtggctgctt ttttggccag acaggggagg
gaggggatgg
2401 gatcagggag tcctggaatg ggaactaggc aataaaaaaa aaaaaatgtc
agaagcaggg
2461 cggcgggagg tgggggcagg gccagctgtc cttaccaggg ataaaaggct
ttgccagtgt
2521 gactaggaag agagacacct cccctccttc cttcatcaag acatcaagga
gggacctgtg
2581 ccctgctcca catcctccca cctgccgccc gcagagcctg caggccccgc
ccccctcgtc
2641 tctggtccct acctctctgc tgtgtcttca tgtccctgag ggtcttgggc
tctgggtaag
2701 tgccccttgc tgtctctgcc tctcagcccc cggttctgtt gaaggttcct
tctctctcac
2761 tttttctctg catttgacag gacctggccc tcagccccta aaatgttcct
cctgctgaca
2821 gcacttcaag tcctggctat aggtaagaga acggttgggt atgacacaag
ggggtcccct
2881 ggagactctg agaagagatg gggatgggtc cttggggccc ctggatgctc
atggtgacct
2941 cataagaaag agcagggagt ggtttggggg tcatggtggg ggaacgtgct
ggaggcctaa
3001 attcctagtt gtggaggtgc tagggaattg tggggccggg gagagaggtg
tttataaggt
3061 ctggtgcaaa atacataagg aatcttaggg aactattagg tcctgagtgg
gtcatagcag
3121 aaagatcacg gggctctacc tgactgtgtt aggaaagaaa caatgtcaga
aagatgtttt
3181 gttgtcagag ggaaggtgga gaaggatgat gggatggcgg gatcgtggca
tggggtggcg
3241 ggatcgtggc atgggtgtgt gaggtggatg ggggcaagtg tggggcaaga
gatggcggat
3301 ccttggggtc ccactgagtg ggaacgttgg ggaggagaca gggaggtcct
tgaatgtgtt
3361 ggggaaggac tcattggggg gaaatgtggc atatttcgag aagtgatcac
agaaattatg
3421 ggagcataga gctaagggtc gtagatgtag caaggccctg gataaggtgg
ccacggcaca
3481 aaataagaga tgctacggag gtgacttggg aggtgagtca gaaagctctc
cgtgctgggg

3541 caataacggg gtcaatattg ggcatgtctc accctgggtg ggacagatag
aggcgggcag
3601 tttaggggtt agaccaaaag gaaggggatt tgtcagtttt ggaatcctac
aaacttgtgg
3661 agtggagagt gtttgctcat ctactttccc cacccaatcc tgtccactcc
tagccatgac
3721 acagagccaa gaggatgaga acaagataat tggtggccat acgtgcaccc
ggagctccca
3781 gccgtggcag gcggccctgc tggcgggtcc caggcgccgc ttcctctgcg
gaggcgccct
3841 gctttcaggc cagtgggtca tcactgctgc tcactgcggc cgcccgtaag
tgacccccctc
3901 ccctgtccct gtacctagtg aattccagag tctaaagccc tagagctgag
ctgagaacct
3961 ggatctctgt atagaaccca atgtagtggc tggctcctgg tttgaggtct
agagaagagc
4021 ctggaacaaa aacacagctc gggatgtggg ctcctccata aatctcgaac
tcagcatagg
4081 ttctgaaagc agatgggcag cttggaaccc atggacctgc tgagaaccga
acatctgatc
4141 cagtgattct tccagaggcc acacattaca tcgagaccaa gcttagccca
ttccagattg
4201 gtggctgaat tcaggacccc gtctacattc agaaactcag gacactacgt
agaactcaga
4261 gcccagttca ggacctgcag tctagccata aatccagaac tagaacgctg
ctcacagctg
4321 gaacatacaa ctctaagaat agaggcaaaa cctggaggct gtttcacacc
caaggtttag
4381 ttcagagtct agtctatagc tccgctatga gcagacttca acccagtgtt
tgaatcccag
4441 aatgtggcgg gtgcggtggc tcatgcctat aatcctagca ctttgggatg
ctgaggcagg
4501 cagatcacct gaggtcagga gttcgagacc agcctgagca acatagagaa
accctgtctc
4561 tactaaaaat gcaaaattag ccaggcatgg tggcacatgc ctgtaatccc
agccactcgg
4621 gaggctgagg caggagaatc acttgaacct gggaggcgga ggttgcagtg
agtcaagatc
4681 gcaccattgc actccaggct aggcaacaag agcgaaactc catatcaatc
aatcaatcaa
4741 taaatcccag aatgcagatc ctaatcagaa gccccatata aaacctagac
ccctcctaaa
4801 ttctagatct gaacttacaa cccagacccc agccaagagg tcaaaatgcc
tataagccat
4861 atctatgcca taaacaggtc agtctagaac ctagagatca aagctcaggc
cagagtctag
4921 aatataaagg ccagaatgca aaccagactc tagaatcttg gatccgggcc
ataacctaga
4981 gctccaacta gaacccagag cccaacctga ggtcaagggc tagggccaga
gtccagaacc
5041 aagagcccta taatccaata tgaaacagac ctgtagaggc tgggtgcggt
ggctcacgcc
5101 tgtaatccca gcactttggg aggctgaggc gggagaatca cttgaactgg
gagttggagg
5161 tcgagagtga gctgagatcg tgccactgca ctccagccta ggtgacagag
cgagactcca
5221 tcacaaaaaa aaaataaata aataaatcaa gtcataatcc aggttcgatc
tagaatcctg
5281 atcttagcat agagtcaaaa gtttaagatg tctagaactc agaacccagg
ctagaaacag

5341 aatggtgcct actccggaat atcagttccg atttagagcc tagactcata
acgcagtttc
5401 gcttaggact caatgcaccg agcccagcac agaccctggc acggagccaa
gctctcccaa
5461 tcatcacctt cttcccaagc caggagctgg agcccagccc aagagcggaa
ggagaggcag
5521 ctggggctgg gccgagagaa tgccctggcc atggggaagg gcacaggagg
ccaagaatgc
5581 tcggcctgca gttagtgaga agcaggctag acctcgggga agactcgtca
cccggccagg
5641 gaaccgggct ggagggtggg gaggagtctc tggctcagac cctgagcagc
gcttctcttg
5701 ggggtcgtgg ccaggatcct tcaggttgcc ctgggcaagc acaacctgag
gaggtgggag
5761 gccacccagc aggtgctgcg cgtggttcgt caggtgacgc accccaacta
caactcccgg
5821 acccacgaca acgacctcat gctgctgcag ctacagcagc ccgcacggat
cgggagggca
5881 gtcaggccca ttgaggtcac ccaggcctgt gccagccccg ggacctcctg
ccgagtgtca
5941 ggctggggaa ctatatccag ccccatcggt gaggactcct gcgtcttgga
aagcagggga
6001 ctgggcctgg gctcctgggt ctccaggagg tggagctggg gggactgggg
ctcctgggtc
6061 tgagggagga ggggctgggc ctggactcct gggtctgagg gaggaggggg
ctgaggcctg
6121 gactcctggg tctcaaggag gaggagctgg gcctggactc atacgtctga
gggaggaggg
6181 gctggagcct ggactcctgg gtctcaagga ggaggggctg ggcctggact
tctgggtctg
6241 agggaggagg ggctggggac ctggactccc gggtctgagg gaggagggac
tggggggtctg
6301 gactcctggg tctgagggag gaggggctgg gggcctggac tcctgggtct
gagggaggag
6361 gtgctggggc tggactcctg ggtcggaagg aggaggggct gggggcctgg
acccttgggt
6421 cttatgggag ggtagaccca gttataaccc tgcagtgtcc cccagccagg
taccccgcct
6481 ctctgcaatg cgtgaacatc aacatctccc cggatgaggt gtgccagaag
gcctatccta
6541 gaaccatcac gcctggcatg gtctgtgcag gagttcccca gggcgggaag
gactcttgtc
6601 aggtaaggcc caggatggga gctgtggtag ggattatttg ggactgggat
ttaagcaaat
6661 gatgtcagga gcatggaagt ctgcagaggt cttcagaaga gagtgaaccg
caggcacaga
6721 gagattccga tagccaggcc accctgcttc ctagccctgt gccccctggg
taatggactc
6781 agagcattca tgcctcagtt tcctcatctg tcaggtggga gtaaccctct
tagggtagtt
6841 ggtggaatgg gatgaggcag gttggggaaa gatcgcagag tggcctctgc
tcatatgggt
6901 ctgggaaagg ctgtgctgag gcttctagaa atcttaatgc atccttgagg
gaggcagaga
6961 tggggaaata gaaaaagaga gacacacaaa tgttctacag ttggagcgaa
cagagagggg
7021 cctggtgaga ttcaagggac aggcaggtgc acacagagac agagccagac
ccagcggaga
7081 gggaaggaag tgccccgacc tccggggctg agacctcaga gctggggcag
gactgtgtcc

7141 ctaactgtcc accagtgtct ctgcctgtct ccctgtgtct gcttctcggg
ttctctgtgc
7201 catggtggct ctggctacct gtccatcagt gtctccattt ctgttcctcc
ccctcagggt
7261 gactctgggg gacccctggt gtgcagagga cagctccagg gcctcgtgtc
ttggggaatg
7321 gagcgctgcg ccctgcctgg ctaccccggt gtctacacca acctgtgcaa
gtacagaagc
7381 tggattgagg aaacgatgcg ggacaaatga tggtcttcac ggtgggatgg
acctcgtcag
7441 ctgcccaggc cctcctctct ctactcagga cccaggagtc caggccccag
cccctcctcc
7501 ctcagaccca ggagtccagg cccccagccc ctcctccctc agacccggga
gtccaggccc
7561 ccagcccctc ctccctcaga cccaggagtc caggccccag cccctcctcc
ctcagacccg
7621 ggagtccagg cccccagccc ctcctccctc agacccagga gtccaggccc
cagtccctcc
7681 tccctcagac ccaggagtcc aggcccccag cccctcctcc ctcagaccca
ggaatccagg
7741 cccagcccct cctccctcag acccaggagc cccagtcccc cagcccctcc
tccttgagac
7801 ccaggagtcc aggcccagcc cctcctccct cagacccagg agccccagtc
cccagcatcc
7861 tgatctttac tccggctctg atctctcctt tcccagagca gttgcttcag
gcgttttctc
7921 cccaccaagc ccccaccctt gctgtgtcac catcactact caagaccgga
ggcacagagg
7981 gcaggagcac agacccctta aaccggcatt gtattccaaa gacgacaatt
tttaacacgc
8041 ttagtgtctc taaaaaccga ataaataatg acaataaaaa tggaatcatc
ctaaattgta
8101 ttcattcatc catgtgttta ctttttattt tttgagacaa ggtcttgctc
agtctcctgg
8161 tgaaatgctg taacgcaatc atagctcact gcaaccgtga cctcctgggc
tccagtgatc
8221 ctcttacctc agcctcccga gtagctggga ccacaggtgc ccgtcaccat gccccgctac

SEQ.ID.NO. 66

KLK-L6 AA

MTQSQEDENKIIGGHTCTRSSQPWQAALLAGPRRRFLCGGALLSGQWVITAA
HCGRPILQVALGKHNLRRWEATQQVLRVVRQVTHPNYNSRTHDNDLMLLQL
QQPARIGRAVRPIEVTQACASPGTSCRVSGWGTISSPIARYPASLQCVNINISPD
EVCQKAYPRTITPGMVCAGVPQGGKDSCQGDSGGPLVCRGQLQGLVSWGM
ERCALPGYPGVYTNLCKYRSWIEETMRDK

SEQ.ID.NO. 67

KLK-L6 AA

MFLLLTALQVLAIAMTQSQEDENKIIGGHTCTRSSQPWQAALLA
GPRRRFLCGGALLSGQWVITAAHCGRPILQVALGKHNLRRWEATQQVLRVV
RQVTHPNYNSRTHDNDLMLLQLQQPARIGRAVRPIEVTQACASPGTSCRVSG
WGTISSPIARYPASLQCVNINISPDEVCQKAYPRTITPGMVCAGVPQGGKDSC

QGDSGGPLVCRGQLQGLVSWGMERCALPGYPGVYTNLCKYRSWIEETMRD
K

SEQ.ID.NO. 68

Figure 9

prostase

MATAGNPWGWFLGYLILGVAGSLVSGSCSQIINGEDCSPHSQPWQAALVME
NELFCSGVLVHPQWVLSAAHCFQNSYTIGLGLHSLEADQEPGSQMVEASLSV
RHPEYNRPLLANDLMLIKLDESVSESDTIRSISIASQCPTAGNSCLVSGWGLLA
NGRMPTVLQCVNVSVVSEEVCSKLYDPLYHPSMFCAGGGHDQKDSCNGDSG
GPLICNGYLQGLVSFGKAPCGQVGVPGVYTNLCKFTEWIEK

SEQ.ID.NO. 69

Figure 9

EMSP

MATAGNPWGWFLGYLILGVAGSLVSGEMSPSCSQIINGEDCSPHSQPWQAAL
VMENELFCSGVLVHPQWVLSAAHCFQNSYTIGLGLHSLEADQEPGSQMVEAS
LSVRHPEYNRPLLANDLMLIKLDESVSESDTIRSISIASQCPTAGNSCLVSGWG
LLANGRMPTVLQCVNVSVVSEEVCSKLYDPLYHPSMFCAGGGHDQKDSCNG
DSGGPLICNGYLQGLVSFGKAPCGQVGVPGVYTNLCKFTEWIEK 249

SEQ.ID.NO. 70

Figure 9

KLK-L2

MATARPPWMWVLCALITALLLGVTEHVLANNDVSCDHPSNTVPSGSNQDLG
AGAGEDARSDDSSSRIINGSDCDMHTQPWQAALLLRPNQLYCGAVLVHPQW
LLTAAHCRKKVFRVRLGHYSLSPVYESGQQMFQGVKSIPHPGYSHPGHSNDL
MLIKLNRRIRPTKDVRPINVSSHCPSAGTKCLVSGWGTTKSPQVHFPKVLQCL
NISVLSQKRCEDAYPRQIDDTMFCAGDKAGRDSCQGDSGGPVVCNGSLQGL
VSWGDYPCARPNRPGVYTNLCKFTKWIQE

SEQ.ID.NO. 71

Figure 9

zyme

MKKLMVVLSLIAAAWAEEQNKLVHGGPCDKTSHPYQAALYTSGHLLCGGV

LIHPLWVLTAAHCKKPNLQVFLGKHNLRQRESSQEQSSVVRAVIHPDYDAAS
HDQDIMLLRLARPAKLSELIQPLPLERDCSANTTSCHILGWGKTADGDFPDTI
QCAYIHLVSREECEHAYPGQITQNMLCAGDEKYGKDSCQGDSGGPLVCGDH
LRGLVSWGNIPCGSKEKPGVYTNVCRYTNWIQK

SEQ.ID.NO. 72

Figure 9

neuropsin

MGRPRPRAAKTWMFLLLLGGAWAGHSRAQEDKVLGGHECQPHSQPWQAA
LFQGQQLLCGGVLVGGNWVLTAAHCKKPKYTVRLGDHSLQNKDGPEQEIPV
VQSIPHPCYNSSDVEDHNHDLMLLQLRDQASLGSKVKPISLADHCTQPGQKC
TVSGWGTVTSPRENFPDTLNCAEVKIFPQKKCEDAYPGQITDGMVCAGSSKG
ADTCQGDSGGPLVCDGALQGITSWGSDPCGRSDKPGVYTNICRYLDWIKKTL
SPMRILQLILLALATGLVG

SEQ.ID.NO. 73

Figure 9

TLSP

GETRIIKGFECKPHSQPWQAALFEKTRLLCGATLIAPRWLLTAAHCLKPRYIV
HLGQHNLQKEEGCEQTRTATESFPHPGFNNSLPNKDHRNDIMLVKMASPVSI
TWAVRPLTLSSRCVTAGTSCLISGWGSTSSPQLRLPHTLRCANITIIEHQKCEN
AYPGNITDTMVCASVQEGGKDSCQGDSGGPLVCNQSLQGIISWGQDPCAITR
KPGVYTKVCKYVDWIQE

SEQ.ID.NO. 74

Figure 9

PSA

MWVPVVFLTLSVTWIGAAPLILSRIVGGWECEKHSQPWQVLVASRGRAVCG
GVLVHPQWVLTAAHCIRNKSVILLGRHSLFHPEDTGQVFQVSHSFPHPLYDM
SLLKNRFLRPGDDSSHDLMLLRLSEPAELTDAVKVMDLPTQEPALGTTCYAS
GWGSIEPEEFLTPKKLQCVDLHVISNDVCAQVHPQKVTKFMLCAGRWTGGK
STCSGDSGGPLVCNGVLQGITSWGSEPCALPERPSLYTKVVHYRKWIKD

SEQ.ID.NO. 75

Figure 9

KLK2

MWDLVLSIALSVGCTGAVPLIQSRIVGGWECEKHSQPWQVAVYSHGWAHCG
GVLVHPQWVLTAAHCLKKNSQVWLGRHNLFEPEDTGQRVPVSHSFPHPLYN
MSLLKHQSLRPDEDSSHDLMLLRLSEPAKITDVVKVLGLPTQEPALGTTCYAS
GWGSIEPEEFLRPRSLQCVSLHLLSNDMCARAYSEKVTEFMLCAGLWTGGKD
TCGGDSGGPLVCNGVLQGITSWGPEPCALPEKPAVYTKVVHYRKWIKD

SEQ.ID.NO. 76

Figure 9

KLK1

MWFLVLCLALSLGGTGAAPPIQSRIVGGWECEQHSQPWQAALYHFSTFQCGG
ILVHRQWVLTAAHCISDNYQLWLGRHNLFDDENTAQFVHVSESFPHPGFNMS
LLENHTRQADEDYSHDLMLLRLTEPADTITDAVKVVELPTEEPEVGSTCLASG
WGSIEPENFSFPDDLQCVDLKILPNDECKKAHVQKVTDFMLCVGHLEGGKDT
CVGDSGGPLMCDGVLQGVTSWGYVPCGTPNKPSVAVRVLSYVKWIED

SEQ.ID.NO. 77

Figure 9

trypsinogen

MNPLLILTFVAAALAAPFDDDDKIVGGYNCEENSVPYQVSLNSGYHFCGGSLI
NEQWVVSAGHCYKSRIQVRLGEHNIEVLEGNEQFINAAKIIRHPQYDRKTLNN
DIMLIKLSSRAVINARVSTISLPTAPPATGTKCLISGWGNTASSGADYPDELQC
LDAPVLSQAKCEASYPGKITSNMFCVGFLEGGKDSCQGDSGGPVVCNGQLQ
GVVSWG-DGCAQKNKPGVYTKVYNYVKWIKN

SEQ.ID.NO. 78

Figure 17, 27, 36, 43

PSA

MWVPVVFLTLSVTWIGAAPLILSRIVGGWECEKHSQPWQVLVASRGRAVCG
GVLVHPQWVLTAAHCIRNKSVILLGRHSLFHPEDTGQVFQVSHSFPHPLYDM
SLLKNRFLRPGDDSSHDLMLLRLSEPAELTDAVKVMDLPTQEPALGTTCYAS
GWGSIEPEEFLTPKKLQCVDLHVISNDVCAQVHPQKVTKFMLCAGRWTGGK
STCSGDSGGPLVCNGVLQGITSWGSEPCALPERPSLYTKVVHYRKWIKDTIVA
NP

SEQ.ID.NO. 79

Figure 17, 27, 36, 43

KLK2

MWDLVLSIALSVGCTGAVPLIQSRIVGGWECEKHSQPWQVAVYSHGWAHCG
GVLVHPQWVLTAAHCLKKNSQVWLGRHNLFEPEDTGQRVPVSHSFPHPLYN
MSLLKHQSLRPDEDSSHDLMLLRLSEPAKITDVVKVLGLPTQEPALGTTCYAS
GWGSIEPEEFLRPRSLQCVSLHLLSNDMCARAYSEKVTEFMLCAGLWTGGKD
TCGGDSGGPLVCNGVLQGITSWGPEPCALPEKPAVYTKVVHYRKWIKDTIAA
NP

SEQ.ID.NO. 80

Figure 17, 27, 36, 43

KLK1

MWFLVLCLALSLGGTGAAPPIQSRIVGGWECEQHSQPWQAALYHFSTFQCGG
ILVHRQWVLTAAHCISDNYQLWLGRHNLFDDENTAQFVHVSESFPHPGFNMS
LLENHTRQADEDYSHDLMLLRLTEPADTITDAVKVVELPTEEPEVGSTCLASG
WGSIEPENFSFPDDLQCVDLKILPNDECKKAHVQKVTDFMLCVGHLEGGKDT
CVGDSGGPLMCDGVLQGVTSWGYVPCGTPNKPSVAVRVLSYVKWIEDTIAE
NS

SEQ.ID.NO. 81

Figure 17, 27, 36, 43

prostase

MATAGNPWGWFLGYLILGVAGSLVSGSCSQIINGEDCSPHSQPWQAALVME
NELFCSGVLVHPQWVLSAAHCFQNSYTIGLGLHSLEADQEPGSQMVEASLSV
RHPEYNRPLLANDLMLIKLDESVS-
ESDTIRSISIASQCPTAGNSCLVSGWGLLANG—
RMPTVLQCVNVSVVSEEVCSKLYDPLYHPSMFCAGGGHDQKDSCNGDSGGP
LICNGYLQGLVSFGKAPCGQVGVPGVYTNLCKFTEWIEKTVQAS

SEQ.ID.NO. 82

Figure 17, 27

trypsinogen







SEQ.ID.NO. 83

Figure 17, 27, 36, 43

neuropsin

MGRPRPRAAKTWMFLLLLGGAWAGHSRAQEDKVLGGHECQPHSQPWQAA
LFQGQQLLCGGVLVGGNWVLTAAHCKKPKYTVRLGDHSLQNKDGPEQEIPV
VQSIPHPCYNSSDVEDHNHDLMLLQLRDQASLGSKVKPISLADHCTQPGQKC
TVSGWGTVTSPRENFPDTLNCAEVKIFPQKKCEDAYPGQITDGMVCAGSSKG
ADTCQGDSGGPLVCDGALQGITSWGSDPCGRSDKPGVYTNICRYLDWIKKIIG
SKG

SEQ.ID.NO. 84

Figure 17, 27, 36, 43

zyme

MKKLMVVLSLIAAAWAEEQNKLVHGGPCDKTSHPYQAALYTSGHLLCGGV
LIHPLWVLTAAHCKKPNLQVFLGKHNLRQRESSQEQSSVVRAVIHPDYDAAS
HDQDIMLLRLARPAKLSELIQPLPLERDCSANTTSCHILGWGKTADGDFPDTI
QCAYIHLVSREECEHAYPGQITQNMLCAGDEKYGKDSCQGDSGGPLVCGDH
LRGLVSWGNIPCGSKEKPGVYTNVCRYTNWIQKTIQAK

SEQ.ID.NO. 85

Figure 27

EMSP

MATAGNPWGWFLGYLILGVAGSLVSGEMSPSCSQIINGEDCSPHSQPWQAAL
VMENELFCSGVLVHPQWVLSAAHCFQNSYTIGLGLHSLEADQEPGSQMVEAS
LSVRHPEYNRPLLANDLMLIKLDESVSESDTIRSISIASQCPTAGNSCLVSGWG
LLANGRMPTVLQCVNVSVVSEEVCSKLYDPLYHPSMFCAGGGHDQKDSCNG
DSGGPLICNGYLQGLVSFGKAPCGQVGVPGVYTNLCKFTEWIEKTVQAS

SEQ.ID.NO. 86

Figure 27

TLSP

MRI-LQLILLALATGLVGGETRIIKGFECKPHSQPWQAALFEKTRLLC
GATLIAPRWLLTAAHCLKPRYIVHLGQHNLQKEEGCEQTRTATESFPHPGFNN
SLPNKDHRNDIMLVKMASPVSITWAVRPLTLSSRCVTAGTSCLISGWGSTSSP
QLRLPHTLRCANITIIEHQKCENAYPGNITDTMVCASVQEGGKDSCQGDSGGP
LVCNQSLQGIISWGQDPCAITRKPGVYTKVCKYVDWIQETMKNN

SEQ.ID.NO. 87

Figure 27

HSCEE

MARSLLLPLQILLLSLALETAGEEAQGDKIIDGAPCARGSHPWQVALLSGNQL
HCHSCCEGGVLVNERWVLTAAHCKMNEYTVHLGSDTLGDRRAQRIKASKSF
RHPGYSTQTHVNDLMLVKLNSQARLSSMVKKVRLPSRCEPPGTTCTVSGWG
TTTSPDVTFPDLMCVDVKLISPQDCTKVYKDLLENSMLCAGIPDSKKNACNG
DSGGPLVCRGTLQGLVS WGTFPCGQPNDPGVYTQVCKFTKWINDTMKKHR

SEQ.ID.NO. 88

Figure 27

NES1

MRAPHLHLSAASGARALAKLLPLLMAQLWAAEAALLPQNDTRLDPEAYGAP
CARG SQPWQVSLFNGLSFH CAGVLVDQSWVLTAAHCGNKPLWARVGDDH
LL-LLQG-EQLRRTT RSVVHPKYHQGSGPI LPRRTDEHDLML LKLARPVV-
PGPRVR ALQLPYR-CAQPGDQ CQVAGWGTTAARRVK YNKGLTCSSITILSP
KECEVFYPGVVTNNM ICAGLDR-GQDPCQS DSGGPLVCDETLQGI LSWG-
VYPCGSAQHPAVYTQICKYMSWINK VIRSN

## Claims

1. An isolated KLK-L1. KLK-L2, KLK-L3, KLK-L4, KLK-L5, or KLK-L6 nucleic acid molecule of at least 30 nucleotides which hybridizes to SEQ ID NO. 1, 13, 21, 43, 56, or 65, respectively, or the complement of SEQ ID NO. 1, 13, 21, 43, 56, or 65, under stringent hybridization conditions

2. An isolated nucleic acid molecule which comprises:
(i) a nucleic acid sequence encoding a protein having substantial sequence identity with an amino acid sequence of a KLK-L1, KLK-L2, KLK-L3, KLK-L4, KLK-L5, or KLK-L6 protein as shown in SEQ.ID.NO. 2, 3, 14, 22, 23, 44, 45, 57, 58, 59, 60, 66, or 67, respectively;
(ii) a nucleic acid sequence encoding a protein comprising an amino acid sequence of a KLK-L1, KLK-L2, KLK-L3, KLK-L4, KLK-L5, or KLK-L6 protein as shown in SEQ.ID.NO. 2, 3, 14, 22, 23, 44, 45, 57, 58, 59, 60, 66, or 67, respectively;
(iii) nucleic acid sequences complementary to (i);
(iv) a degenerate form of a nucleic acid sequence of (i);
(v) a nucleic acid sequence capable of hybridizing under stringent conditions to a nucleic acid sequence in (i), (ii) or (iii);
(vi) a nucleic acid sequence encoding a truncation, an analog, an allelic or species variation of a protein comprising an amino acid sequence of a KLK-L1, KLK-L2, KLK-L3, KLK-L4, KLK-L5, or KLK-L6 protein as shown in SEQ.ID.NO. 2, 3, 14, 22, 23, 44, 45, 57, 58, 59, 60, 66, or 67, respectively; or
(vii) a fragment, or allelic or species variation of (i), (ii) or (iii).

3. A purified and isolated nucleic acid molecule of the invention comprises:
(i) a nucleic acid sequence comprising the sequence of SEQ.ID.NO. 1, 13, 21, 43, 56, or 65 wherein T can also be U;
(ii) nucleic acid sequences complementary to (i), preferably complementary to the full nucleic acid sequence of SEQ.ID.NO. 1, 13, 21, 43, 56, or 65;
(iii) a nucleic acid capable of hybridizing under stringent conditions to a nucleic acid of (i) or (ii) and preferably having at least 18 nucleotides; or
(iv) a nucleic acid molecule differing from any of the nucleic acids of (i) to (iii) in codon sequences due to the degeneracy of the genetic code.

4. An isolated nucleic acid molecule which encodes a protein which binds an antibody of a KLK-L1, KLK-L2, KLK-L3, KLK-L4, KLK-L5, or KLK-L6 protein.

5. A regulatory sequence of an isolated nucleic acid molecule as claimed in any of the preceding claims fused to a nucleic acid which encodes a heterologous protein.

6. A vector comprising a nucleic acid molecule of any of the preceding claims.

7. A host cell comprising a nucleic acid molecule of any of the preceding claims.

8. An isolated KLK-L1 protein comprising an amino acid sequence of SEQ. ID. NO. 2 or 3.

9. An isolated KLK-L2 protein comprising an amino acid sequence of SEQ. ID. NO. 14.

10. An isolated KLK-L3 protein comprising an amino acid sequence of SEQ. ID. NO. 22 or 23.

11. An isolated KLK-L4 protein comprising an amino acid sequence of SEQ. ID. NO. 44 or 45.

12. An isolated KLK-L5 protein comprising an amino acid sequence of SEQ. ID. NO. 57, 58, 59, or 60.

13. An isolated KLK-L6 protein comprising an amino acid sequence of SEQ. ID. NO. 66 or 67.

14. An isolated protein having at least 65% amino acid sequence identity to an amino acid sequence of SEQ. ID. NO. 2, 3, 14, 22, 23, 44, 45, 57, 58, 59, 60, 66, or 67.

15. A method for preparing a protein as claimed in any of the preceding claimd comprising:
(a) transferring a vector as claimed in claim 6 into a host cell;
(b) selecting transformed host cells from untransformed host cells;
(d) culturing a selected transformed host cell under conditions which allow expression of the protein; and
(e) isolating the protein.

16. A protein prepared in accordance with the method of claim 15.

17. An antibody having specificity against an epitope of a polypeptide as claimed in claim 8, 9, 10, 11, 12, or 13.

18. An antibody as claimed in claim 17 labeled with a detectable substance and used to detect the protein in biological samples, tissues, and cells.

19. A probe comprising a sequence encoding a protein as claimed in claim 8, 9, 10, 11, 12, or 13, or a part thereof.

20. A method of diagnosing and monitoring conditions mediated by a protein as claimed in claim 8, 9, 10, 11, 12, or 13, by determining the presence of a nucleic acid molecule encoding the protein as claimed in any of the preceding claims or determining the presence of the protein.

21. A method as claimed in claim 20 wherein the condition is cancer.

22. A method for identifying a substance which associates with a protein as claimed in claim 8, 9, 10, 11, 12, or 13 comprising (a) reacting the protein with at least one substance which potentially can associate with the protein, under conditions which permit the association between the substance and protein, and (b) removing or detecting protein associated with the substance, wherein detection of associated protein and substance indicates the substance associates with the prtoein.

23. A method for evaluating a compound for its ability to modulate the biological activity of a protein as claimed in claim 8, 9, 10, 11, 12, or 13 comprising providing a known concentration of the protein with a substance which associates with the protein and a test compound under conditions which permit the formation of complexes between the substance and protein, and removing and/or detecting complexes.

24. A method for detecting a nucleic acid molecule encoding a protein comprising an amino acid sequence of SEQ. ID. NO. 2, 3, 14, 22, 23, 44, 45, 57, 58, 59, 60, 66, or 67 in a biological sample comprising the steps of:
(a) hybridizing a nucleic acid molecule of claim 2 to nucleic acids of the biological sample, thereby forming a hybridization complex; and
(b) detecting the hybridization complex wherein the presence of the hybridization complex correlates with the presence of a nucleic acid molecule encoding the protein in the biological sample.

25. A method as claimed in claim 24 wherein nucleic acids of the biological sample are amplified by the polymerase chain reaction prior to the hybridizing step.

26. A method for treating a condition mediated by a protein as claimed in claim 8, 9, 10, 11, 12, or 13 comprising administering an effective amount of an antibody as claimed in claim 17 or a substance or compound identified in accordance with a method claimed in claim 22 or 23.

27. A method as claimed in claim 26 wherein the condition is cancer.

28. A composition comprising one or more of a nucleic acid molecule or protein claimed in any of the preceding claims, or a substance or compound identified using a method as claimed in any of the preceding claims, and a pharmaceutically acceptable carrier, excipient or diluent.

29. Use of one or more of a nucleic acid molecule or protein claimed in any of the preceding claims, or a substance or compound identified using a method as claimed in any of the preceding claims in the preparation of a pharmaceutical composition for treating a condition mediated by a protein as claimed in any of the preceding claims.

30. A transgenic non-human mammal which doe not express a KLK-L1, KLK-L2, KLK-L3, KLK-L4, KLK-L5, or KLK-L6 protein as claimed in claim 8, 9, 10, 11, 12, or 13, respectively, resulting in a KLK-L1, KLK-L2, KLK-L3, KLK-L4, KLK-L5, or KLK-L6 protein associated pathology, respectively.

31. A transgenic animal assay system which provides a model system for testing for an agent that reduces or inhibits an a KLK-L1, KLK-L2, KLK-L3, KLK-L4, KLK-L5, or KLK-L6 protein associated pathology comprising
(a) administering the agent to a transgenic non-human animal as claimed in claim 26; and
(b) determining whether said agent reduces or inhibits a KLK-L1, KLK-L2, KLK-L3, KLK-L4, KLK-L5, or KLK-L6 protein associated pathology in the transgenic non-human animal relative to a transgenic non-human animal of step (a) which has not been administered the agent.


SEQUENCE LISTING

<110> Mount Sinai Hospital

<120> Novel Human Kallikrein-Like Genes

<130> P012400EPA

<140> 06076156.6
<141> 2000-03-09

<150> US 60/124,260
<151> 1999-03-11

<150> US 60/127,386
<151> 1999-04-01

<150> US 60/144,919
<151> 1999-07-21

<160> 88

<170> PatentIn version 3.3

<210> 1
<211> 4740
<212> DNA
<213> Homo sapiens

<400> 1
tatctcatga gagagaataa gaacatgaaa agagaaagaa tgagagagag agagagaaag 60

aaaaaggaga gtggagtcta ggatctgggc aggggtctcc tccctgggtc cctagaccct 120

gctgccagcc ccttctgggc ccccaaccac tgcctggtca gagttgaggc agcctgagag 180

agttgagctg gaagtttgca gcacctgacc cctggaacac atcccctggg ggcaggccag 240

cccaggctga ggatgcttat aagccccaag gaggcccctg cggaggcagc aggctggagc 300

tcagcccagc agtggaatcc aggagcccag aggtggccgg gtaagaggcc tggtggtccc 360

ccactaaaag cctgcagtgt tcatgatcca actctcccta cagctccatg tcgctggatt 420

ctcagcctct gtgccttctg tctccacatc tctctagaca gatctctcac tgtctctagt 480

taggagtcac tgtctctagt taggggtctc tctgtctctc tgaatctata tctccatgtc 540

taactctcag actgtctctg aggatatctc tcaagcactc tgtctctccg gctctgattc 600

tctgtgtgtc ttccctccat gcttgtttgt gggtggctag acaccatctc tccccattca 660

cagatggcta gatgctttct ctaaactttc ctttctacct agttctctct ctctctcttt 720

tcccatctct ctctctcttt ttctctctca gtctctaaat ctgtctctct aggttctggg 780

tccatggatg ggagaggggg tagatggtct aggctcttgc ctacctaata acgtcccaga 840

gggaagaaag ggagggacaa agagagggat ggagagactt gggctgaaga tccccagaca 900

cggctaagtc tcagtcctca tccccaggtg ctgacgtgat ggccacagca ggaaatccct 960

ggggctggtt cctggggtac ctcatccttg gtgtcgcagg tatctgagta tgcgtgtgtg 1020

tgtctgtccg tgcttggggg cacagtgttt gttaatgttc aggtgtgact cagtgtcctc 1080
ttgcttgtga ctgcaaagct gcctgtgaga cggtaccgtg ttatccgtcc gccatggctg 1140
tgcccctgca actccttgta tcgtggtaaa tttgtgtgtg gcagtgtgcc tgggtgtgtg 1200
gttgtacctg tgagactctg acagtttgtg cctctgaata tctggtggag tgacaacagt 1260
gtaatgatga tatggggaca ggggaagccg agggtgcagg agattgtgct tcctggggcg 1320
tgatccattg ctgggaatct gtgcctgctt cctgggtctt cagtcctgag atccccctct 1380
cccatcccca aggaactcac ctcacaggac tataaaacgg tgttttggtg tgcatgggct 1440
tgtggcttgg tgtgactgtg ggcaaggctg ggagaggata ggagtgactc ggcgcaggac 1500
cgactctttg agcatcagtc tgcgcagaca agtgacccga tccttgctcc cagcaacaac 1560
tccaccccct gagctttaat tcaccccgaa ggacccgatc ctaccgctat gagcctagac 1620
tcctctgttg aacccctcct gaccgtggct ttgcaccgcg atggcaccag tctcacctcc 1680
agagctcacc ccagagccct gactccgccc cagaagccct ggtcccacct tctgagactg 1740
cctctagcca taacccagct cttgaagcct tgatggcgcc cctgcgctgt aaccccaacc 1800
ctaggagcac tgatcccgcc ttctcagccc acccccatgc cctgactctc ctcccaggag 1860
ccctgactac cctgaatccc tgaccaggct cctgcaccgt gatcaccgcc cctgggagcc 1920
ctaggcctat atcctggacc agcccctgaa gctccgatca tgacccctgc accataaccc 1980
cacccccagg agccctgggt ccgccccctg ggcccgcccc cagccctgac tcggcccccc 2040
aagagtcctg actgctcctg aagccctgac cacgcccctg ctcggtaacc cctcccccaa 2100
gagccctggg cccgcctcct gagcccgttc ccagccctga ctccgccccg aggagccctg 2160
actgctcctg aacctctgac cacgcccctg ctcggtaagc ccacccccag gaaccctggg 2220
cccgcctcct ggtcccgatc ccatccctga ctccgccctc aggatctctc gtctctggta 2280
gctgcagcca aatcataaac ggcgaggact gcagcccgca ctcgcagccc tggcaggcgg 2340
cactggtcat ggaaaacgaa ttgttctgct cgggcgtcct ggtgcatccg cagtgggtgc 2400
tgtcagccgc acactgtttc cagaagtgag tgcagaggta gggggagtgg gcagggcctg 2460
ggtccggggg cggggcctaa tatcaggctc atcttggggt gctcaggggg aaacagcggt 2520
gaaggctctg ggaggaggac ggaatgagcc tggatccggg gagcccagag ggaagggctg 2580
ggaggcggga atcttgcttc ggaaggactc agagagccct gacttgaaat ctcagcccag 2640
tgctgagtct ctagtgaact aaggcaagtt cttgtccctg aatttttgtg aatgaggatt 2700
tgagaccatg gttaagtagc tcttagggtg tttagcgaag agggtggggt tggggttagg 2760
agatggggat gggaatgggg ttgaagatga gaatggaggt aaggatgtag ttgccacaaa 2820
actgacctgc cctccgtggc ccacagctcc tacaccatcg ggctgggcct gcacagtctt 2880
gaggccgacc aagagccagg gagccagatg gtggaggcca gcctctccgt acggcaccca 2940

gagtacaaca gacccttgct cgctaacgac ctcatgctca tcaagttgga cgaatccgtg 3000

tccgagtctg acaccatccg gagcatcagc attgcttcgc agtgccctac cgcggggaac 3060

tcttgcctcg tttctggctg gggtctgctg gcgaacggtg agctcacggg tgtgtgtctg 3120

ccctcttcaa ggaggtcctc tgcccagtcg cgggggctga cccagagctc tgcgtcccag 3180

gcagaatgcc taccgtgctg cagtgcgtga acgtgtcggt ggtgtctgag gaggtctgca 3240

gtaagctcta tgacccgctg taccacccca gcatgttctg cgccggcgga gggcaagacc 3300

agaaggactc ctgcaacgtg agagagggga aaggggaggg caggcgactc agggaagggt 3360

ggagaagggg gagacagaga cacacagggc cgcatggcga gatgcagaga tggagagaca 3420

cacagggaga cagtgacaac tagagagaga aactgagaga aacagagaaa taaacacagg 3480

aataaagaga agcaaaggaa gagagaaaca gaaacagaca tggggaggca gaaacacaca 3540

cacatagaaa tgcagttgac cttccaacag catggggcct gagggcggtg acctccaccc 3600

aatagaaaat cctcttataa cttttgactc cccaaaaacc tgactagaaa tagcctactg 3660

ttgacgggga gccttaccaa taacataaat agtcgattta tgcatacgtt ttatgcattc 3720

atgatatacc tttgttggaa ttttttgata tttctaagct acacagttcg tctgtgaatt 3780

tttttaaatt gttgcaactc tcctaaaatt tttctgatgt gtttattgaa aaaatccaag 3840

tataagtgga cttgtgcagt tcaaaccagg gttgttcaag ggtcaactgt gtacccagag 3900

ggaaacagtg acacagattc atagaggtga aacacgaaga gaaacaggaa aaatcaagac 3960

tctacaaaga ggctgggcag ggtggctcat gcctgtaatc ccagcacttt gggaggcgag 4020

gcaggcagat cacttgaggt aaggagttca agaccagcct ggccaaaatg gtgaaatcct 4080

gtctgtacta aaaatacaaa agttagctgg atatggtggc aggcgcctgt aatcccagct 4140

acttgggagg ctgaggcagg agaattgctt gaatatggga ggcagaggtt gaagtgagtt 4200

gagatcacac cactatactc cagctggggc aacagagtaa gactctgtct caaaaaaaaa 4260

aaaaaaaaag actttacaaa gagatgcaga gacactgaga cagataaaca agccacaaag 4320

gagacaaagg agagacagac aaacagaaac agacagacca caagcccaag agaagcagcc 4380

agcattcagg acataggaca tcgggaagca ggattagatg aagtcaggga tctggaatgg 4440

gacttccaac agatatgttg ctgggctatg ttgttattga tgatggttct gtctttgttt 4500

ctcagtctca tttagttcct ttctgagccc atatccattt ccacctctct gtgttttgaa 4560

ttctgactct ccctctcttc acaacagggt gactctgggg ggcccctgat ctgcaacggg 4620

tacttgcagg gccttgtgtc tttcggaaaa gccccgtgtg gccaagttgg cgtgccaggt 4680

gtctacacca acctctgcaa attcactgag tggatagaga aaaccgtcca ggccagttaa 4740

<210> 2
<211> 237
<212> PRT

<213>  Homo sapiens

<400>  2

Ser Leu Val Ser Gly Ser Cys Ser Gln Ile Ile Asn Gly Glu Asp Cys
1               5                   10                  15

Ser Pro His Ser Gln Pro Trp Gln Ala Ala Leu Val Met Glu Asn Glu
            20                  25                  30

Leu Phe Cys Ser Gly Val Leu Val His Pro Gln Trp Val Leu Ser Ala
        35                  40                  45

Ala His Cys Phe Gln Asn Ser Tyr Thr Ile Gly Leu Gly Leu His Ser
    50                  55                  60

Leu Glu Ala Asp Gln Glu Pro Gly Ser Gln Met Val Glu Ala Ser Leu
65                  70                  75                  80

Ser Val Arg His Pro Glu Tyr Asn Arg Pro Leu Leu Ala Asn Asp Leu
                85                  90                  95

Met Leu Ile Lys Leu Asp Glu Ser Val Ser Glu Ser Asp Thr Ile Arg
            100                 105                 110

Ser Ile Ser Ile Ala Ser Gln Cys Pro Thr Ala Gly Asn Ser Cys Leu
        115                 120                 125

Val Ser Gly Trp Gly Leu Leu Ala Asn Gly Glu Leu Thr Gly Arg Met
    130                 135                 140

Pro Thr Val Leu Gln Cys Val Asn Val Ser Val Val Ser Glu Glu Val
145                 150                 155                 160

Cys Ser Lys Leu Tyr Asp Pro Leu Tyr His Pro Ser Met Phe Cys Ala
                165                 170                 175

Gly Gly Gly Gln Asp Gln Lys Asp Ser Cys Asn Gly Asp Ser Gly Gly
            180                 185                 190

Pro Leu Ile Cys Asn Gly Tyr Leu Gln Gly Leu Val Ser Phe Gly Lys
        195                 200                 205

Ala Pro Cys Gly Gln Val Gly Val Pro Gly Val Tyr Thr Asn Leu Cys
    210                 215                 220

Lys Phe Thr Glu Trp Ile Glu Lys Thr Val Gln Ala Ser
225                 230                 235

<210>  3
<211>  254
<212>  PRT
<213>  Homo sapiens

<400>  3

Met Ala Thr Ala Gly Asn Pro Trp Gly Trp Phe Leu Gly Tyr Leu Ile
1               5                   10                  15

Leu Gly Val Ala Gly Ser Leu Val Ser Gly Ser Cys Ser Gln Ile Ile
            20                  25                  30

Asn Gly Glu Asp Cys Ser Pro His Ser Gln Pro Trp Gln Ala Ala Leu
        35                  40                  45

Val Met Glu Asn Glu Leu Phe Cys Ser Gly Val Leu Val His Pro Gln
    50                  55                  60

Trp Val Leu Ser Ala Ala His Cys Phe Gln Asn Ser Tyr Thr Ile Gly
65                  70                  75                  80

Leu Gly Leu His Ser Leu Glu Ala Asp Gln Glu Pro Gly Ser Gln Met
                85                  90                  95

Val Glu Ala Ser Leu Ser Val Arg His Pro Glu Tyr Asn Arg Pro Leu
            100                 105                 110

Leu Ala Asn Asp Leu Met Leu Ile Lys Leu Asp Glu Ser Val Ser Glu
        115                 120                 125

Ser Asp Thr Ile Arg Ser Ile Ser Ile Ala Ser Gln Cys Pro Thr Ala
    130                 135                 140

Gly Asn Ser Cys Leu Val Ser Gly Trp Gly Leu Leu Ala Asn Gly Arg
145                 150                 155                 160

Met Pro Thr Val Leu Gln Cys Val Asn Val Ser Val Val Ser Glu Glu
                165                 170                 175

Val Cys Ser Lys Leu Tyr Asp Pro Leu Tyr His Pro Ser Met Phe Cys
            180                 185                 190

Ala Gly Gly Gly Gln Asp Gln Lys Asp Ser Cys Asn Gly Asp Ser Gly
        195                 200                 205

Gly Pro Leu Ile Cys Asn Gly Tyr Leu Gln Gly Leu Val Ser Phe Gly
    210                 215                 220

Lys Ala Pro Cys Gly Gln Val Gly Val Pro Gly Val Tyr Thr Asn Leu

225                 230                 235                 240

Cys Lys Phe Thr Glu Trp Ile Glu Lys Thr Val Gln Ala Ser
                245                 250

<210>  4
<211>  278
<212>  DNA
<213>  Homo sapiens

<400>  4
tgacccgctg taccacccca gcatgttctg cgccggcgga gggcaagacc agaaggactc      60

ctgcaacggt gactctgggg ggcccctgat ctgcaacggg tacttgcagg gccttgtgtc     120

tttcggaaaa gccccgtgtg gccaagttgg cgtgccaggt gcctacacca acctctgcaa     180

attcactgag tggatagaga aaaccgtcca ggccagttaa ctctggggac tgggaaccca     240

tgaaattgac ccccaaatac atcctgcgga aggaattc                             278

<210>  5
<211>  20
<212>  DNA
<213>  Artificial

<220>
<223>  Oligonucleotide primer

<400>  5
tgacccgctg taccacccca                                                  20

<210>  6
<211>  20
<212>  DNA
<213>  Artificial

<220>
<223>  Oligonucleotide primer

<400>  6
gaattccttc cgcaggatgt                                                  20

<210>  7
<211>  21
<212>  DNA
<213>  Artificial

<220>
<223>  Oligonucleotide primer

<400>  7
ggtgatctgc gccctggtcc t                                                21

<210>  8
<211>  21
<212>  DNA
<213>  Artificial

<220>
<223> Oligonucleotide primer

<400> 8
aggtgtccgg tggaggtggc a 21

<210> 9
<211> 18
<212> DNA
<213> Artificial

<220>
<223> Oligonucleotide primer

<400> 9
tgcgcaagtt caccctca 18

<210> 10
<211> 19
<212> DNA
<213> Artificial

<220>
<223> Oligonucleotide primer

<400> 10
ccctctcctt acttcatcc 19

<210> 11
<211> 20
<212> DNA
<213> Artificial

<220>
<223> Oligonucleotide primer

<400> 11
acaatgagct gcgtgtggct 20

<210> 12
<211> 21
<212> DNA
<213> Artificial

<220>
<223> Oligonucleotide primer

<400> 12
tctccttaat gtcacgcacg a 21

<210> 13
<211> 11570
<212> DNA
<213> Homo sapiens

<400> 13
gggcccagag tgaaggcaag agaaggagtt gagagctccc tctgcaaagt ggcttgagtc 60

tcccctgcct aaaatgcagg gagagggagg cagaaagaca gggaagagga aggggtgggg 120
aagaaagaga gagagagaga gagacagaat aacacaacta cagaaacaca gagagaacac 180
acagagagcc tgggacacag ggacacacag agtcagagag aaaagagaag atagagaaag 240
acacaaatgg agacacagag gtgtaaagaa agagagatta acagagtccc agatacacgc 300
aaaggggcag aagcacagtt ttcagggtgg tgtctatgat catcttcttt tttttttttt 360
tttttttttt tttttgagac ggagtctcgc tctgtcgccc aggctggagt gcagtggcgg 420
gatctcggct cactgcaagc tccgcctccc gggttcacgc cattctcctg cctcagcctc 480
ccaagtagct gggactacag gcgcccgcca ctacgcccgg ctaatttttt tgtattttta 540
gtagagacgg ggtttcaccg ttttagccgg gatggcctcg atctcctgac ctcgtgatcc 600
gcccgcctcg gcctcccaaa gtgctgggat tacaggcgtg agccaccgcg cccggccatg 660
atcatcttct tgactatgct gatgtgacaa gtacctaaag ccatcagact ctacccttta 720
aatatgcagt ttgggccagg caccgtggct catgcctgta attccagcac tttgggaggc 780
agaggtgggt gaatcacttg aggccaggag tttgagacca gcctggccaa catggtgaaa 840
ctctgtcttt actaaaaaaa aaaaaaaaaa aaaaaaaatc agccgggtgt cgtggggcac 900
acctgtaatc ccagctatgc tggaggctga ggcacgagag tcacttgaac cctggaggcg 960
gaggttgcag tgggccgaga tcacatcacc gccctccagc ctgggcgaca gagcaagact 1020
ctgtctcaaa taaataaata aacaaacgaa caagcagttt gttgtacctt agttatatct 1080
aaaaaaaaaa tgctgtcaac aaatagagca gaagtgaaat aaaggaaaat aaatgggcca 1140
agaactctaa ggtatatttg acaaatcatt cagaaccttt aaaaaagaaa gaatcacaga 1200
ggcatagaaa gacagggagg aacagggaga cagaaacacc tgtggcccaa ggagaacaaa 1260
acaaggctcc taagacagac aggaggagag agagagagag tgagtgagag acagacagag 1320
aaaaagacag agagagagag acagagacag agagacagag aggcgagagg gatagaaaga 1380
gagagagggg tggagagaga cacgagatat tgagagagac tcagaaagat agccgaggga 1440
gaaccacaga gagatggaag aagactctga gaaaaaacca gagacaaaga tggaaagagg 1500
agtatcgagg gtgaacagac agtggtggaa tgagcaaaat gcagagaaga aagcaagcaa 1560
tccaggcgcc aagaatagtg acccagagtt ggtgagaagc cagatcctta aggctggggg 1620
aggcagggaa ggggctggcc tggcttccgg agacccctcc ccattctccg ggccagggag 1680
gtagggagtg acattccgga ctgggtgggg ggtgctctgg gggtggagat agggggagca 1740
ggaggagcta ttgctaaggc ccgataggca cctcattgcc cgggaatgtg ccccagggag 1800
cagtgggtgg ttataactca ggcccggtgc ccagagccca ggaggaggca gtggccagga 1860
aggcacaggc ctgagaagtc tgcggctgag ctgggagcaa atcccccacc ccctacctgg 1920
gggacagggc aagtgagacc tggtgagggt ggctcagcag gcagggaagg agaggtgtct 1980

gtgcgtcctg cacccacatc tttctctgtc ccctccttgc cctgtctgga ggctgctaga 2040

ctcctatctt ctgaattcta tagtgcctgg gtctcagcgc agtgccgatg gtggcccgtc 2100

cttgtggttc ctctctacct ggggaaataa ggtaggggag ggaggggaag tgggttaagg 2160

gctccccgga tcgcctgggc ctcccaaccc tctgacattc cccatccagg tgcagcggcc 2220

atggctacag caagaccccc ctggatgtgg gtgctctgtg ctctgatcac agccttgctt 2280

ctgggggtca caggtaacca gaactctggg gtgggagggt tgtgggattg ggaggactgt 2340

ctctgcggca ctagagcgcc tgtcccctgg ggaactgtgt gagcctgggc atgactccgg 2400

gaccgggtga atgtgagtct ctgtctgtac ttgtggttgt gcgatcgtat gtggccctgt 2460

gactgccacg gtgtgtgtcg gggaggggga tgccttttcc catatcaggt gactgtgcgg 2520

caggtggcac tgaccctttg aggctgtgtg tgtggttttg tgattgtgtg tgcatttaag 2580

attgtgtgtg gctccacagc tgtgtgggtg aatgcatgta gcactggggg tgttcactgt 2640

gtgtttggct gtgtgtggtg acttggcatt gtatatgact gcaggtatct gcagttcctg 2700

tccctgaggt cccgggattg cgtgcaacaa aagtggtcat caccatggaa agctgtgact 2760

gtgtgctgct tgcaggcgat tatgtgattg tggctgagtg tgacgttatg gatgcccgta 2820

tttgtgaccg tgtgactacc tgaagctctg tgtaggggtg actgtatgtg actgtgtgtg 2880

tctgtgtgag gccgtgtaaa tgctactgta tgtgtgatgg tgcagctgtg tgtctggagt 2940

ttctgtctct gcctggaggg atagagggtg caggggtagc tatctctggg agatgggtgc 3000

caggtgactg acttgcagtg tgtgcctgtg tgcagaagag tatgtggcag tctgaacatc 3060

tgtgcacaca cggcatctgt gcgtggcact gagacactgt ggatgagggt gtgcgatccc 3120

gctaggctgc ccgggagcgt gtgtacctgg agacagagct gtatgttagc tgcacctgtg 3180

gaggcaacat gggcgtgtct gcagaactgc gtgcgtgctt ggctgttact gctgttgtgc 3240

gcgtggttct tggggtgagt tcgtgaatga tggtggtgcc agggccatca gcaagggtaa 3300

gaaccaggcc gggcgcggtg gctcacgcct gtaatcccag ccctttggga ggccgaggca 3360

ggcggatcac ctgaggtcgg gagatcgagg ccagcctgac caacatggag aaccccgtct 3420

ctactaaaaa tacaaaaaat tagctggtgt ggtggcgcgt gcctgtaatc ccagctactc 3480

gggagactgg ggcagaaaaa tcgcttgaac ccgggaggtg gaggttgcgg tgagccgaga 3540

tcgcgccatt gcactccagc ctgggcaaca agagcgaaac tccgtctcga aagaaaaaaa 3600

gaaaaaaaaa agggtaagaa ccagtgaatg ggcacgggag gactgatgat ggagtggggc 3660

atgcatgtag tctgtaggtc tgtgtgtgag aggaggagat tgacaggatt gagaaggcat 3720

gttttcatct gagaattcag aaacctaggc ctgctcttcc cctccatgtg gccccctaag 3780

ctgagccctt ctttcctggt cctgctttcg gaaccctagc tccgcccatg agctctgacc 3840

ccacctcctt tcctcaacca cgcccctagg ccagactcta gtggaccccg cctaaggcca 3900

cacccctttg ggccaggctc caccccctat tctgtgggta ccttctagaa cccccttcaa 3960

agtcagagct tttttttttt tttttttgga gacagtcttg ctctctctcc caggctggag 4020

tgcagtggcg tgatctcggc tcactgcaac ctctgcctcc caggttcaag tgattctcgt 4080

gcctccacct cctgagtagc tgggattaca ggtgcgcgcc accacgcctg gctaattttt 4140

gtgtctttag tagagacagg gtttcacctt gttggccagg ctggtctcaa actcccaacc 4200

tcaggtgatc cgcccacctc ggcctcccag agtgctgggg ttacaggcgt gagccaccgc 4260

ccccagccca aagtcagagc tctttatagg agactctaac atgtaaccct gaccctggcc 4320

ctaactaagt caattccaaa ccccttcctg cctccagccc tgaccccact cactgaggcc 4380

tgaccccact tcttgagacc agttccatcc ctaaagccct ggtctccctc ccatccccag 4440

gctccagccc ccacagcttt ggcactaccc ctgagcttgt ccaggaatcc tgtacccaat 4500

tttaccctca catgtagttc tagccaattc caggaatctg tgaggtccag ttagagtcca 4560

gtaaccctac ctgagcctgg gctctgtcct tgagcttgag cctgggcttg agaggtgcca 4620

ctcttattct ccaggccctg cccctgcccc ctcagcatgt cagacaccca ccctctagct 4680

ggtctggcct cttgagtctg aaacccaccc ccagcccaag ccccgcctct gagccccgcc 4740

caacccattt tccgttccca gagcatgttc tcgccaacaa tgatgtttcc tgtgaccacc 4800

cctctaacac cgtgccctct gggagcaacc aggacctggg agctggggcc ggggaagacg 4860

cccggtcgga tgacagcagc agccgcatca tcaatggatc cgactgcgat atgcacaccc 4920

agccgtggca ggccgcgctg ttgctaaggc ccaaccagct ctactgcggg gcggtgttgg 4980

tgcatccaca gtggctgctc acggccgccc actgcaggaa gaagtgagtg ggagttccaa 5040

gaggagggtt ggtggggacg gggaagtggg ggtgggggtg gggaagtggg ggtgggggtg 5100

tcatggaggt gagggctggt ggggacgggg aagtggggtt gggggtgtca tggaaggtga 5160

gggttggtgg ggatgggttg gggatgtggg agcaggagga ggtcgagttg gggataggac 5220

taaggatgga gttttgcggg ggagcaaggt gggaggatga ggttggagag gggagagtgt 5280

tgtggtaggg aatgggaagg agccaaggat gggttggatt tggggttagg agcatatatt 5340

tgttgaatgg tttgggatgg aggtggaatt gggattggct ttagaattgg gggtgggtga 5400

aaatcgggct ggggtggaaa tgaagatagc atggagatag ggttgagatt gggagcagat 5460

atagaatgaa ggatggggat tggagttttg ggtggggttg gagatggttg gatttgggct 5520

tgagaatgca tatggtgatg gcttctgggt agggaaagaa ttagggttgg gaatgggatg 5580

ggtttggaat tgtgactggg atggggacag gcatgggatt ggagaccaag agggagttga 5640

ggatggtttg gggaccgggg gtggggatgg gggtggggct ggggctgggt gtggggttgg 5700

gattggcgtt ggacgtggag atagagatca gggttggtgg tgacctgccc catcttcctc 5760

agagttttca gagtccgtct cggccactac tccctgtcac cagtttatga atctgggcag 5820

cagatgttcc agggggtcaa atccatcccc caccctggct actcccaccc tggccactct 5880

aacgacctca tgctcatcaa actgaacaga agaattcgtc ccactaaaga tgtcagaccc 5940

atcaacgtct cctctcattg tccctctgct gggacaaagt gcttggtgtc tggctggggg 6000

acaaccaaga gcccccaagg tgagtgtcca ggttcttctt gataccgacc catctctgcc 6060

gccttccatc tttctccact tctcattgtg ttcctgtttg acagtgcact tccctaaggt 6120

cctccagtgc ttgaatatca gcgtgctaag tcagaaaagg tgcgaggatg cttacccgag 6180

acagatagat gacaccatgt tctgcgccgg tgacaaagca ggtagagact cctgccaggt 6240

gaggacacct ctctttattc agcagataca cactgagtgc caactcggta acatggagcg 6300

ttgccaaatt ctgagaatcc agcaattgcc aagacagtca ggacccctgt tctcacagag 6360

ctcataccct agagtagtgg tgtttagtag aaataatgct gagctgctta tgtcatttcc 6420

agttttttag tagccacatt aaaacaggta aaaaaggctg ggcgcagtgg ctcacacctg 6480

taatcccagc actttgggag gctgaggcag gcagatcacc tttggtcagg agtttgagac 6540

tagcctggcc aacatggcga aactctgtct ctaaaaaaaa atacaaaaat tagcctggca 6600

tggtggcggg cgcctgtaat ctcagctgct caggaggccg agacacaaga atcacttaaa 6660

cccaggaggt ggaggttgca gtgagctgag atcgtgccac tcactccaac ctgggagaca 6720

gagtgacact tttgtctcaa aaagaaaaaa aaaaacaagt aaaaaagaaa caggtgaagt 6780

taactttaat aacccaatgt atcccaaata caatcatttc aaagtgtaat taatataaaa 6840

caattatgaa tgagatactt tacattcttt tcttgttttc atattaagtc tttgaaagtg 6900

agtatatatg ttatgctgac agcacatctc aatttggact agctacattt caggtgctca 6960

gtagccacat gtggctagca gttactgtat tggatggcac ggatctagag ggaaagatca 7020

gggctgtttt gtatggttgg gcaggttgtg cactgcataa agataccata tctaataggg 7080

gcactccgtg ttacagatgt cagttttggc agttttcagg cgtgtggtag ttaagtgtct 7140

tgtttcaaca aaatctgtaa tatgacagtt ttctagcaag tgctggtaaa atatcttgag 7200

gaaggaaaag agaaatctgg taggtatttt tacaagagaa tatttaatac aggggattaa 7260

ttgcaaagct gctggaaggg ctggaggaac aaagttaaaa aataaaaaac tctgtggtca 7320

agaatctgca taaatagggc aatttcagag agtggtaaag gttaacccca aaataaaaca 7380

tggttttagg atagtaaaca ataagggcca atattcaaaa aggtggtcag gggagcctcc 7440

ttggagaggt ggcatttgag cagagaatgg atgacacaaa gaagctaaac tcgtgaagtt 7500

taaggggaaa gaaaaggcac gtgcaaaggc cctgaggcag taaggaattt ggctgattca 7560

aagaagaaga ggaaaccaat gcaactggag aacaaaagtg ggggcaacag tagaaagtga 7620

cgctggaggt gtaggcaggg gcgaatgctc tgcaagtatt tcttggtcac caacacagag 7680

cttccctatg ttctaatgga agctgtatct gttgaggaag acagaattta aaatcaaact 7740

gttacatcaa ccagcaccct tctctgtatt caggctccca agggatctag aaggacgtaa 7800
gttaacaagc tctcattagc agggtgtgtg tttcaacagt agttaggaag ctggggattc 7860
aggagtactc cagtcccatg gctatgaaaa gctcccccca aattgtacaa acctgacaaa 7920
tgcaacacct ccccagctct ccccatttct tctctgtgcc ctgggtgtgg gggggtgggt 7980
tgcgaggggg aaaactttta acagaagaaa gcacatctcg gccgggcgtg gtggctcaca 8040
cctgtaatcc caacactttg ggaggccgag gcgggtggat cactaggtca ggagatggag 8100
accatcctgg ctgacacggt gaaaccctgt ctctactaaa aacacaaaaa attagccggg 8160
cgtggtggca ggcgcctgta gtcccagcta ctcgggaggc tgaggcagga gaatggcctg 8220
aacccgggag gcggaacttg cagtgagccg aggttgcacc actgcactcc agcctgggca 8280
acacagtgag actccgtctc aaaaaaaaaa aaagaaaaga aaagaaatca catctcattc 8340
aagtggtggc atttaaaact atttagcctt tctgtaggca aggttagtat cttgtttttc 8400
cagacctcaa ggtgtttttt tgtttgtttt ttcataccgg tgtgtggtct gggtgtggcc 8460
actaaaagct acaagcaaga aataataaca actacaacaa tactaatacc aatagtataa 8520
aaataatagc atctggctaa ttgctggaca ctgttttaag tggtttgcat gcctcagctc 8580
attaactcat ttacctgtta ttattggccc tattttacaa acaaggagcc aaggctcaga 8640
gcagttaact aacagcctct caaaagaaac tctgcagaga tattaaattt aaaaaataat 8700
gagagaaatt aaaccacaag aaagttgaaa tttagaggta caggcagcta agcttgtttg 8760
ctttgaaaca gtgtctgcta ctgggaaaaa ggcaagtctt ggctttccta ataattgata 8820
ccaggactct gtaattcata ttttgcatgc atgtaagtaa gaaatgaagc cgggtgcaat 8880
ggcacatgcc agtaatccca gcactctggg agactgaagt gggaagatca cttgagctca 8940
ggagttcaag accagcctgg gcaactaaaa attaaaaaaa taaaaatact aattgttttt 9000
attttagtag attttattca taccacttac atcattattg tagtatgtac atatttattt 9060
cttttctttt cttttctttt cttttttgag acggagtctc gctctgtcac ccaggctgga 9120
gtgcaatggc accatatcag ctcactgcag catgcgcctc ctgggttcaa gcatttcttc 9180
cacctcagcc tcccaagtag ctgggataac aggcacccac caccatgcct ggctattttt 9240
ttttttccgt agagatgggg ttccaccatg ttggccaggc tggtcttgaa ctcctgacct 9300
ccagtgatct gcctgcctcg gcctcccaaa ttgctggtat tacaggtgtg agccaccgtg 9360
cccaggtggg agatagacat ttctctctac ctcaaacaga ggtccactca agctactttt 9420
cattttcttc ataaatatta gccgagtggc tattttgcac caggaatggt tccaggtgct 9480
gtggatatgg catcaggcaa aacagaccaa aaacttcctg ccgcgtggac ctcatgttcc 9540
ccaagtggaa gacaggcaat aaagagatag ataaatatgt agtaaattaa aaaaaaaaaa 9600
aattagccgg gtgtggtggc ttgcacctgt agttccagct acttgggagg ctgaggtggg 9660

agaattgctt gagcccaaac gtttgaggct gcggtaagcc atgactgcac tgctgcactc 9720

cagacagcag cctgggtgac aaagcaagac gtttttgtca gaaagaaaaa aaaaagagac 9780

gaagggagga aggagagaga aaggaaggaa ggaaggagaa agaaaggaag gaaggagaaa 9840

gaaaggaagg aaggaaggag aaagaaagga agaaagagaa agaaagaaaa agaaagaaag 9900

aaagaagaaa gaaaagagag aggaaggaag gaaagaagga aaagagggaa aaaaatgact 9960

gttgaagagc agtgagtatt attataggag ggtaattata gggaggtatg gggaattgaa 10020

gacaggaaac acaaattagt ccaagcgaat ggatttctat tgggagtgat tctgccccta 10080

gaagacactg gcaataccag gagacatttt tggttgtcac aactatatgg aggggcatta 10140

ctggcaacta atggatagat gccaagtgtg ctgttcaaca tgctatgatg cacacggcag 10200

gcctccacaa caaaccatta tccagcttca gatgcccaca gtgcccagat cgaggaaccc 10260

tcatccaggg gctgagaacc gtatttttgc agaagggagg tataaggatg ggttggtgga 10320

gaatggggaa ggaaggtgtg tgtccagtaa gagaaataag gcctgcacag gctggagggg 10380

agagtgagag agaaagggag gcggagagat acacgatgag ggagacaggc tggaacagaa 10440

agtagagacg aagattcgag atgtggagag gaagggtcac agaccccccc gaaatgatgt 10500

gtggacaaca ggaatctgga agaggaagat ggagtggaga gtgacaaatg gggtctaaag 10560

gttgaacttg gaggccaggc atggtggctc acgcctgtaa tcccaacact ttggaggctg 10620

aggtgggcga atcacttgag gccaggagtt cgagaccagc ctggccaaca tggtgaaacc 10680

ccgtctctac aaaaaaaata caaaaaatta gccgggtgtg gtgatggaca cctgtagtca 10740

cagctacttg ggaggctgag gcaggagaat tgcttgaacc cgggagatgg aggctgcagt 10800

gagctgaggt caggccactg cgctccaacc tgggcaacag agtaagactc catctcaaaa 10860

aaaaaaaagc tggatttgga gtgaaatatt aataacattc tccctctctc tccttttgcc 10920

tgtgtctcca tctctgtctt tttctgcatt tcttcatctc tgtactttcc atctctgtgt 10980

gtctgttccc atctgcttct ccatctatgg gcatctctgg gtctctcatg tctccttctg 11040

cccactttgc cacatctctg cctctctcat gccccccttt ctctcctgca gggtgattct 11100

ggggggcctg tggtctgcaa tggctccctg cagggactcg tgtcctgggg agattaccct 11160

tgtgcccggc ccaacagacc gggtgtctac acgaacctct gcaagttcac caagtggatc 11220

caggaaacca tccaggccaa ctcctgagtc atcccaggac tcagcacacc ggcatcccca 11280

cctgctgcag ggacagccct gacactcctt tcagaccctc attccttccc agagatgttg 11340

agaatgttca tctctccagc ccctgacccc atgtctcctg gactcagggt ctgcttcccc 11400

cacattgggc tgaccgtgtc tctctagttg aaccctggga acaatttcca aaactgtcca 11460

gggcgggggt tgcgtctcaa tctccctggg gcactttcat cctcaagctc agggcccatc 11520

ccttctctgc agctctgacc caaatttagt cccagaaata aactgagaag 11570

<210>  14
<211>  293
<212>  PRT
<213>  Homo sapiens

<400>  14

Met Ala Thr Ala Arg Pro Pro Trp Met Trp Val Leu Cys Ala Leu Ile
1               5                   10                  15

Thr Ala Leu Leu Leu Gly Val Thr Glu His Val Leu Ala Asn Asn Asp
            20                  25                  30

Val Ser Cys Asp His Pro Ser Asn Thr Val Pro Ser Gly Ser Asn Gln
        35                  40                  45

Asp Leu Gly Ala Gly Ala Gly Glu Asp Ala Arg Ser Asp Asp Ser Ser
    50                  55                  60

Ser Arg Ile Ile Asn Gly Ser Asp Cys Asp Met His Thr Gln Pro Trp
65                  70                  75                  80

Gln Ala Ala Leu Leu Leu Arg Pro Asn Gln Leu Tyr Cys Gly Ala Val
                85                  90                  95

Leu Val His Pro Gln Trp Leu Leu Thr Ala Ala His Cys Arg Lys Lys
            100                 105                 110

Val Phe Arg Val Arg Leu Gly His Tyr Ser Leu Ser Pro Val Tyr Glu
        115                 120                 125

Ser Gly Gln Gln Met Phe Gln Gly Val Lys Ser Ile Pro His Pro Gly
    130                 135                 140

Tyr Ser His Pro Gly His Ser Asn Asp Leu Met Leu Ile Lys Leu Asn
145                 150                 155                 160

Arg Arg Ile Arg Pro Thr Lys Asp Val Arg Pro Ile Asn Val Ser Ser
                165                 170                 175

His Cys Pro Ser Ala Gly Thr Lys Cys Leu Val Ser Gly Trp Gly Thr
            180                 185                 190

Thr Lys Ser Pro Gln Val His Phe Pro Lys Val Leu Gln Cys Leu Asn
        195                 200                 205

Ile Ser Val Leu Ser Gln Lys Arg Cys Glu Asp Ala Tyr Pro Arg Gln
    210                 215                 220

Ile Asp Asp Thr Met Phe Cys Ala Gly Asp Lys Ala Gly Arg Asp Ser
225                 230                 235                 240

Cys Gln Gly Asp Ser Gly Gly Pro Val Val Cys Asn Gly Ser Leu Gln
                245                 250                 255

Gly Leu Val Ser Trp Gly Asp Tyr Pro Cys Ala Arg Pro Asn Arg Pro
            260                 265                 270

Gly Val Tyr Thr Asn Leu Cys Lys Phe Thr Lys Trp Ile Gln Glu Thr
        275                 280                 285

Ile Gln Ala Asn Ser
    290

<210>  15
<211>  20
<212>  DNA
<213>  Artificial

<220>
<223>  Oligonucleotide primer

<400>  15
ggatgcttac ccgagacaga                                                   20

<210>  16
<211>  22
<212>  DNA
<213>  Artificial

<220>
<223>  Oligonucleotide primer

<400>  16
gctggagaga gatgaacatt ct                                                22

<210>  17
<211>  21
<212>  DNA
<213>  Artificial

<220>
<223>  Oligonucleotide primer

<400>  17
ggtgatctgc gccctggtcc t                                                 21

<210>  18
<211>  21
<212>  DNA
<213>  Artificial

<220>
<223>  Oligonucleotide primer

<400>  18
aggtgtccgg tggaggtggc a                                                21


<210>  19
<211>  28
<212>  DNA
<213>  Artificial

<220>
<223>  Oligonucleotide primer

<400>  19
ccgagacgga ctctgaaaac tttcttcc                                         28


<210>  20
<211>  28
<212>  DNA
<213>  Artificial

<220>
<223>  Oligonucleotide primer

<400>  20
tgaaaacttt cttcctgcag tgggcggc                                         28


<210>  21
<211>  8622
<212>  DNA
<213>  Homo sapiens

<400>  21
cttgaaccca ggaggcagag gttgcagtga gctgagatcg cgccactgta cttcagcctg      60

ggtgtcagag caatactccg ttttggaaaa caaacaaaca aacaaacaaa caaaaaacag     120

atggagcaac tgagagaggt cttgtgactt gcccaaagtc acacacctca tcactaatca     180

cacctaatca ttgagatttg gacacacatg gttcagttcc agagtccatg ctccaaacca     240

tgacgacaca gtgagagaac attcaagggg agcccagacc cagcttcata accaggcctg     300

tgagcaggag aaagtggaag ggatcgtaag tgcccagggg aggcaaagat ggactctgcc     360

tgaggatctc agagatttcc tggaggaggg agaattgagg ttgggtgttg aaggatgagt     420

gggagttcac caggaaaaga aggatatgga gaaagacatt cactcattca atgaacatct     480

cctgaggact tctgcaagcc ctgttccgcc tggaacgggg tgatgctggg acacagagat     540

gagtcagacc tgggcccagc cctccagaag ctgtccacct ggtgagaagg aatgatgagg     600

agagaggcag ggaggatggg gtgatggaag ggacaatggg gtggggggca gggagatgga      660

tgaaaaaaat atatagcaaa tgttctcagg atttggcaaa gatcaggatg tattaagaga     720

gagcacaggg cacttgctac ctggaaggtt gggcacctgg gtccttgggt ggtggagccg     780

tggggaaggg ggcaggttat gacaagagtg ggttaatcca gatggaacca gatttctcaa     840

cattctagga gagggccttg tccttgtggg aagaggccca aatccccagg gcagggaagg     900

ttctgcaagg tgtgtaaacc tgtgcagctg cctgtggtct ctgcctcact ccacctggat 960
ttccctcaat ctttcccgtg ttctgtctcc tcctcccact cctcctctca tcttgggtcc 1020
ttctgtgcct gtacctccct ctctttgtat cttttgctct tgtgtctgag tcctgactct 1080
gtcttccacc cctcgcctcc tttctgggtg gtccccctgc acatccctcc agcctgccgt 1140
gggaggttgg tctctgcaca ccactgcttt atccaaaata aacctgctgc accccaggac 1200
cttaggcttc aaggatctcc ctccttttcc aggacacaaa agattctgta tcttgtagcc 1260
taaggtgatg aggaatgagg tctcccactc tgaagacccc agaggaggtg cccacaacct 1320
ctccacaccc ccagcactcc tcctccattc agtcaagctc tggcccagca agccgccagt 1380
tcatcccaaa aggggggtcc ccctgcactt acctcctctc ccaaggcccc tgtcacagcc 1440
ccagggcttc cccctccccc aggtacattt cccaaccccg attaatcaca ggggcggccc 1500
catggaggag gaaggagatg gcatggctta ccataaagaa gcactggacg ccgggtgcac 1560
gttccaggat ccaggtgccc aggggtcatg aagctgggac tcctctgtgc tctgctctct 1620
ctgctggcag gtgaggctcc caggctggct gccccttcac ggctgtacta aggtcacctt 1680
gctcttccct cccatcccag gcttctgcct cctgccctct aggcttctca gcatcctctc 1740
cctgccctcc cagcctgctc ttcgctgacc cctttgtccc tcatccccac cccagggcat 1800
ggctgggcag acacccgtgc catcggggcc gaggaatgtc gccccaactc ccagccttgg 1860
caggccggcc tcttccacct tactcggctc ttctgtgggg cgaccctcat cagtgaccgc 1920
tggctgctca cagctgccca ctgccgcaag ccgtgagtga cccaggctgg ccatgctggg 1980
gagggacaga ggctgggggt caggagaggg tgaggggtgc tttaggccag aagtgcggag 2040
cctccacttc tgataccaca agttcaactc ttagaagtag gaagggtagc ctcccaaatc 2100
ctaaaattct agagaccagc aatatctcat ttgagaagtc taagattcga aacttaggct 2160
cttcgaatcc gagactgacc cagagaaatc cagaatcgta gaatcctaaa atcttgaatt 2220
tatgaaattc tgcaatagcc tcagcaaatt ttagaatcat agattcgcag actattagaa 2280
tcttagcagt ctgggtcagc actgcccaga ggaattatga tgccagccac atgtgtaagt 2340
ttaaatttct ggtggacaca tttaaaaaat aaggaatgag taaaattaat tctaatagat 2400
ttaacttgac atacccaaaa acttattttg acatgtaatc aatttttaaa tacgtatgaa 2460
cgatacagtt tacttttgtt ttggtactaa gcctttgaaa tctgttctgt attttacaca 2520
catagcctgt tacaaaatgg actagccaca tttcaagtgt tcaatagcca taatggctag 2580
tgtgatccta gaatcttaaa ttcagagctt tctagattca ttgaatattg aaactcacag 2640
tactagaatc tttgattcac agtatcctag aatattgaga ttcagataat tctgtagtct 2700
taaactattt gaatcccaga ctcttaaatt tctaaggtta tagatttata gaatgatgac 2760
attctagtct ttcttttttt tttttttttt tttttttgag acagagtctc cctctatctc 2820

ccaggctgga gtgcagtggc acaatctcag ctcactgcaa cctctgcctc tcgggttcaa 2880
gcaattctcc tgcctcagcc tcctgagtag ctgggattac aggtatgcac caccatgcca 2940
ggctattttt tttttttttt tttttttagt agagacgggg gtttcaccat attggccagg 3000
ctggtcttga actcctgacc ttgtgatctg cccgcctcgg cctcccaaag tgctgggatt 3060
acaggcgtga gccaccgcgc ccagccaaaa ttctagtctt tttgtcctag aacattaaaa 3120
ttctatgttc aaatcttaga tttaattcag ataatgttag aatcctggag tttttttgat 3180
ccaggggaat ctggaatgtt agaatcttgg attcataaaa ctctaaacct tgagcctcta 3240
gattctagaa tcatggataa tagtgtgtcg gaatctgaga attctagaat cttaggttct 3300
gggcattcta atagtatcct ggaatccacc tgatgcagga atcctctctc cattgcctct 3360
gaaaagtgac catccatact gttccaattt tcttccctcc atgagtaaag cactgattgt 3420
ggtaagagat gctgtgtggg aatttcccat catgcattgc tccatgatgg aacctccttt 3480
aacttaagcc tatacatcag actgggagaa cgatgttcag atttcagccg aaagtgaagc 3540
aggagaaatg cagagatatg aaggtggaag agagtgagag gcaggggaag ggtaggggga 3600
tgaagggatg taggggtgag gactactttt ccagatccag agccaagaca gcaagaatga 3660
cagagagaga cagacacaga tgtttctggt tccccaaccc tgaattcgca gtcattagcc 3720
tgctgcctaa tgtcagaggt cagaggctgg ggaatggact tgtcatcccc gaaaggatcc 3780
cagctgtcta gggcatggac cagaaatgaa acaagtgcgc tgagactgtg gtgagggctt 3840
aaggttagac accaggaaga catgcattga agggtgaagg atatgataga caggaaaagc 3900
tgaggccaga gatgaccccc aatttgggga ttttccatat cccatcccct ttcatacaca 3960
cgcacacgta tacacacaca ccacttagac atacagagcc gctcccacag aagccaccag 4020
acctgtgggg gcaggggtgg ggcggttgtt atgtggtagg tggggtcccc cgtgcccaca 4080
ccgttcctag ggacccaagt caccaccaag gctccaggtg agtagggagg aaggtggctc 4140
actcagcctg ggactaggag cgggggcttt gtggggagag ctacaaagat ggagacacac 4200
aaaacatcag agtggggacc agggacccag aggaggtgtg tgcctcgctt aaaatcacag 4260
taccctgggc cagacataga tgatgagggt gcagagaggg tgtgtggctt gcagagggtc 4320
acacagcacc ctgatggaca ggaaaagagg gctggggctg aaaggacttt tacctttccc 4380
ccagcttgac ctctgaggcc tgtcccagca ggtatctgtg ggtccgcctt ggagagcacc 4440
acctctggaa atgggagggt ccggagcagc tgttccgggt tacggacttc ttcccccacc 4500
ctggcttcaa caaggacctc agcgccaatg accacaatga tgacatcatg ctgatccgcc 4560
tgcccaggca ggcacgtctg agtcctgctg tgcagcccct caacctcagc cagacctgtg 4620
tctccccagg catgcagtgt ctcatctcag gctgggggcc cgtgtccagc cccaagggta 4680
tgacctggcc cagaactctc tctgaaactt gctccctcac ccctctgtct ctgccttttc 4740

atctctgtct tctccttttc tctctcctct ctctctctgt cagtctatct atctgccaat 4800
cgatatattt aaccaaatat aagatgctag catttttaag atgtgccatt atttcatgaa 4860
ctgcgaagaa gtggaagaag gaggaggagg agaagaaaaa aaggaggagg aggaaagatc 4920
ccattagatc ccattgatta tataacacca ttttctggaa gacacattct aatttcagag 4980
tgtttgtttg tttgtttgtt tgtttgtttt tgagacaggg tctcgctttg ttgctcaggc 5040
tggagtgcag cggtgtgatc acggctcatt gcagctttga actcctgggc tcaagtgatc 5100
ctctcgcctc aacctcccaa gtagctggga ttacagatat gcaccaccac atcccacacc 5160
ggggtcattt ttttattatt tattattatt attattatta tctttttttt tgtattttta 5220
gtagagacag aggtttcacc atattggcca ggctggtctc aaattcctga cctggtgatc 5280
tgcccgcctt ggactcccaa agtgctggga aaacaggcat gagccactgc acccagccaa 5340
aattctagtc ttttttaaat ctagtcatat cttagattta attcagataa tgttagaatc 5400
ctggagtttt ttgatccagg ggaatctgga atgttagaat cttggattca taaaactcta 5460
aacgttgagc ctctagattc tagaatcatg gatactagtg tgtcagaatc tgagaattct 5520
agaatcttag attctgggca ttctaatagt atcctggaat ccacctgatg caggaatcct 5580
ctctccattg cctctgaaaa gtgaccatcc atactgttcc aattttcttc cctccatgaa 5640
taaagcactg attctggtaa aagatgctgg gtgggaattt cccatcatgc attgctccat 5700
gatgggacct cctttaactt aagccttatg ctaaaaattt ttattatttt tagcaaagat 5760
gaggtcttgc tatgttgtcc aggctagtct caaactcctg gcctcccaaa gtgctgagat 5820
tacaagtgtg agccactgta cctggcccag agatgtttaa atgtgaaatg cgttcatctt 5880
agaatgggaa taagaccatg tctctcagag tcacggatca ctgacccatt agccaaattg 5940
ggtcagtgga ttggaaaaac agtctgaatt tgttgctgcc aatatctaaa acttggaaag 6000
ttttatacaa aagccaggtt tctggattca cctgaaaaag tttgaagaac tcacattccc 6060
aaaatagcaa gcattgggct gagtcaatgg aggctgcccc cttcagccaa gataagttct 6120
ctgattcact ccaatggacc caaatggctc ctgtctccct gcacagcccc cgtccccgac 6180
ttctgtttac caattctgtt tatcatatcc cttgatgcat cggagcctgc acccatgtct 6240
tatatagatg cacatgtgta ttatatatcc atatccacat ctatactgac tacactgtat 6300
ctggtatctc tgtctatgtc tctgtctcca tcagtgacca tcttcctgca aatctctttc 6360
cttttatctc actgccttca ttccacccct tgaggtctgg gtctttttct atttcttttt 6420
tttttttttt taagagactg agtcttgctc ttgttgccca ggctggagtg cagtggtgtg 6480
atctcggctc actgcaacct ccacctcctg ggttttaagt gatcctcctg cctcagcctc 6540
ccgagtagct gggactacag gtgtgcaaca gcatgcccag ctgatttttt gtattttcag 6600
tagagacgga gtttcaccat gttggccagg atggtctcaa tctcttgacc ttgtgatccg 6660

cccgcctcag cctcccaaag tgctagggag ttatatatgc atctcctctt atctcttggc 6720

tctctgcatg catctttctg tttctcttcc ttcctttctt tttttttttt tttttttttt 6780

tttttttttt ttttttgaga cggagtcttg ctctgtctcc caggctggag tgcagtgacc 6840

agtctcggct cactgcaacc tccacctccc aggttcaagt gattctcgtg cctcagcctc 6900

ccgagtagct gggattacag gcgcctgcca ccatgcctgg ctaatttttg tatatttagc 6960

agagatgggg tttcaccatg ttggctgggc tggtctcaaa ctcctgacct caagcgatcc 7020

gccggcctcg gcctccaaaa cactgggatt acaggcatga gccacggtgc ccggccagcc 7080

tctctctcta cttggccctc ttcctccttg tctccatttg tttctcttgt gtgctatgac 7140

tgtctgtctg tcactgtctc ttgtctctat ctttgagagt cctaaatgtg gctccattgg 7200

tcctttggaa aagctgcagg gaggactcag ggcagtgggg tgctgagtgt gttggagaca 7260

gttgcagatc cttgacagtt ctcttccctg acagcgctgt ttccagtcac actgcagtgt 7320

gccaacatca gcatcctgga gaacaaactc tgtcactggg cataccctgg acacatctcg 7380

gacagcatgc tctgtgcggg cctgtgggag gggggccgag gttcctgcca ggtgagacct 7440

tactctgggg aaaatgaggc tgtcctgcca agttttctag gatttagggg agcagagggg 7500

tcggccccca gccttcctgg gtcaaaatga gaaggagact gggatacctg gttcctggga 7560

gaggacggga ccagggcctg gactccttag tgtaaaagag aaaaggtctg gaggtccaga 7620

cttctggatc tacaggagga gtgggctggg cgtccagagt ctgagtcctc ggggaggagg 7680

aggttaggtc ctgcggggag gtgggccctc tgagctttta ctcctgggtc tgaggaagaa 7740

gaggctggag atggaggact ctcggatgtt ggaggaggaa ggggctgggg cctttctggg 7800

agggaggaag tggcccgtgt aattgtcatg aacagagtgg cctaacagtt cctctgccct 7860

tctctcgcgt acagggtgac tctgggggcc ccctggtttg caatggaacc ttggcaggcg 7920

tggtgtctgg gggtgctgag ccctgctcca gaccccggcg ccccgcagtc tacaccagcg 7980

tatgccacta ccttgactgg atccaagaaa tcatggagaa ctgagcccgc gcgccacggg 8040

ggcaccttgg aagaccaaga gaggccgaag ggcacggggt agggggttct cgtagggtcc 8100

cagcctcaat ggttcccgcc ctggacctcc agctgccctg actcccctct ggacactaag 8160

actccgcccc tgaggctccg ccccctcacg aggtcaagca agacacagtc gcgccccctc 8220

ggaacggagc agggacacgc ccttcagagc ccgtctctat gacgtcaccg acagccatca 8280

cctccttctt ggaacagcac agcctgtggc tccgccccaa ggaaccactt acacaaaata 8340

gctccgcccc tcggaacttt gcccagtggg acttcccctc gggactccac cccttgtggc 8400

cccgcctcct tcaccagaga tctcgcccct cgtgatgtca ggggcgcagt agctccgccc 8460

acgtggagct cgggcggtgt agagctcagc cccttgtggc cccgtcctgg gcgtgtgctg 8520

ggtttgaatc ctggcggaga cctgggggga aattgaggga gggtctggat acctttagag 8580

ccaatgcaac ggatgatttt tcagtaaacg cgggaaacct ca 8622

<210> 22
<211> 296
<212> PRT
<213> Homo sapiens

<400> 22

Val His Phe Pro Thr Pro Ile Asn His Arg Gly Gly Pro Met Glu Glu
1               5                   10                  15

Glu Gly Asp Gly Met Ala Tyr His Lys Glu Ala Leu Asp Ala Gly Cys
            20                  25                  30

Thr Phe Gln Asp Pro Ala Cys Ser Ser Leu Thr Pro Leu Ser Leu Ile
        35                  40                  45

Pro Thr Pro Gly His Gly Trp Ala Asp Thr Arg Ala Ile Gly Ala Glu
    50                  55                  60

Glu Cys Arg Pro Asn Ser Gln Pro Trp Gln Ala Gly Leu Phe His Leu
65                  70                  75                  80

Thr Arg Leu Phe Cys Gly Ala Thr Leu Ile Ser Asp Arg Trp Leu Leu
                85                  90                  95

Thr Ala Ala His Cys Arg Lys Pro Leu Thr Ser Glu Ala Cys Pro Ser
            100                 105                 110

Arg Tyr Leu Trp Val Arg Leu Gly Glu His His Leu Trp Lys Trp Glu
        115                 120                 125

Gly Pro Glu Gln Leu Phe Arg Val Thr Asp Phe Phe Pro His Pro Gly
    130                 135                 140

Phe Asn Lys Asp Leu Ser Ala Asn Asp His Asn Asp Asp Ile Met Leu
145                 150                 155                 160

Ile Arg Leu Pro Arg Gln Ala Arg Leu Ser Pro Ala Val Gln Pro Leu
                165                 170                 175

Asn Leu Ser Gln Thr Cys Val Ser Pro Gly Met Gln Cys Leu Ile Ser
            180                 185                 190

Gly Trp Gly Ala Val Ser Ser Pro Lys Ala Leu Phe Pro Val Thr Leu
        195                 200                 205

Gln Cys Ala Asn Ile Ser Ile Leu Glu Asn Lys Leu Cys His Trp Ala
    210                 215                 220

Tyr Pro Gly His Ile Ser Asp Ser Met Leu Cys Ala Gly Leu Trp Glu
225                 230                 235                 240

Gly Gly Arg Gly Ser Cys Gln Gly Asp Ser Gly Gly Pro Leu Val Cys
                245                 250                 255

Asn Gly Thr Leu Ala Gly Val Val Ser Gly Gly Ala Glu Pro Cys Ser
            260                 265                 270

Arg Pro Arg Arg Pro Ala Val Tyr Thr Ser Val Cys His Tyr Leu Asp
        275                 280                 285

Trp Ile Gln Glu Ile Met Glu Asn
    290                 295

<210>  23
<211>  250
<212>  PRT
<213>  Homo sapiens

<400>  23

Met Lys Leu Gly Leu Leu Cys Ala Leu Leu Ser Leu Leu Ala Gly His
1               5                   10                  15

Gly Trp Ala Asp Thr Arg Ala Ile Gly Ala Glu Glu Cys Arg Pro Asn
            20                  25                  30

Ser Gln Pro Trp Gln Ala Gly Leu Phe His Leu Thr Arg Leu Phe Cys
        35                  40                  45

Gly Ala Thr Leu Ile Ser Asp Arg Trp Leu Leu Thr Ala Ala His Cys
    50                  55                  60

Arg Lys Pro Tyr Leu Trp Val Arg Leu Gly Glu His His Leu Trp Lys
65                  70                  75                  80

Trp Glu Gly Pro Glu Gln Leu Phe Arg Val Thr Asp Phe Phe Pro His
                85                  90                  95

Pro Gly Phe Asn Lys Asp Leu Ser Ala Asn Asp His Asn Asp Asp Ile
            100                 105                 110

Met Leu Ile Arg Leu Pro Arg Gln Ala Arg Leu Ser Pro Ala Val Gln
        115                 120                 125

Pro Leu Asn Leu Ser Gln Thr Cys Val Ser Pro Gly Met Gln Cys Leu
    130                 135                 140

Ile Ser Gly Trp Gly Ala Val Ser Ser Pro Lys Ala Leu Phe Pro Val
145                 150                 155                 160

Thr Leu Gln Cys Ala Asn Ile Ser Ile Leu Glu Asn Lys Leu Cys His
                165                 170                 175

Trp Ala Tyr Pro Gly His Ile Ser Asp Ser Met Leu Cys Ala Gly Leu
            180                 185                 190

Trp Glu Gly Gly Arg Gly Ser Cys Gln Gly Asp Ser Gly Gly Pro Leu
        195                 200                 205

Val Cys Asn Gly Thr Leu Ala Gly Val Val Ser Gly Gly Ala Glu Pro
    210                 215                 220

Cys Ser Arg Pro Arg Arg Pro Ala Val Tyr Thr Ser Val Cys His Tyr
225                 230                 235                 240

Leu Asp Trp Ile Gln Glu Ile Met Glu Asn
                245                 250

<210>  24
<211>  20
<212>  DNA
<213>  Artificial

<220>
<223>  Oligonucleotide primer

<400>  24
catgcagtgt ctcatctcag                                                   20

<210>  25
<211>  20
<212>  DNA
<213>  Artificial

<220>
<223>  Oligonucleotide primer

<400>  25
catggaggag gaaggagatg                                                   20

<210>  26
<211>  20
<212>  DNA
<213>  Artificial

<220>
<223>  Oligonucleotide primer

<400>  26
cttcggcctc tcttggtctt                                                   20

<210> 27
<211> 21
<212> DNA
<213> Artificial

<220>
<223> Oligonucleotide primer

<400> 27
gaccctgaca ttggacatct a 21


<210> 28
<211> 21
<212> DNA
<213> Artificial

<220>
<223> Oligonucleotide primer

<400> 28
gccactgcct gatggagact g 21


<210> 29
<211> 20
<212> DNA
<213> Artificial

<220>
<223> Oligonucleotide primer

<400> 29
aacatcagca tcctggagaa 20


<210> 30
<211> 20
<212> DNA
<213> Artificial

<220>
<223> Oligonucleotide primer

<400> 30
cttcggcctc tcttggtctt 20


<210> 31
<211> 20
<212> DNA
<213> Artificial

<220>
<223> Oligonucleotide primer

<400> 31
gggtcagagc tgcagagaag 20


<210> 32
<211> 20
<212> DNA

<213>  Artificial

<220>
<223>  Oligonucleotide primer

<400>  32
gggcctgtcg tctgcaatgg                                                    20


<210>  33
<211>  20
<212>  DNA
<213>  Artificial

<220>
<223>  Oligonucleotide primer

<400>  33
atggccacag caggaaatcc                                                    20


<210>  34
<211>  20
<212>  DNA
<213>  Artificial

<220>
<223>  Oligonucleotide primer

<400>  34
ggtcacttgt ctgcgcagac                                                    20


<210>  35
<211>  20
<212>  DNA
<213>  Artificial

<220>
<223>  Oligonucleotide primer

<400>  35
cccaaccctg tgtttttctc                                                    20


<210>  36
<211>  17
<212>  DNA
<213>  Artificial

<220>
<223>  Oligonucleotide primer

<400>  36
ggccctcctc cctcaga                                                       17


<210>  37
<211>  20
<212>  DNA
<213>  Artificial

<220>
<223>  Oligonucleotide primer

<400>  37
atccctccat tcccatcttt                                                    20

<210>  38
<211>  20
<212>  DNA
<213>  Artificial

<220>
<223>  Oligonucleotide primer

<400>  38
cacatacaat tctctggttc                                                    20

<210>  39
<211>  20
<212>  DNA
<213>  Artificial

<220>
<223>  Oligonucleotide primer

<400>  39
agtgacactg tctcagaatt                                                    20

<210>  40
<211>  20
<212>  DNA
<213>  Artificial

<220>
<223>  Oligonucleotide primer

<400>  40
ccccaatctc accagtgcac                                                    20

<210>  41
<211>  19
<212>  DNA
<213>  Artificial

<220>
<223>  Oligonucleotide primer

<400>  41
gcttccctac cgctgtgct                                                     19

<210>  42
<211>  19
<212>  DNA
<213>  Artificial

<220>
<223>  Oligonucleotide primer

<400>  42
cactctggca agggtcctg                                                     19

<210> 43
<211> 10080
<212> DNA
<213> Homo sapiens

<400> 43
caggaggttg cacactgttc ctcccacctc gccactgcac ccccaccaag gatggaattg 60

gaggcggggg gcagattcca gggtcagggc tgtcaagagt gaatgaggcg aggagacatt 120

caggagcaga gaggtttcag acgcggaggt tccgggcacg ccctcaacac ccccttcacc 180

ttctcctcag gccccgcccg ccctgccctc ccctcccgat cccggagcca tgtggcccct 240

ggccctagtg atcgcctccc tgaccttggc cttgtcagga ggtaagaatg cgcgggggtg 300

gaggcgcggc ggccattcgg gacaatggta ggaggggtca ggccggaggg ggagggggcg 360

tgggagccgc gagctccgcc ccccgcccac tcggggccgg gtccagtggg gacagctcag 420

agctcttcct gcttgtccct gggtgacctg gtttcccggc tgaggttggc cctccgaccc 480

cagacccttc acctcccaaa ataccctcgc agcagcccct cccgcgttca aggcttcctg 540

tcctctctgg aaagctgaaa gacatgggtt cgcgtcctga cgctgccgct ttgagccagt 600

agcctagcag ctgctttgtg cctaaattgt tttcatctgg aaaatgggct taatctataa 660

gtgcttacca gagaaggtca ctgtgaatat tgaaacgagg taatgcgtcg agccttcagt 720

atgtcgcagg tagaagggac ttgaaagtta gccacttagc cgttattact ttattagtag 780

tattcctttt tttttttttt tttttttttg agatggaacc ttgctctgtc tcccaggctg 840

gaaggcagtg gcacgatctt ggcttactga aacctccgcc tcccgggttg aagcgattct 900

cctgcctcag cctcccgagt agctgggatt acaggcgccc gccaccacgc ccaactaatt 960

tttgtatttt cagcagagac ggggtttcgc catgttggtt aggctggtct cgaacttcta 1020

acttcaagta gcccgcgtca gcctcccaaa gtgccaggat tacaggcatg agccaccgag 1080

cccggcctct agtattctgt cttcatactc agccccttcc agaaccttct agattgttat 1140

tttaatcctt gggttgaccc caaacctatg tgacctcacc ccaaattggt agtccttaag 1200

atccttatgg atctttccca tctttccctg ccgttgtagg caggttctct ggaaaccccg 1260

ttcatgaatc atttattcat tcaacaaaca gctattaaac accggccact gtgctgggtg 1320

ctgtacaagc agagacacag tccctgctct cagcacctgg agtctagcgg ggacagacgc 1380

agatgttatt caaacaatta tccaaataat tagttaataa ttatcttgac atgaggtgaa 1440

gacttcaagg agccaagcca ggggcctaga gatgtaatgg cggcttcccg accagaggcc 1500

ttcccaaagg gcttgaccct tgagccaaga cctgaaaaag gagggatctg tgggtgcctg 1560

gcacctggca ccatccttgg cctgaaggtg gggtggcttt tctcctctgg cgacactccc 1620

tggattcatg cccgtgccac tcctgagtgc cacaccctag gctaggagac ccacacgcta 1680

cgccttgtgg agtcctcaac aacctggcga ggtaggtgca ttgtaattac tccaatttca 1740

tggcagagaa acctaggact caaagacaga aggctcctgc tccaatgaca ccggcgatgc 1800
ctgagtcaga atcctaatca aggttgtttt ccctgtccat atcctggact tgaggctctg 1860
aaaaccattt ttataacttt tgacctaatc atttgcttaa agttagcttt ttttcttctt 1920
ttttcactca aacaaaagca tgttcaactt tatattactg tcctgaatag agaatagaat 1980
tctttgtcat aaatagaagg taaggaagga aataaatcct gcacaatgaa aagaaaataa 2040
tatgtttatt gggttggacc acctgaaatt gctgatactt gacccttttt gaccttccta 2100
aaacaacttt tgcagatggt tcagtgtaat aaatgttagg tggcctgatg aggcttctgt 2160
gtcctcctgg ctttgaaaag tgagctcagt gaggattagg gaggtgttaa aaccatatta 2220
gcaccatcct gagactttat ccttgacaaa atcaggttta aaagagaact ggatgctggt 2280
tcagcgtctg agtgtgcgat ttaacgttac ttaaatctca tctctctacc atctaaaatg 2340
atcctgtgct caccgacaac ttctgtccct aactgcaaac cactgagcta atccaactgc 2400
ttgccctgta gttggggaaa ctagctaggg aggcagaggg acctcctgtt gtagctaata 2460
attaataata acatttccca ctgactgagt gctctccatg ccacctgctg tgctgcacgg 2520
tttgaaatgc aggatcatct tgaattcttc aactgcgcaa tgagagatga actattactt 2580
tttctacttg acagctgggg aaactgaggc tggtgatttg cataaggtca cacagtcaca 2640
aaatggcatg catgttcagg attggattct ccctgtccca cggacccctg ctgtgctttc 2700
aatgccagac acagtgcctg gcacacacag catttattta ttgagccccc attgtgtgcc 2760
aggcgctgtg ttaggtcctg ggaatatggt actgaataaa gcagttaagg tgcctgttgt 2820
caatggagct tacagtcaaa gtggagagat ttttaaaaac gaatacatac aaatgtgaag 2880
agaaatgaat agcaatcatt gttctgatga agaccaactg gaagaatgta atgggggagg 2940
agtcgggacc aggagagtca acattagacc aggtggtcag ggaaggcctt tctgaagagg 3000
agacatttga gctgacctct cagaattaag aaggacccag acatacaacc tctaaattct 3060
gagggtcatc cagtagaata ttccatatat gtatatatga aatatcctat atctgtgctg 3120
tccaattatc cactagcccc ttcaggctat tgaacatttg aaatatggct ggtgtgactt 3180
aagaactgaa tttttaattt agttttactt cattttaatt agtttaaatt taaatagcca 3240
catgtagcta gtggctacca tattaaacaa cataggtctg gagaaaggac tgtgcagaga 3300
gaggaaatag caagtataaa atgtctagta tgggggcatc caagatgatt taaattcttc 3360
ttttctttaa atgcctggtg tgtttgaaga acaggcccat gaggctggac tagaggaagt 3420
cagaagaaag aggttggaga tggggtcaaa gaggctggca agggccagac agcacagagt 3480
cctgcacacc ttgggaaggc tttttggatt ttattttaaa gaaagttgag cctgggaaca 3540
acatctgact ttctttgttt gaagagtcct cagcctactt tgagaagact ggatcggagg 3600
gatgtaaaag tggaaggatt taggttaatg ttgtagtcat ttgggctaca gaagatgggg 3660

catggaccaa gatggtggca gaagtgtgga gataactgga tatttgggag ataaaaccaa 3720

taggaactgg ttgtgagtga tgaaggaaag aagagaagca aagatgactc ccaggtttgg 3780

ggctgagcac tgaggtggga aatactggag cgaacagttt tgattgagaa gaatcaagtt 3840

gggaatacaa agcttaagat gcctgtaagg catccaaatc aacagtgttt gagttttgag 3900

cttaaagaag agttcagggc tggagatgat tagcctatag ctggtattta aagccatgga 3960

ggcaaccagt atatatgcag tgaaaggata gagagatggg tggaaagatg attggatgga 4020

tgcatggatg gatatatgga tagatggatg gatggatggt tggattggat ggatggatgg 4080

atggatggat ggatggatgg atggatggat ggatgaataa atggaccagt ggatggaggg 4140

acagatgagt ggatggatgg ttggatggat ggatggatgg atggatggat agatggttag 4200

atgactacct aaatggatga atggatagat ggatgagtag acggatggac aaatcaatag 4260

gatgaatggg ggatggatga ttggatagat tgatggatag atattgccta ggtggatgtg 4320

taggtcagtc tcacttctac ctcctgaaat ccatcttctg gtagaatgat ataaaaaatg 4380

catgtggaga gaaagtcagg ctcctgctta cctatcagca acatcctcat tttgtgaact 4440

cttctgttaa cccccagtgg aggatttggt acttcctgag aaaataatgt cacccctttg 4500

ccctaattca tctccacttg gtcaagaata gcaactgcca taggtcggca aattcatctt 4560

cagttcctgg tcacccaggg caataatccg acccttaccc caaacccaga aaccacaacc 4620

ccagggctcc tctgccccct ggatcccagt tttctaacaa tctctcttct ttaccaggtg 4680

tctcccagga gtcttccaag gttctcaaca ccaatgggac cagtgggttt ctcccaggtg 4740

gctacacctg cttcccccac tctcagccct ggcaggctgc cctactagtg caagggcggc 4800

tactctgtgg gggagtcctg gtccacccca aatgggtcct cactgccgca cactgtctaa 4860

aggagtatgt gggggccggg ggagcatggg gtagggatga gaatgggact gggattgtgg 4920

atggggtaga gttggatttg aggatggagt tggagttagg gttggggatg gacatgggag 4980

tgagaatgag gtttggggtt gagatatggg gattgggtat gggaatagaa tcaaagtagg 5040

ggatttggat gggattgaag ttgaggatgg gggagatgta tttggagatg aggaaggtag 5100

gatggagaag aagttaggtt ggggatggga agaggttggg gctgggatgg ggatggaaat 5160

gggctcatct tctttcctaa ccaccttctt tctgcaccca cagggggctc aaagtttacc 5220

taggcaagca cgccctaggg cgtgtggaag ctggtgagca ggtgagggaa gttgtccact 5280

ctatccccca ccctgaatac cggagaagcc ccacccacct gaaccacgac catgacatca 5340

tgcttctgga gctgcagtcc ccggtccagc tcacaggcta catccaaacc ctgccccttt 5400

cccacaacaa ccgcctaacc cctggcacca cctgtcgggt gtctggctgg ggcaccacca 5460

ccagccccca gggtatgcac ccacacaggt ggcctgaggc cccataggag tggctgggga 5520

aacaggggca gagatgggag ggaaggtctg aggtaggttc ctttatatat aaaaatataa 5580

ataagtaaat aaatatatat atttaaagtt agctgtatcc tttatataaa tataaattca 5640
tgaatatata aaaatatgag tatataaatt catgaatata tagaaatata aatagatcta 5700
atatatgaat atattatatg atgtatatta tgtattatat agtaatataa ttatatatta 5760
tacaaaaagt atacaaatta aatgtatttt ataaattata aaatttatca attatgtatt 5820
ttaaatatgt atttctgcat aatgtatata ttatatataa tctatattta aattatatat 5880
tataaatgta ttttataaat gtatacattt atatatttat atactgtaaa tgaattttat 5940
catttataat atataaatca tacatataaa atgtttatat ttctataatt tataaaatgt 6000
ttaatatatt aaatatggtt attaatgaaa tgtctaataa ttcaatgtaa taattaattc 6060
tatatcatta cttagtaagt ataatacatt atatatgtga atataaagtt gatgtatata 6120
ccgacaagag ccctttgcat ctccctagca atccctgact ctctcccagc ctcatgtttg 6180
tatctttctc ctcaacatgc cctgtctctc ttcctaccat tctatccaac tctcccgtaa 6240
ctcttcccat ccctgttcct gcttttccca tctttaattc tctatttctg accatctccc 6300
tattccaact ccctctctcc aactttctct ccccaccgct ggctccacca ctctccttat 6360
caaccttcca ttctcttgtc ccttccctcc ttgtccttcc ctccactttt ctcctcatct 6420
ctcccttcgc ctctctccca tgtccctcca tatttctgtc acttccgttg ctttacccag 6480
ataggtgctc atctcttctc ccatctttct cttcccatct caattttcta tctactcttt 6540
acccattcaa ctcgcctatt tcaccttcat cccatatcct atccaggtcg gataccttag 6600
accttctctt tcttctcccc agtgaattac cccaaaactc tacaatgtgc caacatccaa 6660
cttcgctcag atgaggagtg tcgtcaagtc tacccaggaa agatcactga caacatgttg 6720
tgtgccggca caaaagaggg tggcaaagac tcctgtgagg tgaggccggg aggctggtgg 6780
gtgccttgga caggatagaa agccagaatg gaagtgacag atgctgggga aaaagctttg 6840
tttccagcct taggggaacc aatctttata agatacaatg tcccctcaca taggaggtca 6900
agacaaaaag gggtacccag ggatggcagg aataattcat cataagcccc agctttgact 6960
gagtggctgc caagatccct gtgttgagat gcataaaggt tggtattctt tcacttgtga 7020
gtgatagaca accaactcaa actggcttaa acaaaatgca ggcttttgta actgaaaatc 7080
caggttgtct ggctttaggc acagatggat ccaggtatgc aaattgtgtg tttggaattc 7140
tgtctttctt ttaactctca gctcttcttt attctgtttt ggcttcattc tcggttagat 7200
tcttcccatg acaagatggc cccagcagct ttgagcttac atcctaccct ctaggcaacc 7260
ctattagaaa gagaacctct cttttccaat agttcacaca aaagtcttaa gcatgattct 7320
cactaggctg acctaagtca tgtgtcttga gccatcactc caccagagct gtgggattct 7380
ctgatgggcc aagcctgagt cacatagtta actgtgggtg ctggagaggg gcagggacaa 7440
actgcatgga ttggaagtgg agaagggcag ttccccaaat gaaaaaatca ggagaggctg 7500

ttaccaaaat aaggggaaat ggccaagtac agtagttcat gcctgtaatc ccagcacttt 7560

gggaggctga ggtgagagga ttacttgagc ccaggagttt gagaccagcc tgggcaacat 7620

agtgagactc tgtctctaca aaaagaaaaa aaagttttta aattagccag gtgtggtgga 7680

gtacaactgc agtcctagtt actcgggagg ctgaggcaga aggactattt gaacccagga 7740

gttcaaggct gcagtgaggt atgatcatgc cactgcactc cagcctgggt gatagagcaa 7800

ggccctgtct ctaaaacaaa aagaaataaa tagagcaaga cactgtctct aataaataaa 7860

taaataaaaa tttaaaaatg aatgtttaat tttttaaaaa taagaggaaa tggatactac 7920

atgagcaaaa aatagccttc atcaataaag aagttgagat tggattcagt gagaaagagt 7980

atgatactat attaatgata tgtgccttga tcgattagtg atgtctgcct tgggcccagg 8040

aagagaaata gacttacacg tgtgttgcat accctgccca gatatgaatg ggttcactca 8100

atagtgagag acacaaatga gccttaaata ggagcagggt cagctggtgt ggggcagggg 8160

gtgatttagt accagggaaa caaaaatggg tatgaagtaa gttgttacca ttttaatgaa 8220

actgaggaac agagaaaaac acagaaattt ctctgtgtct ctctttctct gggcctatct 8280

ctgtctttct gtccctattt ctgtctcttg ctgtctgtcc ctctgtgttt gtcttcttgt 8340

ctgtttctca ctgtcttcat tgctttctct cacactgtgt gtgtctgact ctgcctctct 8400

gagtctcctt ctctgtgtgt gtctctctcc atctttcact ctctccccac acctccctgt 8460

ccctgccttg tttagcccca gcaaggaccc acctctctct ctctttcttt ccccaactca 8520

gggtgactct gggggccccc tggtctgtaa cagaacactg tatggcatcg tctcctgggg 8580

agacttccca tgtgggcaac ctgaccggcc tggtgtctac acccgtgtct caagatacgt 8640

cctgtggatc cgtgaaacaa tccgaaaata tgaaacccag cagcaaaaat ggttgaaggg 8700

cccacaataa aagttgagaa atgtaccggc ttccatcctg tcaccatgac ttcctcacat 8760

ggtctgctta gcccttctct gctccttatt cccagtgttc catttgaacc agtgatccat 8820

gtcctgaaaa atgctcaatc tcagctaaca ttccatgttt cagaagcatt caggcactgc 8880

caggcttgca gtctcccaga tgttgcatcc ctgaaacatc tcaacaacct gaatgtccca 8940

acccagacaa tggcccaggt ctctcaactt catcagtgtg gcttctatga gcccagatca 9000

ccacctgaac gttctgtctg tggcacattc ttaaatattt ccatcagccc atctcaacaa 9060

tatatgtcct ataaatggac catccttgac aacatcctct aactcttcaa gtatttattc 9120

aatgccagta tcctagacct tctatttttt gcactcaaga aggctctaga ctcccatgat 9180

agttcatcct gaaaatattc tcttatgccc acaatcttct gccctgacaa cattctgtgt 9240

acctctgtga ctcaccacag ctaacattgg atcctcagaa tatttcattc tcacactgtt 9300

atgggtgtct cagaagtccc aacccaacct acatcccaca ttcttccaat accccacctc 9360

tgccaacatt ccctctctga atcaatggca ccctagtctc tagagttata gggttcagta 9420

taccaaaggg tcttcttgcc tgaactttat tgtctaccaa atattccgtc ttgtatcccc 9480

tccatgaaca tccttggtca gtgtcccttg ctgttacatc tttgtgcatg accctaaaat 9540

gtagtgcaaa tccttgcttt ggacaagtta taaaactcac agtctctgtg ctttctcatc 9600

tgtaaaatgg gttcataatt ttttttaatt gtaacattat tacaagaata aatgtcaagc 9660

atttatcact attattattt gcatggttcc cataaaatat taccttagaa tgttaataac 9720

agcccttcga atttgcagag tgtccaaaaa aagtgttgca ctgatttatt ttcctcagga 9780

gacatttctt cagtgttgac tatgtgcaag cactctcctg ggtgttgtta aatatagttt 9840

atttactcaa caaatatttg tacctatcaa gagccaggca ctgttgcaga gacaagtgat 9900

aaccaatgag ttaaacagat aaaaacttct gcccttgtag aacttacatt cttttcaaga 9960

agtctccata acaatgaata aagaaatagg ctgtcaggtg gtgctgcaag ccatagcaag 10020

aaatgaaaca agggccatat gtggtagctc atgcctgtaa taccaacact gggaggccaa 10080

<210> 44
<211> 256
<212> PRT
<213> Homo sapiens

<400> 44

Glu Ser Ser Lys Val Leu Asn Thr Asn Gly Thr Ser Gly Phe Leu Pro
1               5                   10                  15

Gly Gly Tyr Thr Cys Phe Pro His Ser Gln Pro Trp Gln Ala Ala Leu
            20                  25                  30

Leu Val Gln Gly Arg Leu Leu Cys Gly Gly Val Leu Val His Pro Lys
        35                  40                  45

Trp Val Leu Thr Ala Ala His Cys Leu Lys Glu Gly Leu Lys Val Tyr
    50                  55                  60

Leu Gly Lys His Ala Leu Gly Arg Val Glu Ala Gly Glu Gln Val Arg
65                  70                  75                  80

Glu Val Val His Ser Ile Pro His Pro Glu Tyr Arg Arg Ser Pro Thr
                85                  90                  95

His Leu Asn His Asp His Asp Ile Met Leu Leu Glu Leu Gln Ser Pro
            100                 105                 110

Val Gln Leu Thr Gly Tyr Ile Gln Thr Leu Pro Leu Ser His Asn Asn
        115                 120                 125

Arg Leu Thr Pro Gly Thr Thr Cys Arg Val Ser Gly Trp Gly Thr Thr

130                 135                 140

Thr Ser Pro Gln Val Asn Tyr Pro Lys Thr Leu Gln Cys Ala Asn Ile
145                 150                 155                 160

Gln Leu Arg Ser Asp Glu Glu Cys Arg Gln Val Tyr Pro Gly Lys Ile
                165                 170                 175

Thr Asp Asn Met Leu Cys Ala Gly Thr Lys Glu Gly Gly Lys Asp Ser
            180                 185                 190

Cys Glu Gly Asp Ser Gly Gly Pro Leu Val Cys Asn Arg Thr Leu Tyr
        195                 200                 205

Gly Ile Val Ser Trp Gly Asp Phe Pro Cys Gly Gln Pro Asp Arg Pro
    210                 215                 220

Gly Val Tyr Thr Arg Val Ser Arg Tyr Val Leu Trp Ile Arg Glu Thr
225                 230                 235                 240

Ile Arg Lys Tyr Glu Thr Gln Gln Gln Lys Trp Leu Lys Gly Pro Gln
                245                 250                 255

<210>  45
<211>  277
<212>  PRT
<213>  Homo sapiens

<400>  45

Met Trp Pro Leu Ala Leu Val Ile Ala Ser Leu Thr Leu Ala Leu Ser
1               5                   10                  15

Gly Gly Val Ser Gln Glu Ser Ser Lys Val Leu Asn Thr Asn Gly Thr
            20                  25                  30

Ser Gly Phe Leu Pro Gly Gly Tyr Thr Cys Phe Pro His Ser Gln Pro
        35                  40                  45

Trp Gln Ala Ala Leu Leu Val Gln Gly Arg Leu Leu Cys Gly Gly Val
    50                  55                  60

Leu Val His Pro Lys Trp Val Leu Thr Ala Ala His Cys Leu Lys Glu
65                  70                  75                  80

Gly Leu Lys Val Tyr Leu Gly Lys His Ala Leu Gly Arg Val Glu Ala
                85                  90                  95

Gly Glu Gln Val Arg Glu Val Val His Ser Ile Pro His Pro Glu Tyr
            100                 105                 110

Arg Arg Ser Pro Thr His Leu Asn His Asp His Asp Ile Met Leu Leu
        115                 120                 125

Glu Leu Gln Ser Pro Val Gln Leu Thr Gly Tyr Ile Gln Thr Leu Pro
    130                 135                 140

Leu Ser His Asn Asn Arg Leu Thr Pro Gly Thr Thr Cys Arg Val Ser
145                 150                 155                 160

Gly Trp Gly Thr Thr Thr Ser Pro Gln Val Asn Tyr Pro Lys Thr Leu
                165                 170                 175

Gln Cys Ala Asn Ile Gln Leu Arg Ser Asp Glu Glu Cys Arg Gln Val
            180                 185                 190

Tyr Pro Gly Lys Ile Thr Asp Asn Met Leu Cys Ala Gly Thr Lys Glu
        195                 200                 205

Gly Gly Lys Asp Ser Cys Glu Gly Asp Ser Gly Gly Pro Leu Val Cys
    210                 215                 220

Asn Arg Thr Leu Tyr Gly Ile Val Ser Trp Gly Asp Phe Pro Cys Gly
225                 230                 235                 240

Gln Pro Asp Arg Pro Gly Val Tyr Thr Arg Val Ser Arg Tyr Val Leu
                245                 250                 255

Trp Ile Arg Glu Thr Ile Arg Lys Tyr Glu Thr Gln Gln Gln Lys Trp
            260                 265                 270

Leu Lys Gly Pro Gln
        275

<210>  46
<211>  19
<212>  DNA
<213>  Artificial

<220>
<223>  Oligonucleotide primer

<400>  46
aactctacaa tgtgccaca                                                    19

<210>  47
<211>  20
<212>  DNA
<213>  Artificial

<220>

<223>  Oligonucleotide primer

<400>  47
ttattgtggg cccttcaacc                                                    20


<210>  48
<211>  20
<212>  DNA
<213>  Artificial

<220>
<223>  Oligonucleotide primer

<400>  48
ggatggtcca tttataggac                                                    20


<210>  49
<211>  20
<212>  DNA
<213>  Artificial

<220>
<223>  Oligonucleotide primer

<400>  49
aggctgccct actagtgcaa                                                    20


<210>  50
<211>  20
<212>  DNA
<213>  Artificial

<220>
<223>  Oligonucleotide primer

<400>  50
atattgccta ggtggatgtg                                                    20


<210>  51
<211>  20
<212>  DNA
<213>  Artificial

<220>
<223>  Oligonucleotide primer

<400>  51
aagacttcaa ggagccaagc                                                    20


<210>  52
<211>  20
<212>  DNA
<213>  Artificial

<220>
<223>  Oligonucleotide primer

<400>  52
gacccttcac ctcccaaaat                                                    20

<210>  53
<211>  20
<212>  DNA
<213>  Artificial

<220>
<223>  Oligonucleotide primer

<400>  53
ctagtgatcg cctccctgac                                                    20


<210>  54
<211>  21
<212>  DNA
<213>  Artificial

<220>
<223>  Oligonucleotide primer

<400>  54
ggtgatctgc gccctggtcc t                                                  21


<210>  55
<211>  21
<212>  DNA
<213>  Artificial

<220>
<223>  Oligonucleotide primer

<400>  55
aggtgtccgg tggaggtggc a                                                  21


<210>  56
<211>  11820
<212>  DNA
<213>  Homo sapiens

<400>  56
attgaggggg gatcccagca ggtcccattt gttcagattc ttctgggcct ttctgtgttg      60

catttcttcc tctgcaggac acctgtcaca tgagggtctt caggggaaaa ggaattctag     120

tgtctgtgac tgcttcaaag gagaagtaag gggagaggag aggaaagcag gaggaggttg     180

gagagaacct cttgttcctg aggtcttcca atctccttca gctcaaagca ctcagcatgc     240

tgaagttcca gactctgggc tatcacgttc tgacatgcaa cacaggcaac accccagctc     300

catccacgtt tctccaaaag cacaggcatg acgtcatatg gtgacaaaca cccttgtcca     360

aaggaagccc ataggatacg ctaattctag attcacaaat actctagagg aactcacaca     420

atgggatggg ccagtgcccc acacagagta tgaggcctcc caccttggtt gaatatcttt     480

ttcttttttt tctttttttt tttttttgag acggagtctc actctgtcac ccaggctgga     540

gtgcagtggc tcgatctcgg ctcactgcaa cctctgcctc ccaggttcaa gtgattctcc     600

tgcctcagcc tctggagtag ctggaattac aggtgcccac caccacaact ggctaatttt 660
tgtattttta gtagagacga gggtccacca tgttgaccag gcttgtctcg aactcctggt 720
ctcaagtgat ctgcccacct cggcctagtg ctgggattac agacgtgagt caccacgccc 780
ggccccacct tggttaaatt tctgaaaatc atttggtaaa gtgaggaccc ctccagctga 840
gacactgcca ggaaacagct attgagtctc ttagcaccca cagcattaaa acaaacccaa 900
aacattttag gcctcgttga gttctggagg caaaatattc ctcatctaca aatttatttt 960
attttatttt ttaataaaag ttattattat ttttttttat agagagacag ggtcttgctc 1020
tgtgacccag actagagtgc agtggtgtga ccatggttca ctgcagcctc cacctcctgg 1080
gctcaagcga tcctcccacc tcagcctgcc aagtagctgg gaccacaggt atgcccctcc 1140
cccaggctaa ctttttttta ctttttgtgg agatgaggtc tcactatgtt gcccatgctg 1200
gtcttttttt tttttttttt tttttttgag acgggagtgc aggctggagt gcccaggctg 1260
gagtgcagtg gcacaatcat aactcactgc agcctcaaac tcctggggtc aagtgatcct 1320
cccacctcag cctcccacat aactaggact acaggcctgt gtcaccataa tgcctggtta 1380
atttttttag tttttgtttg tagaaacagg gtctcactat gttacccagg ctggcctcta 1440
actcctggcc tcaatccatc ctcccatctc aacctcccaa agtgctagga ttatagccac 1500
gagccaccat gcccggcccc atacttatat ttacttgtag tgagaacact taaaccctac 1560
tcggtcagta attttcaagt acacaataca ttgttactaa ctatatacat ggtattttta 1620
atttgcatga atgctcctta aaatcagcga gcccgcctta ttttttgtat tcaattttat 1680
tggatataaa ttccacatca gtaaacctga ttctcttaaa aatctacaca gaaaaaaaaa 1740
aagagagaga ggttgagttg ggtagttgtt ggtatttttg tttttgtttc tttgcttatt 1800
cagttgaggt ttattgtaaa acttgacacc agaaaaagga agaagtggcg tttttgtgct 1860
gtgagcgtgt gacgtgtgtc ctttccagag aaaggacagt catggtgctt ttttatcctc 1920
tctgcccaaa gaaggaaagc tcttaacagc cagcaggagg ctttgtaggg accagcgtta 1980
tcaacgccag tcgcgctgac caatgatgag ttaaagcagt taggtcgttt ctaagagcaa 2040
atcaaaagct aaggttctgt gattctgaaa atgagacacg gacagagact ggagacccag 2100
agagaaagtg aaggactaaa agacagtcat agggtgggag tttgctctcc tctgttttgt 2160
tctgggtttt tttgtttgtt tgtgcgctct gtctgaccgc ttttcttttt tttctttttc 2220
tttctttttt tttttttttt ttttgagatg gagtcttgct ctgccgccca ggctggagtg 2280
cggtggcaca atctcggctc actgcaagct ccgcctccca cgttcaaggg attctcctgc 2340
ctcagcctcc cgagcagctg ggattacagg catgcaccac cacactcagc taatttttgt 2400
atttttagta gagatggggt ttcaccatat tgaccaggct ggtcttgaac tcctggcgtc 2460
aagtgacttg cccgcctcgg cctcccaaag tgttgggatt acaggcgtga gccaccgcgc 2520

ctggccctga ctgcttttct ccttggtttg tttgtcaatc ccccttcctc tgagccgaat 2580

tccctttttg ttctcatttt ctctctctgt cccctctctc tctccttctt tctctccttc 2640

cattcctcct agatgaagca aaaactcaga taaaccagca cagaggccag gtatggtggc 2700

tcacacctgt aatcccggca ctttgggaag ccaaggcagg caggttgctt gaggccagga 2760

gttcaagacc agcctggccc acatggtgaa accccgtctc tactaaaaat acaaaaatta 2820

gccggacatg gtggcacgtg cctgtaatcc caggtactca agaggtggag gttgcagtga 2880

gcggagatca cagccctgca ctccagcctg ggtgacagag cgagactcca tctcaaaacg 2940

aaaaacaaaa aacagcacaa agttcccttg tcctgtgact cattctctct ctctctttct 3000

accatttctc cttccctgtg tctttttttt ttctctctgt gggttttatt taagcaatag 3060

aagttcttag caaagaaaaa ctttatggaa ttagattgat ccacttcata tgtacatata 3120

tgaactcagt tcagaaactc tcttctaccc ctgcctgatc acctatttgg aagtctgttc 3180

cttcaactct tcttctcttt ctgggactct ttctagcttg ggcttcctgc ccctcccgtc 3240

cactctcctg ctttcacagc ctctccttcc ccctgcccct cccctgcact gcatggggat 3300

gggccccagg tgtccaaggt ctccccaccc tcctttgtca ctggagtcag gattagaacc 3360

cagctcccta gtcaccttga gtcatcagtc ctggggctgc tgacgggctt gcagaggaga 3420

gagggagtgg ggctgggtct tcccaccctg ggtcctttcc tccttcccca ctccgtttag 3480

ctgtaaagct caattaagtg tgattagctg agaagagttt ctgcagaatt agagcacgcc 3540

ccacccctgt cttcgtggtc cccttccctt aacccggaaa ctggatgggc caggacaaag 3600

agagttaaga gctttgtcag tggtctgtct ggagcgacag atggaaggaa agggaccggt 3660

tgagcaacat gacaggtggc tgaggagcca ggtgcagagt ggtagagttg gctggcggag 3720

tggccagcac atgagaagac aggcaggtag gtggacggag agatagcagc gacgaggaca 3780

ggccaaacag tgacagccac gtagaggatc tggcagacaa agagacaagg tgagaaggag 3840

gtaggcgact gccaatgagg gagtgacaca caggggagca ggtagagaga ggacaagcag 3900

gtcatcccct tggtgacctt caaagagaag cagagagggc agaggtgggg ggcacaggga 3960

aagggtgacc tctgagattc ccctttcccc ccagactttg gaagtgaccc accatggggc 4020

tcagcatctt tttgctcctg tgtgttcttg gtgagttctc ccggagcagg gagagggcag 4080

gactgcgact ggatcccttc acccccatga ggaggcccca ccaccctccc catctcagct 4140

ctggccccca gcctggtggt gaggaggaga ggggctttct ctgtgcctcc atttacctgc 4200

agctctcagg gtactgctca cctcggtctc ccctattttt tgatccctct tcccttctgt 4260

ccctctctga atctctgtct ctccatttcc ctcctatgtg taagcatctt tctccctggg 4320

tgtctttgat gtttcatggt ctttttctat cactgggtct ctctctcttt ctctctcttt 4380

ctcgtctctc tttctcctct ctctctcctg cctgtttctc tctctcactc tgtgtgtctc 4440

tccatctctg tatcttttct tcctctctct gacccatgcc cctgtctgtc tccagggctc 4500

agccaggcag ccacaccgaa gattttcaat ggcactgagt gtgggcgtaa ctcacagccg 4560

tggcaggtgg ggctgtttga gggcaccagc ctgcgctgcg ggggtgtcct tattgaccac 4620

aggtgggtcc tcacagcggc tcactgcagc ggcaggtaag tcccttcctg gggtgggcga 4680

agggaggact atgggaaggc aagcgctggg ggtaggatca caagggaggg tggtgcccac 4740

tgggaagaag ctgatcctgc aacaagagag tctgaggtta gaccaggagt ggaacttcct 4800

tagcagtggg cctggggtgg tgctgggcag ggtgaggtat gttgggtgga gggccgggga 4860

gggtcctgga acctgccctc ctgcctctcc cattcctgca tgtacccttt ctttcctata 4920

tgacatctgc cactcacccc agccattcct tgacccagtc tgggcccggg gcccaggtct 4980

cacccaagct ctttttcttt ttcttttttt tatttttttg agacagggtc tcgctctgtc 5040

gcccaggctg ctgtgcaatg gcgtgatcac agctcactgc tgtctctgcc tcccaggttc 5100

aagtgattct cctgccccag cctcctgagt agctgggatt acaggcaccc gccaccatgc 5160

ccagctaatt tttgtatttt ttgtagagac agggttttgc catgttggcc aggctggtct 5220

cgaactcctg gcctcaaatg acctgcccgt cttggcctcc caaagtgctg ggattacagg 5280

tgtgagccac tgcacccggc caacatgacc caaactcttt gtgcaacttc agaatctatg 5340

cctggcacct ctctgggcct cagtagactg atgttctgga atttttttct ttttctttct 5400

tttttttttt ttttggagac agagtcttgc tctttctgtc atccaagctg gagtgcagtg 5460

atgctatctt ggctcactac agcctcaacc acctgggctc aagtgatcct cacacctcag 5520

cctcccaagg agctaagact acaggcctgc gccaccacac ctggctaatt tttaaatttt 5580

ttttgtagag acagggtttt gctatgttac ccaggctggt ctcaaactcc tcagctcaag 5640

caatcttcct gccttgacct cccaaagtgc tgggattaca ggcatgagcc actgtgcctg 5700

gcctggaact ttttttgtga aaggggagat cagatgcaaa gaaacagaga ctcagggaga 5760

gagagggcca gcagcaggat gcagagaggc cattcatcaa cccactcgtt caatcatgaa 5820

cccactcgtc cacgcatgag catggagggc acatgctccg tgccaggcgg tgggaataag 5880

gcagtgaaca aggtccactg atgtccctgc cttcatgggc ttcaccagcc gagagaatca 5940

gaaagagagg cctggcgcgg tggctcacac ctgtaatccc agcactttgg gaggccgagg 6000

cgggcggatc acttgaggtc aggagtttga gaccagcctg acacacatgg tgaaacctta 6060

tctctactaa aaatacaaaa attagctggg catggtggca tgcttctgta atcccagcta 6120

cttgggaggc tgaggcaggt gaattgcttg aacctgggag gtggaggttg tagtgagcca 6180

agatggtgcc actgcactcc agcctgggcg acagagcgag actcggtctt gaaaaaaaaa 6240

aaaaaaaaaa aaaggagaga gagagacaca gatgcaggga catggtagga gaaacaggga 6300

acacccaaga tggaaagagg gtgatggagg ttgggaataa gagcctgtaa gagagactcg 6360

gagaatgaga gttgcgggtg agaggacaga cagtgagggg cagaacagtg gggagcggca 6420
ggagcgcctg agtgtccgtg gaggggtgca aggtggggga ctgcgtgcct gccacccgct 6480
cagccgtcgc caccggcagc aggtactggg tgcgcctggg ggaacacagc ctcagccagc 6540
tcgactggac cgagcagatc cggcacagcg gcttctctgt gacccatccc ggctacctgg 6600
gagcctcgac gagccacgag cacgacctcc ggctgctgcg gctgcgcctg cccgtccgcg 6660
taaccagcag cgttcaaccc ctgcccctgc ccaatgactg tgcaaccgct ggcaccgagt 6720
gccacgtctc aggctggggc atcaccaacc acccacggag taagggcccc agggccaggg 6780
gtcaggggtc aggatgggta caagtctggg atgcagggcg agaggtcgaa tcatgacacc 6840
tcagaggaag gatgggtaaa gggtcagggt gtgggatggg acatcaggat catggtttgg 6900
ggtcagagat tatggtggat tggggtcttg ggagccaaag gggttaaagg actgggtatg 6960
aagtcaggga tcagaggtca gaggtcagag tgtgtcagag gtcatcacac tggagcaaaa 7020
ggcatatata tatatatatg tatgtatagg atatgggcat tgtgggtcat gggtctgggg 7080
ttagaggtca ccgtagaatt aaggtcatgg gatccagagg ttgtacaatc tggtcaaaat 7140
ctgaggatgg aaattgggat tctatccaaa atcacatatc tgagattgga ggtcatagcg 7200
tttggggtgt ggggcccgaa gtttggggtc atggaggctg gggcccaata aactaggatc 7260
aggggacact ggcgttggaa gcagtgaggt ttggaagatg cagagctgag gttggaggtt 7320
aaggtaaaga cagggacatg gggtcaggag acagaagata tgagatcaag ctgggatcat 7380
aaggtaataa gacagaaggt caaagatcac agtagctggc attgaagagg gtcaggtctg 7440
gattcgttgt ctctgacgct ggagagacaa gaaagttctt gagttatgcc actcaaagtc 7500
aaatgtcaaa gatcaaagag accgtcaatc atctggggtc atgattcata tgaaattaag 7560
tcataaatat gtaacttgga ggtttcggga ttgtagtaca ggtcggtgag gggcaggggt 7620
attgacatgg atgggccaca tccagggaag agggacgtgg cctcaaagtg gggagattta 7680
ggggaccctg cagcacgcat gttctctctc cagacccatt cccggatctg ctccagtgcc 7740
tcaacctctc catcgtctcc catgccacct gccatggtgt gtatcccggg agaatcacga 7800
gcaacatggt gtgtgcaggc ggcgtcccgg ggcaggatgc ctgccaggtg agccagtgca 7860
ggcagcgtgc gtggtcacca ggacaggaag tgaaggggag gggctggaag caggagggga 7920
actgatggag gatgaatcag ggaaagggga tgctgcagag agacggggtc aaaaaggaag 7980
ggagaggctg gttacggagg ctcacacctg taatcccagc actttgggag gccgaggcgg 8040
gcggatcact tgaggtcagg agttcaagac aagcctggcc aacacggtga gactctgaat 8100
ctactaaaaa taccagaatt agccgggggt ggtggtgcaa gcctgtggcc ccagctactt 8160
ggaaggctga ggcaggagaa tcgcttgatc ccgggaggcg gaggttgcag tgagctgaga 8220
tcacgccact gcactccagc ctgggcgaca gagccagact ctgtctcaaa acaaaataat 8280

taataataat aataataata ataataataa taatggagga gaggcccagg ataagggagg 8340
gagagagaca gggagtaaaa gggaggaccg gggaatggag gagggggagg ggcagggaga 8400
gagagggagg aagggaacag agaaggaaag atggggcagg ggttacagag agagacagca 8460
aaacagacgg agaggactgg gagcccagac agggaaccag ctgtttctgg ggctctaagt 8520
ctttcccata ccatcctcca gttggtgctg tcccagactg agagagattt gaggatggcg 8580
gtctctcccc tcattggtca gggccccagc cattgtcctt gagagaactc tgtgcttttg 8640
atggagtcct gcccaccttc cctgggattg gtcatttttg atggcactct ctcccctcat 8700
tggtcagaac cccaggcatt gtccttgaga gaacctctat cctttatgga gtcccaccct 8760
cctcccctgg gattggtcat tgataatagt gttctctctc ctcattggtc agggccccag 8820
ccattgtcct tgagagaatg ctcgactctt tatgttgtct tgacagcctc ccctgagatt 8880
ggtcattaat gactgtgctc tctctcctca ttggtcaggg ccccagccat tgtccttgag 8940
agaacctctg tcctttatgg agttccaccc ttcttccctg ggattggccc ctagagacag 9000
tggttcttct cttttggtta gccattgcca ttgtcctccg ggaaagtgat tatactcttt 9060
tgtctaatga ccagacttgg agccctcccc aaggcccagg actgggttga agggttgggg 9120
aggaaaacag aaataagatg tctcccttgt tcagacagta cttctcttcc cttccagggt 9180
gattctgggg gccccctggt gtgtggggga gtccttcaag gtctggtgtc ctgggggtct 9240
gtggggccct gtggacaaga tggcatccct ggagtctaca cctatatttg caagtatgtg 9300
gactggatcc ggatgatcat gaggaacaac tgacctgttt cctccacctc cacccccacc 9360
ccttaacttg ggtacccctc tggccctcag agcaccaata tctcctccat cacttcccct 9420
agctccactc ttgttggcct gggaacttct tggaacttta actcctgcca gcccttctaa 9480
gacccacgag cggggtgaga gaagtgtgca atagtctgga ataaatataa atgaaggagg 9540
ggccatgtct gtccatttga agtcctcatg ctggttgaga ctggaagaag gactcagcag 9600
tttccctatc tcataggagt agaaacagag ctcaaataag gccaggcaca gtggctcaca 9660
cctgtaatcc catcactttg ggaagctgag gcaggtggat cacctgaggt caggaactcg 9720
ggaccagcct ggtcaacata gtgaaacccc aactctacta aaaatgcaaa aattagccag 9780
gcatggtggc gcatgcctgt aatcccagct actcaggagg ctgagacagg agaatagcat 9840
gaacccgtga ggcagaggct gcagcgagcc gagattgaac cattacactc cagcctgggc 9900
gacagagcga gactccatct caaaaacaaa caaacaaaaa acccagtgct caaataggat 9960
gagggtcttc cctgagtagt tactcagaaa tggagtagaa aaagttactt ttaataatat 10020
aggccgggtg cagtggccca cgcctgtaat cccagcactt tgggaggccg aggtgggagg 10080
atggcttgag ctcagatttc gagatcagcc tggcaacaca gtgaaatctt gtcactacaa 10140
aaacacaaaa aattagctgg gtgtggtggt gcgtgcctgt agtcccagct acttgggaag 10200

ctgaggtggg aggatcaccc gagccgggga ggtggaggct gcaaagagcc gagatcatgc 10260

cactgcactc cagcctgggc aataaagtga gaccttgtct caaaaacaaa aacccagcaa 10320

tataaataag acacatgttt cttcatctgg cataatagaa atagtgccca gagcttataa 10380

gcttttcaag agtccacaaa agacccgaaa aagaaaaaga aaattgttag ctccaaaata 10440

ccagatgaaa gctgcaaagt caacatttat gaccatttaa tccaatgtcc ataaaacgta 10500

gcattctttc cactagccaa ctgcagttta ctttcttgta atgaagcata cattgtatct 10560

ttaatgtggg acgtggcttt gttctaataa gacgaagggt ggagtgcagg cttggaaagc 10620

aggagagctc agcctacgtc tttaatcctc ctgcccaccc cttggattct gtctccactg 10680

ggactcaaga ggtgaggaga gaccatctcc ccaaatgcac tgaagggaaa ctggaggagg 10740

gagggagtga ggggtgatca taccagcgga ggcacatttg ctgagccccc ccgcagtctg 10800

ctctttccaa gtggaccctc ctggaagcct gatcccaacc tcccctgcaa gcaggtctgt 10860

cacccccatc tctcagatga agaaactgag ccttgcaggg gtggagtccc ttgtccccac 10920

gtcataaggg tagtcatagt agtaggaaga ggaagcacct aggtttgagg ccagggctgg 10980

ctgctgtcag aacctaggcc ctcccctgcc ttgctccaca cctggtcagg ggagagaggg 11040

gaggaaagcc aagggaaggg acctaactga aaacaaacaa gctgggagaa gcaggaatct 11100

gcgctcgggt tccgcagatg cagaggttga ggtggctgcg ggactggaag tcatcgggca 11160

gaggtctcac agcagccagt aagtgaacag ctggactcgg gctgcctggg cggcagggag 11220

aagcgggcag gggaagggtc agcagaggag cgaggcccca gaggagccct ggggtggagc 11280

acagccaagg gctctgttcc ctttcctgga ctcggcttcc acaggccctg acctgcctcc 11340

cccaccctcc ggtcctgccc ctgtgcctgg cagcagcccc acctgtgtga catcccagca 11400

caccccccct ctccttgcaa aggagaaggg agcggcctag gggaggccag gggcccacct 11460

gggctggggc tgtggagagg gagtggctgg gacgggagga aaaagagaga cggagattag 11520

atggaagaag agggatttca agacaaattg ccagagatgc agtcagagag actgactgag 11580

agacacaaag atagaaggaa ttagagaaag ggccacacag agccagacag agagagaaga 11640

gtggagatgg agacagggac gaggacagag aaaggcagac agacacatag ggacagaaag 11700

agaaaaatca cacaaagtca gaattactga atgacaggga atgacacata gaacgagaca 11760

cagattcaga gactcagggc agggaaagga aggctgcaga cagacagaca gacagaggga 11820

<210> 57
<211> 184
<212> PRT
<213> Homo sapiens

<400> 57

Leu Ser Gln Ala Ala Thr Pro Lys Ile Phe Asn Gly Thr Glu Cys Gly
1               5                   10                  15

Arg Asn Ser Gln Pro Trp Gln Val Gly Leu Phe Glu Gly Thr Ser Leu
            20                  25                  30

Arg Cys Gly Gly Val Leu Ile Asp His Arg Trp Val Leu Thr Ala Ala
        35                  40                  45

His Cys Ser Gly Ser Arg Tyr Trp Val Arg Leu Gly Glu His Ser Leu
    50                  55                  60

Ser Gln Leu Asp Trp Thr Glu Gln Ile Arg His Ser Gly Phe Ser Val
65                  70                  75                  80

Thr His Pro Gly Tyr Leu Gly Ala Ser Thr Ser His Glu His Asp Leu
                85                  90                  95

Arg Leu Leu Arg Leu Arg Leu Pro Val Arg Val Thr Ser Ser Val Gln
            100                 105                 110

Pro Leu Pro Leu Pro Asn Asp Cys Ala Thr Ala Gly Thr Glu Cys His
        115                 120                 125

Val Ser Gly Trp Gly Ile Thr Asn His Pro Arg Asn Pro Phe Pro Asp
    130                 135                 140

Leu Leu Gln Cys Leu Asn Leu Ser Ile Val Ser His Ala Thr Cys His
145                 150                 155                 160

Gly Val Tyr Pro Gly Arg Ile Thr Ser Asn Met Val Cys Ala Gly Gly
                165                 170                 175

Val Pro Gly Gln Asp Ala Cys Gln
            180

<210>  58
<211>  111
<212>  PRT
<213>  Homo sapiens

<400>  58

Met Gly Leu Ser Ile Phe Leu Leu Leu Cys Val Leu Gly Leu Ser Gln
1               5                   10                  15

Ala Ala Thr Pro Lys Ile Phe Asn Gly Thr Glu Cys Gly Arg Asn Ser
            20                  25                  30

Gln Pro Trp Gln Val Gly Leu Phe Glu Gly Thr Ser Leu Arg Cys Gly
        35                  40                  45

Gly Val Leu Ile Asp His Arg Trp Val Leu Thr Ala Ala His Cys Ser
    50                  55                  60

Gly Arg Pro Ile Pro Gly Ser Ala Pro Val Pro Gln Pro Leu His Arg
65                  70                  75                  80

Leu Pro Cys His Leu Pro Trp Cys Val Ser Arg Glu Asn His Glu Gln
                85                  90                  95

His Gly Val Cys Arg Arg Arg Pro Gly Ala Gly Cys Leu Pro Gly
            100                 105                 110

<210>  59
<211>  254
<212>  PRT
<213>  Homo sapiens

<400>  59

Met Gly Leu Ser Ile Phe Leu Leu Leu Cys Val Leu Gly Leu Ser Gln
1               5                   10                  15

Ala Ala Thr Pro Lys Ile Phe Asn Gly Thr Glu Cys Gly Arg Asn Ser
            20                  25                  30

Gln Pro Trp Gln Val Gly Leu Phe Glu Gly Thr Ser Leu Arg Cys Gly
        35                  40                  45

Gly Val Leu Ile Asp His Arg Trp Val Leu Thr Ala Ala His Cys Ser
    50                  55                  60

Gly Ser Arg Tyr Trp Val Arg Leu Gly Glu His Ser Leu Ser Gln Leu
65                  70                  75                  80

Asp Trp Thr Glu Gln Ile Arg His Ser Gly Phe Ser Val Thr His Pro
                85                  90                  95

Gly Tyr Leu Gly Ala Ser Thr Ser His Glu His Asp Leu Arg Leu Leu
            100                 105                 110

Arg Leu Arg Leu Pro Val Arg Val Thr Ser Ser Val Gln Pro Leu Pro
        115                 120                 125

Leu Pro Asn Asp Cys Ala Thr Ala Gly Thr Glu Cys His Val Ser Gly
    130                 135                 140

Trp Gly Ile Thr Asn His Pro Arg Asn Pro Phe Pro Asp Leu Leu Gln
145                 150                 155                 160

Cys Leu Asn Leu Ser Ile Val Ser His Ala Thr Cys His Gly Val Tyr
                165                 170                 175

Pro Gly Arg Ile Thr Ser Asn Met Val Cys Ala Gly Gly Val Pro Gly
            180                 185                 190

Gln Asp Ala Cys Gln Gly Asp Ser Gly Gly Pro Leu Val Cys Gly Gly
        195                 200                 205

Val Leu Gln Gly Leu Val Ser Trp Gly Ser Val Gly Pro Cys Gly Gln
    210                 215                 220

Asp Gly Ile Pro Gly Val Tyr Thr Tyr Ile Cys Asn Ser Thr Leu Val
225                 230                 235                 240

Gly Leu Gly Thr Ser Trp Asn Phe Asn Ser Cys Gln Pro Phe
                245                 250

<210>  60
<211>  248
<212>  PRT
<213>  Homo sapiens

<400>  60

Met Gly Leu Ser Ile Phe Leu Leu Leu Cys Val Leu Gly Leu Ser Gln
1               5                   10                  15

Ala Ala Thr Pro Lys Ile Phe Asn Gly Thr Glu Cys Gly Arg Asn Ser
            20                  25                  30

Gln Pro Trp Gln Val Gly Leu Phe Glu Gly Thr Ser Leu Arg Cys Gly
        35                  40                  45

Gly Val Leu Ile Asp His Arg Trp Val Leu Thr Ala Ala His Cys Ser
    50                  55                  60

Gly Ser Arg Tyr Trp Val Arg Leu Gly Glu His Ser Leu Ser Gln Leu
65                  70                  75                  80

Asp Trp Thr Glu Gln Ile Arg His Ser Gly Phe Ser Val Thr His Pro
                85                  90                  95

Gly Tyr Leu Gly Ala Ser Thr Ser His Glu His Asp Leu Arg Leu Leu
            100                 105                 110

Arg Leu Arg Leu Pro Val Arg Val Thr Ser Ser Val Gln Pro Leu Pro
        115                 120                 125

Leu Pro Asn Asp Cys Ala Thr Ala Gly Thr Glu Cys His Val Ser Gly

130                 135                 140

Trp Gly Ile Thr Asn His Pro Arg Asn Pro Phe Pro Asp Leu Leu Gln
145                 150                 155                 160

Cys Leu Asn Leu Ser Ile Val Ser His Ala Thr Cys His Gly Val Tyr
                165                 170                 175

Pro Gly Arg Ile Thr Ser Asn Met Val Cys Ala Gly Gly Val Pro Gly
            180                 185                 190

Gln Asp Ala Cys Gln Gly Asp Ser Gly Gly Pro Leu Val Cys Gly Gly
        195                 200                 205

Val Leu Gln Gly Leu Val Ser Trp Gly Ser Val Gly Pro Cys Gly Gln
    210                 215                 220

Asp Gly Ile Pro Gly Val Tyr Thr Tyr Ile Cys Lys Tyr Val Asp Trp
225                 230                 235                 240

Ile Arg Met Ile Met Arg Asn Asn
                245

<210>  61
<211>  20
<212>  DNA
<213>  Artificial

<220>
<223>  Oligonucleotide primer

<400>  61
tcagccaggc agccacaccg                                                   20

<210>  62
<211>  20
<212>  DNA
<213>  Artificial

<220>
<223>  Oligonucleotide primer

<400>  62
ttggtgatgc cccagcctga                                                   20

<210>  63
<211>  20
<212>  DNA
<213>  Artificial

<220>
<223>  Oligonucleotide primer

<400>  63

ccacaccgaa gattttcaat 20

<210> 64
<211> 18
<212> DNA
<213> Artificial

<220>
<223> Oligonucleotide primer

<400> 64
gcccctcctt catttata 18

<210> 65
<211> 8280
<212> DNA
<213> Homo sapiens

<400> 65
atcgtgtaat caccgccaca tccagtgcaa agctgattcg tcaccacaga gcagctccct 60

cctgccaccc catccctggg tcccaagaga accctttctt aaaagaggga gttcttgacg 120

ggtgtggtgg ctcatgcctg taatccttgc actttgggag gccaaggagg gtggatcatt 180

tgaggtcagg agtttgagac cagactggcc aacatggtga aaccctgtct ttactaaaaa 240

tacaaaaaaa tgagcggggc atggtggtgg gtgcctatag ccccagctac tcaggaggct 300

gaggcaggag aatcgcttga acccaggagg cagaggttgc agtgagccga gattgagcca 360

ctgcactcca gccggggcta aagagtgaga ctctgtctca aaaaaaaaaa aaagaaaaag 420

aaaaaaagaa aaaaaaataa aataaataaa taaataaaat aaatttaaaa atttaaaaat 480

aaagaggggg ttcttgtgtt gatgccgagc ctgaaccaag gcagaggagg ccgggaaggc 540

ttcccaaggc cttcagctca aagcagggag gcccatagtt aaacagaaac agttcaggaa 600

tcacagaaag gcacctgggg agagatgggt gtgtggctcc agatgcaggt gcccagacag 660

tgcgtcccca ggtgtacaga cagacccagg ccaagctcca gctcaaagag ccagcctagg 720

ggggtgccga ggtggaggga ggctgagtca ggctgaggcc ggggaacagt tggggtagcc 780

aagggaggca agcagcctcc tgagtcacca cgtggtccag gtacggggct gcccaggccc 840

agagacggac acaagcactg gggaatttaa ggggctaggg gaggggctga ggagggtagg 900

ccctccccca aatgaggatg gaaccccccc aactccagaa cccccctgca ggctggccag 960

aatccttccc catctcattc actctgtctc tcctgctctc tgccgtctcc tattttgaat 1020

ttccaacccc gtctgttaag actgtccttc tgtctctgaa tctctgtccc cttctctttc 1080

tgggtctctc tccctctccc tctgggtctc tgtccccctc tctgggtctc tgtcactctc 1140

tctttgcatc tccagctctc actttgtctc tgcacctagc agatcccaag ctggggaatg 1200

ccagttctgg caccaacctt cctgctccct gctggggcct ctgctccccc atctctcagg 1260

agtcgaaagt gagaaagcaa ggtgggcagc tctgctccag gtccaggtat ctcccgccca 1320

cctcctgccc gtcctctatc ccacccctcc tctccatctc tccctggcgc tgccatctct 1380

catctaggcc tccgtctcct ctgtcattgt ccccatcccc tgtaggtgcc catccttccc 1440

gtctcccctc tgccatcggc ctgcctgtcc catcctcttt ctcccaccat gtcccgttct 1500

cttccacgtc tcatgcccgc actgccttca tcatcatcgc tgttgttctg tgtgtgtttg 1560

tggtgagtgc cgcatggtgg gggcgtctcg gcctctctcc tctctctcca ctgttttctc 1620

tttctgtgtg tctgtttcca ttctatctcc accttcttcc ctccgtcttt tgcttttcta 1680

tctccacttc tccacacccc tctctccctg cgtctctgtg tctccctctt cctctgtctt 1740

gtttttttcc caccgtctgc ctcttctgtt ccctgtcaca tccaacttcc accggtttct 1800

ccagctctct cctcagttcc ttctctcatg agcacacctg cctctgtgct cgtattcctg 1860

gactcctctc tctccactgt catatcttct cattcatttt cccagtctct ctctgtctct 1920

tgctctcccc ctctctgtca ctctgtctct gtctctctct ttctctctct ctctctgtgt 1980

ctctctgtct ggctctctct ctgtctctct ctccatctct ctctctctct cccccccgtc 2040

accctgtctc tgtctctctc tgtctgtgtg tctctctgtc tttctctctc tccatctctc 2100

tctgtctctc tctctctctc tctctctctc cctctctccc tcctcccgtg actccctctc 2160

tcagtccatc tcttcctccc tctctcagcc ccttcgtgcc ctttcctctg acactcccca 2220

ccctggtttc ctgactccac cactagatcc accacctcca gcaactggga accctcccct 2280

gcccaccctg ccctggggtc ccctcccagg attccttcta gattatagca tcttccctgg 2340

gcgggttctc atgaacaatt gtggctgctt ttttggccag acaggggagg gaggggatgg 2400

gatcagggag tcctggaatg ggaactaggc aataaaaaaa aaaaaatgtc agaagcaggg 2460

cggcgggagg tgggggcagg gccagctgtc cttaccaggg ataaaaggct ttgccagtgt 2520

gactaggaag agagacacct cccctccttc cttcatcaag acatcaagga gggacctgtg 2580

ccctgctcca catcctccca cctgccgccc gcagagcctg caggccccgc ccccctcgtc 2640

tctggtccct acctctctgc tgtgtcttca tgtccctgag ggtcttgggc tctgggtaag 2700

tgccccttgc tgtctctgcc tctcagcccc cggttctgtt gaaggttcct tctctctcac 2760

tttttctctg catttgacag gacctggccc tcagccccta aaatgttcct cctgctgaca 2820

gcacttcaag tcctggctat aggtaagaga acggttgggt atgacacaag ggggtcccct 2880

ggagactctg agaagagatg gggatgggtc cttggggccc ctggatgctc atggtgacct 2940

cataagaaag agcagggagt ggtttggggg tcatggtggg ggaacgtgct ggaggcctaa 3000

attcctagtt gtggaggtgc tagggaattg tggggccggg gagagaggtg tttataaggt 3060

ctggtgcaaa atacataagg aatcttaggg aactattagg tcctgagtgg gtcatagcag 3120

aaagatcacg gggctctacc tgactgtgtt aggaaagaaa caatgtcaga aagatgtttt 3180

gttgtcagag ggaaggtgga gaaggatgat gggatggcgg gatcgtggca tggggtggcg 3240

ggatcgtggc atgggtgtgt gaggtggatg ggggcaagtg tggggcaaga gatggcggat 3300
ccttggggtc ccactgagtg ggaacgttgg ggaggagaca gggaggtcct tgaatgtgtt 3360
ggggaaggac tcattggggg gaaatgtggc atatttcgag aagtgatcac agaaattatg 3420
ggagcataga gctaagggtc gtagatgtag caaggccctg gataaggtgg ccacggcaca 3480
aaataagaga tgctacggag gtgacttggg aggtgagtca gaaagctctc cgtgctgggg 3540
caataacggg gtcaatattg ggcatgtctc accctgggtg ggacagatag aggcgggcag 3600
tttaggggtt agaccaaaag gaaggggatt tgtcagtttt ggaatcctac aaacttgtgg 3660
agtggagagt gtttgctcat ctactttccc cacccaatcc tgtccactcc tagccatgac 3720
acagagccaa gaggatgaga acaagataat tggtggccat acgtgcaccc ggagctccca 3780
gccgtggcag gcggccctgc tggcgggtcc caggcgccgc ttcctctgcg gaggcgccct 3840
gctttcaggc cagtgggtca tcactgctgc tcactgcggc cgcccgtaag tgaccccctc 3900
ccctgtccct gtacctagtg aattccagag tctaaagccc tagagctgag ctgagaacct 3960
ggatctctgt atagaaccca atgtagtggc tggctcctgg tttgaggtct agagaagagc 4020
ctggaacaaa aacacagctc gggatgtggg ctcctccata aatctcgaac tcagcatagg 4080
ttctgaaagc agatgggcag cttggaaccc atggacctgc tgagaaccga acatctgatc 4140
cagtgattct tccagaggcc acacattaca tcgagaccaa gcttagccca ttccagattg 4200
gtggctgaat tcaggacccc gtctacattc agaaactcag gacactacgt agaactcaga 4260
gcccagttca ggacctgcag tctagccata aatccagaac tagaacgctg ctcacagctg 4320
gaacatacaa ctctaagaat agaggcaaaa cctggaggct gtttcacacc caaggtttag 4380
ttcagagtct agtctatagc tccgctatga gcagacttca acccagtgtt tgaatcccag 4440
aatgtggcgg gtgcggtggc tcatgcctat aatcctagca ctttgggatg ctgaggcagg 4500
cagatcacct gaggtcagga gttcgagacc agcctgagca acatagagaa accctgtctc 4560
tactaaaaat gcaaaattag ccaggcatgg tggcacatgc ctgtaatccc agccactcgg 4620
gaggctgagg caggagaatc acttgaacct gggaggcgga ggttgcagtg agtcaagatc 4680
gcaccattgc actccaggct aggcaacaag agcgaaactc catatcaatc aatcaatcaa 4740
taaatcccag aatgcagatc ctaatcagaa gccccatata aaacctagac ccctcctaaa 4800
ttctagatct gaacttacaa cccagacccc agccaagagg tcaaaatgcc tataagccat 4860
atctatgcca taaacaggtc agtctagaac ctagagatca aagctcaggc cagagtctag 4920
aatataaagg ccagaatgca aaccagactc tagaatcttg gatccgggcc ataacctaga 4980
gctccaacta gaacccagag cccaacctga ggtcaagggc tagggccaga gtccagaacc 5040
aagagcccta taatccaata tgaaacagac ctgtagaggc tgggtgcggt ggctcacgcc 5100
tgtaatccca gcactttggg aggctgaggc gggagaatca cttgaactgg gagttggagg 5160

tcgagagtga gctgagatcg tgccactgca ctccagccta ggtgacagag cgagactcca 5220
tcacaaaaaa aaaataaata aataaatcaa gtcataatcc aggttcgatc tagaatcctg 5280
atcttagcat agagtcaaaa gtttaagatg tctagaactc agaacccagg ctagaaacag 5340
aatggtgcct actccggaat atcagttccg atttagagcc tagactcata acgcagtttc 5400
gcttaggact caatgcaccg agcccagcac agaccctggc acggagccaa gctctcccaa 5460
tcatcacctt cttcccaagc caggagctgg agcccagccc aagagcggaa ggagaggcag 5520
ctggggctgg gccgagagaa tgccctggcc atggggaagg gcacaggagg ccaagaatgc 5580
tcggcctgca gttagtgaga agcaggctag acctcgggga agactcgtca cccggccagg 5640
gaaccgggct ggagggtggg gaggagtctc tggctcagac cctgagcagc gcttctcttg 5700
ggggtcgtgg ccaggatcct tcaggttgcc ctgggcaagc acaacctgag gaggtgggag 5760
gccacccagc aggtgctgcg cgtggttcgt caggtgacgc accccaacta caactcccgg 5820
acccacgaca acgacctcat gctgctgcag ctacagcagc ccgcacggat cgggagggca 5880
gtcaggccca ttgaggtcac ccaggcctgt gccagccccg ggacctcctg ccgagtgtca 5940
ggctggggaa ctatatccag ccccatcggt gaggactcct gcgtcttgga aagcagggga 6000
ctgggcctgg gctcctgggt ctccaggagg tggagctggg gggactgggg ctcctgggtc 6060
tgagggagga ggggctgggc ctggactcct gggtctgagg gaggaggggg ctgaggcctg 6120
gactcctggg tctcaaggag gaggagctgg gcctggactc atacgtctga gggaggaggg 6180
gctggagcct ggactcctgg gtctcaagga ggaggggctg ggcctggact tctgggtctg 6240
agggaggagg ggctggggac ctggactccc gggtctgagg gaggagggac tgggggtctg 6300
gactcctggg tctgagggag gaggggctgg gggcctggac tcctgggtct gagggaggag 6360
gtgctggggc tggactcctg ggtcggaagg aggaggggct gggggcctgg acccttgggt 6420
cttatgggag ggtagaccca gttataaccc tgcagtgtcc cccagccagg taccccgcct 6480
ctctgcaatg cgtgaacatc aacatctccc cggatgaggt gtgccagaag gcctatccta 6540
gaaccatcac gcctggcatg gtctgtgcag gagttcccca gggcgggaag gactcttgtc 6600
aggtaaggcc caggatggga gctgtggtag ggattatttg ggactgggat ttaagcaaat 6660
gatgtcagga gcatggaagt ctgcagaggt cttcagaaga gagtgaaccg caggcacaga 6720
gagattccga tagccaggcc accctgcttc ctagccctgt gccccctggg taatggactc 6780
agagcattca tgcctcagtt tcctcatctg tcaggtggga gtaaccctct tagggtagtt 6840
ggtggaatgg gatgaggcag gttggggaaa gatcgcagag tggcctctgc tcatatgggt 6900
ctgggaaagg ctgtgctgag gcttctagaa atcttaatgc atccttgagg gaggcagaga 6960
tggggaaata gaaaaagaga gacacacaaa tgttctacag ttggagcgaa cagagagggg 7020
cctggtgaga ttcaagggac aggcaggtgc acacagagac agagccagac ccagcggaga 7080

gggaaggaag tgccccgacc tccggggctg agacctcaga gctggggcag gactgtgtcc 7140

ctaactgtcc accagtgtct ctgcctgtct ccctgtgtct gcttctcggg ttctctgtgc 7200

catggtggct ctggctacct gtccatcagt gtctccattt ctgttcctcc ccctcagggt 7260

gactctgggg gacccctggt gtgcagagga cagctccagg gcctcgtgtc ttggggaatg 7320

gagcgctgcg ccctgcctgg ctaccccggt gtctacacca acctgtgcaa gtacagaagc 7380

tggattgagg aaacgatgcg ggacaaatga tggtcttcac ggtgggatgg acctcgtcag 7440

ctgcccaggc cctcctctct ctactcagga cccaggagtc caggccccag cccctcctcc 7500

ctcagaccca ggagtccagg cccccagccc ctcctccctc agacccggga gtccaggccc 7560

ccagcccctc ctccctcaga cccaggagtc caggccccag cccctcctcc ctcagacccg 7620

ggagtccagg cccccagccc ctcctccctc agacccagga gtccaggccc cagtccctcc 7680

tccctcagac ccaggagtcc aggcccccag cccctcctcc ctcagaccca ggaatccagg 7740

cccagcccct cctccctcag acccaggagc cccagtcccc cagcccctcc tccttgagac 7800

ccaggagtcc aggcccagcc cctcctccct cagacccagg agccccagtc cccagcatcc 7860

tgatctttac tccggctctg atctctcctt tcccagagca gttgcttcag gcgttttctc 7920

cccaccaagc ccccaccctt gctgtgtcac catcactact caagaccgga ggcacagagg 7980

gcaggagcac agacccctta aaccggcatt gtattccaaa gacgacaatt tttaacacgc 8040

ttagtgtctc taaaaaccga ataaataatg acaataaaaa tggaatcatc ctaaattgta 8100

ttcattcatc catgtgttta ctttttattt tttgagacaa ggtcttgctc agtctcctgg 8160

tgaaatgctg taacgcaatc atagctcact gcaaccgtga cctcctgggc tccagtgatc 8220

ctcttacctc agcctcccga gtagctggga ccacaggtgc ccgtcaccat gccccgctac 8280

<210> 66
<211> 237
<212> PRT
<213> Homo sapiens

<400> 66

Met Thr Gln Ser Gln Glu Asp Glu Asn Lys Ile Ile Gly Gly His Thr
1               5                   10                  15

Cys Thr Arg Ser Ser Gln Pro Trp Gln Ala Ala Leu Leu Ala Gly Pro
            20                  25                  30

Arg Arg Arg Phe Leu Cys Gly Gly Ala Leu Leu Ser Gly Gln Trp Val
        35                  40                  45

Ile Thr Ala Ala His Cys Gly Arg Pro Ile Leu Gln Val Ala Leu Gly
    50                  55                  60

Lys His Asn Leu Arg Arg Trp Glu Ala Thr Gln Gln Val Leu Arg Val
65                  70                  75                  80

Val Arg Gln Val Thr His Pro Asn Tyr Asn Ser Arg Thr His Asp Asn
                85                  90                  95

Asp Leu Met Leu Leu Gln Leu Gln Gln Pro Ala Arg Ile Gly Arg Ala
            100                 105                 110

Val Arg Pro Ile Glu Val Thr Gln Ala Cys Ala Ser Pro Gly Thr Ser
        115                 120                 125

Cys Arg Val Ser Gly Trp Gly Thr Ile Ser Ser Pro Ile Ala Arg Tyr
    130                 135                 140

Pro Ala Ser Leu Gln Cys Val Asn Ile Asn Ile Ser Pro Asp Glu Val
145                 150                 155                 160

Cys Gln Lys Ala Tyr Pro Arg Thr Ile Thr Pro Gly Met Val Cys Ala
                165                 170                 175

Gly Val Pro Gln Gly Gly Lys Asp Ser Cys Gln Gly Asp Ser Gly Gly
            180                 185                 190

Pro Leu Val Cys Arg Gly Gln Leu Gln Gly Leu Val Ser Trp Gly Met
        195                 200                 205

Glu Arg Cys Ala Leu Pro Gly Tyr Pro Gly Val Tyr Thr Asn Leu Cys
    210                 215                 220

Lys Tyr Arg Ser Trp Ile Glu Glu Thr Met Arg Asp Lys
225                 230                 235

<210>  67
<211>  251
<212>  PRT
<213>  Homo sapiens

<400>  67

Met Phe Leu Leu Leu Thr Ala Leu Gln Val Leu Ala Ile Ala Met Thr
1               5                   10                  15

Gln Ser Gln Glu Asp Glu Asn Lys Ile Ile Gly Gly His Thr Cys Thr
            20                  25                  30

Arg Ser Ser Gln Pro Trp Gln Ala Ala Leu Leu Ala Gly Pro Arg Arg
        35                  40                  45

Arg Phe Leu Cys Gly Gly Ala Leu Leu Ser Gly Gln Trp Val Ile Thr
    50                  55                  60

Ala Ala His Cys Gly Arg Pro Ile Leu Gln Val Ala Leu Gly Lys His
65                  70                  75                  80

Asn Leu Arg Arg Trp Glu Ala Thr Gln Gln Val Leu Arg Val Val Arg
                85                  90                  95

Gln Val Thr His Pro Asn Tyr Asn Ser Arg Thr His Asp Asn Asp Leu
            100                 105                 110

Met Leu Leu Gln Leu Gln Gln Pro Ala Arg Ile Gly Arg Ala Val Arg
        115                 120                 125

Pro Ile Glu Val Thr Gln Ala Cys Ala Ser Pro Gly Thr Ser Cys Arg
    130                 135                 140

Val Ser Gly Trp Gly Thr Ile Ser Ser Pro Ile Ala Arg Tyr Pro Ala
145                 150                 155                 160

Ser Leu Gln Cys Val Asn Ile Asn Ile Ser Pro Asp Glu Val Cys Gln
                165                 170                 175

Lys Ala Tyr Pro Arg Thr Ile Thr Pro Gly Met Val Cys Ala Gly Val
            180                 185                 190

Pro Gln Gly Gly Lys Asp Ser Cys Gln Gly Asp Ser Gly Gly Pro Leu
        195                 200                 205

Val Cys Arg Gly Gln Leu Gln Gly Leu Val Ser Trp Gly Met Glu Arg
    210                 215                 220

Cys Ala Leu Pro Gly Tyr Pro Gly Val Tyr Thr Asn Leu Cys Lys Tyr
225                 230                 235                 240

Arg Ser Trp Ile Glu Glu Thr Met Arg Asp Lys
                245                 250

<210>  68
<211>  249
<212>  PRT
<213>  Homo sapiens

<400>  68

Met Ala Thr Ala Gly Asn Pro Trp Gly Trp Phe Leu Gly Tyr Leu Ile
1               5                   10                  15

Leu Gly Val Ala Gly Ser Leu Val Ser Gly Ser Cys Ser Gln Ile Ile

20                  25                  30

Asn Gly Glu Asp Cys Ser Pro His Ser Gln Pro Trp Gln Ala Ala Leu
        35                  40                  45

Val Met Glu Asn Glu Leu Phe Cys Ser Gly Val Leu Val His Pro Gln
    50                  55                  60

Trp Val Leu Ser Ala Ala His Cys Phe Gln Asn Ser Tyr Thr Ile Gly
65                  70                  75                  80

Leu Gly Leu His Ser Leu Glu Ala Asp Gln Glu Pro Gly Ser Gln Met
                85                  90                  95

Val Glu Ala Ser Leu Ser Val Arg His Pro Glu Tyr Asn Arg Pro Leu
            100                 105                 110

Leu Ala Asn Asp Leu Met Leu Ile Lys Leu Asp Glu Ser Val Ser Glu
        115                 120                 125

Ser Asp Thr Ile Arg Ser Ile Ser Ile Ala Ser Gln Cys Pro Thr Ala
    130                 135                 140

Gly Asn Ser Cys Leu Val Ser Gly Trp Gly Leu Leu Ala Asn Gly Arg
145                 150                 155                 160

Met Pro Thr Val Leu Gln Cys Val Asn Val Ser Val Val Ser Glu Glu
                165                 170                 175

Val Cys Ser Lys Leu Tyr Asp Pro Leu Tyr His Pro Ser Met Phe Cys
            180                 185                 190

Ala Gly Gly Gly His Asp Gln Lys Asp Ser Cys Asn Gly Asp Ser Gly
        195                 200                 205

Gly Pro Leu Ile Cys Asn Gly Tyr Leu Gln Gly Leu Val Ser Phe Gly
    210                 215                 220

Lys Ala Pro Cys Gly Gln Val Gly Val Pro Gly Val Tyr Thr Asn Leu
225                 230                 235                 240

Cys Lys Phe Thr Glu Trp Ile Glu Lys
                245

<210>  69
<211>  253
<212>  PRT
<213>  Homo sapiens

<400>  69

Met Ala Thr Ala Gly Asn Pro Trp Gly Trp Phe Leu Gly Tyr Leu Ile
1               5                   10                  15

Leu Gly Val Ala Gly Ser Leu Val Ser Gly Glu Met Ser Pro Ser Cys
            20                  25                  30

Ser Gln Ile Ile Asn Gly Glu Asp Cys Ser Pro His Ser Gln Pro Trp
        35                  40                  45

Gln Ala Ala Leu Val Met Glu Asn Glu Leu Phe Cys Ser Gly Val Leu
    50                  55                  60

Val His Pro Gln Trp Val Leu Ser Ala Ala His Cys Phe Gln Asn Ser
65                  70                  75                  80

Tyr Thr Ile Gly Leu Gly Leu His Ser Leu Glu Ala Asp Gln Glu Pro
                85                  90                  95

Gly Ser Gln Met Val Glu Ala Ser Leu Ser Val Arg His Pro Glu Tyr
            100                 105                 110

Asn Arg Pro Leu Leu Ala Asn Asp Leu Met Leu Ile Lys Leu Asp Glu
        115                 120                 125

Ser Val Ser Glu Ser Asp Thr Ile Arg Ser Ile Ser Ile Ala Ser Gln
    130                 135                 140

Cys Pro Thr Ala Gly Asn Ser Cys Leu Val Ser Gly Trp Gly Leu Leu
145                 150                 155                 160

Ala Asn Gly Arg Met Pro Thr Val Leu Gln Cys Val Asn Val Ser Val
                165                 170                 175

Val Ser Glu Glu Val Cys Ser Lys Leu Tyr Asp Pro Leu Tyr His Pro
            180                 185                 190

Ser Met Phe Cys Ala Gly Gly Gly His Asp Gln Lys Asp Ser Cys Asn
        195                 200                 205

Gly Asp Ser Gly Gly Pro Leu Ile Cys Asn Gly Tyr Leu Gln Gly Leu
    210                 215                 220

Val Ser Phe Gly Lys Ala Pro Cys Gly Gln Val Gly Val Pro Gly Val
225                 230                 235                 240

Tyr Thr Asn Leu Cys Lys Phe Thr Glu Trp Ile Glu Lys
                245                 250

<210>  70
<211>  287
<212>  PRT
<213>  Homo sapiens

<400>  70

Met Ala Thr Ala Arg Pro Pro Trp Met Trp Val Leu Cys Ala Leu Ile
1               5                   10                  15

Thr Ala Leu Leu Leu Gly Val Thr Glu His Val Leu Ala Asn Asn Asp
            20                  25                  30

Val Ser Cys Asp His Pro Ser Asn Thr Val Pro Ser Gly Ser Asn Gln
        35                  40                  45

Asp Leu Gly Ala Gly Ala Gly Glu Asp Ala Arg Ser Asp Asp Ser Ser
    50                  55                  60

Ser Arg Ile Ile Asn Gly Ser Asp Cys Asp Met His Thr Gln Pro Trp
65                  70                  75                  80

Gln Ala Ala Leu Leu Leu Arg Pro Asn Gln Leu Tyr Cys Gly Ala Val
                85                  90                  95

Leu Val His Pro Gln Trp Leu Leu Thr Ala Ala His Cys Arg Lys Lys
            100                 105                 110

Val Phe Arg Val Arg Leu Gly His Tyr Ser Leu Ser Pro Val Tyr Glu
        115                 120                 125

Ser Gly Gln Gln Met Phe Gln Gly Val Lys Ser Ile Pro His Pro Gly
    130                 135                 140

Tyr Ser His Pro Gly His Ser Asn Asp Leu Met Leu Ile Lys Leu Asn
145                 150                 155                 160

Arg Arg Ile Arg Pro Thr Lys Asp Val Arg Pro Ile Asn Val Ser Ser
                165                 170                 175

His Cys Pro Ser Ala Gly Thr Lys Cys Leu Val Ser Gly Trp Gly Thr
            180                 185                 190

Thr Lys Ser Pro Gln Val His Phe Pro Lys Val Leu Gln Cys Leu Asn
        195                 200                 205

Ile Ser Val Leu Ser Gln Lys Arg Cys Glu Asp Ala Tyr Pro Arg Gln
    210                 215                 220

Ile Asp Asp Thr Met Phe Cys Ala Gly Asp Lys Ala Gly Arg Asp Ser
225                 230                 235                 240

Cys Gln Gly Asp Ser Gly Gly Pro Val Val Cys Asn Gly Ser Leu Gln
                245                 250                 255

Gly Leu Val Ser Trp Gly Asp Tyr Pro Cys Ala Arg Pro Asn Arg Pro
            260                 265                 270

Gly Val Tyr Thr Asn Leu Cys Lys Phe Thr Lys Trp Ile Gln Glu
        275                 280                 285

<210>  71
<211>  239
<212>  PRT
<213>  Homo sapiens

<400>  71

Met Lys Lys Leu Met Val Val Leu Ser Leu Ile Ala Ala Ala Trp Ala
1               5                   10                  15

Glu Glu Gln Asn Lys Leu Val His Gly Gly Pro Cys Asp Lys Thr Ser
            20                  25                  30

His Pro Tyr Gln Ala Ala Leu Tyr Thr Ser Gly His Leu Leu Cys Gly
        35                  40                  45

Gly Val Leu Ile His Pro Leu Trp Val Leu Thr Ala Ala His Cys Lys
    50                  55                  60

Lys Pro Asn Leu Gln Val Phe Leu Gly Lys His Asn Leu Arg Gln Arg
65                  70                  75                  80

Glu Ser Ser Gln Glu Gln Ser Ser Val Val Arg Ala Val Ile His Pro
                85                  90                  95

Asp Tyr Asp Ala Ala Ser His Asp Gln Asp Ile Met Leu Leu Arg Leu
            100                 105                 110

Ala Arg Pro Ala Lys Leu Ser Glu Leu Ile Gln Pro Leu Pro Leu Glu
        115                 120                 125

Arg Asp Cys Ser Ala Asn Thr Thr Ser Cys His Ile Leu Gly Trp Gly
    130                 135                 140

Lys Thr Ala Asp Gly Asp Phe Pro Asp Thr Ile Gln Cys Ala Tyr Ile
145                 150                 155                 160

His Leu Val Ser Arg Glu Glu Cys Glu His Ala Tyr Pro Gly Gln Ile
                165                 170                 175

Thr Gln Asn Met Leu Cys Ala Gly Asp Glu Lys Tyr Gly Lys Asp Ser
            180                 185                 190

Cys Gln Gly Asp Ser Gly Gly Pro Leu Val Cys Gly Asp His Leu Arg
        195                 200                 205

Gly Leu Val Ser Trp Gly Asn Ile Pro Cys Gly Ser Lys Glu Lys Pro
    210                 215                 220

Gly Val Tyr Thr Asn Val Cys Arg Tyr Thr Asn Trp Ile Gln Lys
225                 230                 235

<210>  72
<211>  275
<212>  PRT
<213>  Homo sapiens

<400>  72

Met Gly Arg Pro Arg Pro Arg Ala Ala Lys Thr Trp Met Phe Leu Leu
1               5                   10                  15

Leu Leu Gly Gly Ala Trp Ala Gly His Ser Arg Ala Gln Glu Asp Lys
            20                  25                  30

Val Leu Gly Gly His Glu Cys Gln Pro His Ser Gln Pro Trp Gln Ala
        35                  40                  45

Ala Leu Phe Gln Gly Gln Gln Leu Leu Cys Gly Gly Val Leu Val Gly
    50                  55                  60

Gly Asn Trp Val Leu Thr Ala Ala His Cys Lys Lys Pro Lys Tyr Thr
65                  70                  75                  80

Val Arg Leu Gly Asp His Ser Leu Gln Asn Lys Asp Gly Pro Glu Gln
                85                  90                  95

Glu Ile Pro Val Val Gln Ser Ile Pro His Pro Cys Tyr Asn Ser Ser
            100                 105                 110

Asp Val Glu Asp His Asn His Asp Leu Met Leu Leu Gln Leu Arg Asp
        115                 120                 125

Gln Ala Ser Leu Gly Ser Lys Val Lys Pro Ile Ser Leu Ala Asp His
    130                 135                 140

Cys Thr Gln Pro Gly Gln Lys Cys Thr Val Ser Gly Trp Gly Thr Val

145                 150                 155                 160

Thr Ser Pro Arg Glu Asn Phe Pro Asp Thr Leu Asn Cys Ala Glu Val
                165                 170                 175

Lys Ile Phe Pro Gln Lys Lys Cys Glu Asp Ala Tyr Pro Gly Gln Ile
            180                 185                 190

Thr Asp Gly Met Val Cys Ala Gly Ser Ser Lys Gly Ala Asp Thr Cys
        195                 200                 205

Gln Gly Asp Ser Gly Gly Pro Leu Val Cys Asp Gly Ala Leu Gln Gly
    210                 215                 220

Ile Thr Ser Trp Gly Ser Asp Pro Cys Gly Arg Ser Asp Lys Pro Gly
225                 230                 235                 240

Val Tyr Thr Asn Ile Cys Arg Tyr Leu Asp Trp Ile Lys Lys Thr Leu
                245                 250                 255

Ser Pro Met Arg Ile Leu Gln Leu Ile Leu Leu Ala Leu Ala Thr Gly
            260                 265                 270

Leu Val Gly
        275

<210>  73
<211>  228
<212>  PRT
<213>  Homo sapiens

<400>  73

Gly Glu Thr Arg Ile Ile Lys Gly Phe Glu Cys Lys Pro His Ser Gln
1               5                   10                  15

Pro Trp Gln Ala Ala Leu Phe Glu Lys Thr Arg Leu Leu Cys Gly Ala
            20                  25                  30

Thr Leu Ile Ala Pro Arg Trp Leu Leu Thr Ala Ala His Cys Leu Lys
        35                  40                  45

Pro Arg Tyr Ile Val His Leu Gly Gln His Asn Leu Gln Lys Glu Glu
    50                  55                  60

Gly Cys Glu Gln Thr Arg Thr Ala Thr Glu Ser Phe Pro His Pro Gly
65                  70                  75                  80

Phe Asn Asn Ser Leu Pro Asn Lys Asp His Arg Asn Asp Ile Met Leu
                85                  90                  95

Val Lys Met Ala Ser Pro Val Ser Ile Thr Trp Ala Val Arg Pro Leu
            100                 105                 110

Thr Leu Ser Ser Arg Cys Val Thr Ala Gly Thr Ser Cys Leu Ile Ser
        115                 120                 125

Gly Trp Gly Ser Thr Ser Ser Pro Gln Leu Arg Leu Pro His Thr Leu
    130                 135                 140

Arg Cys Ala Asn Ile Thr Ile Ile Glu His Gln Lys Cys Glu Asn Ala
145                 150                 155                 160

Tyr Pro Gly Asn Ile Thr Asp Thr Met Val Cys Ala Ser Val Gln Glu
                165                 170                 175

Gly Gly Lys Asp Ser Cys Gln Gly Asp Ser Gly Gly Pro Leu Val Cys
            180                 185                 190

Asn Gln Ser Leu Gln Gly Ile Ile Ser Trp Gly Gln Asp Pro Cys Ala
        195                 200                 205

Ile Thr Arg Lys Pro Gly Val Tyr Thr Lys Val Cys Lys Tyr Val Asp
    210                 215                 220

Trp Ile Gln Glu
225

<210>  74
<211>  255
<212>  PRT
<213>  Homo sapiens

<400>  74

Met Trp Val Pro Val Val Phe Leu Thr Leu Ser Val Thr Trp Ile Gly
1               5                   10                  15

Ala Ala Pro Leu Ile Leu Ser Arg Ile Val Gly Gly Trp Glu Cys Glu
            20                  25                  30

Lys His Ser Gln Pro Trp Gln Val Leu Val Ala Ser Arg Gly Arg Ala
        35                  40                  45

Val Cys Gly Gly Val Leu Val His Pro Gln Trp Val Leu Thr Ala Ala
    50                  55                  60

His Cys Ile Arg Asn Lys Ser Val Ile Leu Leu Gly Arg His Ser Leu
65                  70                  75                  80

Phe His Pro Glu Asp Thr Gly Gln Val Phe Gln Val Ser His Ser Phe
                85                  90                  95

Pro His Pro Leu Tyr Asp Met Ser Leu Leu Lys Asn Arg Phe Leu Arg
            100                 105                 110

Pro Gly Asp Asp Ser Ser His Asp Leu Met Leu Leu Arg Leu Ser Glu
        115                 120                 125

Pro Ala Glu Leu Thr Asp Ala Val Lys Val Met Asp Leu Pro Thr Gln
    130                 135                 140

Glu Pro Ala Leu Gly Thr Thr Cys Tyr Ala Ser Gly Trp Gly Ser Ile
145                 150                 155                 160

Glu Pro Glu Glu Phe Leu Thr Pro Lys Lys Leu Gln Cys Val Asp Leu
                165                 170                 175

His Val Ile Ser Asn Asp Val Cys Ala Gln Val His Pro Gln Lys Val
            180                 185                 190

Thr Lys Phe Met Leu Cys Ala Gly Arg Trp Thr Gly Gly Lys Ser Thr
        195                 200                 205

Cys Ser Gly Asp Ser Gly Gly Pro Leu Val Cys Asn Gly Val Leu Gln
    210                 215                 220

Gly Ile Thr Ser Trp Gly Ser Glu Pro Cys Ala Leu Pro Glu Arg Pro
225                 230                 235                 240

Ser Leu Tyr Thr Lys Val Val His Tyr Arg Lys Trp Ile Lys Asp
                245   .             250                 255

<210>  75
<211>  255
<212>  PRT
<213>  Homo sapiens

<400>  75

Met Trp Asp Leu Val Leu Ser Ile Ala Leu Ser Val Gly Cys Thr Gly
1               5                   10                  15

Ala Val Pro Leu Ile Gln Ser Arg Ile Val Gly Gly Trp Glu Cys Glu
            20                  25                  30

Lys His Ser Gln Pro Trp Gln Val Ala Val Tyr Ser His Gly Trp Ala
        35                  40                  45

His Cys Gly Gly Val Leu Val His Pro Gln Trp Val Leu Thr Ala Ala
    50                  55                  60

His Cys Leu Lys Lys Asn Ser Gln Val Trp Leu Gly Arg His Asn Leu
65                  70                  75                  80

Phe Glu Pro Glu Asp Thr Gly Gln Arg Val Pro Val Ser His Ser Phe
                85                  90                  95

Pro His Pro Leu Tyr Asn Met Ser Leu Leu Lys His Gln Ser Leu Arg
            100                 105                 110

Pro Asp Glu Asp Ser Ser His Asp Leu Met Leu Leu Arg Leu Ser Glu
        115                 120                 125

Pro Ala Lys Ile Thr Asp Val Val Lys Val Leu Gly Leu Pro Thr Gln
    130                 135                 140

Glu Pro Ala Leu Gly Thr Thr Cys Tyr Ala Ser Gly Trp Gly Ser Ile
145                 150                 155                 160

Glu Pro Glu Glu Phe Leu Arg Pro Arg Ser Leu Gln Cys Val Ser Leu
                165                 170                 175

His Leu Leu Ser Asn Asp Met Cys Ala Arg Ala Tyr Ser Glu Lys Val
            180                 185                 190

Thr Glu Phe Met Leu Cys Ala Gly Leu Trp Thr Gly Gly Lys Asp Thr
        195                 200                 205

Cys Gly Gly Asp Ser Gly Gly Pro Leu Val Cys Asn Gly Val Leu Gln
    210                 215                 220

Gly Ile Thr Ser Trp Gly Pro Glu Pro Cys Ala Leu Pro Glu Lys Pro
225                 230                 235                 240

Ala Val Tyr Thr Lys Val Val His Tyr Arg Lys Trp Ile Lys Asp
                245                 250                 255

<210>  76
<211>  256
<212>  PRT
<213>  Homo sapiens

<400>  76

Met Trp Phe Leu Val Leu Cys Leu Ala Leu Ser Leu Gly Gly Thr Gly
1               5                   10                  15

Ala Ala Pro Pro Ile Gln Ser Arg Ile Val Gly Gly Trp Glu Cys Glu

            20                  25                  30

Gln His Ser Gln Pro Trp Gln Ala Ala Leu Tyr His Phe Ser Thr Phe
        35                  40                  45

Gln Cys Gly Gly Ile Leu Val His Arg Gln Trp Val Leu Thr Ala Ala
    50                  55                  60

His Cys Ile Ser Asp Asn Tyr Gln Leu Trp Leu Gly Arg His Asn Leu
65                  70                  75                  80

Phe Asp Asp Glu Asn Thr Ala Gln Phe Val His Val Ser Glu Ser Phe
                85                  90                  95

Pro His Pro Gly Phe Asn Met Ser Leu Leu Glu Asn His Thr Arg Gln
            100                 105                 110

Ala Asp Glu Asp Tyr Ser His Asp Leu Met Leu Leu Arg Leu Thr Glu
        115                 120                 125

Pro Ala Asp Thr Ile Thr Asp Ala Val Lys Val Val Glu Leu Pro Thr
    130                 135                 140

Glu Glu Pro Glu Val Gly Ser Thr Cys Leu Ala Ser Gly Trp Gly Ser
145                 150                 155                 160

Ile Glu Pro Glu Asn Phe Ser Phe Pro Asp Asp Leu Gln Cys Val Asp
                165                 170                 175

Leu Lys Ile Leu Pro Asn Asp Glu Cys Lys Lys Ala His Val Gln Lys
            180                 185                 190

Val Thr Asp Phe Met Leu Cys Val Gly His Leu Glu Gly Gly Lys Asp
        195                 200                 205

Thr Cys Val Gly Asp Ser Gly Gly Pro Leu Met Cys Asp Gly Val Leu
    210                 215                 220

Gln Gly Val Thr Ser Trp Gly Tyr Val Pro Cys Gly Thr Pro Asn Lys
225                 230                 235                 240

Pro Ser Val Ala Val Arg Val Leu Ser Tyr Val Lys Trp Ile Glu Asp
                245                 250                 255

<210> 77
<211> 241
<212> PRT
<213> Homo sapiens

<400> 77

Met Asn Pro Leu Leu Ile Leu Thr Phe Val Ala Ala Ala Leu Ala Ala
1               5                   10                  15

Pro Phe Asp Asp Asp Asp Lys Ile Val Gly Gly Tyr Asn Cys Glu Glu
            20                  25                  30

Asn Ser Val Pro Tyr Gln Val Ser Leu Asn Ser Gly Tyr His Phe Cys
        35                  40                  45

Gly Gly Ser Leu Ile Asn Glu Gln Trp Val Val Ser Ala Gly His Cys
    50                  55                  60

Tyr Lys Ser Arg Ile Gln Val Arg Leu Gly Glu His Asn Ile Glu Val
65                  70                  75                  80

Leu Glu Gly Asn Glu Gln Phe Ile Asn Ala Ala Lys Ile Ile Arg His
                85                  90                  95

Pro Gln Tyr Asp Arg Lys Thr Leu Asn Asn Asp Ile Met Leu Ile Lys
            100                 105                 110

Leu Ser Ser Arg Ala Val Ile Asn Ala Arg Val Ser Thr Ile Ser Leu
        115                 120                 125

Pro Thr Ala Pro Pro Ala Thr Gly Thr Lys Cys Leu Ile Ser Gly Trp
    130                 135                 140

Gly Asn Thr Ala Ser Ser Gly Ala Asp Tyr Pro Asp Glu Leu Gln Cys
145                 150                 155                 160

Leu Asp Ala Pro Val Leu Ser Gln Ala Lys Cys Glu Ala Ser Tyr Pro
                165                 170                 175

Gly Lys Ile Thr Ser Asn Met Phe Cys Val Gly Phe Leu Glu Gly Gly
            180                 185                 190

Lys Asp Ser Cys Gln Gly Asp Ser Gly Gly Pro Val Val Cys Asn Gly
        195                 200                 205

Gln Leu Gln Gly Val Val Ser Trp Gly Asp Gly Cys Ala Gln Lys Asn
    210                 215                 220

Lys Pro Gly Val Tyr Thr Lys Val Tyr Asn Tyr Val Lys Trp Ile Lys
225                 230                 235                 240

Asn

<210>  78
<211>  261
<212>  PRT
<213>  Homo sapiens

<400>  78

Met Trp Val Pro Val Val Phe Leu Thr Leu Ser Val Thr Trp Ile Gly
1               5                   10                  15

Ala Ala Pro Leu Ile Leu Ser Arg Ile Val Gly Gly Trp Glu Cys Glu
            20                  25                  30

Lys His Ser Gln Pro Trp Gln Val Leu Val Ala Ser Arg Gly Arg Ala
        35                  40                  45

Val Cys Gly Gly Val Leu Val His Pro Gln Trp Val Leu Thr Ala Ala
    50                  55                  60

His Cys Ile Arg Asn Lys Ser Val Ile Leu Leu Gly Arg His Ser Leu
65                  70                  75                  80

Phe His Pro Glu Asp Thr Gly Gln Val Phe Gln Val Ser His Ser Phe
                85                  90                  95

Pro His Pro Leu Tyr Asp Met Ser Leu Leu Lys Asn Arg Phe Leu Arg
            100                 105                 110

Pro Gly Asp Asp Ser Ser His Asp Leu Met Leu Leu Arg Leu Ser Glu
        115                 120                 125

Pro Ala Glu Leu Thr Asp Ala Val Lys Val Met Asp Leu Pro Thr Gln
    130                 135                 140

Glu Pro Ala Leu Gly Thr Thr Cys Tyr Ala Ser Gly Trp Gly Ser Ile
145                 150                 155                 160

Glu Pro Glu Glu Phe Leu Thr Pro Lys Lys Leu Gln Cys Val Asp Leu
                165                 170                 175

His Val Ile Ser Asn Asp Val Cys Ala Gln Val His Pro Gln Lys Val
            180                 185                 190

Thr Lys Phe Met Leu Cys Ala Gly Arg Trp Thr Gly Gly Lys Ser Thr
        195                 200                 205

Cys Ser Gly Asp Ser Gly Gly Pro Leu Val Cys Asn Gly Val Leu Gln
    210                 215                 220

Gly Ile Thr Ser Trp Gly Ser Glu Pro Cys Ala Leu Pro Glu Arg Pro
225                 230                 235                 240

Ser Leu Tyr Thr Lys Val Val His Tyr Arg Lys Trp Ile Lys Asp Thr
                245                 250                 255

Ile Val Ala Asn Pro
            260

<210>  79
<211>  261
<212>  PRT
<213>  Homo sapiens

<400>  79

Met Trp Asp Leu Val Leu Ser Ile Ala Leu Ser Val Gly Cys Thr Gly
1               5                   10                  15

Ala Val Pro Leu Ile Gln Ser Arg Ile Val Gly Gly Trp Glu Cys Glu
            20                  25                  30

Lys His Ser Gln Pro Trp Gln Val Ala Val Tyr Ser His Gly Trp Ala
        35                  40                  45

His Cys Gly Gly Val Leu Val His Pro Gln Trp Val Leu Thr Ala Ala
    50                  55                  60

His Cys Leu Lys Lys Asn Ser Gln Val Trp Leu Gly Arg His Asn Leu
65                  70                  75                  80

Phe Glu Pro Glu Asp Thr Gly Gln Arg Val Pro Val Ser His Ser Phe
                85                  90                  95

Pro His Pro Leu Tyr Asn Met Ser Leu Leu Lys His Gln Ser Leu Arg
            100                 105                 110

Pro Asp Glu Asp Ser Ser His Asp Leu Met Leu Leu Arg Leu Ser Glu
        115                 120                 125

Pro Ala Lys Ile Thr Asp Val Val Lys Val Leu Gly Leu Pro Thr Gln
    130                 135                 140

Glu Pro Ala Leu Gly Thr Thr Cys Tyr Ala Ser Gly Trp Gly Ser Ile
145                 150                 155                 160

Glu Pro Glu Glu Phe Leu Arg Pro Arg Ser Leu Gln Cys Val Ser Leu
                165                 170                 175

His Leu Leu Ser Asn Asp Met Cys Ala Arg Ala Tyr Ser Glu Lys Val
            180                 185                 190

Thr Glu Phe Met Leu Cys Ala Gly Leu Trp Thr Gly Gly Lys Asp Thr
        195                 200                 205

Cys Gly Gly Asp Ser Gly Gly Pro Leu Val Cys Asn Gly Val Leu Gln
    210                 215                 220

Gly Ile Thr Ser Trp Gly Pro Glu Pro Cys Ala Leu Pro Glu Lys Pro
225                 230                 235                 240

Ala Val Tyr Thr Lys Val Val His Tyr Arg Lys Trp Ile Lys Asp Thr
                245                 250                 255

Ile Ala Ala Asn Pro
            260

<210>  80
<211>  262
<212>  PRT
<213>  Homo sapiens

<400>  80

Met Trp Phe Leu Val Leu Cys Leu Ala Leu Ser Leu Gly Gly Thr Gly
1               5                   10                  15

Ala Ala Pro Pro Ile Gln Ser Arg Ile Val Gly Gly Trp Glu Cys Glu
            20                  25                  30

Gln His Ser Gln Pro Trp Gln Ala Ala Leu Tyr His Phe Ser Thr Phe
        35                  40                  45

Gln Cys Gly Gly Ile Leu Val His Arg Gln Trp Val Leu Thr Ala Ala
    50                  55                  60

His Cys Ile Ser Asp Asn Tyr Gln Leu Trp Leu Gly Arg His Asn Leu
65                  70                  75                  80

Phe Asp Asp Glu Asn Thr Ala Gln Phe Val His Val Ser Glu Ser Phe
                85                  90                  95

Pro His Pro Gly Phe Asn Met Ser Leu Leu Glu Asn His Thr Arg Gln
            100                 105                 110

Ala Asp Glu Asp Tyr Ser His Asp Leu Met Leu Leu Arg Leu Thr Glu
        115                 120                 125

Pro Ala Asp Thr Ile Thr Asp Ala Val Lys Val Val Glu Leu Pro Thr

130                 135                 140

Glu Glu Pro Glu Val Gly Ser Thr Cys Leu Ala Ser Gly Trp Gly Ser
145                 150                 155                 160

Ile Glu Pro Glu Asn Phe Ser Phe Pro Asp Asp Leu Gln Cys Val Asp
                165                 170                 175

Leu Lys Ile Leu Pro Asn Asp Glu Cys Lys Lys Ala His Val Gln Lys
            180                 185                 190

Val Thr Asp Phe Met Leu Cys Val Gly His Leu Glu Gly Gly Lys Asp
        195                 200                 205

Thr Cys Val Gly Asp Ser Gly Gly Pro Leu Met Cys Asp Gly Val Leu
    210                 215                 220

Gln Gly Val Thr Ser Trp Gly Tyr Val Pro Cys Gly Thr Pro Asn Lys
225                 230                 235                 240

Pro Ser Val Ala Val Arg Val Leu Ser Tyr Val Lys Trp Ile Glu Asp
                245                 250                 255

Thr Ile Ala Glu Asn Ser
            260

<210>  81
<211>  254
<212>  PRT
<213>  Homo sapiens

<400>  81

Met Ala Thr Ala Gly Asn Pro Trp Gly Trp Phe Leu Gly Tyr Leu Ile
1               5                   10                  15

Leu Gly Val Ala Gly Ser Leu Val Ser Gly Ser Cys Ser Gln Ile Ile
            20                  25                  30

Asn Gly Glu Asp Cys Ser Pro His Ser Gln Pro Trp Gln Ala Ala Leu
        35                  40                  45

Val Met Glu Asn Glu Leu Phe Cys Ser Gly Val Leu Val His Pro Gln
    50                  55                  60

Trp Val Leu Ser Ala Ala His Cys Phe Gln Asn Ser Tyr Thr Ile Gly
65                  70                  75                  80

Leu Gly Leu His Ser Leu Glu Ala Asp Gln Glu Pro Gly Ser Gln Met
                85                  90                  95

Val Glu Ala Ser Leu Ser Val Arg His Pro Glu Tyr Asn Arg Pro Leu
            100                 105                 110

Leu Ala Asn Asp Leu Met Leu Ile Lys Leu Asp Glu Ser Val Ser Glu
        115                 120                 125

Ser Asp Thr Ile Arg Ser Ile Ser Ile Ala Ser Gln Cys Pro Thr Ala
    130                 135                 140

Gly Asn Ser Cys Leu Val Ser Gly Trp Gly Leu Leu Ala Asn Gly Arg
145                 150                 155                 160

Met Pro Thr Val Leu Gln Cys Val Asn Val Ser Val Val Ser Glu Glu
                165                 170                 175

Val Cys Ser Lys Leu Tyr Asp Pro Leu Tyr His Pro Ser Met Phe Cys
            180                 185                 190

Ala Gly Gly Gly His Asp Gln Lys Asp Ser Cys Asn Gly Asp Ser Gly
        195                 200                 205

Gly Pro Leu Ile Cys Asn Gly Tyr Leu Gln Gly Leu Val Ser Phe Gly
    210                 215                 220

Lys Ala Pro Cys Gly Gln Val Gly Val Pro Gly Val Tyr Thr Asn Leu
225                 230                 235                 240

Cys Lys Phe Thr Glu Trp Ile Glu Lys Thr Val Gln Ala Ser
                245                 250

<210>  82
<211>  247
<212>  PRT
<213>  Homo sapiens

<400>  82

<210>  83
<211>  260
<212>  PRT
<213>  Homo sapiens

<400>  83

Met Gly Arg Pro Arg Pro Arg Ala Ala Lys Thr Trp Met Phe Leu Leu
1               5                   10                  15

Leu Leu Gly Gly Ala Trp Ala Gly His Ser Arg Ala Gln Glu Asp Lys
            20                  25                  30

Val Leu Gly Gly His Glu Cys Gln Pro His Ser Gln Pro Trp Gln Ala
        35                  40                  45

Ala Leu Phe Gln Gly Gln Gln Leu Leu Cys Gly Gly Val Leu Val Gly
    50                  55                  60

Gly Asn Trp Val Leu Thr Ala Ala His Cys Lys Lys Pro Lys Tyr Thr
65                  70                  75                  80

Val Arg Leu Gly Asp His Ser Leu Gln Asn Lys Asp Gly Pro Glu Gln
                85                  90                  95

Glu Ile Pro Val Val Gln Ser Ile Pro His Pro Cys Tyr Asn Ser Ser
            100                 105                 110

Asp Val Glu Asp His Asn His Asp Leu Met Leu Leu Gln Leu Arg Asp
        115                 120                 125

Gln Ala Ser Leu Gly Ser Lys Val Lys Pro Ile Ser Leu Ala Asp His
    130                 135                 140

Cys Thr Gln Pro Gly Gln Lys Cys Thr Val Ser Gly Trp Gly Thr Val
145                 150                 155                 160

Thr Ser Pro Arg Glu Asn Phe Pro Asp Thr Leu Asn Cys Ala Glu Val
                165                 170                 175

Lys Ile Phe Pro Gln Lys Lys Cys Glu Asp Ala Tyr Pro Gly Gln Ile
            180                 185                 190

Thr Asp Gly Met Val Cys Ala Gly Ser Ser Lys Gly Ala Asp Thr Cys
        195                 200                 205

Gln Gly Asp Ser Gly Gly Pro Leu Val Cys Asp Gly Ala Leu Gln Gly
    210                 215                 220

Ile Thr Ser Trp Gly Ser Asp Pro Cys Gly Arg Ser Asp Lys Pro Gly
225                 230                 235                 240

Val Tyr Thr Asn Ile Cys Arg Tyr Leu Asp Trp Ile Lys Lys Ile Ile
                245                 250                 255

Gly Ser Lys Gly
            260

<210>  84
<211>  244
<212>  PRT
<213>  Homo sapiens

<400>  84

Met Lys Lys Leu Met Val Val Leu Ser Leu Ile Ala Ala Ala Trp Ala
1               5                   10                  15

Glu Glu Gln Asn Lys Leu Val His Gly Gly Pro Cys Asp Lys Thr Ser
            20                  25                  30

His Pro Tyr Gln Ala Ala Leu Tyr Thr Ser Gly His Leu Leu Cys Gly
        35                  40                  45

Gly Val Leu Ile His Pro Leu Trp Val Leu Thr Ala Ala His Cys Lys
    50                  55                  60

Lys Pro Asn Leu Gln Val Phe Leu Gly Lys His Asn Leu Arg Gln Arg
65                  70                  75                  80

Glu Ser Ser Gln Glu Gln Ser Ser Val Val Arg Ala Val Ile His Pro
                85                  90                  95

Asp Tyr Asp Ala Ala Ser His Asp Gln Asp Ile Met Leu Leu Arg Leu
            100                 105                 110

Ala Arg Pro Ala Lys Leu Ser Glu Leu Ile Gln Pro Leu Pro Leu Glu
        115                 120                 125

Arg Asp Cys Ser Ala Asn Thr Thr Ser Cys His Ile Leu Gly Trp Gly
    130                 135                 140

Lys Thr Ala Asp Gly Asp Phe Pro Asp Thr Ile Gln Cys Ala Tyr Ile
145                 150                 155                 160

His Leu Val Ser Arg Glu Glu Cys Glu His Ala Tyr Pro Gly Gln Ile
                165                 170                 175

Thr Gln Asn Met Leu Cys Ala Gly Asp Glu Lys Tyr Gly Lys Asp Ser
            180                 185                 190

Cys Gln Gly Asp Ser Gly Gly Pro Leu Val Cys Gly Asp His Leu Arg
        195                 200                 205

Gly Leu Val Ser Trp Gly Asn Ile Pro Cys Gly Ser Lys Glu Lys Pro
    210                 215                 220

Gly Val Tyr Thr Asn Val Cys Arg Tyr Thr Asn Trp Ile Gln Lys Thr
225                 230                 235                 240

Ile Gln Ala Lys

<210>  85
<211>  258
<212>  PRT
<213>  Homo sapiens

<400>  85

Met Ala Thr Ala Gly Asn Pro Trp Gly Trp Phe Leu Gly Tyr Leu Ile
1               5                   10                  15

Leu Gly Val Ala Gly Ser Leu Val Ser Gly Glu Met Ser Pro Ser Cys
            20                  25                  30

Ser Gln Ile Ile Asn Gly Glu Asp Cys Ser Pro His Ser Gln Pro Trp
        35                  40                  45

Gln Ala Ala Leu Val Met Glu Asn Glu Leu Phe Cys Ser Gly Val Leu
    50                  55                  60

Val His Pro Gln Trp Val Leu Ser Ala Ala His Cys Phe Gln Asn Ser
65                  70                  75                  80

Tyr Thr Ile Gly Leu Gly Leu His Ser Leu Glu Ala Asp Gln Glu Pro
                85                  90                  95

Gly Ser Gln Met Val Glu Ala Ser Leu Ser Val Arg His Pro Glu Tyr
            100                 105                 110

Asn Arg Pro Leu Leu Ala Asn Asp Leu Met Leu Ile Lys Leu Asp Glu
        115                 120                 125

Ser Val Ser Glu Ser Asp Thr Ile Arg Ser Ile Ser Ile Ala Ser Gln
    130                 135                 140

Cys Pro Thr Ala Gly Asn Ser Cys Leu Val Ser Gly Trp Gly Leu Leu
145                 150                 155                 160

Ala Asn Gly Arg Met Pro Thr Val Leu Gln Cys Val Asn Val Ser Val
                165                 170                 175

Val Ser Glu Glu Val Cys Ser Lys Leu Tyr Asp Pro Leu Tyr His Pro
            180                 185                 190

Ser Met Phe Cys Ala Gly Gly Gly His Asp Gln Lys Asp Ser Cys Asn
        195                 200                 205

Gly Asp Ser Gly Gly Pro Leu Ile Cys Asn Gly Tyr Leu Gln Gly Leu
    210                 215                 220

Val Ser Phe Gly Lys Ala Pro Cys Gly Gln Val Gly Val Pro Gly Val
225                 230                 235                 240

Tyr Thr Asn Leu Cys Lys Phe Thr Glu Trp Ile Glu Lys Thr Val Gln
                245                 250                 255

Ala Ser

<210>  86
<211>  250
<212>  PRT
<213>  Homo sapiens

<400>  86

Met Arg Ile Leu Gln Leu Ile Leu Leu Ala Leu Ala Thr Gly Leu Val
1               5                   10                  15

Gly Gly Glu Thr Arg Ile Ile Lys Gly Phe Glu Cys Lys Pro His Ser
            20                  25                  30

Gln Pro Trp Gln Ala Ala Leu Phe Glu Lys Thr Arg Leu Leu Cys Gly
        35                  40                  45

Ala Thr Leu Ile Ala Pro Arg Trp Leu Leu Thr Ala Ala His Cys Leu
    50                  55                  60

Lys Pro Arg Tyr Ile Val His Leu Gly Gln His Asn Leu Gln Lys Glu
65                  70                  75                  80

Glu Gly Cys Glu Gln Thr Arg Thr Ala Thr Glu Ser Phe Pro His Pro
                85                  90                  95

Gly Phe Asn Asn Ser Leu Pro Asn Lys Asp His Arg Asn Asp Ile Met
            100                 105                 110

Leu Val Lys Met Ala Ser Pro Val Ser Ile Thr Trp Ala Val Arg Pro
        115                 120                 125

Leu Thr Leu Ser Ser Arg Cys Val Thr Ala Gly Thr Ser Cys Leu Ile
    130                 135                 140

Ser Gly Trp Gly Ser Thr Ser Ser Pro Gln Leu Arg Leu Pro His Thr
145                 150                 155                 160

Leu Arg Cys Ala Asn Ile Thr Ile Ile Glu His Gln Lys Cys Glu Asn
                165                 170                 175

Ala Tyr Pro Gly Asn Ile Thr Asp Thr Met Val Cys Ala Ser Val Gln
            180                 185                 190

Glu Gly Gly Lys Asp Ser Cys Gln Gly Asp Ser Gly Gly Pro Leu Val
        195                 200                 205

Cys Asn Gln Ser Leu Gln Gly Ile Ile Ser Trp Gly Gln Asp Pro Cys
    210                 215                 220

Ala Ile Thr Arg Lys Pro Gly Val Tyr Thr Lys Val Cys Lys Tyr Val
225                 230                 235                 240

Asp Trp Ile Gln Glu Thr Met Lys Asn Asn
                245                 250

<210>  87
<211>  257
<212>  PRT
<213>  Homo sapiens

<400>  87

Met Ala Arg Ser Leu Leu Leu Pro Leu Gln Ile Leu Leu Leu Ser Leu
1               5                   10                  15

Ala Leu Glu Thr Ala Gly Glu Glu Ala Gln Gly Asp Lys Ile Ile Asp
            20                  25                  30

Gly Ala Pro Cys Ala Arg Gly Ser His Pro Trp Gln Val Ala Leu Leu
        35                  40                  45

Ser Gly Asn Gln Leu His Cys His Ser Cys Cys Glu Gly Gly Val Leu
    50                  55                  60

Val Asn Glu Arg Trp Val Leu Thr Ala Ala His Cys Lys Met Asn Glu
65                  70                  75                  80

Tyr Thr Val His Leu Gly Ser Asp Thr Leu Gly Asp Arg Arg Ala Gln
                85                  90                  95

Arg Ile Lys Ala Ser Lys Ser Phe Arg His Pro Gly Tyr Ser Thr Gln
            100                 105                 110

Thr His Val Asn Asp Leu Met Leu Val Lys Leu Asn Ser Gln Ala Arg
        115                 120                 125

Leu Ser Ser Met Val Lys Lys Val Arg Leu Pro Ser Arg Cys Glu Pro
    130                 135                 140

Pro Gly Thr Thr Cys Thr Val Ser Gly Trp Gly Thr Thr Thr Ser Pro
145                 150                 155                 160

Asp Val Thr Phe Pro Asp Leu Met Cys Val Asp Val Lys Leu Ile Ser
                165                 170                 175

Pro Gln Asp Cys Thr Lys Val Tyr Lys Asp Leu Leu Glu Asn Ser Met
            180                 185                 190

Leu Cys Ala Gly Ile Pro Asp Ser Lys Lys Asn Ala Cys Asn Gly Asp
        195                 200                 205

Ser Gly Gly Pro Leu Val Cys Arg Gly Thr Leu Gln Gly Leu Val Ser
    210                 215                 220

Trp Gly Thr Phe Pro Cys Gly Gln Pro Asn Asp Pro Gly Val Tyr Thr
225                 230                 235                 240

Gln Val Cys Lys Phe Thr Lys Trp Ile Asn Asp Thr Met Lys Lys His
                245                 250                 255

Arg

<210>  88
<211>  276
<212>  PRT
<213>  Homo sapiens

<400>  88

Met Arg Ala Pro His Leu His Leu Ser Ala Ala Ser Gly Ala Arg Ala
1               5                   10                  15

Leu Ala Lys Leu Leu Pro Leu Leu Met Ala Gln Leu Trp Ala Ala Glu
            20                  25                  30

Ala Ala Leu Leu Pro Gln Asn Asp Thr Arg Leu Asp Pro Glu Ala Tyr
        35                  40                  45

Gly Ala Pro Cys Ala Arg Gly Ser Gln Pro Trp Gln Val Ser Leu Phe
    50                  55                  60

Asn Gly Leu Ser Phe His Cys Ala Gly Val Leu Val Asp Gln Ser Trp
65                  70                  75                  80

Val Leu Thr Ala Ala His Cys Gly Asn Lys Pro Leu Trp Ala Arg Val
                85                  90                  95

Gly Asp Asp His Leu Leu Leu Leu Gln Gly Glu Gln Leu Arg Arg Thr

            100                 105                 110

Thr Arg Ser Val Val His Pro Lys Tyr His Gln Gly Ser Gly Pro Ile
        115                 120                 125

Leu Pro Arg Arg Thr Asp Glu His Asp Leu Met Leu Leu Lys Leu Ala
    130                 135                 140

Arg Pro Val Val Pro Gly Pro Arg Val Arg Ala Leu Gln Leu Pro Tyr
145                 150                 155                 160

Arg Cys Ala Gln Pro Gly Asp Gln Cys Gln Val Ala Gly Trp Gly Thr
                165                 170                 175

Thr Ala Ala Arg Arg Val Lys Tyr Asn Lys Gly Leu Thr Cys Ser Ser
            180                 185                 190

Ile Thr Ile Leu Ser Pro Lys Glu Cys Glu Val Phe Tyr Pro Gly Val
        195                 200                 205

Val Thr Asn Asn Met Ile Cys Ala Gly Leu Asp Arg Gly Gln Asp Pro
    210                 215                 220

Cys Gln Ser Asp Ser Gly Gly Pro Leu Val Cys Asp Glu Thr Leu Gln
225                 230                 235                 240

Gly Ile Leu Ser Trp Gly Val Tyr Pro Cys Gly Ser Ala Gln His Pro
                245                 250                 255

Ala Val Tyr Thr Gln Ile Cys Lys Tyr Met Ser Trp Ile Asn Lys Val
            260                 265                 270

Ile Arg Ser Asn
        275